(19) Europäisches Patentamt
European Patent Office
Office européen des brevets

(11) EP 3 682 893 A1

(12) EUROPEAN PATENT APPLICATION
published in accordance with Art. 153(4) EPC

(43) Date of publication:
**22.07.2020 Bulletin 2020/30**

(21) Application number: **18856002.3**

(22) Date of filing: **07.09.2018**

(51) Int Cl.:
***A61K 38/10*** (2006.01) ***A61P 35/00*** (2006.01)
***C07K 17/08*** (2006.01)

(86) International application number:
**PCT/CN2018/104578**

(87) International publication number:
**WO 2019/052405 (21.03.2019 Gazette 2019/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2017 CN 201710826497**

(71) Applicant: **Nanjing Anji Biological Technology Co., Ltd**
**Nanjing, Jiangsu 210000 (CN)**

(72) Inventors:
- **KANG, Zhian**
**Nanjing**
**Jiangsu 211198 (CN)**
- **WANG, Kequan**
**Nanjing**
**Jiangsu 211198 (CN)**

(74) Representative: **karo IP**
**karo IP Patentanwälte**
**Kahlhöfer Rößler Kreuels PartG mbB**
**Platz der Ideen 2**
**40476 Düsseldorf (DE)**

# (54) MALEIMIDE GROUP-MODIFIED ANGIOGENESIS INHIBITOR HM-1 AND USE THEREOF

(57) The present invention discloses a maleimide group-modified angiogenesis inhibitor HM-1 and the use thereof, which fall within the technical field of polypeptide drugs. According to the present invention, the selection of a suitable maleimide group to modify the N-terminal of the angiogenesis inhibitor polypeptide HM-1 obtains an unchanged X1-X90 polypeptide sequence target, also prolongs the in viwo half-life of the polypeptide molecules, has a low clearance rate, and reduces the immunogenicity and antigenicity. The maleimide group-modified angiogenesis inhibitor can be used for preventing and treating tumors, various inflammations and ocular neovascular diseases, and the anti-tumor activity remains unchanged. But the plasma concentration at the lesion site is increased, so that the administration frequency is reduced, that is, the administration frequency of the maleimide group-modified HM-1 is changed from twice a day to once every seven days.

EP 3 682 893 A1

## Description

### TECHNICAL FIELD

The present invention belongs to the technical field of polypeptide drugs, and more specifically, relates to a maleimide group-modified angiogenesis inhibitor HM-1 and use thereof.

### BACKGROUND

Integrins are a family of receptors that recognize a variety of extracellular matrix components. The integrins are widely distributed on the cell surface, they can mediate the adhesion between cells and extracellular matrix and between cells, and can participate in angiogenesis through the interaction between intracellular cytoskeletal proteins and extracellular matrix molecules. Such receptors consist of $\alpha$ and $\beta$ chains. At present, 15 $\alpha$ chains and 9 $\beta$ chains have been found. The combination of different $\alpha$ chains and $\beta$ chains determines the specificity of a ligand. Integrins $\alpha_1\beta_1$, $\alpha_2\beta_1$, $\alpha_3\beta_1$, $\alpha_6\beta_1$, $\alpha_6\beta_4$, $\alpha_5\beta_1$, $\alpha_v\beta_3$ and $\alpha_v\beta_5$ participate in angiogenesis and cell migration, among which $\alpha_v\beta_3$ can affect several key processes in carcinogenesis. $\alpha_v\beta_3$ can be expressed in a variety of cell types. It may recognize an Arg-Gly-Asp (RGD) sequence in ligand molecules, and involve in physiological and pathological processes, such as tumor angiogenesis, invasion, metastasis, inflammation, wound healing, and coagulation. Therefore, the RGD sequence can be used as a carrier for targeted transportation to neovascular endothelial cells, thus achieving more efficient treatment of neovascular diseases. Chinese patent application No. 201410324568.9, entitled MULTIFUNCTIONAL FUSION POLYPEPTIDES, AND PREPARATION METHOD AND USE THEREOF, provides several angiogenesis inhibitor polypeptides, one of which is HM-1, with the sequence of Arg-Gly-Ala-Asp-Arg-Ala-Gly-Gly-Gly-Gly-Arg-Gly-Asp. This sequence includes an integrin ligand sequence (Gly-Gly-Gly-Gly-Arg-Gly-Asp) and an agiogenesis inhibiting sequence (Arg-Gly-Ala-Asp-Arg-Ala). The polypeptide has been repeatedly evaluated in vivo and in vitro to prove its activity in treating solid tumors, rheumatoid arthritis and ocular neovascular diseases. However, the above polypeptide has a short half-life and needs frequent administration, which brings some pain to patients. Chinese patent application No. 201610211000.5, entitled POLYETHYLENE GLYCOL-MODIFIED ANGIOGENESIS INHIBITOR HM-1 AND USE THEREOF, discloses polyethylene glycol (PEG)-modified HM-1, which can prolong the half-life of a polypeptide. Compared with this patent, the present invention provides a more prolonged half-life of polypeptide. Due to their specificity, changes in any one amino acid or environment will directly affect the structures, activities and functions of polypeptide drugs. Moreover, different action sites of the polypeptide drugs also affect the selection of modification methods. The methods for modification of chemical drugs in the prior art do not have any technical enlightenment for the polypeptide drugs. Even the existing methods for modification of polypeptide drugs will not bring inevitable enlightenment because of the difference between polypeptide sequences. That is the reason that the process of developing new drugs is long and arduous.

In the literatures, modification of molecular structure is a common method to solve the problems of short half-life and frequent administration. After binding to the amino group of the side chain of maleimide group, the polypeptide can be bound to the sulfhydryl group at the $34^{th}$ position of albumin through the maleimide group, thereby enhancing the stability in vivo and reducing the sensitivity to peptidase or protease degradation. At the same time, the molecular weight is increased after binding to albumin, thus not only prolonging the half-life of HM-1, but also increasing the plasma concentration at the lesion site. However, after the polypeptide is modified by the maleimide group and bound to albumin in vivo, the biological activity of protein and polypeptide is also affected. For example, the binding of the polypeptide to a target is affected. The degree of influence is related to the properties of the maleimide group, such as the length and flexibility of its side chain. The biological activity of the modified product needs to be determined through a series of in vivo and in vitro pharmacodynamic tests.

### SUMMARY

#### 1. PROBLEMS TO BE SOLVED

In view of the shortcomings of an existing angiogenesis inhibitor polypeptide HM-1 (the sequence is shown in SEQ.NO. 1), such as short half-life, high plasma clearance rate, frequent administration, the present invention provides a maleimide group-modified angiogenesis inhibitor HM-1 and use thereof. The modified polypeptide can rapidly react with the sulfhydryl group at the $34^{th}$ position of albumin at a molar ratio of 1:1, thereby reducing sensitivity to peptidase or protease degradation, increasing the molecular weight, prolonging the half-life of the polypeptide HM-1 and retaining the biological activity of the polypeptide HM-1. The synthesis method of the polypeptide is simple and low cost. It can be implemented by polypeptide solid phase synthesis. Furthermore, the polypeptide is better applied to the prevention and treatment of tumors, various inflammations and ocular neovascular diseases.

## 2. TECHNICAL SOLUTION

In order to solve the foregoing problems, the technical solutions adopted by the present invention are as follows:

A modified angiogenesis inhibitor polypeptide, wherein a maleimide group is used to modify the angiogenesis inhibitor polypeptide, and the carboxyl group of the maleimide group forms an amide bond with the amino group of N-terminal Arg of the polypeptide. For example, there are the following polypeptides P1-P4:

polypeptide P1:

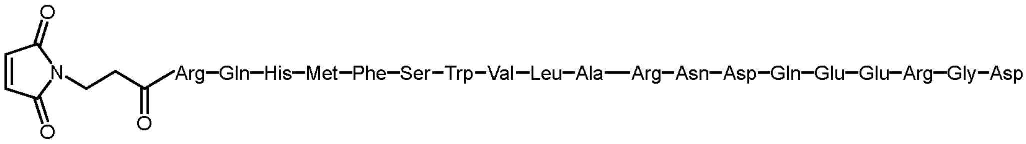

;

polypeptide P2:

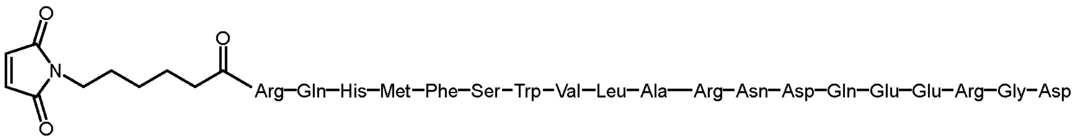

;

polypeptide P3:

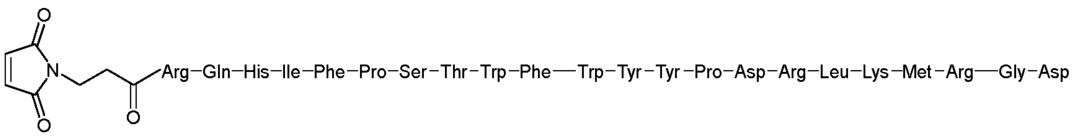

; and

polypeptide P4:

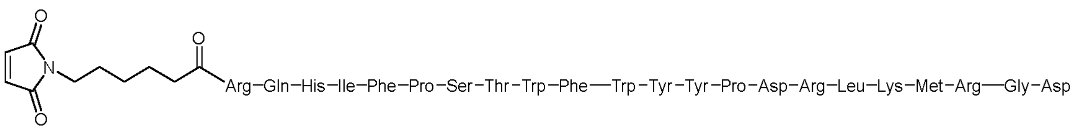

.

Further, a modified angiogenesis inhibitor polypeptide sequence includes two functional groups A and B, wherein the functional group A is Arg-Gly-Ala-Asp-Arg-Ala or a derived polypeptide obtained by substituting, deleting or adding one or two amino acid residues in Arg-Gly-Ala-Asp-Arg-Al, and the derived polypeptide has the same angiogenesis inhibition, anti-tumor and anti-inflammatory activities as Arg-Gly-Ala-Asp-Arg-Ala; the functional group B is Arg-Gly-Asp, wherein the functional groups A and B are ligated with each other through a linker, that is, the modified polypeptide sequence structure is A-linker-B.

Further, the linker is Gly-Gly-Gly-Gly, Glu-Ala-Ala-Ala-Lys or Gly-Ser-Ser-Ser-Ser.

Further, the modified polypeptide sequence is:

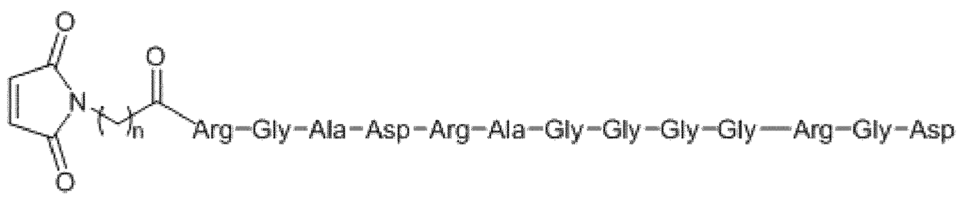

or

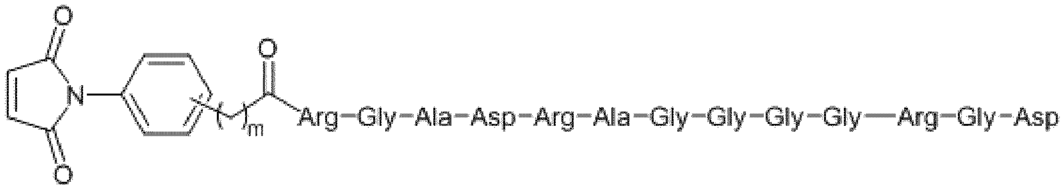

or

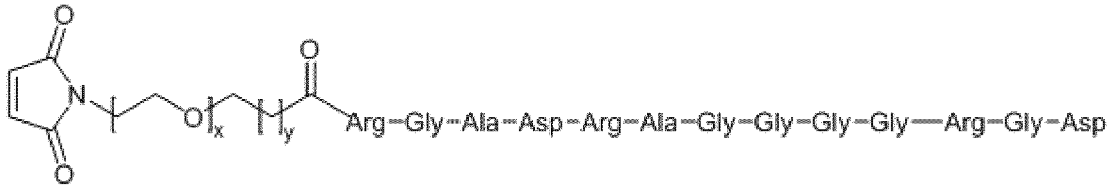

.

Further, a polypeptide chain is ligated with the maleimide group by different linkers, wherein n, m, x and y are the numbers of repeating structural unit methylene, methylene, oxyethylene and methylene respectively in the foregoing structural formulas; the n, m, x and y are all integers, and specific numerical ranges are: n=1-12, m=1-12, x=1-5, y=0-6. The specific polypeptide sequence structures are:

polypeptide X1: 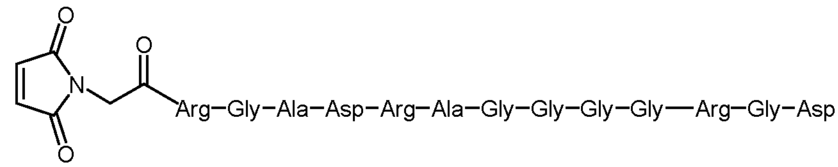 ;

polypeptide X2: 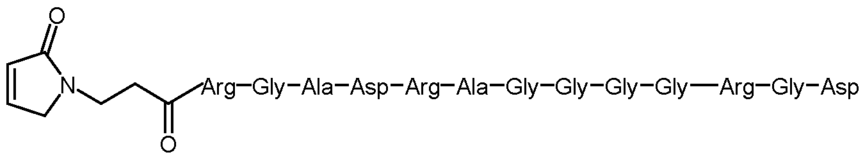 ;

polypeptide X3: 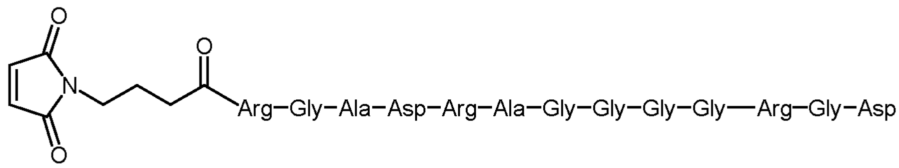 ;

polypeptide X4: 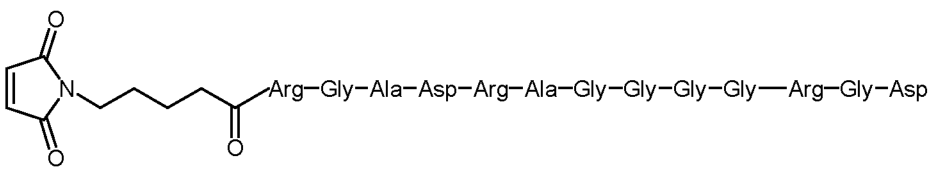 ;

polypeptide X5: 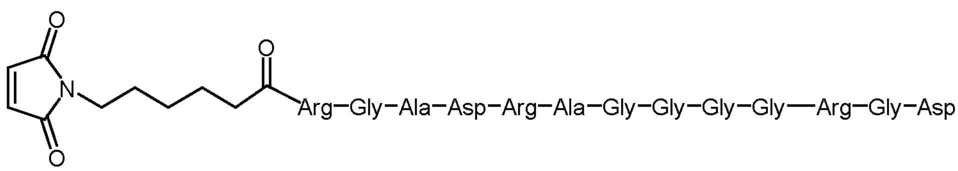 ;

polypeptide X6: 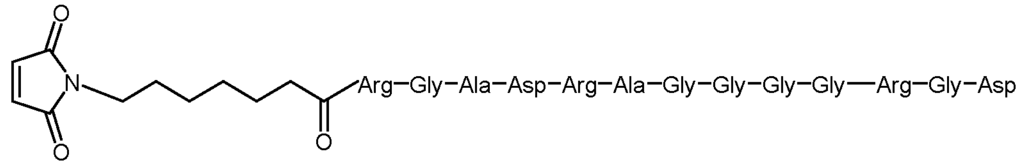 ;

polypeptide X7: 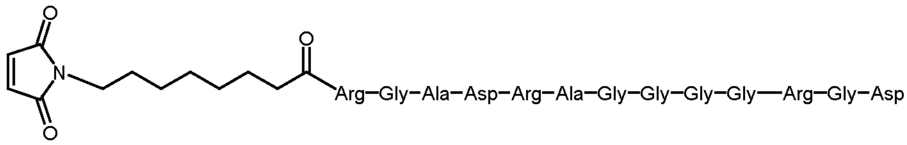 ;

polypeptide X8: 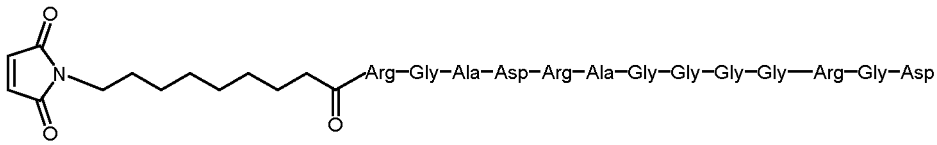 ;

polypeptide X9: 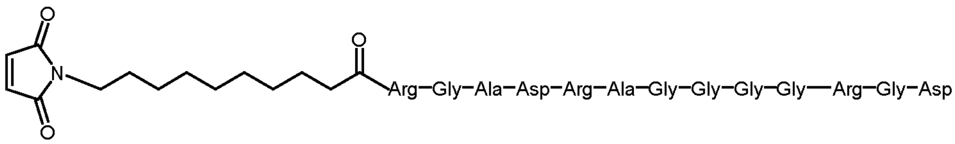 ;

polypeptide X10: 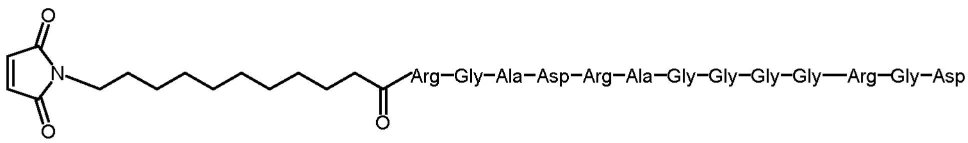 ;

polypeptide X11: 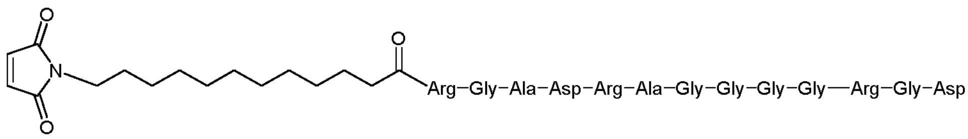 ;

polypeptide X12: 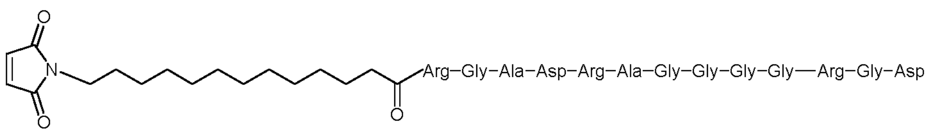 ;

polypeptide X13: 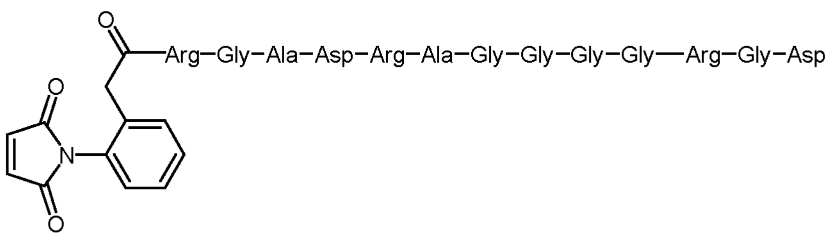 ;

polypeptide X14: 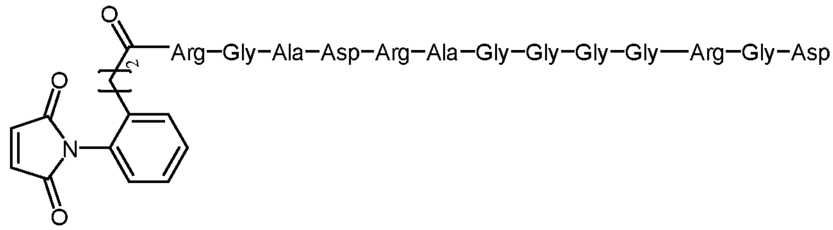 ;

polypeptide X15: 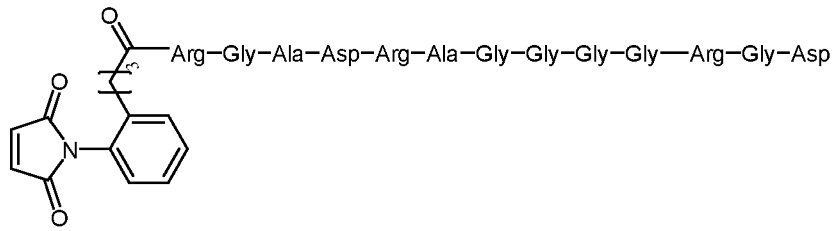 ;

polypeptide X16: 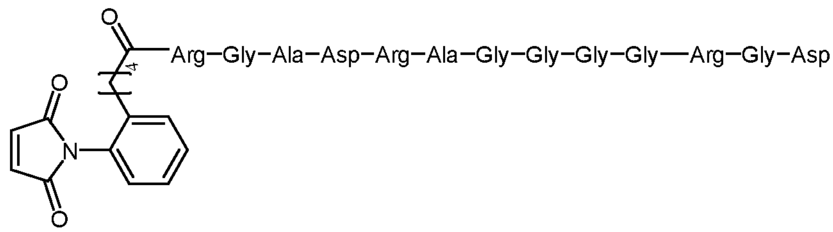 ;

polypeptide X17: 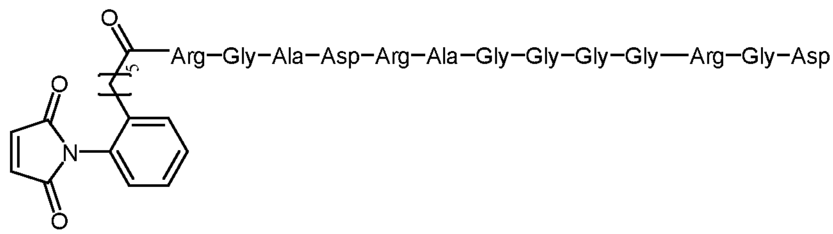 ;

polypeptide X18: 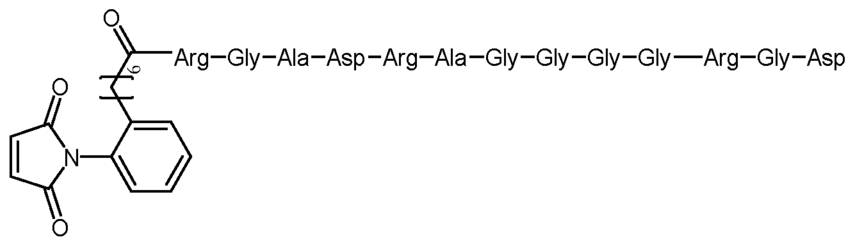 ;

polypeptide X19: 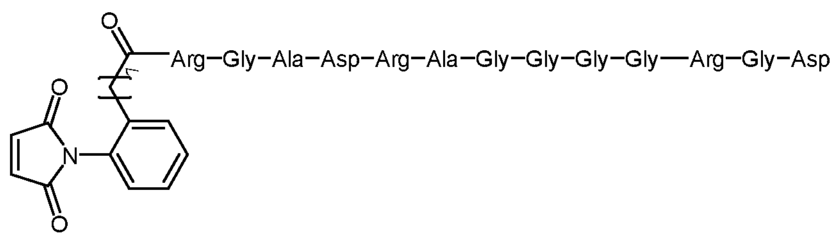 ;

polypeptide X20: 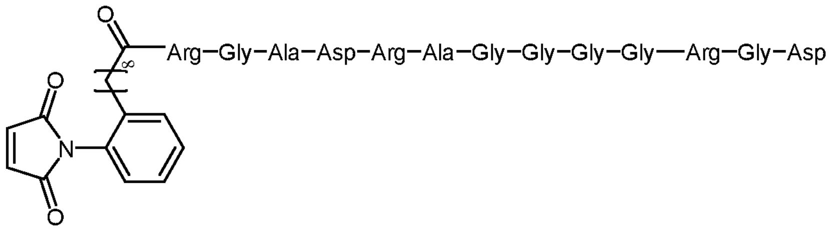 ;

polypeptide X21: 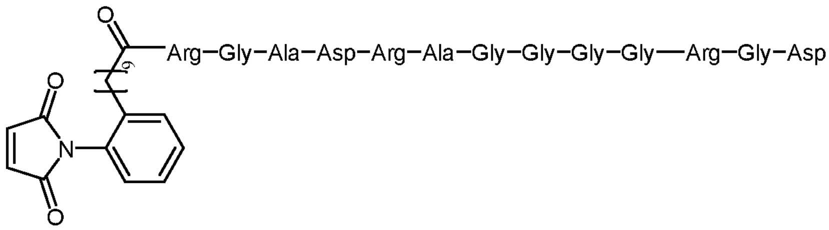 ;

polypeptide X22: 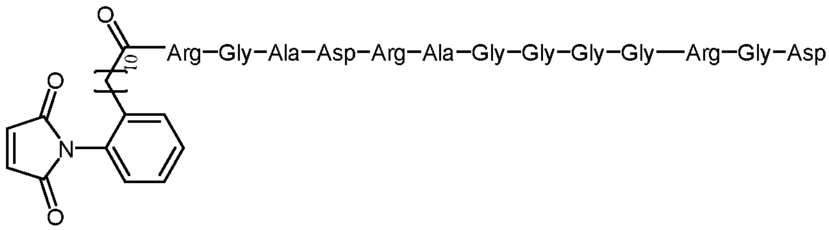 ;

polypeptide X23: 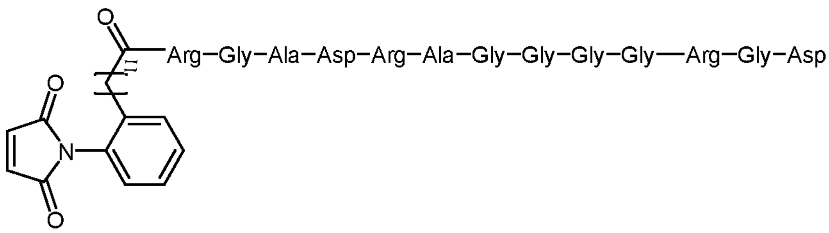 ;

polypeptide X24: 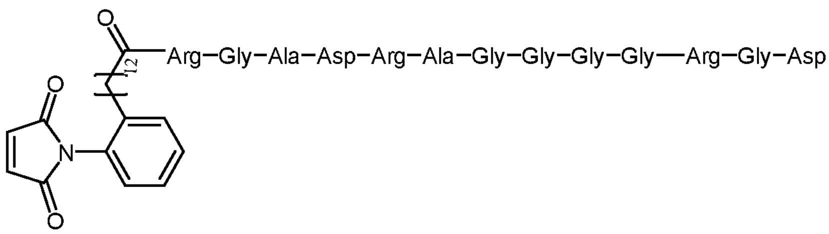 ;

polypeptide X25: 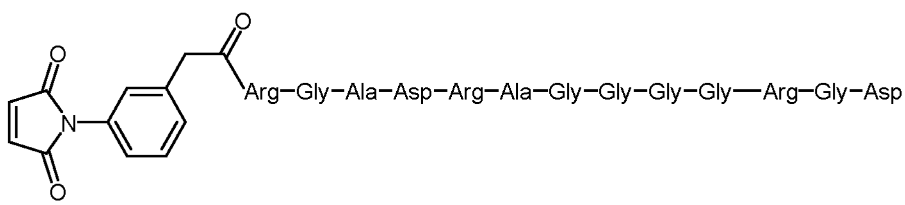 ;

polypeptide X26: 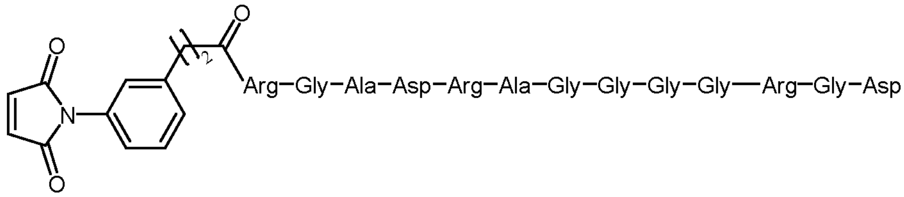 ;

polypeptide X27: 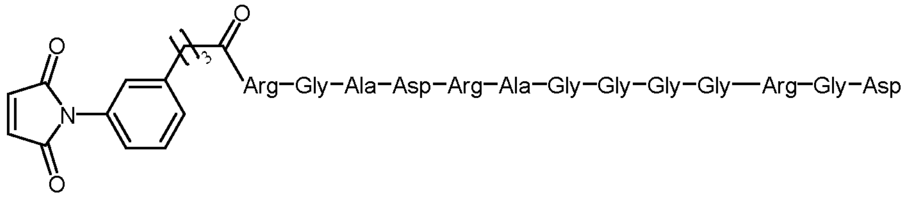 ;

polypeptide X28: 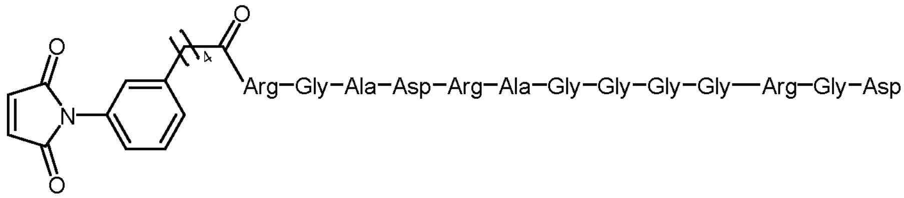 ;

polypeptide X29: 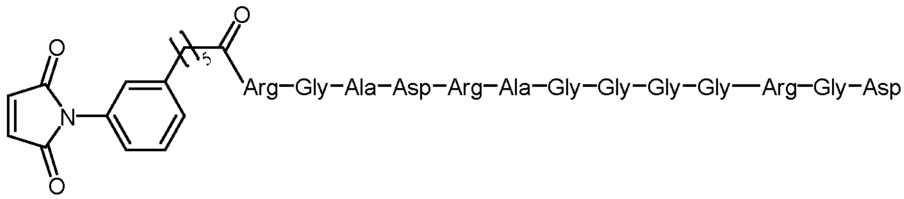 ;

polypeptide X30: 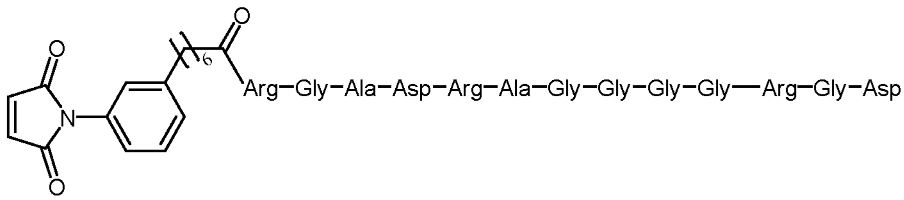 ;

polypeptide X31: 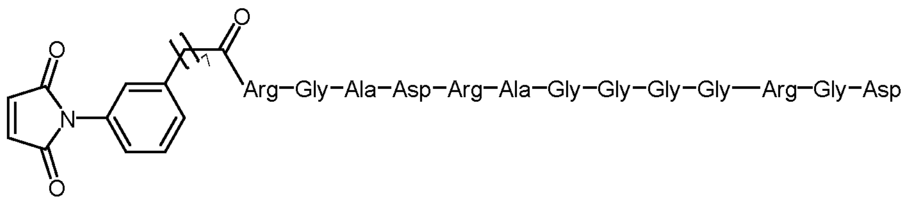 ;

polypeptide X32: 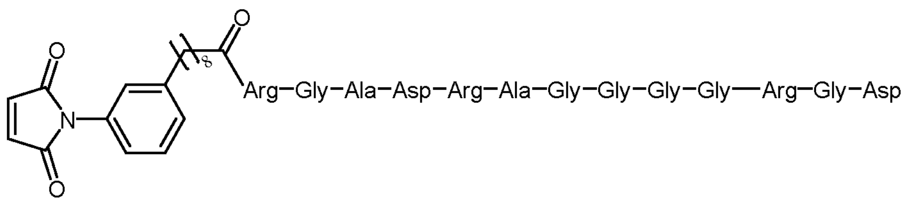 ;

polypeptide X33: 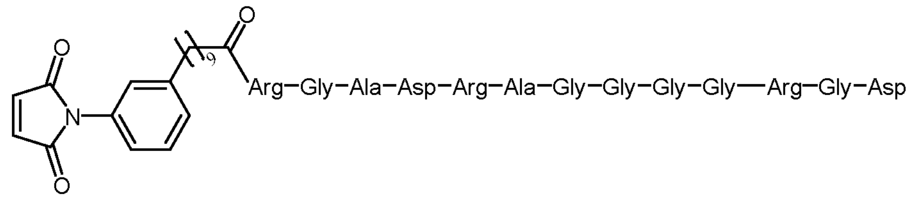 ;

polypeptide X34: 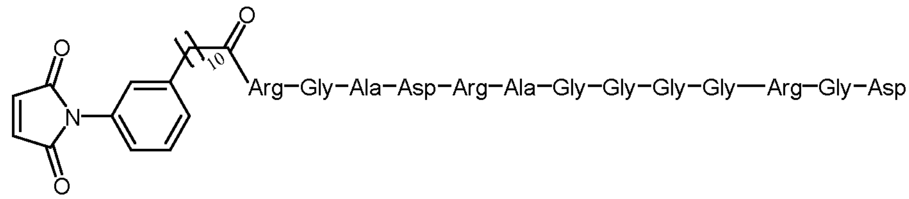 ;

polypeptide X35: 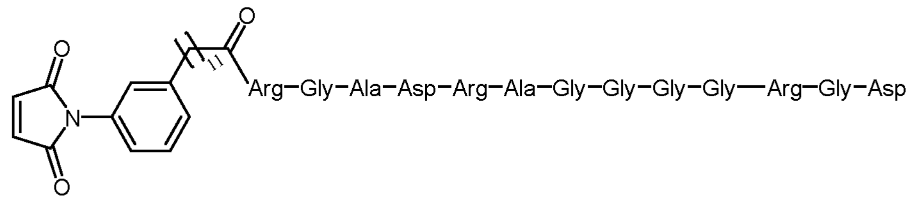 ;

polypeptide X36: 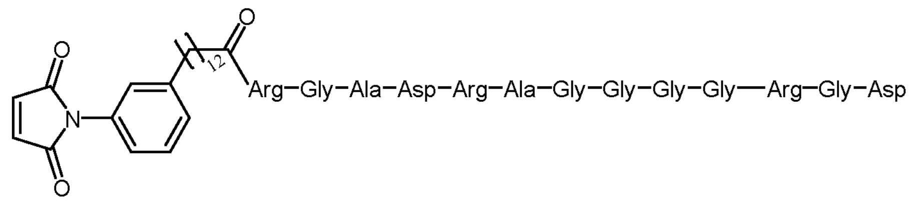 ;

polypeptide X37: 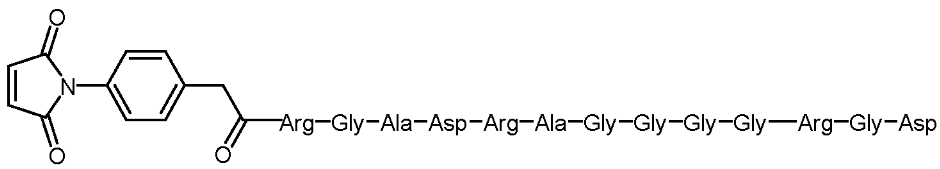 ;

polypeptide X38: 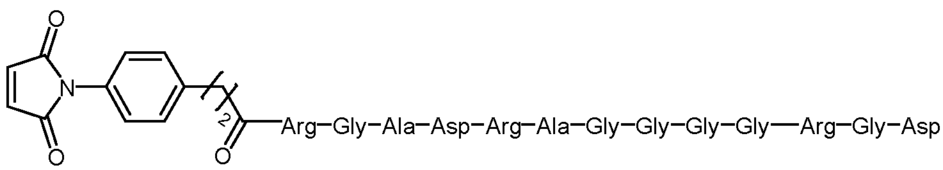 ;

polypeptide X39: 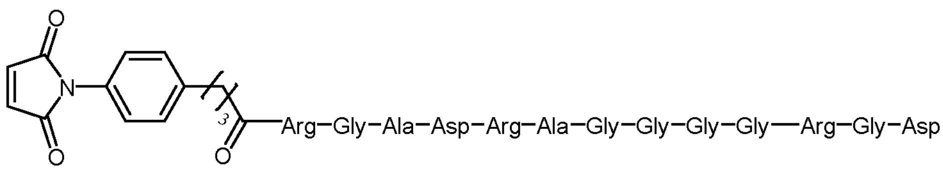 ;

polypeptide X40: 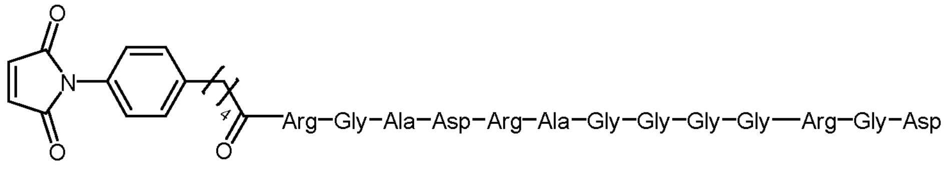 ;

polypeptide X41: 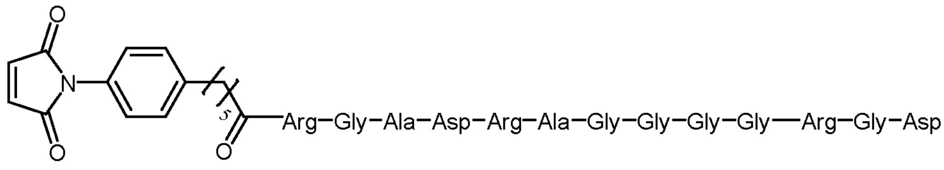 ;

polypeptide X42: 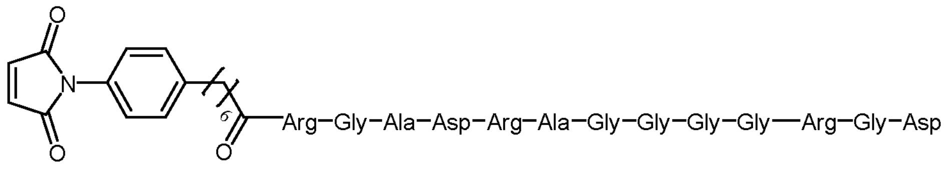 ;

polypeptide X43: 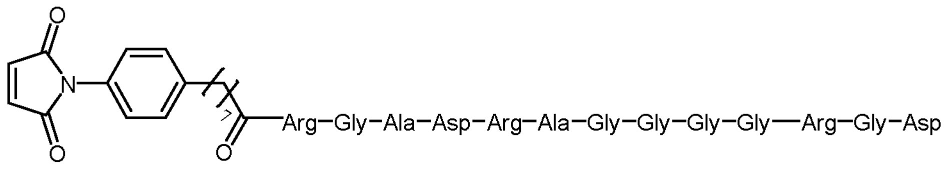 ;

polypeptide X44: 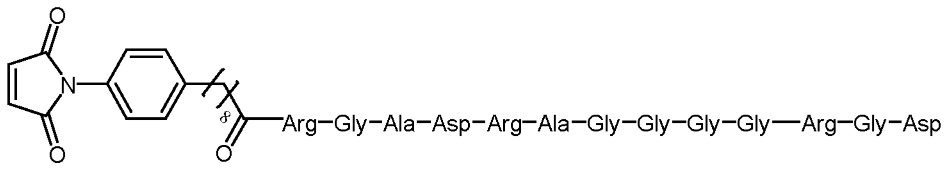 ;

polypeptide X45: 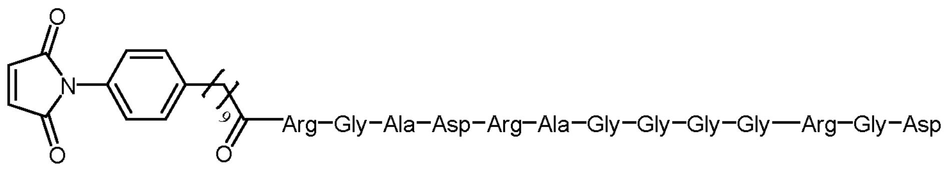 ;

polypeptide X46: 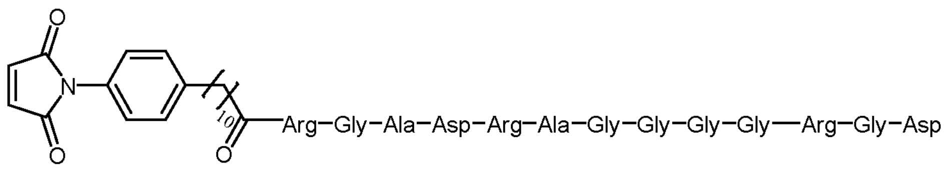 ;

polypeptide X47: 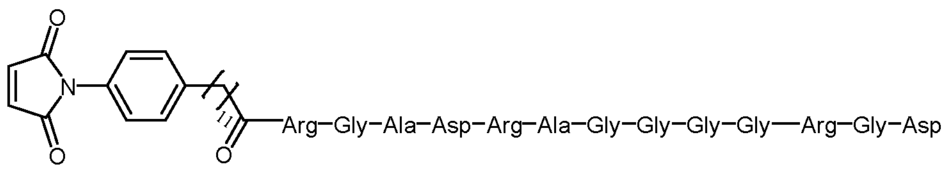 ;

polypeptide X48: 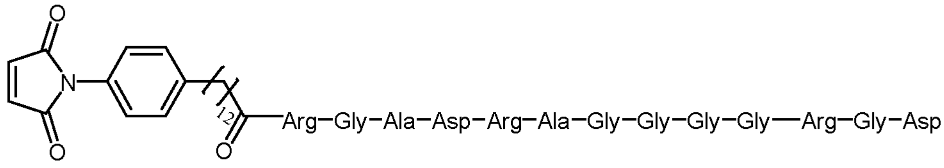 ;

polypeptide X49: 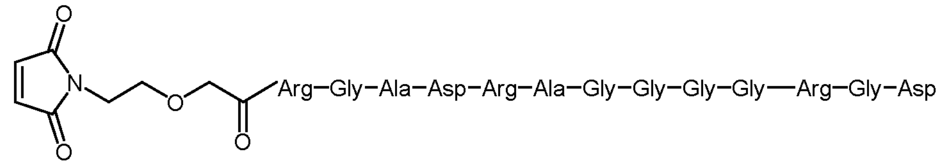 ;

polypeptide X50: 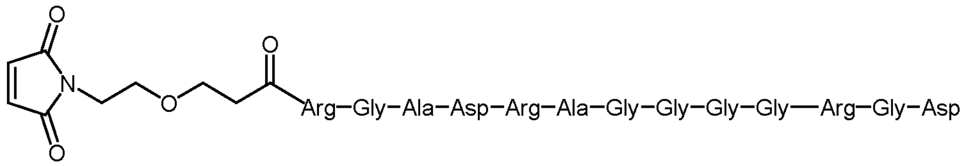 ;

polypeptide X51: 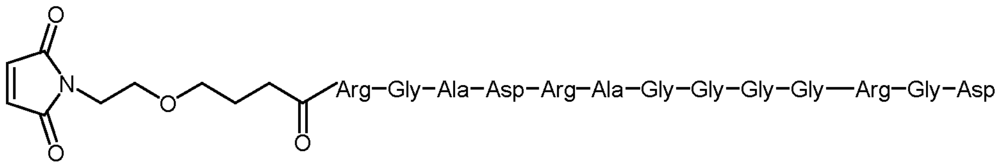 ;

polypeptide X52: 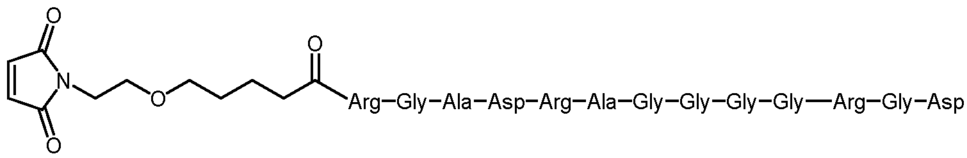 ;

polypeptide X53: 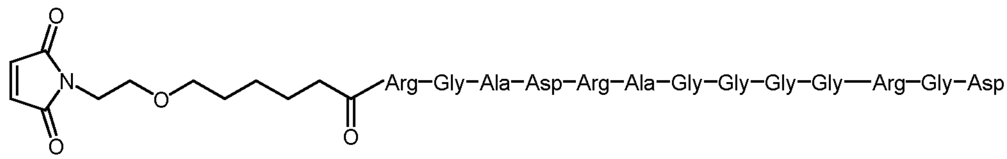 ;

polypeptide X54: 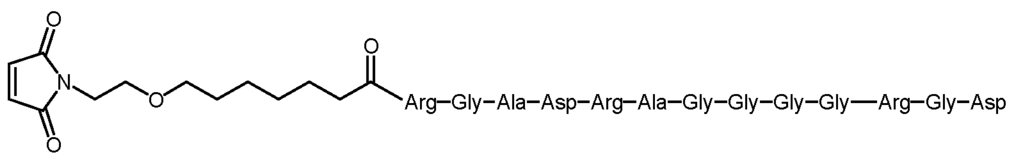 ;

polypeptide X55: 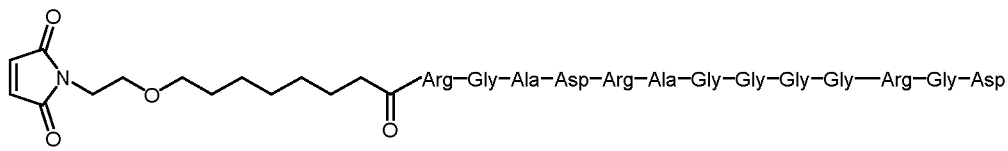 ;

polypeptide X56: 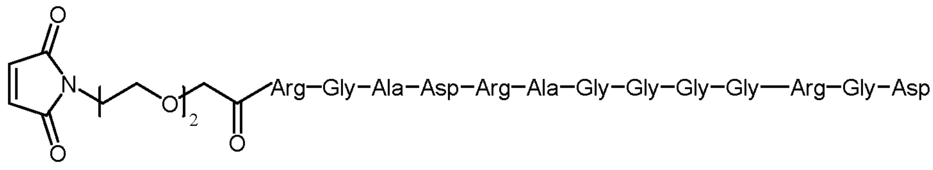 ;

polypeptide X57: 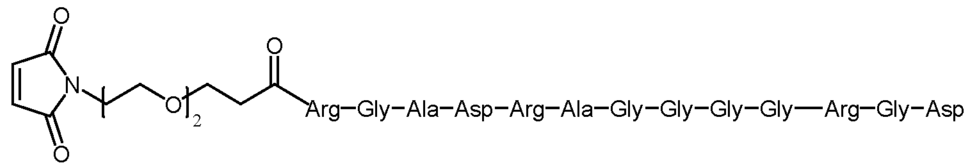 ;

polypeptide X58: 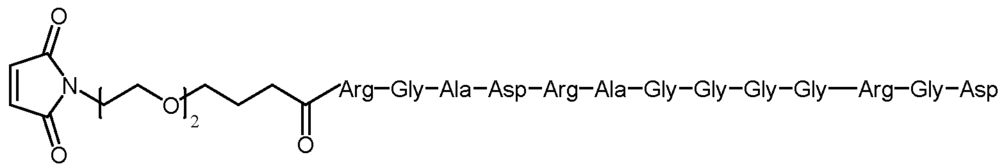 ;

polypeptide X59: 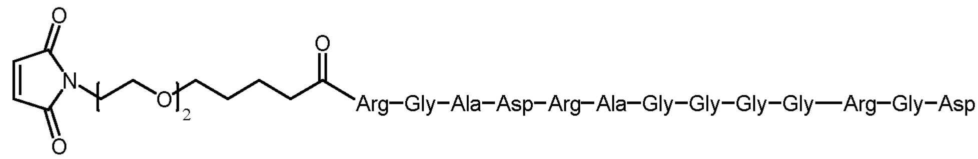 ;

polypeptide X60: 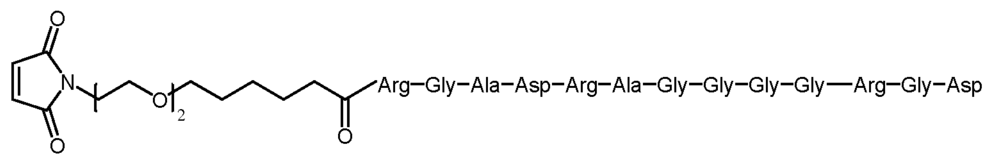 ;

polypeptide X61: 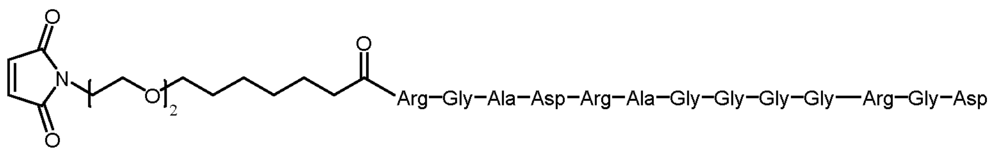 ;

polypeptide X62: 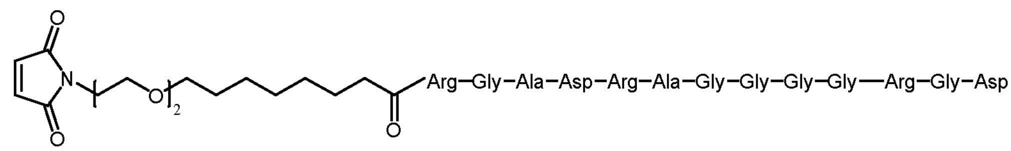 ;

polypeptide X63: 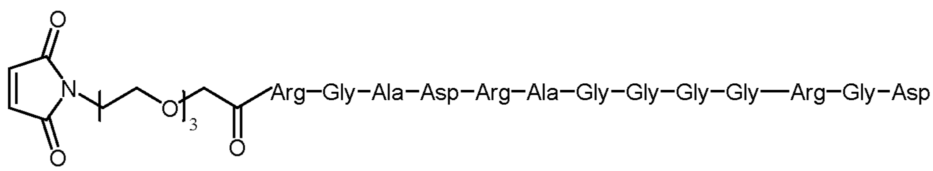 ;

polypeptide X64: 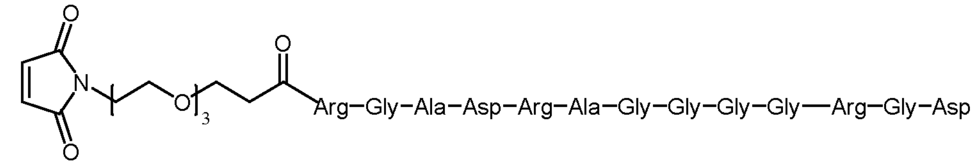 ;

polypeptide X65: 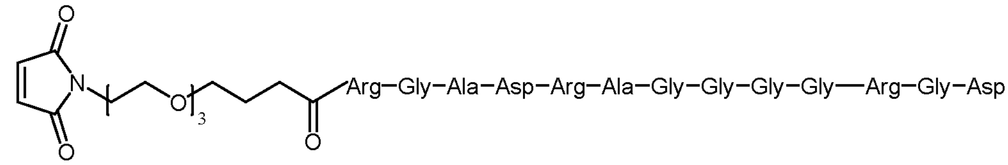 ;

polypeptide X66: 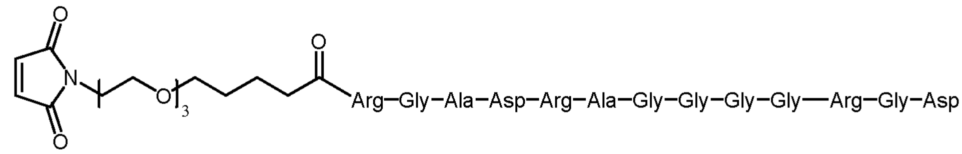 ;

polypeptide X67: 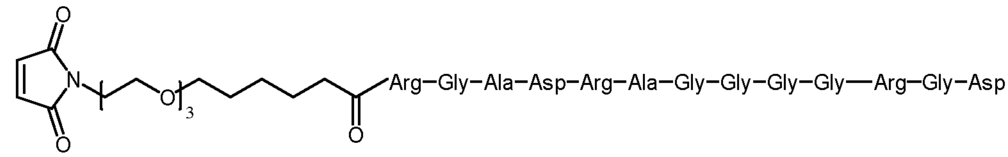 ;

polypeptide X68: 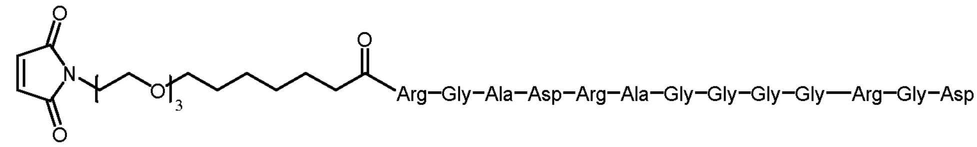 ;

polypeptide X69: 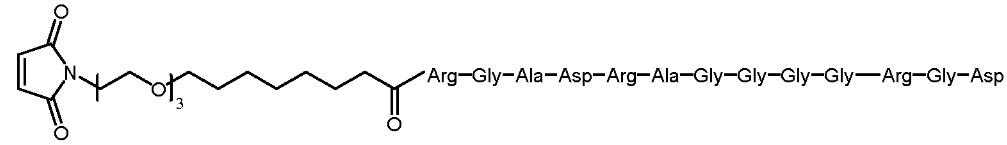 ;

polypeptide X70: 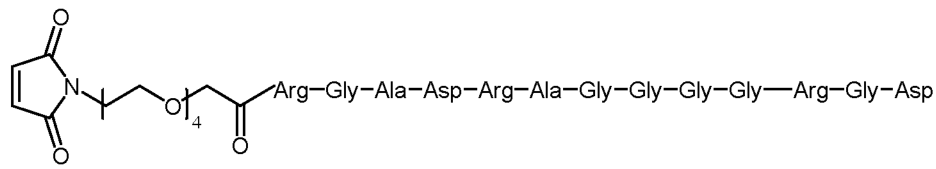 ;

polypeptide X71: 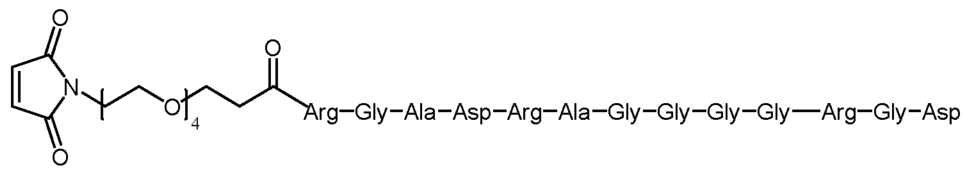 ;

polypeptide X72: 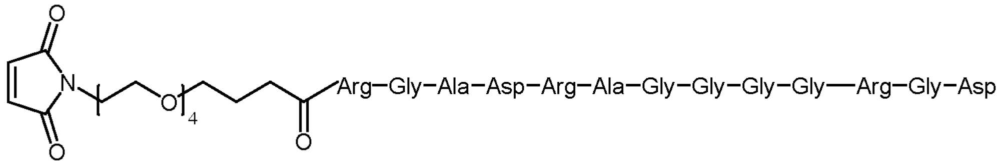 ;

polypeptide X73: 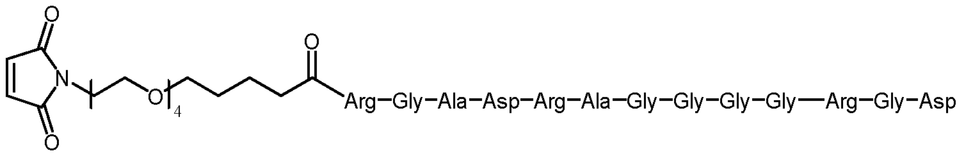 ;

polypeptide X74: 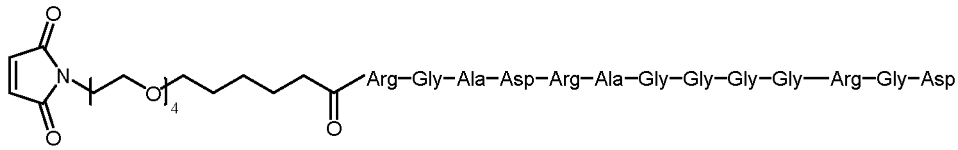 ;

polypeptide X75: 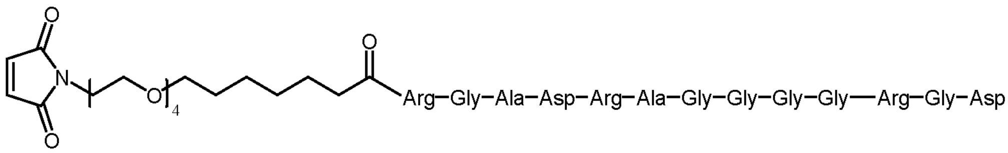 ;

polypeptide X76: 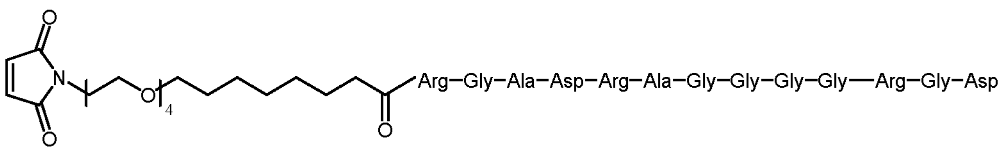 ;

polypeptide X77: 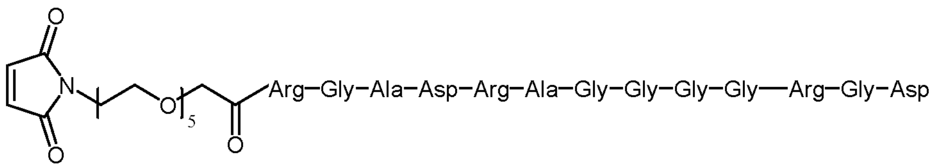 ;

polypeptide X78: 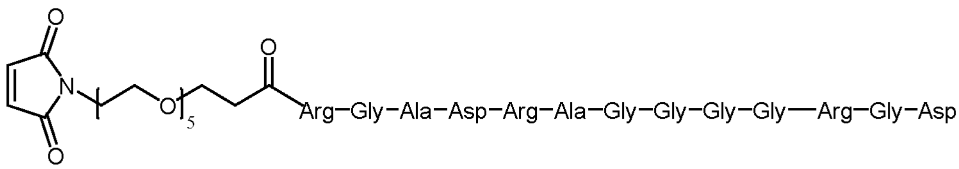 ;

polypeptide X79: 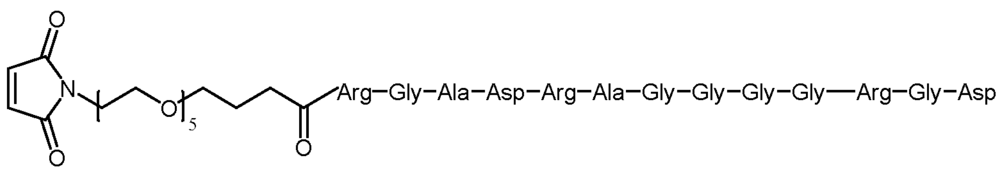 ;

polypeptide X80: 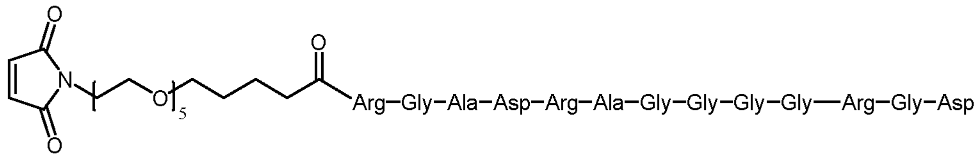 ;

polypeptide X81: 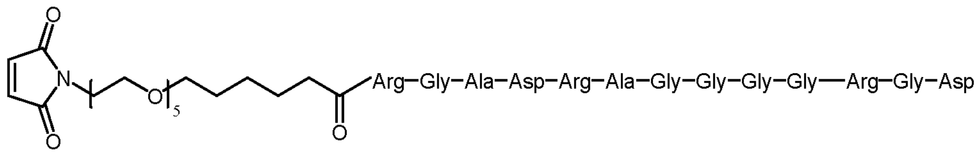 ;

polypeptide X82: 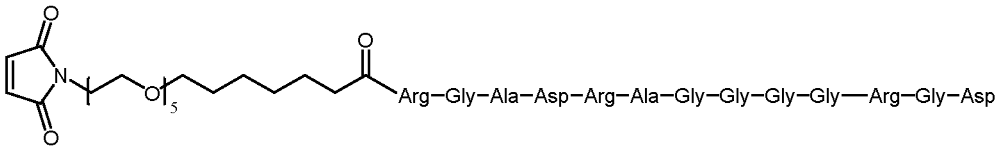 ;

polypeptide X83: 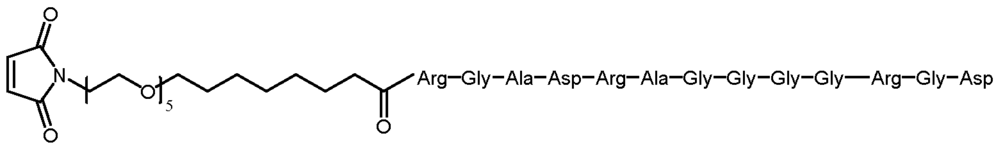 ;

polypeptide X84: 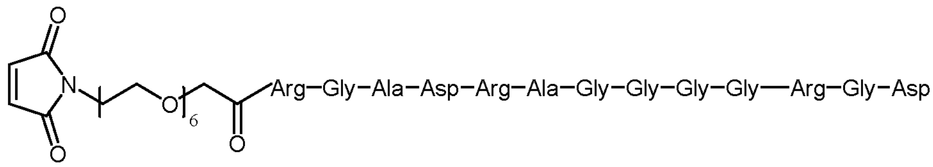 ;

polypeptide X85: 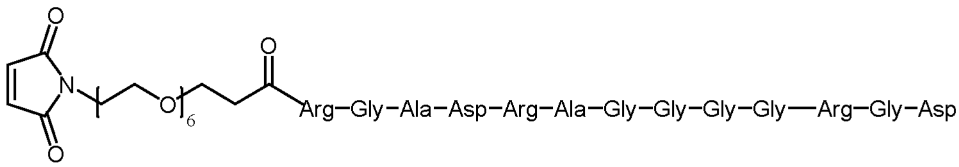 ;

polypeptide X86: 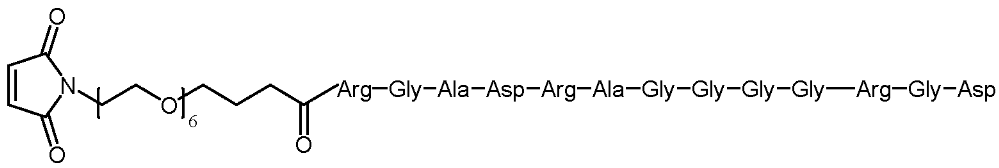 ;

polypeptide X87: 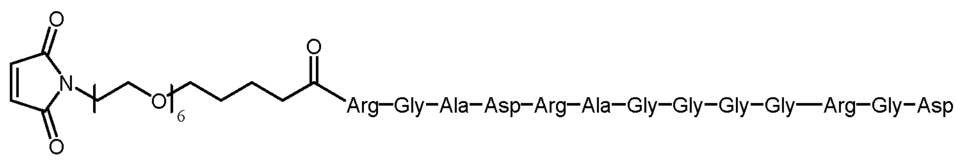 ;

polypeptide X88: 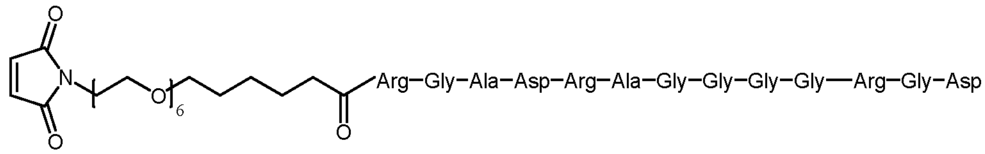 ;

polypeptide X89: 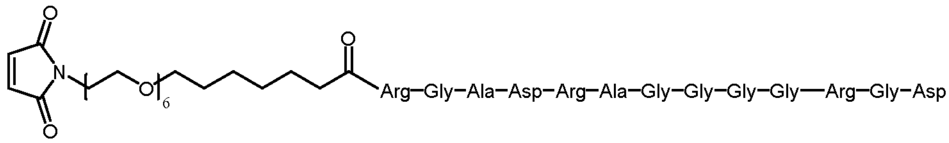 ;

and

polypeptide X90: 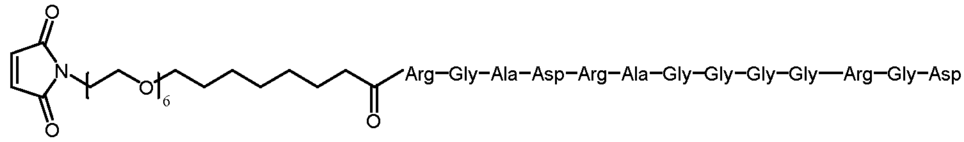 .

The functional group A in the present invention includes a sequence Arg-Gly-Ala-Asp-Arg-Ala and a derived polypeptide obtained by substituting one amino acid with another amino acid, or deleting one amino acid, or adding one or two amino acids between two amino acids. As long as the derived polypeptide has the same angiogenesis inhibition, anti-tumor and anti-inflammatory activities as Arg-Gly-Ala-Asp-Arg-Ala, it can be modified with the maleimide group in the present invention to prolong the half-life and have anti-tumor activities.

For example:

1) polypeptide P5 has a 3-maleimidopropionic acid-modified structure in which amino acid Ala at the third position is substituted by Glu:

polypeptide P5: 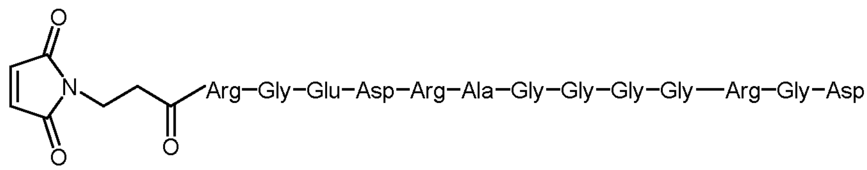 ;

2) polypeptide P6 has a 6-maleimidocaproic acid-modified structure in which amino acid Ala at the third position is substituted by Glu:

polypeptide P6: 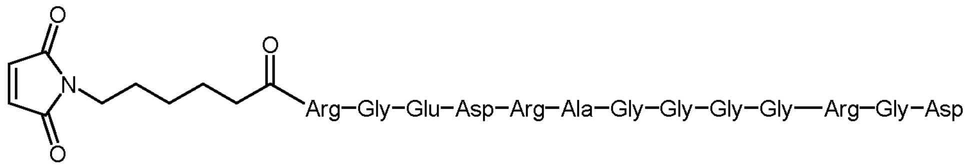 ;

3) polypeptide P7 has a 3-maleimidopropionic acid-modified structure in which amino acid Ala at the third position is deleted:

polypeptide P7: 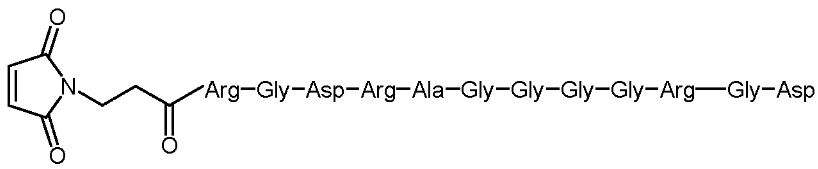 ;

4) polypeptide P8 has a 6-maleimidocaproic acid-modified structure in which amino acid Ala at the third position is deleted:

polypeptide P8: 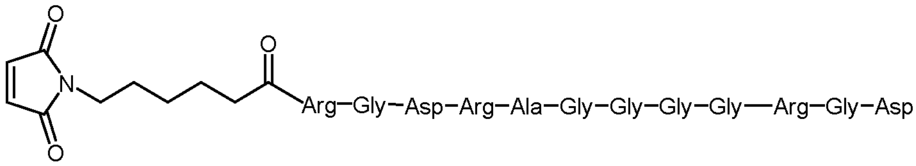 ;

5) polypeptide P9 has a 3-maleimidopropionic acid-modified structure in which a Val amino acid structure is added between an amino acid at the third position and an amino acid at the fourth position:

polypeptide P9: 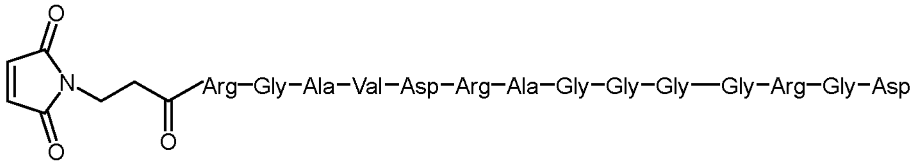 ;

and

6) polypeptide P10 has a 6-maleimidocaproic acid-modified structure in which a Val amino acid structure is added between an amino acid at the third position and an amino acid at the fourth position:

polypeptide P10: 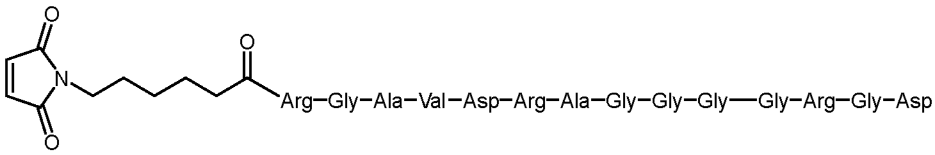 .

The linker between the functional groups A and B mainly plays a ligating role, as long as it is a short peptide that can ligate the functional groups A and B and does not affect the activities of both. The preferred linkers are Gly-Gly-Gly-Gly-Lys, Glu-Ala-Ala-Ala-Lys, and Gly-Ser-Ser-Ser-Ser. For example:

P11 uses Glu-Ala-Ala-Ala-Lys as the linker and is modified by 3-maleimidopropionic acid:

polypeptide P11: 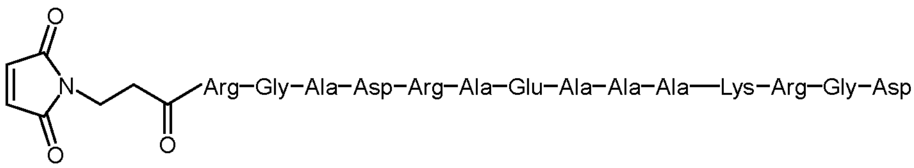 ;

polypeptide P12 uses Glu-Ala-Ala-Ala-Lys as the linker and is modified by 6-maleimidocaproic acid:

polypeptide P12: 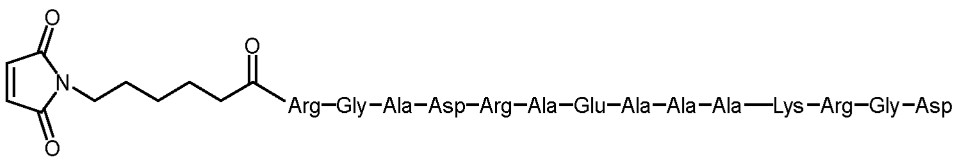 ;

polypeptide P13 uses Gly-Ser-Ser-Ser-Ser as the linker and is modified by 3-maleimidopropionic acid:

polypeptide P13: 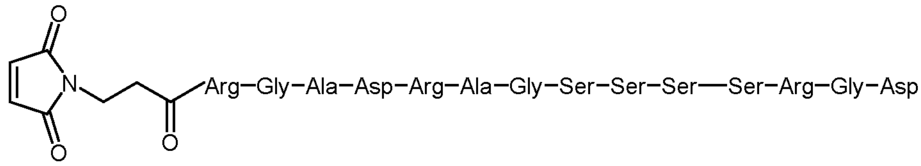 ;

and

polypeptide P14 uses Gly-Ser-Ser-Ser-Ser as the linker and is modified by 6-maleimidocaproic acid:

polypeptide P14: 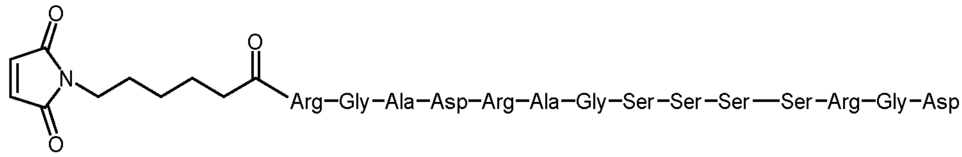 .

Use of the above modified angiogenesis inhibitor polypeptide in the preparation of a medicament for treating tumors, inflammations and ocular neovascular diseases is provided.

Further, the tumors are primary or secondary cancers, melanoma, hemangioma and sarcoma originating from human head and neck, brain, thyroid, esophagus, pancreas, lung, liver, stomach, breast, kidney, gallbladder, colon or rectum, ovary, blood vessel, cervix, prostate, bladder or testis.

Further, the inflammations include rheumatoid arthritis, gouty arthritis, reactive arthritis, osteoarthritis, psoriasis, infectious arthritis, traumatic arthritis and ankylosing spondylitis.

Further, the ocular neovascular diseases include age-related macular degeneration (AMD), iris neovascular eye diseases, choroidal neovascular eye diseases, retinal neovascular eye diseases or corneal neovascular eye diseases.

A medicament for treating tumors, inflammations and/or ocular neovascular diseases comprises the above modified angiogenesis inhibitor polypeptide and pharmaceutically acceptable excipients.

Further, the medicament is administered by injection, including subcutaneous injection, intramuscular injection, intravenous injection, vitreous injection and intravenous drip.

A method for preparing the foregoing maleimide group-modified angiogenesis inhibitor HM-1 comprises the following steps: (1) synthesizing the sequence of the maleimide group-modified angiogenesis inhibitor HM-1; (2) performing separation and purification to obtain the maleimide group-modified angiogenesis inhibitor HM-1 in solution; (3) performing rotary evaporation on the modified product solution obtained by separation and purification, and performing vacuum freeze drying to obtain modified product powder, and storing the modified product powder at -70°C.

The sequence Arg-Gly-Ala-Asp-Arg-Ala has the effect of inhibiting tumor angiogenesis. An RGD sequence is an important ligand of integrin, and therefore, polypeptide Gly-Gly-Gly-Gly-Arg-Gly-Asp containing the RGD sequence can also specifically recognize the integrin. The angiogenesis inhibitor polypeptide HM-1 of the present invention is a polypeptide, which is obtained by ligating a Gly-Gly-Gly-Gly-Arg-Gly-Asp sequence to the C-terminal of an Arg-Gly-Ala-Asp-Arg-Ala sequence having an angiogenesis inhibiting effect. The polypeptide HM-1 has affinity and binding ability with integrins, but it has a short half-life and needs frequent administration. In order to prolong the half-life of the HM-1 sequence, the applicant has conducted a series of studies. Without affecting the target and biological activity of the HM-1 sequence, the N-terminal of the polypeptide HM-1 is modified by maleimide group. The final optimized sequences are the foregoing polypeptides (XI to X90), which each contain a maleimide group and a polypeptide of 13 amino acids. The targets of the RGD sequence in the molecule are integrins $\alpha_v\beta_3$ and $\alpha_5\beta_1$. However, the main binding target is still the integrin $\alpha_v\beta_3$, which is bound to a neovascularization inhibiting sequence in this sequence, thus effectively inhibiting tumor neoangiogenesis and further achieving the effect of inhibiting tumor growth and metastasis.

The target of the polypeptide HM-1 is unchanged after the modification by the maleimide group; meanwhile, the in vivo half-life of the polypeptide molecule is prolonged, the clearance rate is low (see CL(L/h/kg) in Table 1), the immunogenicity and antigenicity are reduced, and the anti-tumor activity remains unchanged (see experimental results in Table 5). However, the plasma concentration at the lesion site is increased, so that the administration frequency is reduced. That is to say, the administration frequency of the maleimide group-modified HM-1 is changed from twice a day to once every seven days. The polypeptide HM-1 modified by the maleimide group mainly functions by reacting with sulfhydryl group at the 34$^{th}$ position of albumin. The reasons why the albumin accumulates in the tumor site are: 1) the vascular endothelial gap of a tumor tissue is large, the reason why macromolecules can penetrate through the vascular wall of the tumor tissue is different from that why the macromolecules can penetrate through blood vessels of a healthy

tissue, and the tumor site lacks lymphatic return, thus resulting in the enhanced permeability and retention (EPR) effect of the tumor tissue; 2) the albumin can be bound to specific receptor gp60 on the surface of the vascular endothelial cell membrane to activate caveolin-1 to form vesicles passing through endothelial cells, and then the vesicles are bound to secreted protein acidic and rich in cysteine (SPARC) in the tumor tissue gap. SPARC can specifically attract and adhere to albumin, so the albumin can be adsorbed and aggregated on the surface of tumor cells to induce tumor cell apoptosis.

According to a large number of experiments, the maleimide group-modified angiogenesis inhibitor in the present invention can obviously inhibit the proliferation and migration of human umbilical vein endothelial cells (HUVECs), and has obvious inhibition effect on the proliferation of tumor cells such as human cervical cancer HeLa cells, human colon cancer HCT 116 cells, human glioma U87 cells, and human breast cancer MDA-MB-231.

According to a large number of experiments, the maleimide group-modified angiogenesis inhibitor in the present invention can effectively treat angiogenesis inflammations. The experiments prove that the maleimide group-modified angiogenesis inhibitor of the present invention can target neovascular endothelium in the process of pannus formation in RA to inhibit neoangiogenesis, thereby achieving the effect of preventing or treating rheumatoid arthritis. Furthermore, rat adjuvant induced rheumatoid arthritis and DBA/1 mouse collagen induced rheumatoid arthritis models prove that the present invention has obvious effect of treating rheumatoid arthritis and has less side effects, less dosage and low costs.

According to a large number of experiments, the maleimide group-modified angiogenesis inhibitor in the present invention can inhibit the proliferation of human retinal capillary endothelial cells (HRCECs), and has a dose-dependent relationship within a certain range. The effect of the angiogenesis inhibitor polypeptide on mouse corneal neovascularization and rabbit iris neovascularization indicates that the maleimide group-modified angiogenesis inhibitor in the present invention can inhibit the growth of cornea and iris neovascularization, has the potential to be developed into drugs for treating corneal neovascular eye diseases and iris neovascular eye diseases, and has the potential effect of treating ocular neovascular diseases.

The choroid is located at the posterior of the eyeball. The experiments prove that the angiogenesis inhibitor can improve choroidal blood flow, which indicates that after doasing, the angiogenesis inhibitor can reach the choroid as soon as possible through systemic circulation or sclera-uvea-optic nerve pathway, and has inhibitory effect on choroidal neoangiogenesis of AMD. It is expected to be used for prevention or treatment of early AMD and other choroidal neovascular diseases. At the same time, the angiogenesis inhibitor has certain therapeutic effect on choroidal neovascular eye diseases including AMD by inhibiting the choroidal neoangiogenesis in rats.

The maleimide group-modified angiogenesis inhibitor in the present invention is scientific, reasonable, feasible and effective and can be used to prepare therapeutic drugs for treating human tumors, various inflammations and ocular neovascular diseases. The therapeutic spectrum of the angiogenesis inhibitor is greatly expanded, which provides new ideas and prospects for future drug development.

The half-life of the pre-modified polypeptide HM-1 is 0.34 h. The half-lives of maleimide group-modified polypeptides are shown in Tables 1-3.

Table 1 Comparison between the half-lives of polypeptides P1-P4 and HM-1 ($t_{1/2}\beta$ is half-life)

| Drug | $T_{1/2}\beta$(h) | CL(L/h/kg) | $AUC_{0-\infty}$(mg/L/h) | $MRT_{0-\infty}$(h) |
|---|---|---|---|---|
| HM-1 | 0.35±0.14 | 1.49±0.31 | 32.76±8.41 | 0.068±0.012 |
| P1 | 36.96 ±0.25 | 53.33 ±14.18 | 0.0336 ±0.0083 | 29.76 ±8.03 |
| P2 | 35.48 ±0.30 | 51.20 ±10.08 | 0.0350 ±0.0076 | 28.57 ±8.42 |
| P3 | 38.89 ±0.30 | 56.12 ±11.65 | 0.0319 ±0.0076 | 31.35 ±7.31 |
| P4 | 31.72 ±0.26 | 45.77 ±11.64 | 0.0391 ±0.0066 | 25.58 ±8.02 |

Table 2 Comparison between the half-lives of polypeptides P5-P14 and HM-1 ($t_{1/2}\beta$ is half-life)

| Drug | $T_{1/2}\beta$(h) | CL(L/h/kg) | $AUC_{0-\infty}$(mg/L/h) | $MRT_{0-\infty}$(h) |
|---|---|---|---|---|
| HM-1 | 0.35±0.14 | 1.49±0.31 | 32.76±8.41 | 0.068±0.012 |
| P5 | 36.28 ±0.25 | 0.0342 ±0.0090 | 29.24 ±7.88 | 52.35 ±12.15 |
| P6 | 34.36 ±0.25 | 0.0361 ±0.0084 | 27.70 ±7.13 | 49.58 ±9.97 |
| P7 | 39.63 ±0.28 | 0.0313 ±0.0091 | 31.95 ±7.82 | 57.19 ±13.90 |
| P8 | 38.67 ±0.27 | 0.0321 ±0.0093 | 31.15 ±8.09 | 55.8 ±13.36 |
| P9 | 34.64 ±0.30 | 0.0358 ±0.0068 | 27.93 ±6.88 | 49.99 ±10.65 |
| P10 | 30.58 ±0.31 | 0.0406 ±0.0071 | 24.63 ±7.03 | 44.13 ±11.03 |
| P11 | 39.16 ±0.31 | 0.0317 ±0.0091 | 31.55 ±8.23 | 56.51 ±11.62 |

(continued)

| Drug | $T_{1/2}\beta$(h) | CL(L/h/kg) | $AUC_{0-\infty}$(mg/L/h) | $MRT_{0-\infty}$(h) |
|---|---|---|---|---|
| P12 | 42.24 ±0.29 | 0.0294 ±0.0080 | 34.01 ±6.88 | 60.95 ±10.06 |
| P13 | 39.44 ±0.27 | 0.0315 ±0.0065 | 31.75 ±8.09 | 56.91 ±14.16 |
| P14 | 39.85 ±0.31 | 0.0311 ±0.0071 | 32.15 ±7.56 | 57.50 ±9.63 |

Table 3 Comparison between the half-lives of polypeptides X1-X90 and HM-1 ($t_{1/2}\beta$ is half-life)

| Drug | $T_{1/2}\beta$(h) | CL(L/h/kg) | $AUC_{0-\infty}$(mg/L/h) | $MRT_{0-\infty}$(h) |
|---|---|---|---|---|
| HM-1 | 0.35±0.14 | 1.49±0.31 | 32.76±8.41 | 0.068±0.012 |
| X1 | 35.99 ±0.29 | 0.0345 ±0.0059 | 28.99 ±6.94 | 51.93 ±10.18 |
| X2 | 34.51 ±0.28 | 0.0360 ±0.0078 | 27.78 ±7.51 | 49.78 ±9.73 |
| X3 | 41.92 ±0.32 | 0.0296 ±0.0064 | 33.78 ±7.66 | 60.46 ±13.17 |
| X4 | 37.44 ±0.27 | 0.0332 ±0.0091 | 30.12 ±7.33 | 53.97 ±10.52 |
| X5 | 34.59 ±0.29 | 0.0359 ±0.0067 | 27.86 ±7.03 | 49.91 ±13.23 |
| X6 | 37.86 ±0.28 | 0.0328 ±0.0076 | 30.49 ±7.17 | 54.63 ±12.19 |
| X7 | 42.83 ±0.29 | 0.0291 ±0.0083 | 34.48 ±6.96 | 61.82 ±14.31 |
| X8 | 34.68 ±0.31 | 0.0358 ±0.0087 | 27.93 ±7.57 | 50.04 ±12.62 |
| X9 | 38.78 ±0.31 | 0.0322 ±0.0074 | 31.25 ±7.35 | 55.96 ±13.35 |
| X10 | 38.64 ±0.25 | 0.0321 ±0.0086 | 31.15 ±7.21 | 55.76 ±12.95 |
| X11 | 31.08 ±0.29 | 0.0399 ±0.0082 | 25.06 ±8.26 | 44.85 ±11.56 |
| X12 | 36.20 ±0.25 | 0.0343 ±0.0084 | 29.15 ±8.18 | 52.24 ±9.78 |
| X13 | 41.58 ±0.34 | 0.0298 ±0.0089 | 33.56 ±7.34 | 60.00 ±12.54 |
| X14 | 36.04 ±0.32 | 0.0344 ±0.0071 | 29.07 ±7.66 | 52.01 ±13.47 |
| X15 | 42.65 ±0.25 | 0.0291 ±0.0063 | 34.36 ±8.52 | 61.54 ±10.04 |
| X16 | 39.00 ±0.24 | 0.0318 ±0.0061 | 31.45 ±8.39 | 56.28 ±13.62 |
| X17 | 37.26 ±0.29 | 0.0333 ±0.0071 | 30.03 ±7.06 | 53.77 ±12.34 |
| X18 | 33.75 ±0.27 | 0.0368 ±0.0078 | 27.17 ±7.31 | 48.70 ±14.06 |
| X19 | 39.95 ±0.28 | 0.0311 ±0.0087 | 32.15 ±6.97 | 57.65 ±10.75 |
| X20 | 39.56 ±0.28 | 0.0314 ±0.0093 | 31.85 ±8.32 | 57.09 ±12.91 |
| X21 | 42.24 ±0.28 | 0.0294 ±0.0075 | 34.01 ±8.19 | 60.89 ±9.41 |
| X22 | 35.03 ±0.27 | 0.0354 ±0.0071 | 28.25 ±7.98 | 50.55 ±13.64 |
| X23 | 31.72 ±0.25 | 0.0391 ±0.0062 | 25.58 ±8.51 | 45.77 ±10.57 |
| X24 | 36.77 ±0.29 | 0.0337 ±0.0061 | 29.67 ±7.58 | 53.06 ±13.51 |
| X25 | 30.56 ±0.32 | 0.0406 ±0.0089 | 24.63 ±7.53 | 44.1 ±10.67 |
| X26 | 42.95 ±0.36 | 0.0289 ±0.0089 | 34.62 ±8.34 | 61.98 ±9.63 |
| X27 | 32.52 ±0.25 | 0.0381 ±0.0082 | 26.25 ±7.25 | 46.93 ±9.74 |
| X28 | 31.19 ±0.26 | 0.0398 ±0.0068 | 25.13 ±7.92 | 45.01 ±13.02 |
| X29 | 39.91 ±0.25 | 0.0311 ±0.0072 | 32.15 ±8.03 | 57.59 ±10.58 |
| X30 | 35.17 ±0.37 | 0.0353 ±0.0067 | 28.33 ±6.88 | 50.75 ±10.28 |
| X31 | 35.58 ±0.31 | 0.0349 ±0.0067 | 28.65 ±6.96 | 51.34 ±11.26 |
| X32 | 38.33 ±0.31 | 0.0324 ±0.0091 | 30.86 ±7.75 | 55.31 ±11.84 |
| X33 | 42.15 ±0.26 | 0.0294 ±0.0087 | 34.01 ±7.62 | 60.82 ±11.96 |
| X34 | 34.35 ±0.26 | 0.0361 ±0.0067 | 27.71 ±8.15 | 49.57 ±10.18 |
| X35 | 39.60 ±0.31 | 0.0313 ±0.0078 | 31.95 ±8.09 | 57.14 ±14.19 |
| X36 | 34.90 ±0.27 | 0.0355 ±0.0084 | 28.17 ±8.09 | 50.36 ±10.11 |
| X37 | 33.76 ±0.28 | 0.0367 ±0.0057 | 27.25 ±7.21 | 48.72 ±13.61 |
| X38 | 38.94 ±0.25 | 0.0319 ±0.0079 | 31.35 ±7.55 | 56.19 ±11.24 |
| X39 | 40.73 ±0.28 | 0.0305 ±0.0065 | 32.79 ±7.11 | 58.77 ±13.08 |
| X40 | 42.23 ±0.27 | 0.0294 ±0.0079 | 34.01 ±7.83 | 60.94 ±10.65 |
| X41 | 32.89 ±0.36 | 0.0377 ±0.0071 | 26.53 ±7.81 | 47.46 ±13.88 |

(continued)

| Drug | $T_{1/2}\beta$(h) | CL(L/h/kg) | $AUC_{0-\infty}$(mg/L/h) | $MRT_{0-\infty}$(h) |
|---|---|---|---|---|
| X42 | 33.58 ±0.24 | 0.0369 ±0.0062 | 27.12 ±8.01 | 48.46 ±10.56 |
| X43 | 33.14 ±0.31 | 0.0374 ±0.0091 | 26.74 ±7.42 | 47.82 ±9.93 |
| X44 | 33.26 ±0.28 | 0.0373 ±0.0067 | 26.81 ±8.44 | 47.99 ±11.55 |
| X45 | 30.15 ±0.26 | 0.0411 ±0.0069 | 24.33 ±8.33 | 43.51 ±12.01 |
| X46 | 39.23 ±0.29 | 0.0316 ±0.0064 | 31.65 ±7.47 | 56.57 ±11.01 |
| X47 | 41.19 ±0.27 | 0.0301 ±0.0092 | 33.22 ±7.13 | 59.44 ±11.98 |
| X48 | 36.64 ±0.27 | 0.0339 ±0.0091 | 29.25 ±7.65 | 52.87 ±12.14 |
| X49 | 35.31 ±0.32 | 0.0351 ±0.0093 | 28.49 ±7.29 | 50.95 ±11.05 |
| X50 | 33.33 ±0.25 | 0.0372 ±0.0092 | 26.88 ±7.09 | 48.13 ±14.21 |
| X51 | 40.28 ±0.24 | 0.0308 ±0.0089 | 32.47 ±7.31 | 58.12 ±9.42 |
| X52 | 34.69 ±0.34 | 0.0358 ±0.0089 | 27.93 ±8.18 | 50.06 ±9.67 |
| X53 | 38.47 ±0.24 | 0.0322 ±0.0078 | 31.06 ±7.94 | 55.51 ±12.43 |
| X54 | 33.83 ±0.25 | 0.0367 ±0.0091 | 27.25 ±7.64 | 48.82 ±11.23 |
| X55 | 30.12 ±0.36 | 0.0412 ±0.0072 | 24.27 ±8.15 | 43.46 ±12.38 |
| X56 | 40.86 ±0.29 | 0.0304 ±0.0057 | 32.89 ±8.07 | 58.96 ±10.05 |
| X57 | 41.93 ±0.24 | 0.0296 ±0.0079 | 33.78 ±8.18 | 60.51 ±10.53 |
| X58 | 32.57 ±0.28 | 0.0381 ±0.0074 | 26.25 ±7.81 | 47.91 ±13.49 |
| X59 | 37.55 ±0.27 | 0.0332 ±0.0083 | 30.32 ±7.11 | 54.18 ±9.93 |
| X60 | 37.51 ±0.25 | 0.0331 ±0.0092 | 30.21 ±7.29 | 54.13 ±11.68 |
| X61 | 31.69 ±0.28 | 0.0391 ±0.0075 | 25.58 ±7.55 | 45.73 ±13.62 |
| X62 | 33.09 ±0.28 | 0.0375 ±0.0092 | 26.67 ±8.16 | 47.75 ±12.28 |
| X63 | 39.97 ±0.31 | 0.0310 ±0.0082 | 32.26 ±7.65 | 57.68 ±13.32 |
| X64 | 39.97 ±0.28 | 0.0310 ±0.0059 | 32.26 ±7.25 | 57.68 ±12.99 |
| X65 | 39.53 ±0.31 | 0.0314 ±0.0083 | 31.85 ±7.28 | 57.12 ±12.24 |
| X66 | 34.38 ±0.25 | 0.0361 ±0.0072 | 27.72 ±7.51 | 49.61 ±9.77 |
| X67 | 39.09 ±0.28 | 0.0317 ±0.0092 | 31.55 ±8.03 | 56.41 ±11.6 |
| X68 | 39.52 ±0.34 | 0.0314 ±0.0088 | 31.85 ±7.92 | 57.03 ±10.29 |
| X69 | 33.38 ±0.32 | 0.0372 ±0.0073 | 26.88 ±8.29 | 48.17 ±14.31 |
| X70 | 34.76 ±0.26 | 0.0357 ±0.0084 | 28.01 ±8.16 | 50.16 ±10.49 |
| X71 | 35.13 ±0.34 | 0.0353 ±0.0057 | 28.33 ±8.14 | 50.69 ±13.41 |
| X72 | 41.92 ±0.29 | 0.0296 ±0.0082 | 33.78 ±7.14 | 60.46 ±12.27 |
| X73 | 33.16 ±0.26 | 0.0374 ±0.0074 | 26.74 ±8.24 | 47.85 ±13.58 |
| X74 | 41.82 ±0.29 | 0.0297 ±0.0061 | 33.67 ±7.09 | 60.35 ±13.71 |
| X75 | 33.15 ±0.25 | 0.0374 ±0.0058 | 26.74 ±6.96 | 47.84 ±13.94 |
| X76 | 42.01 ±0.33 | 0.0295 ±0.0087 | 33.92 ±7.64 | 60.62 ±10.51 |
| X77 | 42.91 ±0.25 | 0.0289 ±0.0061 | 34.63 ±8.18 | 61.92 ±9.54 |
| X78 | 37.02 ±0.31 | 0.0335 ±0.0056 | 29.85 ±8.51 | 53.42 ±9.94 |
| X79 | 37.87 ±0.32 | 0.0328 ±0.0061 | 30.49 ±8.35 | 54.65 ±10.24 |
| X80 | 42.72 ±0.25 | 0.0290 ±0.0073 | 34.48 ±7.33 | 61.65 ±11.67 |
| X81 | 38.06 ±0.28 | 0.0326 ±0.0091 | 30.67 ±8.17 | 54.92 ±11.49 |
| X82 | 31.91 ±0.35 | 0.0389 ±0.0056 | 25.71 ±7.22 | 46.05 ±13.61 |
| X83 | 36.23 ±0.28 | 0.0342 ±0.0073 | 29.24 ±7.64 | 52.28 ±11.73 |
| X84 | 36.09 ±0.28 | 0.0344 ±0.0069 | 29.07 ±8.23 | 52.08 ±10.27 |
| X85 | 40.97 ±0.26 | 0.0303 ±0.0082 | 33.12 ±8.52 | 59.12 ±10.41 |
| X86 | 32.03 ±0.31 | 0.0387 ±0.0079 | 25.84 ±8.05 | 46.22 ±10.23 |
| X87 | 41.42 ±0.31 | 0.0299 ±0.0085 | 33.44 ±8.45 | 59.77 ±10.55 |
| X88 | 39.45 ±0.33 | 0.0314 ±0.0073 | 31.85 ±7.13 | 56.93 ±10.12 |
| X89 | 40.95 ±0.27 | 0.0303 ±0.0091 | 33.11 ±7.15 | 59.09 ±11.83 |
| X90 | 34.18 ±0.31 | 0.0363 ±0.0076 | 27.55 ±8.16 | 49.32 ±11.53 |

### 3. BENEFICIAL EFFECTS

Compared with the prior art, the present invention has the following beneficial effects:

(1) The half-life of the maleimide group-modified polypeptide HM-1 in the present invention is much longer than that of the HM-1 sequence, it is prolonged from 0.34 h to 30 h or more, the effect is very significant. Moreover, the modified polypeptide HM-1 increases the molecular weight and the plasma concentration at the lesion site after being bound to albumin, so that the administration frequency can be reduced from twice a day to once every seven days.

(2) According to the present invention, a solid phase synthesis method is adopted to enable the maleimide group to react with HM-1. Compared with PEG-modified HM-1, the present invention has the advantages of simple reaction, low cost, high yield and few impurities. The modified polypeptide in the present invention mainly functions by reacting the maleimide group with the sulfhydryl group at the $34^{th}$ position of albumin at a molar ratio of 1:1. However, after the polypeptide reacts with the albumin through the maleimide group, due to the molecular weight of the polypeptide is small, while the molecular weight of the albumin is large, the albumin may cover active sites of the polypeptide, causing its activity to decrease or completely lose. This is unpredictable. The ideal molecules can be obtained only by selecting different molecules with maleimide groups. These ideal molecules do not affect the activity and can prolong the half-life.

(3) The present invention provieds a new molecule of the polypeptide HM-1 modified by the maleimide group. A large number of in vivo and in vitro activity studies have been performed on three types of maleimide group-modified angiogenesis inhibitors in the present invention, and their therapeutic effects on various diseases have been studied. It is found that under the condition of reduced administration frequency, various modified products maintain good activities of HM-1, surpassing the polypeptide HM-1 and the PEG-modified HM-1, and increasing their social value and economic value.

(4) For protein polypeptide molecules, a novel molecule is formed once each amino acid changes, which is the characteristic of biological macromolecules. Therefore, for biological macromolecules, including polypeptide molecules, no technology is universal, and it needs to explore and test to find out whether the technology is suitable for this novel molecule. The polypeptide in the present invention is a new molecule designed and discovered by the applicant, which is modified by the maleimide group for the first time. This requires a large number of experiments to obtain the expected effect and cannot be realized by speculation. The product modified by the maleimide group in the present invention also belongs to a novel molecule having different effects on activity from the pre-modified molecule.

### DETAILED DESCRIPTION

The present invention is further described below with reference to specific examples.

### Example 1

Preparation and testing of angiogenesis inhibitor polypeptides X1-X90

The polypeptides X1-X90 were synthesized by solid phase synthesis and separated and purified by preparative HPLC, and their purities were determined by analytical RP-HPLC.

The methods for synthesizing the polypeptides X1-X90 are similar and are all solid phase synthesis methods. The methods each include using Fmoc-wang-resin as a starting material, ligating protective amino acids to dipeptide to tridecapeptide, followed by maleimide group, performing adequate washing after the completion of the synthetic operation, and then cleaving the peptide and performing post treatment to obtain a crude angiogenesis inhibitor; dissolving the crude product, purifying with a preparative high performance liquid chromatography twice, performing concentration and freeze drying to obtain a pure product, and finally purifying for the third time to obtain a refined polypeptide. The methods can not only ensure the synthesis efficiency, but also improve the product purity.

1. The steps of peptide ligation are as follows:

weighing an appropriate amount of Fmoc-wang-resin, pouring the Fmoc-wang-resin into a glass sand core reaction column, adding an appropriate amount of $CH_2Cl_2$ to fully expand the resin, and sequentially ligating the following protected amino acids and maleimide groups which are dissolved in N, N-dimethylformamide (DMF) to the resin: Fmoc-Gly-OH, Fmoc-Arg(pbf)-OH, Fmoc-Gly-OH, Fmoc-Gly-OH, Fmoc-Gly-OH, Fmoc-Gly-OH, Fmoc-Ala-OH, Fmoc-Arg(pbf)-OH, Fmoc-Asp(otBu)-OH, Fmoc-Gly-OH, Fmoc-Arg(pbf)-OH and maleimide groups, and activating with 1-hydroxybenzotriazole (HOBT) and N,N'-diisopropylcarbodiimide (DIC) sequentially; using DMF solution containing 20% piperidine to remove the Fmoc protecting groups for 20 min; using trifluoroacetic acid, phenol, water, thioanisole and EDT at a ratio

of 90:3:3:2:2 to cleave the peptide from the resin, and then precipitating with diethyl ether cooled by dry ice.

A. Deblocking: a proper amount of deblocking liquid of piperidine/DMF is added to react for a period of time, then the deblocking liquid is drained; during the process, the reactants are washed once with DMF, an addtional amount of deblocking liquid is add for reaction to remove Fmoc protecting groups.
B. Washing: the deblocking liquid is drained, and the resin is washed with DMF for several times, thereby fully washing off the by-products.
C. Condensation: the protective amino acids and activators for peptide ligation are dissolved in DMF and a condensing agent, and the mixture is shaked evenly; under the condition that the temperature is controlled at about 34°C, the reaction is fully carried out in a reactor.
D. Washing: the reaction liquid is drained, and the resin is fully washed with DMF, thereby washing off by-products.

2. The steps of peptide cleaving are as follows:
placing the drained resin into a round bottom flask, adding a lysate to fully lyse the synthesized X1-X90 intermediates, and separating the resin from the polypeptide by using a sand core funnel, wherein the lysate is composed of trifluoroacetic acid, phenol, water, thioanisole and EDT at the volume ratio of 90:3:3:2:2.
3. The steps of post treatment are as follows:
adding anhydrous diethyl ether first to the cleavage solution to precipitate the polypeptide, then centrifuging, pouring out the supernatant, then washing the polypeptide with the anhydrous diethyl ether, and draining to obtain the crude polypeptide.
4. The steps of purification are as follows:
A. Dissolution: the crude product is weighed accurately, an appropriate amount of purified water is added to prepare a solution with a concentration of 5-20 g/L, and the solution is ultrasonically stirred until a particle-free clear solution is obtained.
B. Filtration: the crude product solution is filtered with a 0.45 μm mixed filter membrane using a sand core filter.
C. Preparation: the primary purification, secondary purification and tertiary purification are performed by semi-preparative high performance liquid chromatography to obtain a refined qualified polypeptide, wherein the mobile phase comprises: acetonitrile in phase A and aqueous solution in phase B.
① Primary purification: the 10% of acetonitrile and 90% of aqueous solution is used to rinse and equilibrate the preparative column for 10 min at a flow rate of 60 mL/min, and the crude product solution is loaded by an infusion pump. The elution gradient is shown in Table 4.

Table 4 The elution gradient of primary purification

| Time (min) | Flow rate (mL/min) | A% | B% | Wavelength (nm) |
|---|---|---|---|---|
| 0 | 60 | 10 | 90 | 220 |
| 40 | 60 | 25 | 75 | 220 |

The solutions with the UV wavelength of 220 nm and an absorption value greater than 200 mV are collected, and the solutions with purity greater than 95% are pooled as a peak solution, which is subject to secondary separation and purification.
② Secondary purification: the rotary evaporation is performed on the peak solution obtained by the primary purification to remove organic solvent; the 5% of acetonitrile and 95% of aqueous solution is used to rinse and equilibrate the preparative column for 10 min at a flow rate of 60 mL/min; and the product solution is loaded by an infusion pump. The elution gradient is shown in Table 5.

Table 5 The elution gradient of secondary purification

| Time (min) | Flow rate (mL/min) | A% | B% | Wavelength (nm) |
|---|---|---|---|---|
| 0 | 60 | 7 | 95 | 220 |
| 40 | 60 | 15 | 85 | 220 |

The solutions with the UV wavelength of 220 nm and an absorption value greater than 200 mV are collected, and the solutions with purity greater than 98.5% were taken as qualified solutions.
D. Concentration, filtration and freeze-drying: the qualified solutions are concentrated under reduced pressure at 37°C by a rotary evaporator to remove the residual solvent and part of moisture. Finally, the concentrated solutions are filtered

with a 0.22 μm filter membrane, and the filtrate is put into a freeze-drying plate, and freeze-dried with a freeze dryer to obtain pure product.

5. Purity analysis of product

The purity of the freeze-dried product is analyzed by analytical high performance liquid chromatography under the following conditions:

analytical chromatographic column: COSMOSIL, 250 mm×4.6 mm(5 μm filler);
mobile phase: phase A is water and phase B is acetonitrile;
loading: 20 μL;
flow rate: 1 mL/min;
detection wavelength: 220 nm; and
elution gradient: see Table 6.

Table 6 RP-HPLC determination of the purity of polypeptides X1-X90

| Number of polypeptides | Peak area | Purity (%) |
|---|---|---|
| X1 | 12106 | 97.94 |
| X2 | 12100 | 97.44 |
| X3 | 13004 | 97.77 |
| X4 | 12585 | 98.53 |
| X5 | 12791 | 98.62 |
| X6 | 12237 | 98.20 |
| X7 | 12335 | 97.57 |
| X8 | 12866 | 98.58 |
| X9 | 12483 | 98.60 |
| X10 | 13087 | 98.36 |
| X11 | 13564 | 98.19 |
| X12 | 13382 | 98.28 |
| X13 | 12577 | 97.56 |
| X14 | 12289 | 97.94 |
| X15 | 13803 | 98.07 |
| X16 | 13947 | 97.85 |
| X17 | 13286 | 98.71 |
| X18 | 12703 | 98.23 |
| X19 | 12975 | 98.69 |
| X20 | 13564 | 98.17 |
| X21 | 12507 | 98.67 |
| X22 | 12896 | 97.77 |
| X23 | 12580 | 97.65 |
| X24 | 13913 | 98.46 |
| X25 | 12652 | 97.44 |
| X26 | 13897 | 98.59 |
| X27 | 13163 | 98.52 |
| X28 | 12899 | 97.92 |
| X29 | 13266 | 97.65 |
| X30 | 13175 | 97.98 |
| X31 | 12161 | 98.32 |
| X32 | 12667 | 98.12 |
| X33 | 13077 | 98.17 |
| X34 | 12472 | 98.51 |
| X35 | 13212 | 97.65 |
| X36 | 12524 | 97.96 |
| X37 | 12323 | 98.00 |

(continued)

| Number of polypeptides | Peak area | Purity (%) |
|---|---|---|
| X38 | 13814 | 97.50 |
| X39 | 12994 | 98.68 |
| X40 | 13681 | 98.34 |
| X41 | 13071 | 98.34 |
| X42 | 12114 | 97.48 |
| X43 | 13983 | 97.59 |
| X44 | 13478 | 97.70 |
| X45 | 13798 | 98.11 |
| X46 | 13561 | 98.74 |
| X47 | 13536 | 98.43 |
| X48 | 12835 | 97.96 |
| X49 | 12246 | 97.46 |
| X50 | 13169 | 97.77 |
| X51 | 13833 | 98.27 |
| X52 | 13008 | 98.12 |
| X53 | 12150 | 98.70 |
| X54 | 12528 | 98.75 |
| X55 | 13726 | 98.28 |
| X56 | 12280 | 98.14 |
| X57 | 13729 | 97.50 |
| X58 | 13223 | 97.85 |
| X59 | 13617 | 98.68 |
| X60 | 12782 | 97.62 |
| X61 | 13850 | 98.32 |
| X62 | 12497 | 98.65 |
| X63 | 12648 | 97.71 |
| X64 | 13387 | 98.19 |
| X65 | 12216 | 98.75 |
| X66 | 12686 | 97.89 |
| X67 | 13047 | 98.61 |
| X68 | 13175 | 97.69 |
| X69 | 13188 | 98.37 |
| X70 | 12976 | 98.15 |
| X71 | 12312 | 98.49 |
| X72 | 13300 | 97.97 |
| X73 | 12117 | 97.60 |
| X74 | 12797 | 98.32 |
| X75 | 12204 | 97.57 |
| X76 | 12977 | 97.54 |
| X77 | 13314 | 97.98 |
| X78 | 13087 | 97.94 |
| X79 | 12609 | 98.37 |
| X80 | 13924 | 98.25 |
| X81 | 13725 | 97.78 |
| X82 | 13364 | 97.76 |
| X83 | 13564 | 98.44 |
| X84 | 12689 | 98.30 |
| X85 | 12316 | 98.74 |
| X86 | 12902 | 98.21 |

(continued)

| Number of polypeptides | Peak area | Purity (%) |
|---|---|---|
| X87 | 13138 | 97.43 |
| X88 | 13282 | 97.91 |
| X89 | 12533 | 97.69 |
| X90 | 12595 | 97.57 |

Results: The synthesized polypeptides were analyzed by reversed phase liquid chromatography to obtain purity identification results. As shown in Table 6, the purities of the polypeptides X1-X90 are greater than 95%, which meets the design requirements.

**Example 2**

Proliferation inhibition experiment of maleimide group-modified angiogenesis inhibitor polypeptides on various tumor cells

An MTT method was used to detect the inhibitory activity of X1-X90 on the growth of various tumor cells. Tumor cells were digested and collected with trypsin after being cultured in an incubator at 37°C with 5% $CO_2$ to a confluence of 90% or more. The cells were resuspended with a culture solution and counted under a microscope, the cell concentration was adjusted to $2\times10^4$ cells/mL, and the cell suspension was inoculated into a 96-well plate at 100 μL/well, and cultured overnight in an incubator at 37°C with 5% $CO_2$. X1-X90 were diluted to respective predetermined concentrations with the culture solution. Docetaxel was diluted to a final concentration with the culture solution. After the cells were completely adhered to the wall, each diluent was added into the 96-well plate (100 μL/well). Tumor cells with the addition of diluents of polypeptides X1-X90 were used as administration groups, tumor cells with the addition of docetaxel were used as a positive control group, and tumor cells with the addition of the culture solution without any drug were used as a negative control group. The cells were cultured in an incubator at 37°C with 5% $CO_2$ for 48 h. 5 mg/mL MTT was added into the 96-well plate, 20 μL per well, and the cultivation was continued for 4 h. The culture medium was removed, 150 μL of DMSO was added to each well for dissolution, and gent and uniform mixing was performed in a shaker for 10 min. The absorbance was measured at a detection wavelength of 570 nm and a reference wavelength of 630 nm using a microplate reader, and the proliferation inhibition rate (PIR) was calculated by the formula as follows:

$$\text{PIR (\%)} = 1 - \text{administration group/negative group}$$

The experiment was repeated independently three times, and the results were expressed as mean ± SD. The experimental results are shown in Table 7.

Table 7 PIR (%) of polypeptides X1-X90 on various tumor cells

| Tumor cell line source | HM-1 | X1 | X2 | X3 | X4 | X5 | X6 | X7 | X8 | X9 | X10 |
|---|---|---|---|---|---|---|---|---|---|---|---|
| Head and neck cancers | 36.12 ± 11.21 | 45.32 ± 10.94 | 49.44 ± 18.24 | 40.55 ± 17.92 | 45.13 ± 15.15 | 69.15 ± 16.66 | 58.41± 11.59 | 40.15 ± 15.57 | 49.93 ± 18.85 | 37.65 ± 18.92 | 76.62± 17.47 |
| Brain tumor | 41.54 ± 12.43 | 61.34 ± 18.45 | 46.62 ± 15.43 | 47.71 ± 12.42 | 70.81 ± 16.63 | 76.29 ± 17.93 | 62.37± 17.95 | 71.71 ± 12.28 | 73.5 ± 12.73 | 55.98 ± 18.21 | 71.95 ± 17.4 |
| Thyroid cancer | 43.15 ± 14.42 | 55.15 ± 15.82 | 69.33 ± 14.38 | 64.22 ± 12.61 | 62.99 ± 14.78 | 72.3 ± 18.62 | 73.62± 11.02 | 58.03 ± 18.41 | 73.95 ± 12.30 | 42.05 ± 12.36 | 63.94± 18.95 |
| Esophageal cancer | 46.22 ± 18.24 | 62.32 ± 19.64 | 64.37 ± 18.49 | 36.11 ± 10.66 | 38.81 ± 11.36 | 54.16 ± 10.75 | 70.62± 17.02 | 46.78 ± 14.88 | 75.56 ± 16.11 | 69.92 ± 11.67 | 59.44± 18.63 |
| Pancreatic cancer | 45.03 ± 13.18 | 75.04 ± 16.68 | 69.94 ± 19.94 | 68.79 ± 15.74 | 67.47 ± 17.41 | 78.51 ± 14.26 | 67.47± 19.99 | 74.36 ± 15.33 | 70.29 ± 18.12 | 72.1 ± 14.74 | 68.83± 10.76 |
| Lung cancer | 50.35 ± 14.14 | 50.55 ± 15.04 | 51.09 ± 18.62 | 73.31 ± 13.07 | 71.83 ± 17.48 | 63.21 ± 16.98 | 75.24± 10.57 | 65.66 ± 17.89 | 71.42 ± 11.21 | 45.21 ± 14.27 | 49.77± 19.68 |
| Liver cancer | 44.45 ± 13.46 | 47.77 ± 15.96 | 48.67 ± 13.64 | 39.09 ± 19.99 | 63.37 ± 12.42 | 56.49 ± 16.35 | 60.46± 13.32 | 52.51 ± 18.03 | 46.78 ± 11.22 | 70.57 ± 18.17 | 52.17± 13.39 |
| Stomach cancer | 44.43 ± 11.35 | 46.42 ± 10.95 | 66.68 ± 13.89 | 36.88 ± 18.33 | 59.22 ± 15.12 | 74.39 ± 19.66 | 66.17± 19.84 | 70.86 ± 13.36 | 70.39 ± 10.11 | 55.43 ± 18.32 | 67.71± 10.63 |
| Breast cancer | 41.54 ± 14.28 | 50.84 ± 14.38 | 38.08 ± 12.92 | 47.44 ± 13.73 | 62.03 ± 11.14 | 56.98 ± 12.93 | 45.39± 15.53 | 43.24 ± 10.21 | 59.25 ± 13.82 | 41.76 ± 16.73 | 48.69± 17.43 |
| Kidney cancer | 42.45 ± 10.22 | 62.65 ± 11.81 | 42.42 ± 19.73 | 69.87 ± 14.85 | 37.37 ± 15.13 | 64.26 ± 13.95 | 35.68± 16.37 | 39.42 ± 12.53 | 45.77 ± 18.32 | 59.77 ± 10.23 | 55.27± 12.37 |
| Colorectal cancer | 42.64 ± 12.31 | 69.65 ± 12.22 | 68.97 ± 13.48 | 56.23 ± 15.52 | 41.05 ± 11.77 | 78.84 ± 14.67 | 43.61 ± 12.53 | 47.35 ± 12.35 | 43.97 ± 19.5 | 58.82 ± 14.58 | 36.56± 13.66 |
| Ovarian cancer | 44.78 ± 14.06 | 56.91 ± 17.05 | 62.85 ± 17.22 | 45.82 ± 10.23 | 57.25 ± 15.89 | 70.07 ± 17.29 | 35.33± 17.57 | 53.68 ± 11.49 | 66.00 ± 14.31 | 76.51 ± 18.97 | 67.9 ± 11.92 |
| Cervical cancer | 46.03 ± 14.71 | 66.03 ± 16.73 | 68.94 ± 14.21 | 53.82 ± 15.25 | 47.74 ± 17.07 | 65.31 ± 13.87 | 53.83± 18.12 | 50.12 ± 15.57 | 61.36 ± 10.83 | 36.05 ± 13.62 | 43.58± 12.38 |
| Uterine cancer | 46.85 ± 10.21 | 44.85 ± 11.31 | 61.25 ± 19.98 | 37.31 ± 18.32 | 52.66 ± 19.92 | 76.97 ± 16.87 | 47.04 ± 17.32 | 60.03 ± 15.64 | 74.99 ± 18.79 | 55.32 ± 16.01 | 69.42 ± 18.85 |
| Prostate cancer | 50.14 ± 14.13 | 70.12 ± 17.10 | 52.24 ± 18.33 | 63.22 ± 15.99 | 76.32 ± 14.54 | 75.07 ± 13.88 | 55.64± 19.58 | 40.17 ± 15.73 | 40.62 ± 11.39 | 69.35 ± 15.14 | 62.39± 12.52 |
| Bladder cancer | 51.99 ± 14.57 | 52.59 ± 19.54 | 67.44 ± 15.81 | 58.84 ± 14.48 | 36.28 ± 15.22 | 73.55 ± 15.92 | 76.54 ± 13.26 | 69.99 ± 16.57 | 39.36 ± 11.00 | 41.19 ± 11.96 | 41.05± 13.81 |
| Melanoma | 53.62 ± 12.12 | 53.73 ± 18.72 | 66.34 ± 12.43 | 43.28 ± 12.95 | 40.51 ± 14.82 | 68.38 ± 18.49 | 54.92 ± 17.94 | 36.52 ± 13.11 | 66.92 ± 11.56 | 59.72 ± 17.25 | 73.63 ± 14.34 |
| Hemangioma | 46.23 ± 12.09 | 66.23 ± 10.06 | 56.24 ± 19.91 | 59.43 ± 16.89 | 54.64 ± 12.72 | 65.27 ± 10.67 | 41.63± 18.32 | 71.12 ± 13.01 | 42.54 ± 12.49 | 44.06 ± 19.17 | 65.39 ± 18.56 |
| Sarcoma | 49.25 ± 11.03 | 69.22 ± 11.18 | 39.82 ± 17.52 | 47.97 ± 19.23 | 42.74 ± 18.42 | 77.03 ± 12.78 | 47.72± 10.94 | 49.68 ± 10.83 | 48.86 ± 16.97 | 68.92 ± 11.29 | 49.55 ± 11.75 |

Table 7 PIR (%) of polypeptides X1-X90 on various tumor cells (continued)

| Tumor cell line source | X11 | X12 | X13 | X14 | X15 | X16 | X17 | X18 | X19 | X20 |
|---|---|---|---|---|---|---|---|---|---|---|
| Head and neck cancers | 70.38 ± 10.46 | 43.35 ± 10.51 | 52.83 ± 16.12 | 57.41 ± 12.19 | 43.22 ± 12.75 | 45.08 ± 12.39 | 44.20 ± 10.35 | 72.52 ± 13.81 | 46.15 ± 17.34 | 35.32 ± 10.94 |

| | | | | | | | | | | |
|---|---|---|---|---|---|---|---|---|---|---|
| Brain tumor | 43.5 ± 10.29 | 64.6 ± 10.41 | 45.99 ± 10.57 | 65.89 ± 13.7 | 42.86 ± 15.27 | 63.52 ± 15.53 | 57.12 ± 12.71 | 76.72 ± 11.32 | 44.00 ± 15.36 | 61.34 ± 18.45 |
| Thyroid cancer | 61.62 ± 16.5 | 48.73 ± 13.45 | 75.42 ± 14.25 | 69.82 ± 11.28 | 67.00 ± 13.00 | 43.49 ± 17.57 | 65.07 ± 12.16 | 45.28 ± 15.02 | 61.38 ± 14.8 | 55.15 ± 15.82 |
| Esophageal cancer | 75.25 ± 19.36 | 49.55 ± 10.47 | 39.83 ± 18.32 | 63.49 ± 14.04 | 54.44 ± 10.82 | 42.86 ± 18.89 | 38.70 ± 10.38 | 64.11 ± 13.19 | 59.89 ± 10.43 | 62.32 ± 19.64 |
| Pancreatic cancer | 68.62 ± 16.11 | 68.77 ± 13.87 | 73.27 ± 17.24 | 75.33 ± 19.56 | 70.27 ± 17.16 | 65.36 ± 12.63 | 52.33 ± 18.04 | 60.53 ± 13.86 | 66.02 ± 18.91 | 45.04 ± 16.68 |
| Lung cancer | 74.71 ± 13.94 | 57.53 ± 10.47 | 69.83 ± 11.88 | 54.73 ± 16.81 | 55.41 ± 18.03 | 61.82 ± 17.43 | 58.01 ± 13.06 | 56.29 ± 16.98 | 71.72 ± 13.46 | 50.55 ± 15.04 |
| Liver cancer | 39.36 ± 19.9 | 47.27 ± 10.69 | 47.27 ± 16.42 | 39.09 ± 17.13 | 71.54 ± 18.65 | 37.73 ± 13.19 | 48.78 ± 11.01 | 68.38 ± 13.37 | 42.02 ± 15.65 | 47.77 ± 15.96 |
| Stomach cancer | 67.31 ± 14.91 | 40.71 ± 18.16 | 60.66 ± 18.62 | 66.73 ± 19.09 | 51.95 ± 11.45 | 55.87 ± 12.11 | 40.46 ± 17.78 | 72.54 ± 17.92 | 36.74 ± 10.31 | 46.4 ± 10.95 |
| Breast cancer | 72.88 ± 19.79 | 62.51 ± 10.82 | 56.91 ± 13.22 | 65.43 ± 15.75 | 75.76 ± 14.66 | 55.22 ± 15.17 | 73.27 ± 18.97 | 54.26 ± 10.66 | 68.22 ± 15.65 | 50.84 ± 14.38 |
| Kidney cancer | 51.24 ± 11.79 | 73.13 ± 15.26 | 45.54 ± 11.94 | 70.09 ± 19.12 | 45.27 ± 18.12 | 61.25 ± 14.17 | 66.17 ± 15.56 | 55.34 ± 10.48 | 51.05 ± 15.04 | 62.65 ± 11.81 |
| Colorectal cancer | 41.66 ± 10.28 | 40.41 ± 18.93 | 65.38 ± 10.76 | 46.64 ± 12.58 | 50.81 ± 15.59 | 60.82 ± 19.32 | 70.89 ± 11.99 | 57.09 ± 12.27 | 69.75 ± 19.72 | 69.65 ± 12.22 |
| Ovarian cancer | 46.03 ± 17.88 | 70.05 ± 19.95 | 63.65 ± 11.23 | 37.09 ± 18.1 | 59.86 ± 17.18 | 73.44 ± 15.92 | 52.42 ± 14.4 | 52.32 ± 18.59 | 68.98 ± 15.57 | 56.91 ± 17.05 |
| Cervical cancer | 75.33 ± 11.37 | 39.8 ± 11.93 | 38.93 ± 15.38 | 38.36 ± 15.55 | 37.08 ± 13.66 | 54.21 ± 19.74 | 76.64 ± 16.75 | 41.59 ± 10.98 | 74.61 ± 12.49 | 66.03 ± 16.73 |
| Uterine cancer | 40.22 ± 18.4 | 41.28 ± 17.34 | 67.28 ± 19.72 | 50.56 ± 14.78 | 66.27 ± 10.13 | 71.58 ± 18.16 | 67.20 ± 15.42 | 60.27 ± 16.22 | 75.49 ± 17.93 | 44.85 ± 11.31 |
| Prostate cancer | 41.34 ± 15.08 | 46.03 ± 18.52 | 67.61 ± 13.78 | 39.41 ± 17.12 | 61.34 ± 15.92 | 63.08 ± 13.98 | 75.20± 17.35 | 74.09 ± 13.02 | 38.65 ± 13.79 | 70.12 ± 17.21 |
| Bladder cancer | 51.59 ± 12.27 | 42.17 ± 18.37 | 51.93 ± 11.55 | 46.15 ± 16.28 | 74.66 ± 16.55 | 68.71 ± 15.12 | 42.48 ± 16.74 | 53.89 ± 14.29 | 49.44 ± 18.24 | 52.59 ± 19.54 |
| Melanoma | 73.35 ± 17.22 | 42.09 ± 15.84 | 60.97 ± 10.64 | 76.32 ± 19.97 | 56.56 ± 12.71 | 66.03 ± 18.07 | 54.91 ± 11.6 | 63.33 ± 12.77 | 46.62 ± 15.43 | 53.73 ± 18.72 |
| Hemangioma | 51.73 ± 15.04 | 36.27 ± 14.02 | 55.41 ± 19.69 | 63.62 ± 11.34 | 55.46 ± 12.82 | 49.73 ± 10.09 | 57.68 ± 19.18 | 51.93 ± 18.53 | 69.33 ± 14.38 | 66.23 ± 10.06 |
| Sarcoma | 75.26 ± 10.29 | 56.74 ± 12.92 | 59.26 ± 11.12 | 72.57 ± 14.25 | 42.85 ± 11.47 | 56.19 ± 18.81 | 73.67 ± 11.87 | 67.78 ± 14.37 | 64.37 ± 18.49 | 69.22 ± 11.18 |

Table 7 PIR (%) of polypeptides X1-X90 on various tumor cells (continued)

| Tumor cell line source | X21 | X22 | X23 | X24 | X25 | X26 | X27 | X28 | X29 | X30 |
|---|---|---|---|---|---|---|---|---|---|---|
| Head and neck cancers | 65.37 ± 12.94 | 41.81 ± 19.53 | 38.14 ± 11.14 | 47.69 ± 11.08 | 72.17 ± 19.42 | 67.64 ± 14.25 | 35.7 ± 11.09 | 42.99 ± 13.38 | 62.68 ± 18.91 | 39.04 ± 17.98 |
| Brain tumor | 40.99± 17.15 | 43.94 ± 10.64 | 47.74 ± 18.81 | 40.49 ± 11.86 | 68.77 ± 15.58 | 73.72 ± 16.04 | 63.22 ± 12.73 | 47.88 ± 15.33 | 56.05 ± 15.02 | 43.39 ± 12.99 |
| Thyroid cancer | 41.77± 11.86 | 40.29 ± 16.44 | 71.79 ± 11.29 | 37.62 ± 11.49 | 66.85 ± 16.33 | 49.45 ± 19.31 | 59.78 ± 16.48 | 63.91 ± 12.54 | 51.22 ± 11.18 | 73.79 ± 13.35 |
| Esophageal cancer | 43.15± 12.94 | 69.17 ± 15.02 | 65.85 ± 12.51 | 55.87 ± 19.15 | 58.8 ± 18.63 | 36.55 ± 11.12 | 37.45 ± 17.97 | 63.41 ± 17.78 | 54.58 ± 12.69 | 49.43 ± 12.02 |
| Pancreatic cancer | 62.03 ± 17.34 | 70.94 ± 11.72 | 50.79 ± 17.97 | 71.49 ± 16.47 | 42.49 ± 13.11 | 69.55 ± 13.45 | 74.52 ± 11.34 | 74.12 ± 19.3 | 65.63 ± 10.54 | 46.17 ± 14.33 |
| Lung cancer | 62.39± 18.81 | 39.95 ± 14.11 | 35.08 ± 10.52 | 40.66 ± 13.43 | 37.53 ± 14.82 | 57.59 ± 18.82 | 60.92 ± 16.14 | 63.39 ± 14.52 | 38.75 ± 15.08 | 38.85 ± 18.34 |
| Liver cancer | 56.3 ± 19.71 | 43.69 ± 17.77 | 61.83 ± 11.42 | 52.89 ± 11.54 | 59.05 ± 17.63 | 61.04 ± 10.92 | 76.74 ± 15.66 | 67.73 ± 13.72 | 36.33 ± 10.49 | 73.83 ± 16.98 |
| Stomach cancer | 36.47± 14.16 | 59.08 ± 18.93 | 38.39 ± 14.85 | 37.4 ± 13.48 | 75.26 ± 16.03 | 63.35 ± 11.59 | 51.63 ± 17.16 | 54.97 ± 17.82 | 52.53 ± 18.92 | 58.32 ± 11.91 |
| Breast cancer | 38.96± 11.33 | 75.82 ± 12.56 | 39.96 ± 11.75 | 75.56 ± 17.92 | 67.97 ± 11.35 | 37.61 ± 14.38 | 53.33 ± 14.69 | 71.89 ± 12.86 | 37.44 ± 16.04 | 46.39 ± 17.85 |

| | | | | | | | | | | |
|---|---|---|---|---|---|---|---|---|---|---|
| Kidney cancer | 74.18± 19.11 | 57.47 ± 13.26 | 42.09 ± 10.45 | 36.39 ± 11.33 | 68.24 ± 11.93 | 64.84 ± 19.25 | 63.46 ± 17.95 | 73.61 ± 11.16 | 72.79 ± 13.82 | 63.35 ± 14.59 |
| Colorectal cancer | 53.91± 15.15 | 63.73 ± 14.02 | 41.59 ± 16.05 | 69.36 ± 14.11 | 37.01 ± 13.65 | 54.52 ± 15.74 | 49.88 ± 10.16 | 36.83 ± 18.74 | 37.95 ± 12.31 | 59.27 ± 19.00 |
| Ovarian cancer | 52.65± 18.77 | 38.12 ± 17.81 | 59.19 ± 18.4 | 67.71 ± 10.08 | 43.11 ± 15.64 | 36.94 ± 11.86 | 71.02 ± 17.33 | 47.82 ± 17.51 | 38.03 ± 13.59 | 53.44 ± 17.9 |
| Cervical cancer | 69.23± 11.47 | 51.74 ± 14.66 | 54.67 ± 16.68 | 57.26 ± 13.86 | 69.86 ± 13.06 | 57.01 ± 13.74 | 55.75 ± 12.84 | 63.32 ± 12.16 | 43.21 ± 18.53 | 76.45 ± 11.67 |
| Uterine cancer | 63.43 ± 12.15 | 50.89 ± 10.96 | 42.53 ± 17.35 | 42.54 ± 10.76 | 42.89 ± 14.48 | 64.21 ± 13.94 | 54.16 ± 13.46 | 43.3 ± 13.08 | 46.82 ± 10.37 | 56.25 ± 14.73 |
| Prostate cancer | 37.86± 16.92 | 53.39 ± 15.54 | 36.21 ± 12.05 | 35.47 ± 18.64 | 68.48 ± 16.59 | 64.17 ± 17.11 | 67.49 ± 10.57 | 40.94 ± 16.98 | 55.77 ± 13.52 | 59.54 ± 12.23 |
| Bladder cancer | 37.75± 12.45 | 47.84 ± 17.84 | 50.54 ± 19.02 | 63.09 ± 17.99 | 69.61 ± 19.59 | 50.89 ± 13.05 | 76.84 ± 19.86 | 44.24 ± 17.88 | 61.24 ± 13.77 | 39.65 ± 11.16 |
| Melanoma | 47.85± 16.14 | 61.05 ± 12.55 | 69.51 ± 19.87 | 54.36 ± 16.72 | 42.51 ± 19.82 | 38.59 ± 13.49 | 35.73 ± 19.88 | 56.95 ± 13.59 | 64.53 ± 19.46 | 55.41 ± 15.58 |
| Hemangioma | 62.98± 19.47 | 53.14 ± 18.17 | 70.64 ± 15.46 | 37.01 ± 14.49 | 55.61 ± 10.08 | 37.45 ± 15.43 | 37.75 ± 15.17 | 38.68 ± 15.49 | 69.34 ± 15.1 | 48.32 ± 18.65 |
| Sarcoma | 46.06± 11.73 | 46.46 ± 11.38 | 39.37 ± 10.22 | 40.2 ± 16.78 | 58.24 ± 16.24 | 49.91 ± 15.19 | 35.55 ± 15.02 | 70.91 ± 13.58 | 59.14 ± 10.37 | 67.77 ± 10.57 |

Table 7 PIR (%) of polypeptides X1-X90 on various tumor cells (continued)

| Tumor cell line source | X31 | X32 | X33 | X34 | X35 | X36 | X37 | X38 | X39 | X40 |
|---|---|---|---|---|---|---|---|---|---|---|
| Head and neck cancers | 59.09 ± 11.77 | 47.45 ± 10.36 | 37.58 ± 18.81 | 39.94 ± 19.94 | 71.09 ± 13.86 | 56.51± 11.45 | 71.19 ± 18.71 | 39.93 ± 18.52 | 62.9 ± 17.54 | 61.85 ± 14.34 |
| Brain tumor | 37.42 ± 14.99 | 47.30 ± 14.26 | 60.86 ± 16.39 | 51.09 ± 18.62 | 55.15 ± 10.41 | 38.39± 13.28 | 75.85 ± 10.45 | 39.71 ± 17.17 | 64.41 ± 10.78 | 37.43 ± 15.84 |
| Thyroid cancer | 67.18 ± 13.35 | 40.25 ± 18.76 | 72.56 ± 10.91 | 48.67 ± 13.64 | 36.27 ± 17.48 | 46.37 ± 15.24 | 66.79 ± 14.56 | 46.45 ± 16.12 | 64.57 ± 18.46 | 54.26 ± 11.41 |
| Esophageal cancer | 35.00 ± 11.96 | 62.59 ± 17.05 | 69.52 ± 11.28 | 66.68 ± 13.89 | 40.33 ± 10.03 | 50.51± 19.08 | 49.78 ± 13.14 | 56.35 ± 13.87 | 70.94 ± 17.84 | 66.23 ± 10.75 |
| Pancreatic cancer | 36.26 ± 13.15 | 67.82 ± 18.67 | 46.18 ± 18.53 | 38.08 ± 12.92 | 37.68 ± 14.54 | 41.26± 16.28 | 43.04 ± 13.58 | 44.09 ± 19.22 | 74.19 ± 15.88 | 42.92 ± 17.04 |
| Lung cancer | 37.01 ± 19.88 | 64.40 ± 14.44 | 59.87 ± 18.89 | 42.42 ± 19.73 | 61.72 ± 19.05 | 53.94± 11.35 | 64.69 ± 15.31 | 50.43 ± 15.95 | 59.01 ± 14.77 | 59.02 ± 14.13 |
| Liver cancer | 65.28 ± 15.00 | 41.50 ± 14.14 | 47.79 ± 19.83 | 68.97 ± 13.48 | 76.66 ± 18.79 | 53.72± 11.59 | 70.33 ± 16.53 | 74.45 ± 19.05 | 75.48 ± 14.7 | 40.28 ± 15.06 |
| Stomach cancer | 43.64 ± 18.32 | 64.61 ± 11.64 | 74.39 ± 12.63 | 62.85 ± 17.22 | 51.62 ± 13.33 | 42.63± 14.95 | 70.68 ± 15.03 | 38.56 ± 12.55 | 73.13 ± 13.66 | 57.6 ± 19.33 |
| Breast cancer | 59.94 ± 18.74 | 50.80 ± 19.73 | 40.54 ± 16.38 | 68.94 ± 14.21 | 61.67 ± 11.23 | 72.93± 13.26 | 75.33 ± 17.22 | 42.19 ± 14.86 | 49.25 ± 12.62 | 52.71 ± 13.62 |
| Kidney cancer | 58.09 ± 11.56 | 62.76 ± 19.43 | 71.31 ± 19.93 | 61.35 ± 19.98 | 64.65 ± 14.52 | 69.37± 17.47 | 61.78 ± 19.61 | 43.37 ± 10.23 | 72.04 ± 18.36 | 49.08 ± 13.39 |
| Colorectal cancer | 55.85 ± 15.14 | 65.59 ± 16.95 | 50.37 ± 14.42 | 52.24 ± 18.33 | 51.98 ± 12.13 | 53.83 ± 16.52 | 51.57 ± 17.35 | 52.13 ± 11.76 | 75.22 ± 12.29 | 60.02 ± 17.91 |
| Ovarian cancer | 40.61 ± 17.82 | 58.83 ± 14.79 | 44.16 ± 19.51 | 67.44 ± 15.81 | 75.88 ± 16.79 | 46.07± 15.34 | 36.29 ± 15.57 | 70.13 ± 16.95 | 65.05 ± 12.23 | 36.21 ± 13.51 |
| Cervical cancer | 37.69 ± 13.66 | 60.95 ± 10.91 | 59.94 ± 17.51 | 66.34 ± 12.43 | 49.43 ± 19.55 | 47.99± 17.22 | 42.92 ± 12.23 | 57.13 ± 13.93 | 62.28 ± 11.24 | 55.53 ± 13.76 |
| Uterine cancer | 68.32 ± 12.00 | 70.59 ± 10.48 | 53.45 ± 12.20 | 56.24 ± 19.91 | 63.93 ± 19.28 | 38.24 ± 12.39 | 75.62 ± 10.59 | 59.59 ± 11.12 | 70.32 ± 18.57 | 48.62 ± 18.19 |
| Prostate cancer | 58.57 ± 11.14 | 54.52 ± 15.17 | 57.98 ± 11.51 | 39.80 ± 17.52 | 55.13 ± 13.55 | 67.78± 15.22 | 56.05 ± 17.78 | 37.75 ± 15.12 | 67.22 ± 17.94 | 57.83 ± 15.36 |
| Bladder cancer | 68.69 ± 13.76 | 47.88 ± 14.12 | 55.5 ± 16.52 | 39.15 ± 16.66 | 36.37 ± 15.56 | 67.95± 11.94 | 37.03 ± 11.84 | 38.32 ± 18.93 | 50.68 ± 11.3 | 72.35 ± 17.77 |

| | | | | | | | | | | |
|---|---|---|---|---|---|---|---|---|---|---|
| Melanoma | 37.58 ± 13.12 | 55.05 ± 18.49 | 42.06 ± 12.13 | 56.29 ± 17.93 | 72.16 ± 17.97 | 44.02± 17.54 | 45.96 ± 15.52 | 43.72 ± 13.46 | 69.87 ± 10.94 | 71.94 ± 19.98 |
| Hemangioma | 43.57 ± 10.89 | 68.1 ± 11.51 | 58.53 ± 19.84 | 72.30 ± 18.62 | 51.58 ± 14.73 | 68.06± 10.23 | 66.33 ± 15.99 | 41.49 ± 13.11 | 40.71 ± 14.01 | 57.56 ± 16.63 |
| Sarcoma | 75.54 ± 12.27 | 42.81 ± 18.57 | 44.99 ± 15.09 | 54.16 ± 10.75 | 61.40 ± 15.31 | 39.39± 11.23 | 57.04 ± 12.99 | 62.42 ± 16.98 | 36.49 ± 11.27 | 56.97 ± 15.31 |

Table 7 PIR (%) of polypeptides X1-X90 on various tumor cells (continued)

| Tumor cell line source | X41 | X42 | X43 | X44 | X45 | X46 | X47 | X48 | X49 | X50 |
|---|---|---|---|---|---|---|---|---|---|---|
| Head and neck cancers | 43.57 ± 11.52 | 57.82 ± 18.62 | 45.72 ± 17.16 | 47.11 ± 18.32 | 64.08 ± 17.54 | 65.93 ± 11.44 | 71.66 ± 13.54 | 72.66 ± 12.68 | 63.32 ± 16.98 | 35.25 ± 18.05 |
| Brain tumor | 67.93 ± 12.91 | 57.43 ± 18.76 | 38.23 ± 11.62 | 45.99 ± 16.31 | 38.99 ± 17.71 | 40.92 ± 18.18 | 43.77 ± 19.29 | 74.95 ± 12.2 | 56.49 ± 16.35 | 66.21 ± 18.67 |
| Thyroid cancer | 74.41 ± 14.24 | 45.39 ± 12.72 | 70.14 ± 13.78 | 44.32 ± 19.58 | 75.66 ± 17.37 | 43.73 ± 11.29 | 47.17 ± 16.99 | 51.53 ± 14.44 | 74.39 ± 19.66 | 61.88 ± 18.69 |
| Esophageal cancer | 66.6 ± 18.75 | 39.81 ± 19.98 | 70.48 ± 11.52 | 63.23 ± 12.00 | 45.02 ± 17.87 | 75.58 ± 17.98 | 62.93 ± 11.00 | 45.97 ± 19.69 | 56.98 ± 12.93 | 57.62 ± 14.81 |
| Pancreatic cancer | 67.73 ± 18.13 | 68.34 ± 17.07 | 70.71 ± 18.98 | 57.18 ± 15.60 | 52.08 ± 12.11 | 64.04 ± 11.93 | 62.95 ± 15.06 | 54.68 ± 13.49 | 54.36 ± 13.95 | 60.61 ± 14.92 |
| Lung cancer | 54.74 ± 17.54 | 72.97 ± 13.34 | 71.61 ± 12.51 | 73.36 ± 19.38 | 49.37 ± 17.63 | 70.54 ± 19.32 | 46.59 ± 15.12 | 42.00 ± 14.24 | 58.84 ± 14.67 | 45.90 ± 13.01 |
| Liver cancer | 52.24 ± 14.33 | 44.69 ± 17.55 | 58.91 ± 16.57 | 37.45 ± 14.63 | 59.27 ± 17.74 | 40.64 ± 11.24 | 59.38 ± 18.24 | 44.78 ± 17.1 | 70.07 ± 17.29 | 45.05 ± 15.28 |
| Stomach cancer | 61.92 ± 11.95 | 57.72 ± 12.27 | 54.13 ± 11.01 | 37.92 ± 13.15 | 76.56 ± 12.97 | 40.50 ± 15.68 | 61.58 ± 13.24 | 72.03 ± 18.87 | 35.31 ± 13.87 | 62.97 ± 14.24 |
| Breast cancer | 64.46 ± 12.3 | 61.79 ± 19.55 | 57.59 ± 17.99 | 45.85 ± 17.46 | 66.23 ± 17.14 | 39.62 ± 13.82 | 56.85 ± 13.96 | 48.41 ± 10.37 | 76.97 ± 16.87 | 73.69 ± 16.38 |
| Kidney cancer | 67.32 ± 11.68 | 62.18 ± 10.77 | 40.35 ± 14.15 | 36.95 ± 13.61 | 66.68 ± 18.47 | 58.12 ± 10.96 | 65.49 ± 15.66 | 52.92 ± 14.91 | 75.07 ± 13.88 | 76.09 ± 10.99 |
| Colorectal cancer | 42.94 ± 12.14 | 47.39 ± 18.01 | 64.08 ± 11.47 | 50.08 ± 18.11 | 52.53 ± 13.36 | 46.96 ± 17.53 | 50.67 ± 13.52 | 40.83 ± 12.05 | 48.38 ± 18.49 | 64.83 ± 17.73 |
| Ovarian cancer | 39.68 ± 17.13 | 74.11 ± 14.39 | 61.54 ± 19.79 | 44.64 ± 16.9 | 64.13 ± 12.77 | 37.37 ± 19.48 | 44.93 ± 11.12 | 61.05 ± 11.13 | 65.27 ± 10.67 | 75.15 ± 11.54 |
| Cervical cancer | 47.37 ± 18.96 | 72.53 ± 15.56 | 47.07 ± 15.21 | 55.47 ± 19.86 | 56.97 ± 19.56 | 64.16 ± 13.04 | 42.26 ± 11.78 | 55.52 ± 15.41 | 57.03 ± 12.78 | 51.98 ± 17.49 |
| Uterine cancer | 59.93 ± 14.82 | 70.55 ± 12.92 | 38.13 ± 18.83 | 68.83 ± 13.88 | 39.31 ± 13.21 | 74.34 ± 12.93 | 35.35 ± 10.02 | 45.13 ± 15.15 | 40.55 ± 17.92 | 66.93 ± 16.18 |
| Prostate cancer | 54.51 ± 10.24 | 73.74 ± 12.36 | 47.49 ± 11.98 | 35.19 ± 12.08 | 48.17 ± 19.43 | 51.00 ± 10.13 | 70.17 ± 16.96 | 70.81 ± 16.63 | 47.71 ± 12.42 | 39.81 ± 19.13 |
| Bladder cancer | 53.29 ± 11.03 | 60.39 ± 12.52 | 70.11 ± 16.32 | 43.34 ± 14.41 | 73.89 ± 13.81 | 52.83 ± 17.75 | 62.79 ± 10.07 | 62.99 ± 14.78 | 64.22 ± 12.63 | 62.67 ± 18.21 |
| Melanoma | 56.44 ± 15.23 | 74.41 ± 14.21 | 67.28 ± 19.72 | 41.66 ± 10.72 | 52.76 ± 10.94 | 73.83 ± 10.72 | 65.79 ± 17.97 | 38.81 ± 11.36 | 36.11 ± 10.66 | 56.05 ± 18.67 |
| Hemangioma | 40.91 ± 14.12 | 66.21 ± 10.51 | 66.87 ± 15.29 | 74.36 ± 15.31 | 40.65 ± 18.95 | 75.45 ± 18.09 | 59.32 ± 12.16 | 67.47 ± 17.41 | 68.79 ± 15.74 | 39.90 ± 14.29 |
| Sarcoma | 74.05 ± 12.15 | 46.23 ± 12.39 | 44.1 ± 12.29 | 49.22 ± 19.56 | 75.95 ± 14.61 | 55.78 ± 17.99 | 64.54 ± 13.65 | 71.83 ± 17.48 | 73.31 ± 13.07 | 55.03 ± 19.71 |

Table 7 PIR (%) of polypeptides X1-X90 on various tumor cells (continued)

| Tumor cell line source | X51 | X52 | X53 | X54 | X55 | X56 | X57 | X58 | X59 | X60 |
|---|---|---|---|---|---|---|---|---|---|---|
| Head and neck cancers | 71.73± 13.79 | 67.97 ± 14.45 | 50.5 ± 11.07 | 38.31 ± 13.9 | 65.5 ± 19.21 | 35.55 ± 14.28 | 66.36 ± 13.68 | 40.34 ± 13.58 | 49.86 ± 13.23 | 70.78 ± 17.01 |
| Brain tumor | 38.48± 16.23 | 52.23 ± 11.05 | 62.85 ± 14.02 | 66.14 ± 18.76 | 75.73 ± 14.64 | 37.61 ± 16.92 | 48.16 ± 16.82 | 36.05 ± 15.82 | 55.61 ± 14.73 | 50.82 ± 16.24 |
| Thyroid cancer | 56.65± 15.18 | 53.23 ± 19.88 | 47.46 ± 13.43 | 35.55 ± 17.12 | 61.07 ± 16.47 | 41.84 ± 12.29 | 51.93 ± 15.96 | 57.07 ± 12.62 | 48.16 ± 15.78 | 53.48 ± 10.27 |

| | | | | | | | | | | |
|---|---|---|---|---|---|---|---|---|---|---|
| Esophageal cancer | 39.56± 10.25 | 42.57 ± 12.22 | 53.96 ± 19.26 | 75.89 ± 14.54 | 36.31 ± 17.71 | 49.57 ± 15.38 | 57.85 ± 12.32 | 55.44 ± 10.14 | 74.88 ± 17.52 | 45.45 ± 14.17 |
| Pancreatic cancer | 43.25± 19.53 | 46.95 ± 18.35 | 45.87 ± 19.22 | 53.85 ± 11.88 | 58.72 ± 16.24 | 57.21 ± 12.43 | 59.73 ± 10.12 | 73.61 ± 12.93 | 45.52 ± 13.68 | 50.92 ± 19.47 |
| Lung cancer | 54.77± 14.37 | 63.5 ± 14.12 | 39.93 ± 15.43 | 40.15 ± 17.43 | 45.05 ± 13.19 | 51.03 ± 15.64 | 69.87 ± 19.38 | 49.64 ± 14.08 | 45.73 ± 13.94 | 62.47 ± 13.16 |
| Liver cancer | 64.82± 13.19 | 76.28 ± 16.95 | 44.85 ± 18.16 | 68.72 ± 19.36 | 73.57 ± 18.92 | 47.39 ± 11.27 | 57.51 ± 19.37 | 37.68 ± 17.24 | 61.08 ± 19.18 | 57.27 ± 14.89 |
| Stomach cancer | 49.52 ± 13.42 | 71.77 ± 13.87 | 61.27 ± 10.13 | 65.15 ± 12.34 | 57.37 ± 14.73 | 43.77 ± 15.32 | 44.39 ± 18.59 | 69.28 ± 15.69 | 66.37 ± 14.87 | 58.56 ± 14.96 |
| Breast cancer | 45.34± 12.87 | 71.37 ± 10.37 | 55.81 ± 15.77 | 71.24 ± 10.08 | 68.45 ± 12.61 | 45.15 ± 12.83 | 72.88 ± 18.92 | 55.26 ± 13.17 | 35.39 ± 10.86 | 64.38 ± 16.18 |
| Kidney cancer | 68.57± 12.06 | 48.73 ± 12.21 | 50.42 ± 14.19 | 53.57 ± 18.29 | 73.94 ± 13.75 | 52.29 ± 15.37 | 46.83 ± 19.17 | 39.21 ± 10.58 | 57.34 ± 11.06 | 47.11 ± 18.83 |
| Colorectal cancer | 65.66± 12.27 | 73.22 ± 10.18 | 40.62 ± 12.76 | 69.25 ± 12.45 | 58.47 ± 10.27 | 38.34 ± 13.74 | 51.68 ± 12.59 | 49.93 ± 16.52 | 56.47 ± 12.62 | 74.01 ± 16.85 |
| Ovarian cancer | 36.23± 13.19 | 71.63 ± 16.37 | 41.92 ± 10.41 | 61.42 ± 11.94 | 57.68 ± 12.82 | 52.51 ± 16.01 | 66 ± 15.2 | 71.78 ± 19.13 | 59.72 ± 17.77 | 37.71 ± 19.04 |
| Cervical cancer | 64.98± 18.41 | 50.09 ± 13.98 | 63.12 ± 14.09 | 43.25 ± 12.66 | 75.28 ± 14.24 | 62.12 ± 16.54 | 55.66 ± 18.88 | 69.74 ± 11.34 | 56.03 ± 11.47 | 68.51 ± 10.44 |
| Uterine cancer | 36.86± 17.04 | 47.69 ± 19.55 | 49.15 ± 17.93 | 35.81 ± 10.02 | 63.3 ± 18.28 | 61.69 ± 18.28 | 40.69 ± 17.84 | 52.07 ± 10.37 | 56.94 ± 17.27 | 54.36 ± 18.71 |
| Prostate cancer | 64.4 ± 12.04 | 57.92 ± 14.12 | 67.39 ± 17.65 | 73.54 ± 16.66 | 44.26 ± 16.75 | 76.33 ± 15.82 | 73.75 ± 17.92 | 71.72 ± 18.51 | 60.29 ± 12.23 | 59.57 ± 11.48 |
| Bladder cancer | 63.28± 17.47 | 39.17 ± 11.62 | 38.66 ± 14.24 | 48.75 ± 18.54 | 45.75 ± 19.55 | 37.33 ± 14.97 | 43.43 ± 18.63 | 65.05 ± 11.02 | 53.71 ± 12.75 | 57.48 ± 17.09 |
| Melanoma | 36.94± 11.26 | 37.8 ± 18.44 | 42.32 ± 15.63 | 53.92 ± 17.91 | 41.39 ± 13.73 | 41.91 ± 17.71 | 45.92 ± 13.04 | 59.28 ± 17.18 | 45.12 ± 13.72 | 62.45 ± 17.77 |
| Hemangioma | 67.23± 11.25 | 50.46 ± 10.54 | 45.35 ± 14.66 | 67.47 ± 13.18 | 71.29 ± 14.72 | 59.8 ± 13.33 | 45.15 ± 13.98 | 55.89 ± 12.13 | 61.25 ± 19.99 | 37.3 ± 10.21 |
| Sarcoma | 58.32± 13.38 | 48.85 ± 19.52 | 41.78 ± 16.51 | 67.01 ± 12.85 | 41.45 ± 17.28 | 39.26 ± 19.06 | 48.03 ± 10.76 | 35.26 ± 14.88 | 36.57 ± 11.96 | 40.63 ± 19.46 |

Table 7 PIR (%) of polypeptides X1-X90 on various tumor cells (continued)

| Tumor cell line source | X61 | X62 | X63 | X64 | X65 | X66 | X67 | X68 | X69 | X70 |
|---|---|---|---|---|---|---|---|---|---|---|
| Head and neck cancers | 40.64 ± 19.78 | 60.36 ± 16.07 | 63.37 ± 12.42 | 39.09 ± 19.99 | 52.74 ± 15.57 | 38.52 ± 12.49 | 42.23 ± 15.76 | 59.72 ± 17.24 | 51.77 ± 16.13 | 52.03 ± 12.38 |
| Brain tumor | 47.33 ± 13.12 | 69.94 ± 19.70 | 59.22 ± 15.12 | 36.88 ± 18.33 | 47.91 ± 14.38 | 60.72 ± 15.43 | 73.26 ± 14.09 | 60.29 ± 13.47 | 55.81 ± 18.92 | 38.37 ± 13.24 |
| Thyroid cancer | 39.88 ± 12.95 | 52.65 ± 19.20 | 62.03 ± 11.14 | 47.44 ± 13.73 | 73.75 ± 11.45 | 38.37± 18.26 | 55.43 ± 19.84 | 59.88 ± 17.75 | 75.71 ± 17.42 | 37.92 ± 10.46 |
| Esophageal cancer | 63.31 ± 17.56 | 52.77 ± 10.21 | 37.37 ± 15.10 | 69.87 ± 14.85 | 69.58 ± 11.33 | 75.12± 19.14 | 35.62 ± 19.46 | 47.87 ± 13.16 | 38.73 ± 17.62 | 64.28 ± 18.75 |
| Pancreatic cancer | 46.88 ± 10.17 | 35.43 ± 19.91 | 41.05 ± 11.77 | 56.23 ± 15.52 | 47.59 ± 17.96 | 54.27± 19.84 | 58.71 ± 15.04 | 75.22 ± 19.54 | 61.73 ± 10.56 | 56.61 ± 10.79 |
| Lung cancer | 69.90 ± 16.79 | 42.92 ± 15.59 | 57.25 ± 15.89 | 45.82 ± 10.23 | 59.33 ± 18.79 | 52.94± 19.25 | 68.04 ± 19.12 | 39.11 ± 18.74 | 60.18 ± 17.44 | 39.68 ± 19.33 |
| Liver cancer | 66.47 ± 16.50 | 70.65 ± 18.33 | 47.74 ± 17.07 | 53.82 ± 15.25 | 44.70 ± 17.22 | 72.51 ± 18.64 | 52.65 ± 17.17 | 66.85 ± 17.46 | 67.87 ± 18.98 | 66.59 ± 12.84 |
| Stomach cancer | 43.87 ± 12.66 | 74.79 ± 11.78 | 52.66 ± 19.90 | 37.31 ± 18.20 | 49.47 ± 10.74 | 65.63± 11.49 | 38.72 ± 14.66 | 73.08 ± 11.27 | 63.46 ± 17.98 | 41.87 ± 16.14 |
| Breast cancer | 51.9 ± 18.43 | 60.66 ± 13.30 | 76.30 ± 14.50 | 63.22 ± 15.99 | 60.49 ± 10.81 | 56.94± 11.83 | 71.39 ± 10.03 | 37.5 ± 18.46 | 69.52 ± 15.05 | 62.22 ± 13.67 |
| Kidney cancer | 76.61 ± 11.74 | 73.08 ± 12.10 | 36.28 ± 15.22 | 58.84 ± 14.48 | 65.39 ± 17.81 | 58.74± 10.17 | 74.09 ± 11.22 | 69.86 ± 10.14 | 54.11 ± 13.04 | 52.8 ± 12.14 |
| Colorectal cancer | 61.34 ± 10.45 | 41.69 ± 13.05 | 40.50 ± 14.82 | 43.28 ± 12.95 | 45.96 ± 11.00 | 76.92± 18.37 | 55.41 ± 11.72 | 76.38 ± 12.8 | 72.28 ± 15.99 | 74.94 ± 10.12 |

| | | | | | | | | | | |
|---|---|---|---|---|---|---|---|---|---|---|
| Ovarian cancer | 41.79 ± 11.50 | 65.50 ± 12.11 | 54.64 ± 12.72 | 59.43 ± 16.89 | 74.66 ± 19.07 | 65.86± 13.29 | 74.42 ± 12.21 | 40.76 ± 15.85 | 64.39 ± 11.63 | 35.48 ± 15.43 |
| Cervical cancer | 59.80 ± 10.07 | 52.13 ± 10.40 | 42.74 ± 18.42 | 47.97 ± 19.23 | 48.17 ± 16.72 | 72.53 ± 14.78 | 64.38 ± 12.38 | 68.37±14. 74 | 52.37± 15.37 | 71.37± 19.36 |
| Uterine cancer | 65.76 ± 11.21 | 36.40 ± 13.07 | 45.53 ± 10.70 | 51.12 ± 14.37 | 40.45 ± 16.59 | 49.78± 17.98 | 71.37± 19.74 | 59.27± 12.48 | 48.37± 16.37 | 62.28± 13.27 |
| Prostate cancer | 62.95 ± 11.09 | 53.04 ± 18.63 | 82.39 ± 12.84 | 51.33 ± 17.15 | 59.6 ± 13.83 | 81.49± 11.84 | 80.25 ± 11.87 | 78.02 ± 11.55 | 79.09 ± 16.05 | 73.4 ± 10.31 |
| Bladder cancer | 72.48 ± 10.86 | 65.40 ± 13.99 | 64.27 ± 19.88 | 71.51 ± 14.26 | 43.61 ± 11.06 | 69.39 ± 18.29 | 50.89 ± 18.8 | 67.49 ± 17.29 | 55.74 ± 12.17 | 57.92 ± 18.52 |
| Melanoma | 39.81 ± 19.98 | 44.69 ± 17.55 | 76.84 ± 19.86 | 55.75 ± 12.84 | 63.46 ± 17.95 | 60.92 ± 16.14 | 59.78 ± 16.48 | 56.74 ± 12.92 | 46.03 ± 18.52 | 70.05 ± 19.95 |
| Hemangioma | 68.34 ± 17.07 | 57.72 ± 12.27 | 35.73 ± 19.88 | 54.16 ± 13.46 | 49.88 ± 10.16 | 76.74 ± 15.66 | 37.45 ± 17.97 | 35.7 ± 11.09 | 42.17 ± 18.37 | 39.8 ± 11.93 |
| Sarcoma | 72.97 ± 13.34 | 61.79 ± 19.55 | 37.75 ± 15.17 | 67.49 ± 10.57 | 71.02 ± 17.33 | 51.63 ± 17.16 | 74.52 ± 11.34 | 63.22 ± 12.73 | 42.09 ± 15.84 | 41.28 ± 17.34 |

Table 7 PIR (%) of polypeptides X1-X90 on various tumor cells (continued)

| Tumor cell line source | X71 | X72 | X73 | X74 | X75 | X76 | X77 | X78 | X79 | X80 |
|---|---|---|---|---|---|---|---|---|---|---|
| Head and neck cancers | 49.18 ± 10.58 | 43.31 ± 19.08 | 44.27 ± 16.25 | 52.46 ± 11.59 | 63.28 ± 12.44 | 56.29 ± 17.93 | 35.32 ± 10.94 | 71.09 ± 13.86 | 43.61 ± 11.06 | 68.51 ± 10.44 |
| Brain tumor | 47.61 ± 10.56 | 47.06 ± 15.79 | 74.32 ± 11.53 | 54.64 ± 16.96 | 38.52 ± 12.53 | 72.3 ± 18.62 | 61.34 ± 18.45 | 55.15 ± 10.41 | 35.25 ± 18.05 | 54.36 ± 18.71 |
| Thyroid cancer | 63.08 ± 19.04 | 41.47 ± 15.52 | 72.02 ± 10.95 | 56.97 ± 15.87 | 54.93 ± 17.32 | 54.16 ± 10.75 | 55.15 ± 15.82 | 36.27 ± 17.48 | 66.21 ± 18.67 | 59.57 ± 11.48 |
| Esophageal cancer | 49.81 ± 19.55 | 60.94 ± 10.28 | 73.19 ± 15.36 | 66.75 ± 19.92 | 74.21 ± 10.49 | 71.51 ± 14.26 | 62.32 ± 19.64 | 40.3 ± 10.03 | 61.88 ± 18.69 | 57.48 ± 17.09 |
| Pancreatic cancer | 51.23 ± 12.92 | 60.51 ± 13.32 | 67.14 ± 17.12 | 73.62 ± 16.04 | 46.04 ± 19.41 | 63.2 ± 16.98 | 45.04 ± 16.68 | 37.68 ± 14.54 | 57.62 ± 14.81 | 62.45 ± 17.77 |
| Lung cancer | 48.32 ± 13.6 | 37.99 ± 17.69 | 72.63 ± 10.55 | 56.82 ± 12.55 | 62.81 ± 14.67 | 56.49 ± 16.35 | 50.55 ± 15.04 | 61.72 ± 19.05 | 60.6 ± 14.92 | 37.3 ± 10.21 |
| Liver cancer | 51.96 ± 12.99 | 63.54 ± 18.34 | 66.15 ± 17.37 | 56.31 ± 19.46 | 60.89 ± 14.88 | 74.39 ± 19.66 | 47.77 ± 15.96 | 76.66 ± 18.79 | 45.9 ± 13.01 | 40.63 ± 19.46 |
| Stomach cancer | 66.04 ± 12.86 | 47.13 ± 19.77 | 55.77 ± 12.37 | 61.52 ± 17.24 | 70.68 ± 13.04 | 56.98 ± 12.93 | 46.4 ± 10.95 | 51.62 ± 13.33 | 45.05 ± 15.28 | 63.28 ± 12.44 |
| Breast cancer | 46.31 ± 17.26 | 45.63 ± 14.39 | 59.16 ± 19.94 | 41.73 ± 10.57 | 39.66 ± 10.18 | 54.6 ± 13.95 | 50.84 ± 14.38 | 61.67 ± 11.23 | 62.97 ± 14.24 | 38.5 ± 12.53 |
| Kidney cancer | 54.18 ± 17.44 | 41.16 ± 19.27 | 48.74 ± 17.64 | 74.58 ± 10.06 | 43.76 ± 11.78 | 58.84 ± 14.67 | 62.65 ± 11.81 | 64.65 ± 14.52 | 73.69 ± 16.38 | 54.93 ± 17.32 |
| Colorectal cancer | 75.24 ± 14.77 | 58.68 ± 18.55 | 44.31 ± 17.17 | 63.58 ± 13.74 | 64.11 ± 14.09 | 70.07 ± 17.29 | 69.65 ± 12.22 | 51.98 ± 12.13 | 76.09 ± 10.99 | 74.21 ± 10.49 |
| Ovarian cancer | 41.72 ± 15.96 | 55.51 ± 17.71 | 73.97 ± 19.19 | 59.18 ± 19.28 | 44.3 ± 19.11 | 35.31 ± 13.87 | 56.91 ± 17.05 | 75.88 ± 16.79 | 47.86 ± 19.92 | 46.04 ± 19.41 |
| Cervical cancer | 67.28± 15.38 | 62.18± 19.28 | 48.18± 16.28 | 42.18± 15.38 | 48.88 ± 11.99 | 76.97 ± 16.87 | 66.03 ± 16.73 | 49.43 ± 19.55 | 64.83 ± 17.73 | 62.81 ± 14.67 |
| Uterine cancer | 69.28± 18.23 | 71.82± 18.37 | 69.27± 12.82 | 57.18± 15.28 | 49.18± 17.28 | 75.07 ± 13.88 | 44.85 ± 11.31 | 63.93 ± 19.28 | 75.15 ± 11.54 | 60.89 ± 14.88 |
| Prostate cancer | 72.7± 12.83 | 53.41± 10.86 | 69.45 ± 16.98 | 66.53 ± 13.6 | 52.94 ± 14.56 | 43.55 ± 15.92 | 70.12 ± 17.13 | 55.13 ± 13.55 | 51.98 ± 17.49 | 70.68 ± 13.04 |
| Bladder cancer | 52.72 ± 13.44 | 54.85 ± 11.28 | 76.11 ± 19.42 | 42.12 ± 15.58 | 71.44 ± 16.82 | 48.38 ± 18.49 | 52.59 ± 19.54 | 36.37 ± 15.56 | 66.93 ± 16.18 | 39.66 ± 10.18 |
| Melanoma | 62.51 ± 10.82 | 57.53 ± 10.47 | 48.73 ± 13.45 | 66.04 ± 12.86 | 47.61 ± 10.56 | 65.27 ± 10.67 | 53.73 ± 18.72 | 72.16 ± 17.97 | 39.81 ± 19.13 | 43.76 ± 11.78 |
| Hemangioma | 73.13 ± 15.26 | 47.27 ± 10.69 | 49.55 ± 10.47 | 46.31 ± 17.26 | 63.08 ± 19.04 | 57.03 ± 12.78 | 66.23 ± 10.06 | 51.58 ± 14.73 | 62.67 ± 18.21 | 64.11 ± 14.09 |
| Sarcoma | 40.41 ± 18.93 | 40.71 ± 18.16 | 48.77 ± 13.87 | 54.18 ± 17.44 | 49.81 ± 19.55 | 40.55 ± 17.92 | 69.22 ± 11.18 | 61.4 ± 15.31 | 56.05 ± 18.67 | 44.3 ± 19.11 |

Table 7 PIR (%) of polypeptides X1-X90 on various tumor cells (continued)

| Tumor cell line source | X81 | X82 | X83 | X84 | X85 | X86 | X87 | X88 | X89 | X90 | Docetaxel |
|---|---|---|---|---|---|---|---|---|---|---|---|
| Head and neck cancers | 48.17 ± 19.43 | 59.54 ± 12.23 | 50.81 ± 15.59 | 56.47 ± 12.62 | 55.7 ± 19.86 | 43.21 ± 18.53 | 38.36 ± 15.55 | 57.41 ± 12.19 | 63.39 ± 14.52 | 69.83 ± 11.88 | 55.66 ± 18.88 |
| Brain tumor | 73.89 ± 13.81 | 39.65 ± 11.16 | 59.86 ± 17.18 | 59.72 ± 17.77 | 68.83 ± 13.88 | 46.82 ± 10.37 | 50.56 ± 14.78 | 65.89 ± 13.73 | 67.73 ± 13.72 | 47.27 ± 16.42 | 70.69 ± 17.84 |
| Thyroid cancer | 52.76 ± 10.92 | 55.41 ± 15.58 | 37.08 ± 13.66 | 56.03 ± 11.47 | 35.19 ± 12.08 | 55.77 ± 13.52 | 39.41 ± 17.51 | 69.82 ± 11.28 | 54.97 ± 17.83 | 60.66 ± 18.62 | 73.75 ± 17.97 |
| Esophageal cancer | 40.65 ± 18.95 | 48.32 ± 18.65 | 66.27 ± 10.13 | 56.94 ± 17.27 | 43.34 ± 14.41 | 61.24 ± 13.77 | 46.15 ± 16.28 | 63.49 ± 14.04 | 71.89 ± 12.86 | 56.91 ± 13.22 | 53.43 ± 18.63 |
| Pancreatic cancer | 75.95 ± 14.61 | 67.77 ± 10.57 | 61.34 ± 15.92 | 60.29 ± 12.23 | 41.66 ± 10.72 | 64.53 ± 19.46 | 76.32 ± 19.97 | 55.33 ± 19.56 | 73.61 ± 11.16 | 45.54 ± 11.94 | 45.92 ± 13.04 |
| Lung cancer | 71.44 ± 16.82 | 64.08 ± 17.56 | 74.66 ± 16.55 | 53.71 ± 12.75 | 74.36 ± 15.31 | 69.34 ± 15.16 | 63.62 ± 11.34 | 54.73 ± 16.81 | 36.83 ± 18.73 | 65.38 ± 10.76 | 65.15 ± 13.98 |
| Liver cancer | 70.78 ± 17.01 | 38.99 ± 17.57 | 56.56 ± 12.71 | 45.12 ± 13.77 | 49.22 ± 19.56 | 59.1 ± 10.37 | 72.57 ± 14.25 | 39.09 ± 17.13 | 47.82 ± 17.51 | 63.65 ± 11.26 | 48.03 ± 10.76 |
| Stomach cancer | 50.82 ± 16.24 | 75.66 ± 17.37 | 55.46 ± 12.82 | 61.25 ± 19.99 | 42.12 ± 15.58 | 47.11 ± 18.34 | 62.68 ± 18.91 | 66.73 ± 19.09 | 63.32 ± 12.16 | 38.93 ± 15.38 | 43.31 ± 19.08 |
| Breast cancer | 53.48 ± 10.27 | 45.02 ± 17.87 | 42.85 ± 11.47 | 36.57 ± 11.96 | 49.86 ± 13.23 | 45.99 ± 16.31 | 56.05 ± 15.02 | 65.43 ± 15.76 | 43.3 ± 13.08 | 67.28 ± 19.72 | 47.06 ± 15.79 |
| Kidney cancer | 45.45 ± 14.17 | 52.08 ± 12.11 | 39.04 ± 17.98 | 52.46 ± 11.59 | 55.61 ± 14.73 | 44.32 ± 19.58 | 51.22 ± 11.18 | 70.09 ± 19.12 | 40.94 ± 16.98 | 67.61 ± 13.78 | 71.47 ± 15.52 |
| Colorectal cancer | 50.92 ± 19.47 | 49.37 ± 17.65 | 43.39 ± 12.99 | 54.64 ± 16.96 | 48.16 ± 15.78 | 63.23 ± 12.86 | 54.58 ± 12.69 | 46.64 ± 12.58 | 44.2 ± 17.88 | 51.93 ± 11.55 | 60.94 ± 10.28 |
| Ovarian cancer | 62.47 ± 13.16 | 59.7 ± 17.74 | 73.79 ± 13.35 | 56.97 ± 15.87 | 74.88 ± 17.52 | 57.18 ± 15.63 | 65.63 ± 10.54 | 37.09 ± 18.12 | 56.95 ± 13.59 | 60.97 ± 10.65 | 60.51 ± 13.32 |
| Cervical cancer | 57.27 ± 14.89 | 76.56 ± 12.97 | 49.43 ± 12.02 | 66.75 ± 19.92 | 45.52 ± 13.68 | 73.36 ± 19.38 | 38.75 ± 15.08 | 73.19 ± 15.36 | 38.68 ± 15.49 | 55.41 ± 19.69 | 67.99 ± 17.69 |
| Uterine cancer | 58.56 ± 14.96 | 66.23 ± 17.14 | 46.17 ± 14.33 | 73.62 ± 16.04 | 45.73 ± 13.94 | 37.45 ± 14.63 | 36.33 ± 10.49 | 67.14 ± 17.12 | 70.91 ± 13.58 | 59.26 ± 11.12 | 63.54 ± 18.34 |
| Prostate cancer | 64.38 ± 16.18 | 66.68 ± 18.47 | 38.85 ± 18.34 | 56.8 ± 12.55 | 61.08 ± 19.18 | 37.92 ± 13.15 | 52.53 ± 18.92 | 72.6 ± 10.55 | 45.72 ± 17.16 | 42.99 ± 13.38 | 67.13 ± 19.77 |
| Bladder cancer | 47.11 ± 18.83 | 52.53 ± 13.36 | 73.83 ± 16.98 | 56.31 ± 19.46 | 66.37 ± 14.87 | 45.85 ± 17.46 | 37.4 ± 16.04 | 66.15 ± 17.37 | 38.3 ± 11.62 | 47.88 ± 15.33 | 45.63 ± 14.39 |
| Melanoma | 58.42 ± 11.82 | 64.1 ± 12.77 | 58.32 ± 11.91 | 61.52 ± 17.24 | 35.39 ± 10.86 | 36.95 ± 13.61 | 72.79 ± 13.82 | 55.77 ± 12.37 | 70.14 ± 13.78 | 63.91 ± 12.54 | 41.16 ± 19.27 |
| Hemangioma | 74.01 ± 16.85 | 56.97 ± 19.56 | 46.39 ± 17.85 | 41.73 ± 10.57 | 57.34 ± 11.06 | 50.08 ± 18.11 | 37.95 ± 12.31 | 59.16 ± 19.94 | 70.48 ± 11.52 | 63.41 ± 17.78 | 58.68 ± 18.55 |
| Sarcoma | 37.71 ± 19.04 | 39.31 ± 13.21 | 63.35 ± 14.59 | 74.58 ± 10.06 | 59.05 ± 14.27 | 44.64 ± 16.94 | 38.03 ± 13.59 | 48.74 ± 17.64 | 70.71 ± 18.98 | 74.12 ± 19.33 | 75.22± 19.23 |

Results: Compared with negative control, X1-X90 have significant inhibitory effects on the proliferation of various tumor cells, wherein X5 has the best effect, which provides a good prospect for the development of effective anti-tumor drugs for the present invention.

### Example 3

PIR (%) of maleimide group-modified angiogenesis inhibitor polypeptides P1-P4 on various tumor cells

An MTT method was used to detect the inhibitory activity of P1-P4 on the growth of various tumor cells. Tumor cells were digested and collected with trypsin after being cultured in an incubator at 37°C with 5% $CO_2$ to a confluence of 90% or more. The cells were resuspended with a culture solution and counted under a microscope, the cell concentration was adjusted to $2\times10^4$ cells/mL, and the cell suspension was inoculated into a 96-well plate at 100 $\mu$L/well, and cultured overnight in an incubator at 37°C with 5% $CO_2$. P1-P4 were diluted to respective predetermined concentrations with the culture solution. Docetaxel was diluted to a final concentration with the culture solution. After the cells were completely adhered to the wall, each diluent was added into the 96-well plate (100 $\mu$L/well). Tumor cells with the addition of diluents of polypeptides P1-P4 were used as administration groups, tumor cells with the addition of docetaxel were used as a positive control group, and tumor cells with the addition of the culture solution without any drug were used as a negative

control group. The cells were cultured in an incubator at 37°C with 5% $CO_2$ for 48 h. 5 mg/mL MTT was added into the 96-well plate, 20 μL per well, and the culture was continued for 4 h. The culture medium was removed, 150 μL of DMSO was added to each well for dissolution, and gent and uniform mixing was performed in a shaker for 10 min. The absorbance was measured at a detection wavelength of 570 nm and a reference wavelength of 630 nm using a microplate reader, and the PIR was calculated with the formula as follows:

$$\text{PIR (\%)} = 1 - \text{administration group/negative group}$$

The experiment was repeated independently three times, and the results were expressed as mean ± SD. The experimental results are shown in Table 8.

Table 8 PIR (%) of polypeptides P1-P4 on various tumor cells

| Tumor cell line source | HM-1 | P1 | P2 | P3 | P4 |
|---|---|---|---|---|---|
| Head and neck cancers | 36.12 ± 11.21 | 37.06± 13.32 | 66.5± 17.54 | 64.96± 7.08 | 44.15± 17.85 |
| Brain tumor | 41.54 ± 12.43 | 56.51± 6.86 | 54.93± 15.06 | 43.62± 18.25 | 58.25± 12.61 |
| Thyroid cancer | 43.15 ± 14.42 | 43.67± 15.45 | 35.75± 11.63 | 47.86± 14.51 | 48.89± 7.69 |
| Esophageal cancer | 46.22 ± 18.24 | 54.38± 7.77 | 43.58± 12.41 | 59.78± 17.75 | 28.24± 11.35 |
| Pancreatic cancer | 45.03 ± 13.18 | 40.05± 5.25 | 38.38± 18.64 | 54.79± 12.05 | 44.95± 13.72 |
| Lung cancer | 50.35 ± 14.14 | 52.65± 16.08 | 39.91± 15.09 | 32.58± 17.03 | 37.87± 18.18 |
| Liver cancer | 44.45 ± 13.46 | 42.88± 17.37 | 43.41± 6.31 | 34.44± 4.11 | 38.93± 9.36 |
| Stomach cancer | 44.43 ± 11.35 | 53.13± 9.35 | 48.6± 16.54 | 54.71± 6.58 | 56.72± 18.61 |
| Breast cancer | 41.54 ± 14.28 | 57.06± 17.75 | 37.51± 18.1 | 41.01± 15.47 | 44.25± 8.52 |
| Kidney cancer | 42.45 ± 10.22 | 47.06± 17.68 | 48.49± 8.42 | 40.55± 6.43 | 52.61± 11.68 |
| Colorectal cancer | 42.64 ± 12.31 | 62.11± 7.32 | 45.97± 9.16 | 63.43± 17.81 | 62.71± 16.89 |
| Ovarian cancer | 44.78 ± 14.06 | 54.26± 7.87 | 49.75± 7.47 | 44.51± 9.84 | 63.24± 10.17 |
| Cervical cancer | 46.03 ± 14.71 | 40.19± 13.92 | 36.36± 8.62 | 53.94± 7.45 | 53.63± 5.91 |
| Uterine cancer | 46.85 ± 10.21 | 42.01± 9.95 | 40.72± 10.94 | 53.69± 14.84 | 41.41± 13.27 |
| Prostate cancer | 50.14 ± 14.13 | 50.81± 10.89 | 58.59± 12.29 | 47.73± 13.44 | 51.74± 14.43 |
| Bladder cancer | 51.99 ± 14.57 | 55.38± 6.97 | 49.82± 14.66 | 59.56± 13.77 | 30.22± 16.44 |
| Melanoma | 53.62 ± 12.12 | 41.78± 18.03 | 45.42± 9.22 | 49.52± 12.27 | 43.14± 17.36 |
| Hemangioma | 46.23 ± 12.09 | 51.22± 12.61 | 49.89± 14.65 | 48.68± 5.89 | 49.07± 18.92 |
| Sarcoma | 49.25 ± 11.03 | 40.33± 13.89 | 48.34± 4.82 | 47.91± 12.13 | 49.63± 13.17 |

**Example 4**

Migration inhibition effect of polypeptides P1-P4 on HUVECs 10 mg/mL Matrigel was diluted with a culture medium special for HUVECs at a ratio of 1:2, coated on a transwell chamber membrane, and air-dried at room temperature. HUVECs cultured to a logarithmic growth period were digested with a trypsin digestion solution, collected, washed twice with PBS and then resuspended with a blank culture medium special for HUVECs. The cells were counted under a microscope and the cell concentration was adjusted to $1\times10^5$ cells/mL. The test solution of each group was prepared. The test solutions contained polypeptides P1-P4 with different concentrations, and were each diluted to 100 μL with a blank culture medium special for HUVECs. The cells were inoculated into the transwell chamber at 100 μL per well, and each group of test solution was added into the chamber. 0.6 mL of endothelial cell culture solution containing 5% fetal bovine serum and 1% ECGS was added to a 24-well plate to stimulate cell migration, and cultured for 24 h at 37°C with 5% $CO_2$. The culture solution in the well was discarded, the cells were fixed with 90% alcohol at normal temperature for 30 min, dyed with 0.1% crystal violet at normal temperature for 10 min and rinsed with clear water, the non-migrated cells on the upper layer were gently wiped off using cotton swabs. The observation was made under the microscope and four fields were selected to take photos for counting. The migration inhibition rate (MIR) was calculated according to the formula:

$$MI\ (\%) = 1 - \frac{N_{test}}{N_{control}} \times 100\ \%$$

wherein $N_{test}$ is the cell migration number of the test group and $N_{control}$ is the cell migration number of the blank control group.

The experiment was repeated independently three times. Mean ± SD was calculated based on the results obtained from the experiment, and statistical T-test was conducted. *P< 0.05 indicates significant difference, and **P< 0.01 indicates extremely significant difference. The experimental results are shown in Table 9.

Table 9 Migration inhibition effect of polypeptides P1-P4 on HUVECs

| Group | Dose (μM) | Cell migration number (Mean±SD) | Inhibition rate (%) |
|---|---|---|---|
| | 0.05 | 620.19±46.98* | 41.53% |
| X1 | 0.1 | 560.81±53.1* | 47.13% |
| | 0.2 | 555.64±45.23* | 47.62% |
| | 0.05 | 645.98±49.09* | 39.10% |
| X2 | 0.1 | 516.64±46.50** | 51.29% |
| | 0.2 | 486.11±53.63** | 54.17% |
| | 0.05 | 633.68±56.82* | 40.26% |
| X3 | 0.1 | 535.74±59.20* | 49.49% |
| | 0.2 | 570.83±45.74* | 46.19% |
| | 0.05 | 494.57±51.18** | 53.38% |
| X4 | 0.1 | 484.45±54.52** | 54.33% |
| | 0.2 | 626.68±56.40* | 40.92% |
| Avastin | 0.2 | 418.92±61.42** | 60.92% |
| Control | - | 1060.74±31.42 | 0.00% |

**Example 5**

PIR (%) of derived polypeptides P5-P10 on various tumor cells

An MTT method was used to detect the inhibitory activity of P5-P10 on the growth of various tumor cells. Tumor cells were digested and collected with trypsin after being cultured in an incubator at 37°C with 5% $CO_2$ to a confluence of 90% or more. The cells were resuspended with a culture solution and counted under a microscope, the cell concentration was adjusted to $2\times10^4$ cells/mL, and the cell suspension was inoculated into a 96-well plate at 100 μL/well, and cultured overnight in an incubator at 37°C with 5% $CO_2$. P5-P10 were diluted to respective predetermined concentrations with the culture solution. Docetaxel was diluted to a final concentration with the culture solution. After the cells were completely adhered to the wall, each diluent was added into the 96-well plate (100 μL/well). Tumor cells with the addition of diluents of polypeptides P5-P10 were used as administration groups, tumor cells with the addition of docetaxel were used as a positive control group, and tumor cells with the addition of the culture solution without any drug were used as a negative control group. The cells were cultured in an incubator at 37°C with 5% $CO_2$ for 48 h. 5 mg/mL MTT was added into the 96-well plate, 20 μL per well, and the culture was continued for 4 h. The culture medium was removed, 150 μL of DMSO was added to each well for dissolution, and gent and uniform mixing was performed in a shaker for 10 min. The absorbance was measured at a detection wavelength of 570 nm and a reference wavelength of 630 nm using a microplate reader, and the PIR was calculated with the formula as follows:

$$\text{PIR (\%)} = 1 - \text{administration group/negative group}$$

The experiment was repeated independently three times, and the results were expressed as mean ± SD. The experimental results are shown in Table 10.

Table 10 PIR (%) of polypeptides P5-P10 on various tumor cells

| Tumor cell line source | HM-1 | P5 | P6 | P7 | P8 | P9 | P10 |
|---|---|---|---|---|---|---|---|
| Head and neck cancers | 36.12 ± 11.21 | 45.96± 13.95 | 57.32± 16.75 | 27.29± 14.66 | 61.16± 17.39 | 49.01± 10.16 | 54.79± 7.99 |

(continued)

| Tumor cell line source | HM-1 | P5 | P6 | P7 | P8 | P9 | P10 |
|---|---|---|---|---|---|---|---|
| Brain tumor | 41.54 ± 12.43 | 46.07± 18.12 | 42.44± 14.97 | 46.17± 12.06 | 40.85± 10.24 | 60.37± 7.63 | 47.45± 15.83 |
| Thyroid cancer | 43.15 ± 14.42 | 44.99± 16.02 | 42.43± 17.62 | 64.14± 16.02 | 43.42± 10.02 | 50 54± 5.07 | 50.13± 11.91 |
| Esophageal cancer | 46.22 ± 18.24 | 44.34± 10.55 | 63.13± 10.17 | 46.83± 12.66 | 52.34± 14.99 | 54.42± 7.55 | 50.18± 11.45 |
| Pancreatic cancer | 45.03 ± 13.18 | 54.16± 11.51 | 56.09± 16.01 | 52.28± 15.01 | 55.17± 18.97 | 54.01± 9.61 | 55.87± 17.76 |
| Lung cancer | 50.35 ± 14.14 | 46.99± 12.56 | 52.02± 14.84 | 45.16± 12.09 | 50.07± 15.94 | 58.29± 10.65 | 47.14± 17.26 |
| Liver cancer | 44.45 ± 13.46 | 50.79± 13.95 | 48.96± 15.15 | 55.21± 10.23 | 40.06± 16.28 | 52.61± 13.8 | 55.93± 16.66 |
| Stomach cancer | 44.43 ± 11.35 | 47.78±14.89 | 61.74± 10.29 | 64.23± 16.61 | 50.29± 13.73 | 53.19± 16.45 | 47.58± 10.45 |
| Breast cancer | 41.54 ± 14.28 | 54.73± 11.81 | 58.25± 14.52 | 44.79± 14.21 | 53.15± 11.06 | 46.84± 12.73 | 42.43± 10.62 |
| Kidney cancer | 42.45 ± 10.22 | 50.38± 12.35 | 45.24± 13.11 | 46.3 8± 1 4.44 | 60.73± 10.59 | 49.3 3± 16.14 | 58.26± 12.85 |
| Colorectal cancer | 42.64 ± 12.31 | 46.77± 15.91 | 51.82± 12.71 | 58.47± 12.52 | 51.42± 16.59 | 43.47± 13.21 | 52.63± 11.99 |
| Ovarian cancer | 44.78 ± 14.06 | 54.41± 15.45 | 48.86± 16.61 | 47.02± 12.88 | 57.79± 15.43 | 60.34± 16.21 | 43.46± 10.37 |
| Cervical cancer | 46.03 ± 14.71 | 50.07± 13.34 | 60.67± 11.23 | 55.13± 17.75 | 66.19± 16.16 | 60.43± 15.83 | 54.58± 16.81 |
| Uterine cancer | 46.85 ± 10.21 | 54.75± 12.45 | 57.74± 10.61 | 62.55± 18.95 | 43.22± 11.38 | 46.64± 9.21 | 56.92± 17.33 |
| Prostate cancer | 50.14 ± 14.13 | 49.83± 11.08 | 62.66± 15.13 | 53.05± 17.12 | 53.22± 15.58 | 56.53±12.89 | 63.08± 16.07 |
| Bladder cancer | 51.99 ± 14.57 | 42.68± 16.85 | 62.75± 13.88 | 51.72± 11.34 | 55.6± 15.76 | 58.27± 17.31 | 58.86± 13.61 |
| Melanoma | 53.62 ± 12.12 | 63.66± 16.06 | 48.19± 13.69 | 55.82± 11.53 | 57.51± 14.79 | 43.4± 15.79 | 64.81± 13.65 |
| Hemangioma | 46.23 ± 12.09 | 55.17± 13.65 | 55.84± 10.17 | 43.38± 10.01 | 48.29± 12.71 | 51.3± 14.18 | 43.88± 13.38 |
| Sarcoma | 49.25 ± 11.03 | 46.94± 13.83 | 51.45± 12.82 | 53.94± 13.91 | 56.9± 12.53 | 43.47± 14.77 | 43.21± 12.84 |

**Example 6**

Migration inhibition effect of polypeptides P5-P10 on HUVECs

10 mg/mL Matrigel was diluted with a culture medium special for HUVECs at a ratio of 1:2, coated on a transwell chamber membrane, and air-dried at room temperature. HUVECs cultured to a logarithmic growth period were digested with a trypsin digestion solution, collected, washed twice with PBS and then resuspended with a blank culture medium special for HUVECs. The cells were counted under a microscope and the cell concentration was adjusted to $1\times10^5$ cells/mL. The test solution of each group was prepared. The test solutions contained polypeptides P5-P10 with different concentrations, and were each diluted to 100 μL with a blank culture medium special for HUVECs. The cells were inoculated into the transwell chamber at 100 μL per well, and each group of test solution was added into the chamber. 0.6 mL of endothelial cell culture solution containing 5% fetal bovine serum and 1% ECGS was added to a 24-well plate to stimulate cell migration, and cultured for 24 h at 37°C with 5% $CO_2$. The culture solution in the well was discarded, the cells were fixed with 90% alcohol at normal temperature for 30 min, dyed with 0.1% crystal violet at normal temperature

for 10 min and rinsed with clear water, the non-migrated cells on the upper layer were gently wiped off using cotton swabs. The observation was made under the microscope and four fields were selected to take photos for counting. The migration inhibition rate (MIR) was calculated according to the formula:

$$MI\ (\%) = 1 - \frac{N_{test}}{N_{control}} \times 100\ \%$$

wherein $N_{test}$ is the cell migration number of the test group and $N_{control}$ is the cell migration number of the blank control group.

The experiment was repeated independently three times. Mean ± SD was calculated based on the results obtained from the experiment, and statistical T-test was conducted. *P< 0.05 indicates significant difference, and **P< 0.01 indicates extremely significant difference. The experimental results are shown in Table 11.

Table 11 Migration inhibition effect of polypeptides P5-P10 on HUVECs

| Group | Dose (μM) | Cell migration number (Mean±SD) | Inhibition rate (%) |
|---|---|---|---|
| P5 | 0.05 | 568.79±48.49* | 46.38% |
| | 0.1 | 476.01±54.57** | 55.12% |
| | 0.2 | 530.22±56.20** | 50.01% |
| P6 | 0.05 | 454.57±50.52** | 57.15% |
| | 0.1 | 510.26±56.98* | 51.90% |
| | 0.2 | 590.16±48.21* | 44.36% |
| P7 | 0.05 | 631.62±58.2* | 40.45% |
| | 0.1 | 537.49±52.54* | 49.33% |
| | 0.2 | 640.46±50.11* | 39.62% |
| P8 | 0.05 | 483.56±45.37** | 54.41% |
| | 0.1 | 487.55±51.75** | 54.04% |
| | 0.2 | 481.83±45.91** | 54.58% |
| P9 | 0.05 | 516.51±55.24* | 51.31% |
| | 0.1 | 622.28±54.01* | 41.34% |
| | 0.2 | 604.50±46.64* | 43.01% |
| P10 | 0.05 | 534.90±56.85* | 49.57% |
| | 0.1 | 469.94±56.40** | 55.70% |
| | 0.2 | 537.73±56.02* | 49.31% |
| Avastin | 0.2 | 418.92±61.42** | 60.92% |
| Control | - | 1060.74±31.42 | 0.00% |

**Example 7**

PIR (%) of derived polypeptides P11-P14 on various tumor cells

An MTT method was used to detect the inhibitory activity of polypeptides P11-P14 on the growth of various tumor cells. Tumor cells were digested and collected with trypsin after being cultured in an incubator at 37°C with 5% $CO_2$ to a confluence of 90% or more. The cells were resuspended with a culture solution and counted under a microscope, the cell concentration was adjusted to $2\times10^4$ cells/mL, and the cell suspension was inoculated into a 96-well plate at 100 μL/well, and cultured overnight in an incubator at 37°C with 5% $CO_2$. Polypeptides P11-P14 were diluted to respective predetermined concentrations with the culture solution. Docetaxel was diluted to a final concentration with the culture solution. After the cells were completely adhered to the wall, each diluent was added into the 96-well plate (100 μL/well). Tumor cells with the addition of diluents of polypeptides P11-P14 were used as administration groups, tumor cells with the addition of docetaxel were used as positive control groups, and the culture solution without any drug was used as the negative control group. The cells were cultured in an incubator at 37°C with 5% $CO_2$ for 48 h. 5 mg/mL MTT was added into the 96-well plate, 20 μL per well, and the culture was continued for 4 h. The culture medium was removed, 150 μL of DMSO was added to each well for dissolution, and gent and uniform mixing was performed in a shaker for 10 min. The absorbance was measured at a detection wavelength of 570 nm and a reference wavelength of 630 nm

using a microplate reader, and the PIR was calculated with the formula as follows:

$$PIR\ (\%) = 1 - \text{administration group/negative group}$$

The experiment was repeated independently three times, and the results were expressed as mean ± SD. The experimental results are shown in Table 12.

Table 12 PIR (%) of polypeptides P11-P14 on various tumor cells

| Tumor cell line source | HM-1 | P11 | P12 | P13 | P14 |
|---|---|---|---|---|---|
| Head and neck cancers | 36.12 ± 11.21 | 59.36± 17.54 | 54.75± 17.34 | 51.75± 15.03 | 47.82± 9.11 |
| Brain tumor | 41.54 ± 12.43 | 56.47± 7.02 | 46.81± 4.73 | 50.81± 14.26 | 58.27± 16.92 |
| Thyroid cancer | 43.15 ± 14.42 | 45.19± 17.58 | 54.55± 17.65 | 65.81± 15.21 | 65.73± 7.45 |
| Esophageal cancer | 46.22 ± 18.24 | 57.74± 14.71 | 53.83± 18.54 | 51.39± 12.19 | 50.55± 18.34 |
| Pancreatic cancer | 45.03 ± 13.18 | 62.64± 7.73 | 45.24± 15.93 | 56.11± 18.16 | 59.94± 17.29 |
| Lung cancer | 50.35 ± 14.14 | 26.07± 6.88 | 40.73± 11.44 | 42.51± 14.27 | 42.5± 14.82 |
| Liver cancer | 44.45 ± 13.46 | 51.17± 16.14 | 42.21± 16.23 | 65.23± 17.38 | 50.51± 15.19 |
| Stomach cancer | 44.43 ± 11.35 | 40.32± 6.92 | 57.78± 6.27 | 40.24± 5.45 | 53.17± 15.69 |
| Breast cancer | 41.54 ± 14.28 | 50.34± 10.89 | 43.13± 11.96 | 51.61± 13.58 | 57.06± 13.32 |
| Kidney cancer | 42.45 ± 10.22 | 63.87± 9.86 | 58.47± 16.23 | 50.91± 16.49 | 56.51± 6.86 |
| Colorectal cancer | 42.64 ± 12.31 | 61.69± 6.84 | 48.67± 10.76 | 61.95± 11.44 | 43.67± 15.45 |
| Ovarian cancer | 44.78 ± 14.06 | 59.14± 9.11 | 58.57± 7.49 | 41.41± 8.88 | 54.38± 7.77 |
| Cervical cancer | 46.03 ± 14.71 | 55.11± 14.82 | 64.68± 8.41 | 59.93± 15.98 | 40.05± 5.25 |
| Uterine cancer | 46.85 ± 10.21 | 56.32± 8.95 | 50.94± 12.38 | 63.19± 8.87 | 52.65± 16.08 |
| Prostate cancer | 50.14 ± 14.13 | 53.11± 10.21 | 55.15± 5.66 | 62.25± 6.91 | 42.88± 17.37 |
| Bladder cancer | 51.99 ± 14.57 | 57.05± 11.55 | 64.85± 6.21 | 45.11± 4.24 | 53.13± 9.35 |
| Melanoma | 53.62 ± 12.12 | 57.64± 12.56 | 55.39± 7.79 | 51.04± 18.16 | 57.06± 17.75 |
| Hemangioma | 46.23 ± 12.09 | 49.06± 16.98 | 44.54± 16.89 | 53.28± 12.71 | 47.06± 17.68 |
| Sarcoma | 49.25 ± 11.03 | 48.65± 6.94 | 50.79± 15.65 | 50.13± 16.37 | 62.11± 7.32 |

**Example 8**

Migration inhibition effect of polypeptides P11-P14 on HUVECs 10 mg/mL Matrigel was diluted with a culture medium special for HUVECs at a ratio of 1:2, coated on a transwell chamber membrane, and air-dried at room temperature. HUVECs cultured to a logarithmic growth period were digested with a trypsin digestion solution, collected, washed twice with PBS and then resuspended with a blank culture medium special for HUVECs. The cells were counted under a microscope and the cell concentration was adjusted to $1\times10^5$ cells/mL. The test solution of each group was prepared. The test solutions contained polypeptides P11-P14 with different concentrations, and were each diluted to 100 μL with a blank culture medium special for HUVECs. The cells were inoculated into the transwell chamber at 100 μL per well, and each group of test solution was added into the chamber. 0.6 mL of endothelial cell culture solution containing 5% fetal bovine serum and 1% ECGS was added to a 24-well plate to stimulate cell migration, and cultured for 24 h at 37°C with 5% $CO_2$. The culture solution in the well was discarded, the cells were fixed with 90% alcohol at normal temperature for 30 min, dyed with 0.1% crystal violet at normal temperature for 10 min and rinsed with clear water, the non-migrated cells on the upper layer were gently wiped off using cotton swabs. The observation was made under the microscope and four fields were selected to take photos for counting. The migration inhibition rate (MIR) was calculated according to the formula:

$$MI\ (\%) = 1 - \frac{N_{test}}{N_{control}} \times 100\ \%$$

wherein $N_{test}$ is the cell migration number of the test group and $N_{control}$ is the cell migration number of the blank control group.

The experiment was repeated independently three times. Mean ± SD was calculated based on the results

obtained from the experiment, and statistical T-test was conducted. *P< 0.05 indicates significant difference, and **P< 0.01 indicates extremely significant difference. The experimental results are shown in Table 13.

Table 13 Migration inhibition effect of polypeptides P11-P14 on HUVECs

| Group | Dose (μM) | Cell migration number (Mean±SD) | Inhibition rate (%) |
|---|---|---|---|
| | 0.05 | 544.91±55.33* | 48.63% |
| P11 | 0.1 | 573.27±51.88* | 45.96% |
| | 0.2 | 610.30±57.12* | 42.46% |
| | 0.05 | 563.30±45.07* | 46.90% |
| P12 | 0.1 | 631.75±56.63* | 40.44% |
| | 0.2 | 640.32±56.39* | 39.63% |
| | 0.05 | 563.36±55.87* | 46.89% |
| P13 | 0.1 | 604.98±53.51* | 42.97% |
| | 0.2 | 629.42±57.60* | 40.66% |
| | 0.05 | 521.99±46.56** | 50.79% |
| P14 | 0.1 | 464.30±51.86** | 56.23% |
| | 0.2 | 631.30±49.05* | 40.48% |
| Avastin | 0.2 | 418.92±61.42** | 60.92% |
| control | - | 1060.74±31.42 | 0.00% |

**Example 9**

Migration inhibition effect of polypeptides X1-X90 on HUVECs

10 mg/mL Matrigel was diluted with a culture medium special for HUVECs at a ratio of 1:2, coated on a transwell chamber membrane, and air-dried at room temperature. HUVECs cultured to a logarithmic growth period were digested with a trypsin digestion solution, collected, washed twice with PBS and then resuspended with a blank culture medium special for HUVECs. The cells were counted under a microscope and the cell concentration was adjusted to $1\times10^5$ cells/mL. The test solution of each group was prepared. The test solutions contained polypeptides X1-X90 with different concentrations, and were each diluted to 100 μL with a blank culture medium special for HUVECs. The cells were inoculated into the transwell chamber at 100 μL per well, and each group of test solution was added into the chamber. 0.6 mL of endothelial cell culture solution containing 5% fetal bovine serum and 1% ECGS was added to a 24-well plate to stimulate cell migration, and cultured for 24 h at 37°C with 5% $CO_2$. The culture solution in the well was discarded, the cells were fixed with 90% alcohol at normal temperature for 30 min, dyed with 0.1% crystal violet at normal temperature for 10 min and rinsed with clear water, the non-migrated cells on the upper layer were gently wiped off using cotton swabs. The observation was made under the microscope and four fields were selected to take photos for counting. The migration inhibition rate (MIR) was calculated according to the formula:

$$MI\ (\%) = 1 - \frac{N_{test}}{N_{control}} \times 100\ \%$$

wherein $N_{test}$ is the cell migration number of the test group and $N_{control}$ is the cell migration number of the blank control group.

The experiment was repeated independently three times. Mean ± SD was calculated based on the results obtained from the experiment, and statistical T-test was conducted. *P< 0.05 indicates significant difference, and **P< 0.01 indicates extremely significant difference. The experimental results are shown in Table 14.

Table 14 Migration inhibition effect of polypeptides X1 to X90 on HUVECs

| Group | Dose (μM) | Cell migration number (Mean±SD) | Inhibition rate (%) |
|---|---|---|---|
| | 0.05 | 596.50 ± 44.35* | 43.77% |
| X1 | 0.1 | 528.57 ± 48.12** | 50.17% |
| | 0.2 | 514.51 ± 47.11** | 52.00% |
| | 0.05 | 640.75 ± 65.48* | 40.22% |

(continued)

| Group | Dose (μM) | Cell migration number (Mean±SD) | Inhibition rate (%) |
|---|---|---|---|
| X2 | 0.1 | 636.64 ± 63.41* | 40.60% |
| | 0.2 | 499.83 ± 69.77** | 53.37% |
| | 0.05 | 611.01 ± 58.38* | 43.00% |
| X3 | 0.1 | 564.24 ± 46.78* | 47.36% |
| | 0.2 | 555.54 ± 53.63* | 48.17% |
| | 0.05 | 578.69 ± 63.60* | 46.01% |
| X4 | 0.1 | 560.12 ± 50.60* | 47.74% |
| | 0.2 | 554.16 ± 43.38* | 48.30% |
| | 0.05 | 626.21 ± 40.84* | 41.58% |
| X5 | 0.1 | 616.15 ± 57.51* | 42.52% |
| | 0.2 | 559.72 ± 47.65* | 47.78% |
| | 0.05 | 609.98 ± 66.18* | 43.09% |
| X6 | 0.1 | 524.73 ± 53.87** | 51.04% |
| | 0.2 | 498.30 ± 60.64** | 53.51% |
| | 0.05 | 599.78 ± 48.52* | 44.04% |
| X7 | 0.1 | 523.39 ± 41.62** | 51.17% |
| | 0.2 | 522.23 ± 60.37** | 51.28% |
| | 0.05 | 623.21 ± 49.06* | 41.86% |
| X8 | 0.1 | 589.09 ± 51.42* | 45.04% |
| | 0.2 | 580.69 ± 48.61* | 45.82% |
| | 0.05 | 645.91 ± 58.77* | 39.74% |
| X9 | 0.1 | 604.04 ± 58.33* | 43.65% |
| | 0.2 | 459.92 ± 68.65** | 57.09% |
| | 0.05 | 570.02 ± 60.71* | 46.82% |
| X10 | 0.1 | 566.82 ± 47.23* | 47.12% |
| | 0.2 | 536.75 ± 69.25* | 49.92% |
| | 0.05 | 590.60 ± 59.00* | 44.90% |
| X11 | 0.1 | 583.13 ± 46.68* | 45.60% |
| | 0.2 | 555.75 ± 57.95* | 48.15% |
| | 0.05 | 574.61 ± 48.36* | 46.39% |
| X12 | 0.1 | 538.77 ± 53.35* | 49.74% |
| | 0.2 | 478.42 ± 47.91** | 55.37% |
| | 0.05 | 617.61 ± 43.62* | 42.38% |
| X13 | 0.1 | 463.21 ± 46.79** | 56.78% |
| | 0.2 | 460.75 ± 47.48** | 57.01% |
| | 0.05 | 631.58 ± 62.75* | 41.08% |
| X14 | 0.1 | 511.88 ± 65.93** | 52.24% |
| | 0.2 | 482.35 ± 59.07** | 55.00% |
| | 0.05 | 604.84 ± 67.76* | 43.57% |
| X15 | 0.1 | 558.61 ± 50.98* | 47.88% |
| | 0.2 | 470.58 ± 62.38** | 56.10% |
| | 0.05 | 643.45 ± 52.09* | 39.97% |
| X16 | 0.1 | 505.06 ± 52.71** | 52.88% |
| | 0.2 | 469.22 ± 52.56** | 56.22% |
| | 0.05 | 547.07 ± 50.72* | 48.96% |
| X17 | 0.1 | 483.55 ± 66.46** | 54.89% |
| | 0.2 | 455.35 ± 68.52** | 57.52% |
| | 0.05 | 635.26 ± 59.22* | 40.73% |
| X18 | 0.1 | 632.13 ± 55.39* | 41.02% |

(continued)

| Group | Dose (μM) | Cell migration number (Mean±SD) | Inhibition rate (%) |
|---|---|---|---|
| | 0.2 | 594.30 ± 45.38* | 44.55% |
| | 0.05 | 632.53 ± 66.02* | 40.99% |
| X19 | 0.1 | 570.54 ± 51.64* | 46.77% |
| | 0.2 | 488.31 ± 53.35** | 54.44% |
| | 0.05 | 563.29 ± 41.22* | 47.45% |
| X20 | 0.1 | 538.18 ± 64.13* | 49.79% |
| | 0.2 | 506.96 ± 57.65* | 52.70% |
| | 0.05 | 547.26 ± 48.17* | 48.94% |
| X21 | 0.1 | 529.36 ± 68.43** | 50.61% |
| | 0.2 | 512.08 ± 47.73** | 52.23% |
| | 0.05 | 555.03 ± 66.81* | 48.22% |
| X22 | 0.1 | 480.13 ± 67.49** | 55.21% |
| | 0.2 | 453.97 ± 57.23** | 57.65% |
| | 0.05 | 555.01 ± 40.99* | 48.22% |
| X23 | 0.1 | 519.80 ± 57.39** | 51.50% |
| | 0.2 | 513.80 ± 43.43** | 52.06% |
| | 0.05 | 540.49 ± 60.98* | 49.57% |
| X24 | 0.1 | 536.51 ± 42.41* | 49.95% |
| | 0.2 | 525.06 ± 53.33** | 51.01% |
| | 0.05 | 556.73 ± 40.91* | 48.06% |
| X25 | 0.1 | 511.9 ± 68.58** | 52.24% |
| | 0.2 | 450.13 ± 52.7** | 58.00% |
| | 0.05 | 570.08 ± 49.26* | 46.81% |
| X26 | 0.1 | 546.86 ± 60.43* | 48.98% |
| | 0.2 | 494.57 ± 48.88** | 53.86% |
| | 0.05 | 574.65 ± 60.83* | 46.39% |
| X27 | 0.1 | 546.51 ± 46.67* | 49.01% |
| | 0.2 | 499.65 ± 59.06** | 53.38% |
| | 0.05 | 610.89 ± 42.79* | 43.01% |
| X28 | 0.1 | 506.45 ± 65.00** | 52.75% |
| | 0.2 | 502.42 ± 40.72** | 53.13% |
| | 0.05 | 574.88 ± 55.42* | 46.37% |
| X29 | 0.1 | 531.34 ± 47.59** | 50.43% |
| | 0.2 | 494.73 ± 56.78** | 53.84% |
| | 0.05 | 631.10 ± 64.04* | 41.12% |
| X30 | 0.1 | 497.88 ± 65.38** | 53.55% |
| | 0.2 | 497.56 ± 68.24** | 53.58% |
| | 0.05 | 641.17 ± 61.85* | 40.18% |
| X31 | 0.1 | 571.62 ± 42.83* | 46.67% |
| | 0.2 | 480.05 ± 67.72** | 55.21% |
| | 0.05 | 622.57 ± 49.41* | 41.92% |
| X32 | 0.1 | 560.86 ± 50.91* | 47.67% |
| | 0.2 | 465.06 ± 48.23** | 56.61% |
| | 0.05 | 614.91 ± 68.92* | 42.63% |
| X33 | 0.1 | 563.23 ± 57.29* | 47.45% |
| | 0.2 | 490.88 ± 51.22** | 54.20% |
| | 0.05 | 557.95 ± 42.69* | 47.95% |
| X34 | 0.1 | 513.64 ± 43.27** | 52.08% |
| | 0.2 | 496.39 ± 56.37** | 53.69% |

(continued)

| Group | Dose (μM) | Cell migration number (Mean±SD) | Inhibition rate (%) |
|---|---|---|---|
| | 0.05 | 615.18 ± 57.71* | 42.61% |
| X35 | 0.1 | 529.83 ± 68.02** | 50.57% |
| | 0.2 | 461.34 ± 66.82** | 56.96% |
| | 0.05 | 513.79 ± 55.46** | 52.07% |
| X36 | 0.1 | 463.47 ± 50.27** | 56.76% |
| | 0.2 | 451.01 ± 43.85** | 57.92% |
| | 0.05 | 642.52 ± 55.55* | 40.06% |
| X37 | 0.1 | 517.86 ± 41.17** | 51.69% |
| | 0.2 | 511.08 ± 66.22** | 52.32% |
| | 0.05 | 630.97 ± 66.64* | 41.13% |
| X38 | 0.1 | 619.85 ± 44.78* | 42.17% |
| | 0.2 | 531.57 ± 66.12** | 50.41% |
| | 0.05 | 628.13 ± 60.26* | 41.40% |
| X39 | 0.1 | 509.81 ± 59.62** | 52.44% |
| | 0.2 | 460.22 ± 66.58** | 57.06% |
| | 0.05 | 473.99 ± 63.82** | 55.78% |
| X40 | 0.1 | 463.87 ± 51.75** | 56.72% |
| | 0.2 | 462.34 ± 49.75** | 56.87% |
| | 0.05 | 637.22 ± 61.82* | 40.55% |
| X41 | 0.1 | 588.93 ± 44.15* | 45.06% |
| | 0.2 | 587.17 ± 63.28* | 45.22% |
| | 0.05 | 645.21 ± 40.07* | 39.80% |
| X42 | 0.1 | 630.25 ± 53.06* | 41.20% |
| | 0.2 | 619.42 ± 53.04* | 42.21% |
| | 0.05 | 503.90 ± 67.34** | 52.99% |
| X43 | 0.1 | 464.90 ± 41.52** | 56.63% |
| | 0.2 | 457.03 ± 67.04** | 57.36% |
| | 0.05 | 644.35 ± 66.02* | 39.88% |
| X44 | 0.1 | 605.46 ± 67.29* | 43.51% |
| | 0.2 | 482.27 ± 45.88** | 55.01% |
| | 0.05 | 506.46 ± 56.61** | 52.75% |
| X45 | 0.1 | 493.96 ± 48.77** | 53.92% |
| | 0.2 | 491.44 ± 51.16** | 54.15% |
| | 0.05 | 639.42 ± 45.14* | 40.34% |
| X46 | 0.1 | 611.47 ± 46.48* | 42.95% |
| | 0.2 | 606.59 ± 40.95* | 43.41% |
| | 0.05 | 628.14 ± 46.56* | 41.40% |
| X47 | 0.1 | 585.17 ± 45.06* | 45.41% |
| | 0.2 | 453.86 ± 55.76** | 57.66% |
| | 0.05 | 609.83 ± 42.93* | 43.11% |
| X48 | 0.1 | 527.77 ± 47.87** | 50.76% |
| | 0.2 | 454.60 ± 43.39** | 57.59% |
| | 0.05 | 596.49 ± 59.46* | 44.35% |
| X49 | 0.1 | 591.86 ± 58.82* | 44.78% |
| | 0.2 | 564.8 ± 42.28* | 47.31% |
| | 0.05 | 588.02 ± 64.55* | 45.14% |
| X50 | 0.1 | 491.92 ± 40.42** | 54.11% |
| | 0.2 | 484.66 ± 48.92** | 54.78% |
| | 0.05 | 570.95 ± 57.94* | 46.73% |

(continued)

| Group | Dose (μM) | Cell migration number (Mean±SD) | Inhibition rate (%) |
|---|---|---|---|
| X51 | 0.1 | 562.27 ± 69.67* | 47.54% |
| | 0.2 | 509.89 ± 46.32** | 52.43% |
| | 0.05 | 550.22 ± 66.35* | 48.67% |
| X52 | 0.1 | 490.94 ± 45.96** | 54.20% |
| | 0.2 | 479.99 ± 49.33** | 55.22% |
| | 0.05 | 646.26 ± 52.89* | 39.71% |
| X53 | 0.1 | 614.63 ± 58.99* | 42.66% |
| | 0.2 | 508.07 ± 57.46** | 52.60% |
| | 0.05 | 539.50 ± 46.30* | 49.67% |
| X54 | 0.1 | 511.39 ± 55.45** | 52.29% |
| | 0.2 | 458.68 ± 52.66** | 57.21% |
| | 0.05 | 567.43 ± 62.61* | 47.06% |
| X55 | 0.1 | 561.08 ± 58.02* | 47.65% |
| | 0.2 | 546.15 ± 65.38* | 49.05% |
| | 0.05 | 474.41 ± 60.05** | 55.74% |
| X56 | 0.1 | 464.64 ± 66.21** | 56.65% |
| | 0.2 | 453.36 ± 66.8** | 57.70% |
| | 0.05 | 585.89 ± 52.56* | 45.34% |
| X57 | 0.1 | 552.46 ± 63.62* | 48.46% |
| | 0.2 | 537.05 ± 48.43* | 49.90% |
| | 0.05 | 544.75 ± 50.23* | 49.18% |
| X58 | 0.1 | 541.79 ± 47.41* | 49.45% |
| | 0.2 | 534.40 ± 62.82** | 50.14% |
| | 0.05 | 631.77 ± 42.86* | 41.06% |
| X59 | 0.1 | 548.18 ± 51.93* | 48.86% |
| | 0.2 | 450.01 ± 45.32** | 58.02% |
| | 0.05 | 570.05 ± 68.22* | 46.82% |
| X60 | 0.1 | 516.14 ± 59.71** | 51.85% |
| | 0.2 | 487.66 ± 66.17** | 54.50% |
| | 0.05 | 534.34 ± 60.15** | 50.15% |
| X61 | 0.1 | 492.36 ± 56.92** | 54.06% |
| | 0.2 | 472.26 ± 44.23** | 55.94% |
| | 0.05 | 592.43 ± 41.82* | 44.73% |
| X62 | 0.1 | 509.31 ± 64.35** | 52.48% |
| | 0.2 | 455.14 ± 54.72** | 57.54% |
| | 0.05 | 623.23 ± 65.91* | 41.86% |
| X63 | 0.1 | 568.93 ± 60.47* | 46.92% |
| | 0.2 | 530.05 ± 57.17** | 50.55% |
| | 0.05 | 598.19 ± 57.83* | 44.19% |
| X64 | 0.1 | 597.45 ± 57.61* | 44.26% |
| | 0.2 | 491.49 ± 49.8** | 54.15% |
| | 0.05 | 603.59 ± 54.67* | 43.69% |
| X65 | 0.1 | 567.44 ± 60.62* | 47.06% |
| | 0.2 | 527.88 ± 56.82** | 50.75% |
| | 0.05 | 568.71 ± 46.23* | 46.94% |
| X66 | 0.1 | 461.34 ± 55.31** | 56.96% |
| | 0.2 | 457.45 ± 40.12** | 57.32% |
| | 0.05 | 484.76 ± 61.82** | 54.77% |
| X67 | 0.1 | 484.16 ± 69.61** | 54.83% |

(continued)

| Group | Dose (μM) | Cell migration number (Mean±SD) | Inhibition rate (%) |
|---|---|---|---|
| | 0.2 | 461.41 ± 51.33** | 56.95% |
| | 0.05 | 644.14 ± 50.25* | 39.90% |
| X68 | 0.1 | 493.79 ± 45.96** | 53.93% |
| | 0.2 | 457.27 ± 43.22** | 57.34% |
| | 0.05 | 626.86 ± 54.92* | 41.52% |
| X69 | 0.1 | 613.88 ± 66.11* | 42.73% |
| | 0.2 | 609.37 ± 66.42* | 43.15% |
| | 0.05 | 572.09 ± 68.72* | 46.63% |
| X70 | 0.1 | 545.17 ± 59.19* | 49.14% |
| | 0.2 | 471.98 ± 58.66** | 55.97% |
| | 0.05 | 599.46 ± 41.62* | 44.07% |
| X71 | 0.1 | 573.23 ± 46.83* | 46.52% |
| | 0.2 | 563.66 ± 57.47* | 47.41% |
| | 0.05 | 587.78 ± 65.32* | 45.16% |
| X72 | 0.1 | 562.36 ± 68.25* | 47.53% |
| | 0.2 | 543.09 ± 68.53* | 49.33% |
| | 0.05 | 617.65 ± 52.54* | 42.38% |
| X73 | 0.1 | 610.91 ± 47.76* | 43.00% |
| | 0.2 | 526.93 ± 54.91** | 50.84% |
| | 0.05 | 643.13 ± 44.73* | 40.00% |
| X74 | 0.1 | 609.66 ± 40.19* | 43.12% |
| | 0.2 | 532.17 ± 58.72** | 50.35% |
| | 0.05 | 642.82 ± 44.87* | 40.03% |
| X75 | 0.1 | 599.44 ± 52.61* | 44.07% |
| | 0.2 | 548.83 ± 69.17* | 48.80% |
| | 0.05 | 648.21 ± 59.68* | 39.52% |
| X76 | 0.1 | 615.72 ± 44.04* | 42.56% |
| | 0.2 | 531.49 ± 60.48** | 50.41% |
| | 0.05 | 590.26 ± 58.98* | 44.93% |
| X77 | 0.1 | 551.29 ± 67.77* | 48.57% |
| | 0.2 | 530.54 ± 61.73** | 50.50% |
| | 0.05 | 532.89 ± 61.36** | 50.28% |
| X78 | 0.1 | 470.25 ± 45.15** | 56.13% |
| | 0.2 | 451.24 ± 63.99** | 57.89% |
| | 0.05 | 593.18 ± 64.62* | 44.66% |
| X79 | 0.1 | 508.03 ± 45.26** | 52.60% |
| | 0.2 | 486.72 ± 63.61** | 54.59% |
| | 0.05 | 626.73 ± 48.64* | 41.53% |
| X80 | 0.1 | 583.43 ± 64.15* | 45.57% |
| | 0.2 | 469.12 ± 69.73** | 56.23% |
| | 0.05 | 631.38 ± 41.32* | 41.09% |
| X81 | 0.1 | 571.5 ± 63.73* | 46.68% |
| | 0.2 | 486.90 ± 67.98** | 54.57% |
| | 0.05 | 627.14 ± 61.36* | 41.49% |
| X82 | 0.1 | 546.94 ± 64.81** | 48.97% |
| | 0.2 | 480.77 ± 51.82** | 55.15% |
| | 0.05 | 640.61 ± 51.53* | 40.23% |
| X83 | 0.1 | 575.66 ± 68.26* | 46.29% |
| | 0.2 | 563.64 ± 64.56* | 47.41% |

(continued)

| Group | Dose (μM) | Cell migration number (Mean±SD) | Inhibition rate (%) |
|---|---|---|---|
| | 0.05 | 589.96 ± 45.51* | 44.96% |
| X84 | 0.1 | 571.18 ± 44.95* | 46.71% |
| | 0.2 | 466.05 ± 55.06** | 56.52% |
| | 0.05 | 638.88 ± 63.92* | 40.40% |
| X85 | 0.1 | 629.55 ± 51.71* | 41.27% |
| | 0.2 | 590.65 ± 59.92* | 44.89% |
| | 0.05 | 549.03 ± 59.13* | 48.78% |
| X86 | 0.1 | 503.77 ± 64.22** | 53.00% |
| | 0.2 | 497.53 ± 53.15** | 53.58% |
| | 0.05 | 540.03 ± 52.12* | 49.09% |
| X87 | 0.1 | 511.06 ± 40.06** | 51.82% |
| | 0.2 | 484.92 ± 42.69** | 54.28% |
| | 0.05 | 579.07 ± 52.93* | 45.41% |
| X88 | 0.1 | 464.58 ± 55.82** | 56.20% |
| | 0.2 | 454.59 ± 48.87** | 57.14% |
| | 0.05 | 531.58 ± 59.58* | 49.89% |
| X89 | 0.1 | 500.27 ± 62.93** | 52.84% |
| | 0.2 | 488.42 ± 43.23** | 53.95% |
| | 0.05 | 608.29 ± 57.22* | 42.65% |
| X90 | 0.1 | 582.35 ± 67.26* | 45.10% |
| | 0.2 | 573.28 ± 44.52* | 45.95% |
| Avastin | 0.2 | 418.92±61.42** | 60.92% |
| control | - | 1060.74±31.42 | 0.00% |

Results: Under the action of polypeptide X1-X90, the number of migrated endothelial cells decreased significantly. Compared with the blank control group, the administration group can inhibit the migration of HUVECs induced by 5% fetal bovine serum and 1% ECGS. The inhibitory effect of the polypeptide X59 on cell migration at 0.2 μM dose was extremely significantly different from that of the blank control (**P < 0.01), and the inhibition rate was 58.02%.

**Example 10**

Effect of polypeptides X1-X90 on proliferation of splenic lymphocytes in mice

Spleens of mice were taken out under aseptic conditions, washed with blank 1640 culture medium 3 times, ground with a 5 mL syringe core, filtered with a 200-mesh sieve, made into a single cell suspension. The suspension was centrifuged (1000 rpm × 5 min), and supernatant was removed. Red blood cells were lysed by Tris-$NH_4Cl$, and placed in ice water bath for 4 min and centrifuged (1000 rpm × 5 min). The supernatant was removed, and the cells were washed twice with sterile PBS. Finally, RPMI 1640 culture solution (5 mL) containing 10% calf serum was added to suspend the cells. The cells were counted and the cell concentration was adjusted to $5\times10^6$ cells/mL, and the cells were cultured in a 96-well culture plate.

Blank control group, concanavalin A (ConA) group and dexamethasone (Dex) group were set in the experiment, and polypeptide X1-X90 groups were set as test groups. After 100 μL of splenic lymphocytes suspension per well was added into each group, 100 μL of blank 1640 culture solution was added into the blank control group, ConA was added into the ConA group, Dex was added into the Dex group, and ConA was added into the test groups on the basis of adding different concentrations of polypeptides X1-X90. The cells were static cultured at 37°C in a cell incubator for 48 h. After the cultivation was completed, 20 μL of MTT was added to each well, and the cultivation was continued for 4 h. Finally, all the solutions in each well were discarded. 100 μL DMSO was added to each well and shaken, and the OD value at 570 nm was detected with a microplate reader. Five parallel experiments were set in each well. Results are shown in Table 15.

Table 15 Effect of polypeptides X1-X90 on proliferation of splenic lymphocytes in mice

| Group | Dose (μM) | A570 nm/A630 nm | Inhibition rate (%) |
|---|---|---|---|
| X1 | 0.05 | 0.5690 ±0.0501 | 15.75% |
| | 0.1 | 0.5336 ±0.0435 | 20.99% |
| | 0.2 | 0.5002 ±0.1243 | 25.94% |
| X2 | 0.05 | 0.5829 ±0.1189 | 13.70% |
| | 0.1 | 0.5290 ±0.0543 | 21.68% |
| | 0.2 | 0.4767 ±0.0569 | 29.42% |
| X3 | 0.05 | 0.6137 ±0.1127 | 9.14% |
| | 0.1 | 0.4903 ±0.0507 | 27.41% |
| | 0.2 | 0.4790 ±0.0396 | 29.08% |
| X4 | 0.05 | 0.5747 ±0.1223 | 14.91% |
| | 0.1 | 0.5744 ±0.0865 | 14.95% |
| | 0.2 | 0.4501 ±0.0822 | 33.36% |
| X5 | 0.05 | 0.5841 ±0.1208 | 13.52% |
| | 0.1 | 0.5074 ±0.0893 | 24.87% |
| | 0.2 | 0.4354 ±0.0491 | 35.53% |
| X6 | 0.05 | 0.5281 ±0.0787 | 21.81% |
| | 0.1 | 0.5126 ±0.0664 | 24.10% |
| | 0.2 | 0.4603 ±0.0661 | 31.85% |
| X7 | 0.05 | 0.5503 ±0.1123 | 18.52% |
| | 0.1 | 0.5420 ±0.1094 | 19.75% |
| | 0.2 | 0.4359 ±0.0755 | 35.46% |
| X8 | 0.05 | 0.5701 ±0.1244 | 15.59% |
| | 0.1 | 0.5604 ±0.0847 | 17.03% |
| | 0.2 | 0.5499 ±0.0745 | 18.58% |
| X9 | 0.05 | 0.5614 ±0.0763 | 16.88% |
| | 0.1 | 0.5347 ±0.0627 | 20.83% |
| | 0.2 | 0.5162 ±0.0760 | 23.57% |
| X10 | 0.05 | 0.5705 ±0.0488 | 15.53% |
| | 0.1 | 0.4898 ±0.0423 | 27.48% |
| | 0.2 | 0.4713 ±0.0594 | 30.22% |
| X11 | 0.05 | 0.6074 ±0.0783 | 10.07% |
| | 0.1 | 0.6002 ±0.0945 | 11.13% |
| | 0.2 | 0.5755 ±0.1175 | 14.79% |
| X12 | 0.05 | 0.5668 ±0.1043 | 16.08% |
| | 0.1 | 0.4865 ±0.0743 | 27.97% |
| | 0.2 | 0.4381 ±0.0381 | 35.13% |
| X13 | 0.05 | 0.6034 ±0.1082 | 10.66% |
| | 0.1 | 0.5122 ±0.1124 | 24.16% |
| | 0.2 | 0.4334 ±0.0973 | 35.83% |
| X14 | 0.05 | 0.5894 ±0.0562 | 12.73% |
| | 0.1 | 0.5569 ±0.0827 | 17.55% |
| | 0.2 | 0.5045 ±0.0663 | 25.30% |
| X15 | 0.05 | 0.5189 ±0.0761 | 23.17% |
| | 0.1 | 0.4459 ±0.0996 | 33.98% |
| | 0.2 | 0.4413 ±0.0806 | 34.66% |
| X16 | 0.05 | 0.5703 ±0.04654 | 15.56% |
| | 0.1 | 0.5163 ±0.0753 | 23.56% |
| | 0.2 | 0.4425 ±0.1234 | 34.48% |
| | 0.05 | 0.6219 ±0.0629 | 7.92% |

(continued)

| Group | Dose (μM) | A570 nm/A630 nm | Inhibition rate (%) |
|---|---|---|---|
| X17 | 0.1 | 0.5484 ±0.1109 | 18.80% |
| | 0.2 | 0.5025 ±0.0957 | 25.60% |
| | 0.05 | 0.6041 ±0.0524 | 10.56% |
| X18 | 0.1 | 0.5571 ±0.0554 | 17.52% |
| | 0.2 | 0.4402 ±0.0534 | 34.82% |
| | 0.05 | 0.5093 ±0.0372 | 24.59% |
| X19 | 0.1 | 0.5027 ±0.0814 | 25.57% |
| | 0.2 | 0.4978 ±0.0481 | 26.30% |
| | 0.05 | 0.5977 ±0.1129 | 11.50% |
| X20 | 0.1 | 0.5419 ±0.0423 | 19.77% |
| | 0.2 | 0.4947 ±0.1213 | 26.75% |
| | 0.05 | 0.5904 ±0.0399 | 12.59% |
| X21 | 0.1 | 0.5776 ±0.1193 | 14.48% |
| | 0.2 | 0.4947 ±0.1055 | 26.75% |
| | 0.05 | 0.5694 ±0.0561 | 15.69% |
| X22 | 0.1 | 0.4603 ±0.0873 | 31.85% |
| | 0.2 | 0.4521 ±0.1194 | 33.06% |
| | 0.05 | 0.5946 ±0.0378 | 11.96% |
| X23 | 0.1 | 0.4982 ±0.1212 | 26.24% |
| | 0.2 | 0.4363 ±0.0933 | 35.40% |
| | 0.05 | 0.5823 ±0.0474 | 14.12% |
| X24 | 0.1 | 0.5615 ±0.1155 | 16.86% |
| | 0.2 | 0.5142 ±0.0636 | 23.87% |
| | 0.05 | 0.5838 ±0.0861 | 13.56% |
| X25 | 0.1 | 0.5673 ±0.0906 | 16.01% |
| | 0.2 | 0.4576 ±0.1221 | 32.25% |
| | 0.05 | 0.5714 ±0.1002 | 15.46% |
| X26 | 0.1 | 0.5365 ±0.0543 | 20.57% |
| | 0.2 | 0.4991 ±0.0743 | 26.10% |
| | 0.05 | 0.5388 ±0.0643 | 20.23% |
| X27 | 0.1 | 0.5231 ±0.0967 | 22.55% |
| | 0.2 | 0.4792 ±0.0849 | 29.08% |
| | 0.05 | 0.5765 ±0.0594 | 14.64% |
| X28 | 0.1 | 0.5541 ±0.0486 | 17.96% |
| | 0.2 | 0.5087 ±0.0793 | 24.68% |
| | 0.05 | 0.5595 ±0.0932 | 17.16% |
| X29 | 0.1 | 0.5284 ±0.0661 | 21.76% |
| | 0.2 | 0.5094 ±0.1191 | 24.58% |
| | 0.05 | 0.5725 ±0.0671 | 15.24% |
| X30 | 0.1 | 0.4914 ±0.0652 | 27.24% |
| | 0.2 | 0.4759 ±0.0702 | 29.54% |
| | 0.05 | 0.6142 ±0.0512 | 9.06% |
| X31 | 0.1 | 0.6134 ±0.1148 | 9.18% |
| | 0.2 | 0.5450 ±0.0838 | 19.31% |
| | 0.05 | 0.6117 ±0.0672 | 9.43% |
| X32 | 0.1 | 0.5954 ±0.0678 | 11.84% |
| | 0.2 | 0.5428 ±0.0408 | 19.63% |
| | 0.05 | 0.5669 ±0.1137 | 16.06% |
| X33 | 0.1 | 0.5297 ±0.0479 | 21.57% |

(continued)

| Group | Dose (μM) | A570 nm/A630 nm | Inhibition rate (%) |
|---|---|---|---|
| | 0.2 | 0.4492 ±0.1069 | 33.49% |
| | 0.05 | 0.5469 ±0.0655 | 19.03% |
| X34 | 0.1 | 0.4573 ±0.1104 | 32.29% |
| | 0.2 | 0.4431 ±0.1192 | 34.39% |
| | 0.05 | 0.6122 ±0.1219 | 9.36% |
| X35 | 0.1 | 0.5261 ±0.0617 | 22.11% |
| | 0.2 | 0.4415 ±0.0616 | 34.63% |
| | 0.05 | 0.6051 ±0.0633 | 10.41% |
| X36 | 0.1 | 0.4566 ±0.1223 | 32.40% |
| | 0.2 | 0.4401 ±0.051 | 34.84% |
| | 0.05 | 0.5796 ±0.0738 | 14.18% |
| X37 | 0.1 | 0.5792 ±0.1065 | 14.24% |
| | 0.2 | 0.4659 ±0.0948 | 31.02% |
| | 0.05 | 0.5618 ±0.1042 | 16.82% |
| X38 | 0.1 | 0.4558 ±0.0979 | 32.51% |
| | 0.2 | 0.4328 ±0.0631 | 35.92% |
| | 0.05 | 0.6212 ±0.0507 | 8.02% |
| X39 | 0.1 | 0.4416 ±0.0387 | 34.62% |
| | 0.2 | 0.4357 ±0.0863 | 35.49% |
| | 0.05 | 0.5503 ±0.1179 | 18.52% |
| X40 | 0.1 | 0.5416 ±0.0875 | 19.81% |
| | 0.2 | 0.4765 ±0.0889 | 29.45% |
| | 0.05 | 0.5735 ±0.0874 | 15.09% |
| X41 | 0.1 | 0.5607 ±0.1173 | 16.98% |
| | 0.2 | 0.4914 ±0.0759 | 27.24% |
| | 0.05 | 0.5397 ±0.0472 | 20.09% |
| X42 | 0.1 | 0.5107 ±0.0539 | 24.39% |
| | 0.2 | 0.4931 ±0.0829 | 26.99% |
| | 0.05 | 0.579 ±0.0424 | 14.27% |
| X43 | 0.1 | 0.5636 ±0.0746 | 16.55% |
| | 0.2 | 0.5158 ±0.0464 | 23.63% |
| | 0.05 | 0.4618 ±0.0574 | 31.63% |
| X44 | 0.1 | 0.4546 ±0.0849 | 32.69% |
| | 0.2 | 0.4397 ±0.0381 | 34.90% |
| | 0.05 | 0.4925 ±0.0885 | 27.08% |
| X45 | 0.1 | 0.4649 ±0.0648 | 31.17% |
| | 0.2 | 0.4382 ±0.0542 | 35.12% |
| | 0.05 | 0.5862 ±0.0858 | 13.24% |
| X46 | 0.1 | 0.5312 ±0.0611 | 21.35% |
| | 0.2 | 0.4555 ±0.1123 | 32.56% |
| | 0.05 | 0.5974 ±0.0873 | 11.55% |
| X47 | 0.1 | 0.5872 ±0.0457 | 13.06% |
| | 0.2 | 0.5522 ±0.0891 | 18.24% |
| | 0.05 | 0.4919 ±0.1203 | 27.17% |
| X48 | 0.1 | 0.4673 ±0.0677 | 30.81% |
| | 0.2 | 0.4320 ±0.0449 | 36.04% |
| | 0.05 | 0.6195 ±0.0383 | 8.28% |
| X49 | 0.1 | 0.5796 ±0.0546 | 14.18% |
| | 0.2 | 0.4814 ±0.0475 | 28.72% |

(continued)

| Group | Dose (μM) | A570 nm/A630 nm | Inhibition rate (%) |
|---|---|---|---|
| | 0.05 | 0.6230 ±0.1001 | 7.76% |
| X50 | 0.1 | 0.5842 ±0.1217 | 13.50% |
| | 0.2 | 0.5679 ±0.1122 | 15.92% |
| | 0.05 | 0.5059 ±0.1217 | 25.10% |
| X51 | 0.1 | 0.4460 ±0.0609 | 33.97% |
| | 0.2 | 0.4309 ±0.0846 | 36.20% |
| | 0.05 | 0.5636 ±0.0451 | 16.55% |
| X52 | 0.1 | 0.4912 ±0.0789 | 27.27% |
| | 0.2 | 0.4696 ±0.1046 | 30.47% |
| | 0.05 | 0.6080 ±0.1023 | 9.98% |
| X53 | 0.1 | 0.5264 ±0.0724 | 22.06% |
| | 0.2 | 0.5256 ±0.0637 | 22.18% |
| | 0.05 | 0.5155 ±0.0467 | 23.67% |
| X54 | 0.1 | 0.4531 ±0.0826 | 32.91% |
| | 0.2 | 0.4366 ±0.0544 | 35.36% |
| | 0.05 | 0.6206 ±0.1217 | 8.11% |
| X55 | 0.1 | 0.5632 ±0.0636 | 16.61% |
| | 0.2 | 0.4911 ±0.0831 | 27.29% |
| | 0.05 | 0.5792 ±0.0513 | 14.24% |
| X56 | 0.1 | 0.4813 ±0.0694 | 28.74% |
| | 0.2 | 0.4731 ±0.0773 | 29.95% |
| | 0.05 | 0.5872 ±0.0843 | 13.06% |
| X57 | 0.1 | 0.5186 ±0.0898 | 23.22% |
| | 0.2 | 0.4535 ±0.0803 | 32.85% |
| | 0.05 | 0.482 ±0.1197 | 28.63% |
| X58 | 0.1 | 0.4726 ±0.1193 | 30.03% |
| | 0.2 | 0.4484 ±0.0383 | 33.61% |
| | 0.05 | 0.5684 ±0.1051 | 15.84% |
| X59 | 0.1 | 0.4922 ±0.1001 | 27.12% |
| | 0.2 | 0.4490 ±0.0907 | 33.52% |
| | 0.05 | 0.5752 ±0.0946 | 14.84% |
| X60 | 0.1 | 0.5287 ±0.0535 | 21.72% |
| | 0.2 | 0.4516 ±0.0451 | 33.14% |
| | 0.05 | 0.6027 ±0.0421 | 10.76% |
| X61 | 0.1 | 0.5426 ±0.0588 | 19.66% |
| | 0.2 | 0.5396 ±0.0815 | 20.11% |
| | 0.05 | 0.5924 ±0.0545 | 12.29% |
| X62 | 0.1 | 0.5410 ±0.1178 | 19.90% |
| | 0.2 | 0.4946 ±0.1136 | 26.77% |
| | 0.05 | 0.5120 ±0.0424 | 24.19% |
| X63 | 0.1 | 0.4896 ±0.0466 | 27.51% |
| | 0.2 | 0.4802 ±0.0899 | 28.90% |
| | 0.05 | 0.4903 ±0.0999 | 27.41% |
| X64 | 0.1 | 0.4840 ±0.0397 | 28.34% |
| | 0.2 | 0.4626 ±0.1067 | 31.51% |
| | 0.05 | 0.5761 ±0.1145 | 14.70% |
| X65 | 0.1 | 0.5284 ±0.1090 | 21.76% |
| | 0.2 | 0.5263 ±0.0471 | 22.08% |
| | 0.05 | 0.5782 ±0.0621 | 14.39% |

(continued)

| Group | Dose (μM) | A570 nm/A630 nm | Inhibition rate (%) |
|---|---|---|---|
| X66 | 0.1 | 0.5153 ±0.0677 | 23.70% |
| | 0.2 | 0.4344 ±0.0692 | 35.68% |
| | 0.05 | 0.5022 ±0.1074 | 25.64% |
| X67 | 0.1 | 0.4530 ±0.0848 | 32.93% |
| | 0.2 | 0.4439 ±0.0830 | 34.28% |
| | 0.05 | 0.6040 ±0.0785 | 10.57% |
| X68 | 0.1 | 0.5933 ±0.1197 | 12.16% |
| | 0.2 | 0.4581 ±0.1171 | 32.17% |
| | 0.05 | 0.5825 ±0.0498 | 13.75% |
| X69 | 0.1 | 0.5177 ±0.1040 | 23.35% |
| | 0.2 | 0.4498 ±0.0404 | 33.40% |
| | 0.05 | 0.5590 ±0.0745 | 17.23% |
| X70 | 0.1 | 0.5293 ±0.0927 | 21.63% |
| | 0.2 | 0.4868 ±0.1146 | 27.92% |
| | 0.05 | 0.5929 ±0.1211 | 12.21% |
| X71 | 0.1 | 0.4746 ±0.0674 | 29.73% |
| | 0.2 | 0.4359 ±0.1159 | 35.46% |
| | 0.05 | 0.6087 ±0.0473 | 9.88% |
| X72 | 0.1 | 0.5846 ±0.0861 | 13.44% |
| | 0.2 | 0.5793 ±0.0432 | 14.23% |
| | 0.05 | 0.5086 ±0.0538 | 24.70% |
| X73 | 0.1 | 0.4995 ±0.0594 | 26.04% |
| | 0.2 | 0.4403 ±0.0526 | 34.81% |
| | 0.05 | 0.6150 ±0.1195 | 8.94% |
| X74 | 0.1 | 0.5118 ±0.0370 | 24.22% |
| | 0.2 | 0.4796 ±0.1055 | 28.99% |
| | 0.05 | 0.5665 ±0.1103 | 16.12% |
| X75 | 0.1 | 0.5546 ±0.0651 | 17.89% |
| | 0.2 | 0.5280 ±0.0932 | 21.82% |
| | 0.05 | 0.5429 ±0.0374 | 19.62% |
| X76 | 0.1 | 0.4940 ±0.0946 | 26.86% |
| | 0.2 | 0.4538 ±0.0644 | 32.81% |
| | 0.05 | 0.6037 ±0.0672 | 10.62% |
| X77 | 0.1 | 0.5764 ±0.0563 | 14.66% |
| | 0.2 | 0.5425 ±0.0835 | 19.68% |
| | 0.05 | 0.5848 ±0.0468 | 13.41% |
| X78 | 0.1 | 0.5644 ±0.0524 | 16.43% |
| | 0.2 | 0.5128 ±0.1213 | 24.07% |
| | 0.05 | 0.6191 ±0.0595 | 8.34% |
| X79 | 0.1 | 0.5640 ±0.1173 | 16.49% |
| | 0.2 | 0.4454 ±0.0759 | 34.05% |
| | 0.05 | 0.5708 ±0.0931 | 15.49% |
| X80 | 0.1 | 0.5574 ±0.0764 | 17.47% |
| | 0.2 | 0.4658 ±0.0436 | 31.03% |
| | 0.05 | 0.5519 ±0.0526 | 18.29% |
| X81 | 0.1 | 0.5357 ±0.0666 | 20.68% |
| | 0.2 | 0.4510 ±0.0848 | 33.22% |
| | 0.05 | 0.5582 ±0.0773 | 17.35% |
| X82 | 0.1 | 0.5490 ±0.0646 | 18.71% |

(continued)

| Group | Dose (μM) | A570 nm/A630 nm | Inhibition rate (%) |
|---|---|---|---|
| | 0.2 | 0.4826 ±0.0706 | 28.55% |
| | 0.05 | 0.5530 ±0.0905 | 18.12% |
| X83 | 0.1 | 0.5396 ±0.1234 | 20.11% |
| | 0.2 | 0.4676 ±0.0973 | 30.77% |
| | 0.05 | 0.6088 ±0.0952 | 9.86% |
| X84 | 0.1 | 0.5420 ±0.1007 | 19.75% |
| | 0.2 | 0.4782 ±0.0856 | 29.20% |
| | 0.05 | 0.5653 ±0.1097 | 16.30% |
| X85 | 0.1 | 0.5591 ±0.0484 | 17.22% |
| | 0.2 | 0.5044 ±0.0975 | 25.32% |
| | 0.05 | 0.5788 ±0.0467 | 14.30% |
| X86 | 0.1 | 0.5520 ±0.1060 | 18.27% |
| | 0.2 | 0.5355 ±0.0589 | 20.71% |
| | 0.05 | 0.5785 ±0.0641 | 14.35% |
| X87 | 0.1 | 0.5723 ±0.0847 | 15.27% |
| | 0.2 | 0.4514 ±0.0991 | 33.17% |
| | 0.05 | 0.5840 ±0.0712 | 13.53% |
| X88 | 0.1 | 0.5793 ±0.0628 | 14.23% |
| | 0.2 | 0.5360 ±0.1234 | 20.64% |
| | 0.05 | 0.5860 ±0.0529 | 13.24% |
| X89 | 0.1 | 0.5677 ±0.0877 | 15.95% |
| | 0.2 | 0.5529 ±0.0446 | 18.14% |
| | 0.05 | 0.6211 ±0.0533 | 8.04% |
| X90 | 0.1 | 0.5274 ±0.0429 | 21.91% |
| | 0.2 | 0.4483 ±0.1024 | 33.62% |
| ConA | - | 0.6754±0.0312 | - |
| Dex | 20 | 0.3918±0.1127 | 52.65% |
| Negative | - | 0.6172±0.0486 | - |

Results: Different polypeptides X1-X90 could inhibit the proliferation of splenic lymphocytes in mice to some extent. When the dose of X51 was 0.2 μM, the inhibition rate reached 36.20%. And the inhibition effect of each administration group showed a certain dose-dependent relationship.

**Example 11**

Effect of polypeptides X1-X90 on IL-1β production by mouse peritoneal macrophages

(1) IL-1β production: Mice were injected intraperitoneally with 1 mL of broth culture medium (containing 6% of starch). Three days later, mice peritoneal macrophages were taken aseptically and washed twice with 1640 culture medium, and the cell concentration was adjusted to $2\times10^6$ cells/mL. The solutions were injected into 24-well culture plates at 1 mL per well. The cells were incubated in a cell incubator for 3 h and vibrated once every 30 min to make the cells fully adhere to the wall. Then, the cells were washed twice with a culture solution to remove non-adhered cells. PBS was added to the blank group, positive drug Dex was added to the positive group, no drug was added to the model group, and polypeptides X1-X90 with low, medium and high concentrations were added to the test groups; and the cells were continuously cultured for 48 h after administration, and then centrifuged at 1000 r/min for 15 min. The supernatant was collected as a sample to be tested for IL-1β activity.

(2) Determination of IL-1β content: A mouse IL-1β enzyme-linked immunosorbent assay kit from R&D Company was used for detection, and operations were according to the instructions as follows: tested samples and standards with different concentrations were added respectively, the reaction wells were sealed with sealing tap, and the cells

were cultured at 37°C for 90 min; well plates were washed four times; a biotinylated antibody working solution (100 μL/well) was added, the reaction wells were sealed with sealing tap, and the cells were cultured at 37°C for 60 min; the plates were washed four times; an enzyme conjugate working solution (100 μL/well) was added, the reaction wells were sealed with sealing tap, and the cells were cultured at 37°C for 30 min; the plates were washed four times; a chromogenic agent (100 μL/well) was added, and the cells were cultured for 10-20 min at 37°C in the absence of light; a stopping solution (100 μL/well) was added, and OD450 values were measured after uniform mixing.

Table 16 Effect of polypeptides X1-X90 on IL-1β production by mouse peritoneal macrophages

| Group | Dose (μM) | IL-1β (pg/mL) | Inhibition rate (%) |
|---|---|---|---|
| | 0.05 | 713.98 ± 14.81** | 24.14% |
| X1 | 0.1 | 566.07 ± 12.62** | 39.85% |
| | 0.2 | 504.81 ± 19.06** | 46.36% |
| | 0.05 | 760.65 ± 17.43** | 19.18% |
| X2 | 0.1 | 638.49 ± 11.83** | 32.16% |
| | 0.2 | 571.18 ± 17.86** | 39.31% |
| | 0.05 | 661.28 ± 18.24** | 29.74% |
| X3 | 0.1 | 583.99 ± 12.75** | 37.95% |
| | 0.2 | 538.48 ± 18.51** | 42.78% |
| | 0.05 | 738.37 ± 16.5** | 21.55% |
| X4 | 0.1 | 730.52 ± 19.01** | 22.38% |
| | 0.2 | 520.72 ± 12.58** | 44.67% |
| | 0.05 | 687.97 ± 14.45** | 26.90% |
| X5 | 0.1 | 555.41 ± 15.34** | 40.99% |
| | 0.2 | 515.77 ± 13.86** | 45.20% |
| | 0.05 | 721.27±12.54** | 23.36% |
| X6 | 0.1 | 655.58 ± 17.35** | 30.34% |
| | 0.2 | 511.66 ± 17.94** | 45.63% |
| | 0.05 | 784.12 ± 10.28** | 16.68% |
| X7 | 0.1 | 720.89 ± 10.55** | 23.40% |
| | 0.2 | 616.03 ± 11.32** | 34.54% |
| | 0.05 | 548.85 ± 13.67** | 41.68% |
| X8 | 0.1 | 5 65.46 ± 15.31** | 39.92% |
| | 0.2 | 712.66 ± 10.28** | 24.28% |
| | 0.05 | 761.97 ± 10.58** | 19.04% |
| X9 | 0.1 | 720.99 ± 15.53** | 23.39% |
| | 0.2 | 671.04 ± 18.22** | 28.70% |
| | 0.05 | 657.54 ± 13.71** | 30.13% |
| X10 | 0.1 | 543.52 ± 12.38** | 42.25% |
| | 0.2 | 519.81 ± 10.98** | 44.77% |
| | 0.05 | 740.02 ± 18.07** | 21.37% |
| X11 | 0.1 | 737.89 ± 10.66** | 21.60% |
| | 0.2 | 696.04 ± 13.55** | 26.04% |
| | 0.05 | 712.00 ± 19.81** | 24.35% |
| X12 | 0.1 | 559.65 ± 14.15** | 40.53% |
| | 0.2 | 531.24 ± 15.15** | 43.55% |
| | 0.05 | 615.38 ± 19.33** | 34.61% |
| X13 | 0.1 | 594.57 ± 14.52** | 36.82% |
| | 0.2 | 508.42 ± 14.86** | 45.98% |
| | 0.05 | 748.32 ± 12.75** | 20.49% |
| X14 | 0.1 | 665.77 ± 10.48** | 29.26% |
| | 0.2 | 613.65 ± 15.32** | 34.80% |

(continued)

| Group | Dose (μM) | IL-1β (pg/mL) | Inhibition rate (%) |
|---|---|---|---|
| | 0.05 | 756.78 ± 16.91** | 19.59% |
| X15 | 0.1 | 638.51 ± 15.98** | 32.16% |
| | 0.2 | 588.83 ± 13.37** | 37.43% |
| | 0.05 | 796.16 ± 15.1** | 15.40% |
| X16 | 0.1 | 773.34 ± 14.38** | 17.83% |
| | 0.2 | 592.05 ± 18.61** | 37.09% |
| | 0.05 | 790.35 ± 11.07** | 16.02% |
| X17 | 0.1 | 531.11 ± 17.61** | 43.57% |
| | 0.2 | 528.00 ± 10.53** | 43.90% |
| | 0.05 | 721.31 ± 12.75** | 23.36% |
| X18 | 0.1 | 716.58 ± 16.56** | 23.86% |
| | 0.2 | 683.26 ± 19.63** | 27.40% |
| | 0.05 | 591.13 ± 10.54** | 37.19% |
| X19 | 0.1 | 590.42 ± 10.34** | 37.27% |
| | 0.2 | 589.21 ± 12.08** | 37.39% |
| | 0.05 | 772.72 ± 18.78** | 17.90% |
| X20 | 0.1 | 724.50 ± 12.58** | 23.02% |
| | 0.2 | 631.59 ± 19.52** | 32.89% |
| | 0.05 | 773.58 ± 10.52** | 17.80% |
| X21 | 0.1 | 637.22 ± 12.95** | 32.29% |
| | 0.2 | 526.94 ± 17.75** | 44.01% |
| | 0.05 | 663.21 ± 13.73** | 29.53% |
| X22 | 0.1 | 637.07 ± 19.81** | 32.31% |
| | 0.2 | 578.51 ± 14.03** | 38.53% |
| | 0.05 | 793.48 ± 19.42** | 15.69% |
| X23 | 0.1 | 650.17± 14.98** | 30.92% |
| | 0.2 | 623.64 ± 12.33** | 33.74% |
| | 0.05 | 659.97 ± 17.07** | 29.88% |
| X24 | 0.1 | 645.67 ± 11.87** | 31.39% |
| | 0.2 | 548.82 ± 19.58** | 41.69% |
| | 0.05 | 747.54 ± 18.25** | 20.57% |
| X25 | 0.1 | 713.21 ± 19.17** | 24.22% |
| | 0.2 | 659.86 ± 10.24** | 29.89% |
| | 0.05 | 769.33 ± 19.82** | 18.26% |
| X26 | 0.1 | 734.20 ± 13.89** | 21.99% |
| | 0.2 | 663.38 ± 11.96** | 29.51% |
| | 0.05 | 697.32 ± 17.18** | 25.91% |
| X27 | 0.1 | 649.68 ± 18.74** | 30.97% |
| | 0.2 | 624.72 ± 14.38** | 33.62% |
| | 0.05 | 792.64 ± 11.74** | 15.78% |
| X28 | 0.1 | 640.89 ± 14.7** | 31.90% |
| | 0.2 | 637.34 ± 10.85** | 32.28% |
| | 0.05 | 788.74 ± 15.39** | 16.20% |
| X29 | 0.1 | 736.46 ± 14.72** | 21.75% |
| | 0.2 | 557.81 ± 12.11** | 40.73% |
| | 0.05 | 674.82 ± 10.45** | 28.30% |
| X30 | 0.1 | 621.53 ± 19.69** | 33.96% |
| | 0.2 | 589.79 ± 19.66** | 37.33% |
| | 0.05 | 797.34 ± 10.46** | 15.28% |

(continued)

| Group | Dose (μM) | IL-1β (pg/mL) | Inhibition rate (%) |
|---|---|---|---|
| X31 | 0.1 | 764.81 ± 19.08** | 18.74% |
| | 0.2 | 723.57 ± 18.58** | 23.12% |
| | 0.05 | 771.56 ± 13.41** | 18.02% |
| X32 | 0.1 | 676.06 ± 19.82** | 28.17% |
| | 0.2 | 533.56 ± 18.54** | 43.31% |
| | 0.05 | 699.41 ± 11.17** | 25.68% |
| X33 | 0.1 | 613.62 ± 19.23** | 34.80% |
| | 0.2 | 593.32 ± 11.64** | 36.96% |
| | 0.05 | 767.28 ± 18.52** | 18.47% |
| X34 | 0.1 | 753.21 ± 13.97** | 19.97% |
| | 0.2 | 676.31 ± 16.02** | 28.14% |
| | 0.05 | 718.81 ± 11.53** | 23.62% |
| X35 | 0.1 | 651.42 ± 15.51** | 30.79% |
| | 0.2 | 615.43 ± 17.18** | 34.61% |
| | 0.05 | 779.49 ± 11.56** | 17.18% |
| X36 | 0.1 | 620.34 ± 15.26** | 34.09% |
| | 0.2 | 619.62 ± 11.89** | 34.16% |
| | 0.05 | 794.72 ± 11.93** | 15.56% |
| X37 | 0.1 | 687.76 ± 15.11** | 26.92% |
| | 0.2 | 642.75 ± 16.32** | 31.71% |
| | 0.05 | 568.25 ± 16.41** | 39.62% |
| X38 | 0.1 | 541.61 ± 11.54** | 42.45% |
| | 0.2 | 506.70 ± 13.93** | 46.16% |
| | 0.05 | 792.13 ± 13.67** | 15.83% |
| X39 | 0.1 | 658.74 ± 19.08** | 30.01% |
| | 0.2 | 573.90 ± 10.07** | 39.02% |
| | 0.05 | 673.29 ± 16.27** | 28.46% |
| X40 | 0.1 | 607.19 ± 14.25** | 35.48% |
| | 0.2 | 574.54 ± 18.39** | 38.95% |
| | 0.05 | 742.13 ± 13.85** | 21.15% |
| X41 | 0.1 | 738.75 ± 15.58** | 21.50% |
| | 0.2 | 693.24 ± 11.25** | 26.34% |
| | 0.05 | 798.18 ± 18.97** | 15.19% |
| X42 | 0.1 | 639.07 ± 15.55** | 32.10% |
| | 0.2 | 597.39 ± 13.26** | 36.52% |
| | 0.05 | 708.94 ± 18.65** | 24.67% |
| X43 | 0.1 | 619.99 ± 18.36** | 34.12% |
| | 0.2 | 532.86 ± 11.36** | 43.38% |
| | 0.05 | 747.98 ± 18.08** | 20.52% |
| X44 | 0.1 | 629.53 ± 18.12** | 33.11% |
| | 0.2 | 521.12 ± 14.94** | 44.63% |
| | 0.05 | 766.39 ± 19.61** | 18.57% |
| X45 | 0.1 | 556.6 ± 18.09** | 40.86% |
| | 0.2 | 526.65 ± 12.66** | 44.04% |
| | 0.05 | 768.77 ± 17.46** | 18.32% |
| X46 | 0.1 | 714.74 ± 19.14** | 24.06% |
| | 0.2 | 527.65 ± 17.23** | 43.94% |
| | 0.05 | 694.15 ± 18.69** | 26.24% |
| X47 | 0.1 | 658.30 ± 14.95** | 30.05% |

(continued)

| Group | Dose (μM) | IL-1β (pg/mL) | Inhibition rate (%) |
|---|---|---|---|
| | 0.2 | 518.71 ± 14.69** | 44.88% |
| | 0.05 | 747.87 ± 12.65** | 20.54% |
| X48 | 0.1 | 618.52 ± 12.98** | 34.28% |
| | 0.2 | 598.41 ± 17.15** | 36.42% |
| | 0.05 | 667.29 ± 11.38** | 29.10% |
| X49 | 0.1 | 637.37 ± 13.29** | 32.28% |
| | 0.2 | 586.29 ± 16.72** | 37.70% |
| | 0.05 | 566.55 ± 13.38** | 39.80% |
| X50 | 0.1 | 566.54 ± 16.16** | 39.81% |
| | 0.2 | 552.42 ± 15.46** | 41.30% |
| | 0.05 | 778.04 ± 16.84** | 17.33% |
| X51 | 0.1 | 741.14 ± 10.12** | 21.25% |
| | 0.2 | 608.72 ± 11.49** | 35.32% |
| | 0.05 | 737.61 ± 15.15** | 21.63% |
| X52 | 0.1 | 589.49 ± 11.15** | 37.36% |
| | 0.2 | 572.85 ± 13.85** | 39.13% |
| | 0.05 | 633.83 ± 18.23** | 32.65% |
| X53 | 0.1 | 620.13 ± 13.11** | 34.11% |
| | 0.2 | 600.32 ± 15.37** | 36.21% |
| | 0.05 | 749.61 ± 17.43** | 20.35% |
| X54 | 0.1 | 616.42 ± 12.72** | 34.50% |
| | 0.2 | 543.73 ± 18.82** | 42.23% |
| | 0.05 | 739.52 ± 10.19** | 21.43% |
| X55 | 0.1 | 724.78 ± 19.62** | 22.99% |
| | 0.2 | 544.39 ± 15.61** | 42.16% |
| | 0.05 | 781.78 ± 19.36** | 16.93% |
| X56 | 0.1 | 706.46 ± 19.22** | 24.94% |
| | 0.2 | 561.08 ± 19.21** | 40.38% |
| | 0.05 | 672.72 ± 12.56** | 28.52% |
| X57 | 0.1 | 666.13 ± 14.96** | 29.22% |
| | 0.2 | 520.78 ± 16.49** | 44.66% |
| | 0.05 | 717.32 ± 15.84** | 23.78% |
| X58 | 0.1 | 620.62 ± 19.42** | 34.06% |
| | 0.2 | 586.29 ± 10.36** | 37.70% |
| | 0.05 | 695.91 ± 13.75** | 26.06% |
| X59 | 0.1 | 647.53 ± 16.44** | 31.20% |
| | 0.2 | 643.12 ± 11.69** | 31.67% |
| | 0.05 | 630.91 ± 15.16** | 32.96% |
| X60 | 0.1 | 554.56 ± 11.62** | 41.08% |
| | 0.2 | 500.84 ± 17.46** | 46.78% |
| | 0.05 | 797.35 ± 10.72** | 15.28% |
| X61 | 0.1 | 697.13 ± 13.28** | 25.93% |
| | 0.2 | 647.78 ± 12.47** | 31.17% |
| | 0.05 | 628.45 ± 12.52** | 33.22% |
| X62 | 0.1 | 601.72 ± 15.63** | 36.06% |
| | 0.2 | 520.74 ± 18.21** | 44.67% |
| | 0.05 | 765.84 ± 10.96** | 18.63% |
| X63 | 0.1 | 686.65 ± 19.26** | 27.04% |
| | 0.2 | 556.64 ± 13.23** | 40.85% |

(continued)

| Group | Dose (μM) | IL-1β (pg/mL) | Inhibition rate (%) |
|---|---|---|---|
| | 0.05 | 778.79 ± 12.41** | 17.25% |
| X64 | 0.1 | 759.88 ± 19.83** | 19.26% |
| | 0.2 | 626.56 ± 18.32** | 33.43% |
| | 0.05 | 765.38 ± 16.93** | 18.68% |
| X65 | 0.1 | 660.18 ± 10.44** | 29.85% |
| | 0.2 | 624.23 ± 19.35** | 33.67% |
| | 0.05 | 796.38 ± 12.49** | 15.38% |
| X66 | 0.1 | 770.99 ± 16.16** | 18.08% |
| | 0.2 | 537.76 ± 15.01** | 42.86% |
| | 0.05 | 790.87 ± 19.67** | 15.97% |
| X67 | 0.1 | 677.49 ± 17.07** | 28.01% |
| | 0.2 | 502.45 ± 16.43** | 46.61% |
| | 0.05 | 748.68 ± 11.87** | 20.45% |
| X68 | 0.1 | 742.75 ± 11.59** | 21.08% |
| | 0.2 | 621.92 ± 18.74** | 33.92% |
| | 0.05 | 727.04 ± 17.05** | 22.75% |
| X69 | 0.1 | 617.67 ± 13.71** | 34.37% |
| | 0.2 | 582.44 ± 18.89** | 38.11% |
| | 0.05 | 786.31 ± 14.02** | 16.45% |
| X70 | 0.1 | 604.32 ± 14.13** | 35.79% |
| | 0.2 | 574.15 ± 11.84** | 38.99% |
| | 0.05 | 679.18 ± 19.75** | 27.83% |
| X71 | 0.1 | 634.58 ± 19.39** | 32.57% |
| | 0.2 | 592.41 ± 15.53**. | 37.05% |
| | 0.05 | 655.35 ± 18.42** | 30.37% |
| X72 | 0.1 | 604.85 ± 15.84** | 35.73% |
| | 0.2 | 588.45 ± 13.52** | 37.47% |
| | 0.05 | 771.73 ± 13.81 ** | 18.00% |
| X73 | 0.1 | 753.73 ± 15.23** | 19.91% |
| | 0.2 | 747.51 ± 19.92** | 20.58% |
| | 0.05 | 624.73 ± 11.01** | 33.62% |
| X74 | 0.1 | 568.59 ± 14.61** | 39.58% |
| | 0.2 | 503.89 ± 18.51** | 46.46% |
| | 0.05 | 773.81 ± 19.58** | 17.78% |
| X75 | 0.1 | 739.94 ± 18.37** | 21.38% |
| | 0.2 | 506.17 ± 15.62** | 46.22% |
| | 0.05 | 727.25 ± 14.37** | 22.73% |
| X76 | 0.1 | 598.53 ± 10.69** | 36.40% |
| | 0.2 | 509.91 ± 14.79** | 45.82% |
| | 0.05 | 718.21 ± 15.57** | 23.69% |
| X77 | 0.1 | 596.49 ± 14.02** | 36.62% |
| | 0.2 | 542.70 ± 12.94** | 42.34% |
| | 0.05 | 785.31 ± 13.76** | 16.56% |
| X78 | 0.1 | 614.30 ± 15.49** | 34.73% |
| | 0.2 | 550.10 ± 10.22** | 41.55% |
| | 0.05 | 730.61 ± 11.67** | 22.37% |
| X79 | 0.1 | 655.09 ± 13.17** | 30.39% |
| | 0.2 | 538.64 ± 15.15** | 42.77% |
| | 0.05 | 740.71 ± 14.93** | 21.30% |

(continued)

| Group | Dose (μM) | IL-1β (pg/mL) | Inhibition rate (%) |
|---|---|---|---|
| X80 | 0.1 | 731.38 ± 18.08** | 22.29% |
| | 0.2 | 686.4 ± 14.15** | 27.07% |
| | 0.05 | 738.64 ± 12.83** | 21.52% |
| X81 | 0.1 | 691.37 ± 19.49** | 26.54% |
| | 0.2 | 683.57 ± 17.68** | 27.37% |
| | 0.05 | 767.71 ± 15.67** | 18.43% |
| X82 | 0.1 | 635.72 ± 18.53** | 32.45% |
| | 0.2 | 610.38 ± 18.13** | 35.14% |
| | 0.05 | 668.36 ± 12.95** | 28.98% |
| X83 | 0.1 | 657.88 ± 15.28** | 30.10% |
| | 0.2 | 581.18 ± 10.24** | 38.25% |
| | 0.05 | 737.66 ± 15.78** | 21.62% |
| X84 | 0.1 | 646.9 ± 19.52** | 31.26% |
| | 0.2 | 547.63 ± 15.44** | 41.81% |
| | 0.05 | 756.78 ± 14.73** | 19.59% |
| X85 | 0.1 | 618.25 ± 17.66** | 34.31% |
| | 0.2 | 582.91 ± 16.51** | 38.06% |
| | 0.05 | 721.79 ± 12.02** | 23.31% |
| X86 | 0.1 | 562.02 ± 14.18** | 40.28% |
| | 0.2 | 530.47 ± 16.01** | 43.64% |
| | 0.05 | 709.45 ± 19.53** | 24.62% |
| X87 | 0.1 | 577.97 ± 18.15** | 38.59% |
| | 0.2 | 576.76 ± 16.2** | 38.72% |
| | 0.05 | 799.77 ± 14.02** | 15.02% |
| X88 | 0.1 | 798.94 ± 10.54** | 15.11% |
| | 0.2 | 541.63 ± 18.24** | 42.45% |
| | 0.05 | 725.42 ± 10.43** | 22.92% |
| X89 | 0.1 | 641.72 ± 17.95** | 31.82% |
| | 0.2 | 554.33 ± 11.47** | 41.10% |
| | 0.05 | 765.21 ± 19.43** | 18.69% |
| X90 | 0.1 | 685.28 ± 18.66** | 27.19% |
| | 0.2 | 582.61 ± 11.83** | 38.10% |
| Dex | 20 | 352.06 ± 17.29** | 62.95% |
| Model group | - | 941.14±3.28 | - |
| Negative | - | 9.75±0.75 | - |

Results: Polypeptides X1-X90 all had obvious inhibitory effect on the proliferation of splenic lymphocytes in mice, which was significantly different from that of the negative group. When the dose of X60 was 0.2 μM, the inhibition rate reached 46.78%. And there is a certain dose-dependent relationship.

**Example 12**

Effect of polypeptides X1-X90 on xylene-induced mouse ear swelling

Kunming mice were taken, a normal saline group was taken as a blank control group, an aspirin group (200 mg/kg) was taken as a positive control group, and X1-X90 administration groups were taken as test groups. The mice were injected once a day for 5 consecutive days. The blank control group was given an equal volume of normal saline. One hour after the last administration, 0.05 mL of xylene was applied to both sides of the right ear of each mouse to cause inflammation, while no drug was applied to the left ear as a normal ear. Two hours later, the mice were put to death by dislocation. Two ears were cut along the auricle. Ear pieces were taken with a puncher and weighed, and the swelling degree and the swelling rate were calculated. Swelling degree = right ear piece weight-left ear piece weight,

swelling rate = (swelling degree/left ear piece weight) * 100%. Statistical t-test was conducted on the experimental results. *P< 0.05 indicates significant difference, and **P< 0.01 indicates extremely significant difference. The results are shown in Table 17.

Table 17 Effect of polypeptides X1-X90 on xylene-induced mouse ear swelling

| Group | Dose (mg/kg) | Swelling degree (mg) | Inhibition rate (%) |
|---|---|---|---|
| | 2 | 5.75 ± 0.39 | 7.70% |
| X1 | 4 | 4.61 ± 0.63* | 26.00% |
| | 8 | 4.09 ± 0.02** | 34.35% |
| X2 | 2 | 4.52 ± 0.85* | 27.45% |
| | 4 | 4.21 ± 0.82** | 32.42% |
| | 8 | 3.81 ± 0.11** | 38.84% |
| | 2 | 4.28 ± 0.46** | 31.30% |
| X3 | 4 | 3.51 ± 0.46** | 43.66% |
| | 8 | 3.32 ± 0.64** | 46.71% |
| | 2 | 4.62 ± 0.77* | 25.84% |
| X4 | 4 | 4.47 ± 0.87* | 28.25% |
| | 8 | 3.57 ± 0.26** | 42.70% |
| | 2 | 4.23 ± 0.62** | 32.10% |
| X5 | 4 | 3.78 ± 0.37** | 39.33% |
| | 8 | 3.43 ± 0.76** | 44.94% |
| | 2 | 5.83 ± 0.49 | 6.42% |
| X6 | 4 | 5.26 ± 0.18 | 15.57% |
| | 8 | 4.46 ± 0.19** | 28.41% |
| | 2 | 5.17 ± 0.49 | 17.01% |
| X7 | 4 | 4.61 ± 0.21* | 26.00% |
| | 8 | 3.47 ± 0.35** | 44.30% |
| | 2 | 5.87 ± 0.95 | 5.78% |
| X8 | 4 | 5.63 ± 0.56 | 9.63% |
| | 8 | 5.26 ± 0.38* | 15.57% |
| | 2 | 4.11 ± 0.32** | 34.03% |
| X9 | 4 | 3.54 ± 0.73** | 43.18% |
| | 8 | 3.01 ± 0.13** | 51.69% |
| | 2 | 5.69 ± 0.57 | 8.67% |
| X10 | 4 | 3.68 ± 0.47** | 40.93% |
| | 8 | 3.46 ± 0.05** | 44.46% |
| | 2 | 5.52 ± 0.97 | 11.40% |
| X11 | 4 | 3.81 ± 0.42** | 38.84% |
| | 8 | 3.18 ± 0.17** | 48.96% |
| | 2 | 5.87 ± 0.32 | 5.78% |
| X12 | 4 | 5.53 ± 0.09 | 11.24% |
| | 8 | 5.32 ± 0.31 | 14.61% |
| | 2 | 5.78 ± 0.91 | 7.22% |
| X13 | 4 | 4.91 ± 0.71* | 21.19% |
| | 8 | 3.8 ± 0.15** | 39.00% |
| | 2 | 5.55 ± 0.31 | 10.91% |
| X14 | 4 | 4.78 ± 0.04* | 23.27% |
| | 8 | 4.43 ± 0.09* | 28.89% |
| | 2 | 5.76 ± 0.41 | 7.54% |
| X15 | 4 | 5.58 ± 0.08 | 10.43% |
| | 8 | 3.8 ± 0.94** | 39.00% |
| | 2 | 5.00 ± 0.08 | 19.74% |

(continued)

| Group | Dose (mg/kg) | Swelling degree (mg) | Inhibition rate (%) |
|---|---|---|---|
| X16 | 4 | 3.11 ± 0.71** | 50.08% |
| | 8 | 3.10 ± 0.3** | 50.24% |
| | 2 | 4.65 ± 0.79* | 25.36% |
| X17 | 4 | 4.43 ± 0.75* | 28.89% |
| | 8 | 3.53 ± 0.1** | 43.34% |
| | 2 | 5.42 ± 0.18 | 13.00% |
| X18 | 4 | 5.28 ± 0.46 | 15.25% |
| | 8 | 4.18 ± 0.9** | 32.91% |
| | 2 | 4.98 ± 0.72* | 20.06% |
| X19 | 4 | 3.97 ± 0.61** | 36.28% |
| | 8 | 3.8 ± 0.03** | 39.00% |
| | 2 | 5.72 ± 0.01 | 8.19% |
| X20 | 4 | 3.78 ± 0.88** | 39.33% |
| | 8 | 3.47 ± 0.07** | 44.30% |
| | 2 | 5.39 ± 0.39 | 13.48% |
| X21 | 4 | 4.97 ± 0.14* | 20.22% |
| | 8 | 3.15 ± 0.42** | 49.44% |
| | 2 | 4.72 ± 0.29* | 24.24% |
| X22 | 4 | 3.67 ± 0.77** | 41.09% |
| | 8 | 3.42 ± 0.21** | 45.10% |
| | 2 | 5.64 ± 0.84 | 9.47% |
| X23 | 4 | 3.63 ± 0.51** | 41.73% |
| | 8 | 3.47 ± 0.06** | 44.30% |
| | 2 | 5.25 ± 0.06 | 15.73% |
| X24 | 4 | 4.98 ± 0.19* | 20.06% |
| | 8 | 4.34 ± 0.68** | 30.34% |
| | 2 | 5.62 ± 0.64 | 9.79% |
| X25 | 4 | 4.45 0.20* | 28.57% |
| | 8 | 3.3 ± 0.06** | 47.03% |
| | 2 | 5.88 ± 0.72 | 5.62% |
| X26 | 4 | 5.78 ± 0.62 | 7.22% |
| | 8 | 4.54 ± 0.61** | 49.76% |
| | 2 | 5.36 ± 0.32 | 13.96% |
| X27 | 4 | 4.76 ± 0.89* | 23.60% |
| | 8 | 3.13 ± 0.38* | 27.13% |
| | 2 | 5.25 ± 0.56** | 33.87% |
| X28 | 4 | 4.86 ± 0.63 | 15.73% |
| | 8 | 4.12 ± 0.93* | 21.99% |
| | 2 | 5.43 ± 0.91 | 12.84% |
| X29 | 4 | 4.96 ± 0.1* | 20.39% |
| | 8 | 3.86 ± 0.45** | 38.04% |
| | 2 | 5.19 ± 0.57 | 16.69% |
| X30 | 4 | 5.07 ± 0.49 | 18.62% |
| | 8 | 3.77 ± 0.38** | 39.49% |
| | 2 | 3.16 ± 0.55 | 16.85% |
| X31 | 4 | 4.22 ± 0.51** | 32.26% |
| | 8 | 5.18 ± 0.71** | 49.28% |
| | 2 | 5.17 ± 0.06 | 17.01% |
| X32 | 4 | 4.83 ± 0.02* | 22.47% |

(continued)

| Group | Dose (mg/kg) | Swelling degree (mg) | Inhibition rate (%) |
|---|---|---|---|
| | 8 | 3.69 ± 0.56** | 40.77% |
| X33 | 2 | 3.22 ± 0.59 | 17.34% |
| | 4 | 5.15 ± 0.19* | 29.21% |
| | 8 | 4.41 ± 0.9** | 48.31% |
| X34 | 2 | 3.86 ± 0.43** | 38.04% |
| | 4 | 3.36 ± 0.53** | 46.07% |
| | 8 | 3.2 ± 0.39** | 48.64% |
| X35 | 2 | 5.59 ± 0.91 | 10.27% |
| | 4 | 4.78 ± 0.64* | 23.27% |
| | 8 | 3.48 ± 0.28** | 44.14% |
| X36 | 2 | 5.37 ± 0.84 | 13.80% |
| | 4 | 5.37 ± 0.21 | 13.80% |
| | 8 | 4.7 ± 0.97* | 24.56% |
| X37 | 2 | 5.41 ± 0.31 | 13.16% |
| | 4 | 4.79 ± 0.47* | 23.11% |
| | 8 | 3.41 ± 0.03** | 45.26% |
| X38 | 2 | 5.51 ± 0.41 | 11.56% |
| | 4 | 5.30 ± 0.23 | 14.93% |
| | 8 | 3.61 ± 0.69** | 42.05% |
| X39 | 2 | 5.38 ± 0.22 | 13.64% |
| | 4 | 5.09 ± 0.65 | 18.30% |
| | 8 | 4.85 ± 0.74* | 22.15% |
| X40 | 2 | 5.24 ± 0.55 | 15.89% |
| | 4 | 4.63 ± 0.53* | 25.68% |
| | 8 | 4.55 ± 0.37* | 26.97% |
| X41 | 2 | 5.67 ± 0.88 | 8.99% |
| | 4 | 4.94 ± 0.99* | 20.71% |
| | 8 | 4.72 ± 0.57* | 24.24% |
| X42 | 2 | 5.79 ± 0.06 | 7.06% |
| | 4 | 5.65 ± 0.17 | 9.31% |
| | 8 | 3.15 ± 0.17** | 49.44% |
| X43 | 2 | 4.48 0.02* | 28.09% |
| | 4 | 4.41 ± 0.42* | 29.21% |
| | 8 | 3.51 ± 0.27** | 43.66% |
| X44 | 2 | 3.88 ± 0.39** | 37.72% |
| | 4 | 3.79 ± 0.37** | 39.17% |
| | 8 | 3.45 ± 0.24** | 44.62% |
| X45 | 2 | 5.59 ± 0.47 | 10.27% |
| | 4 | 4.73 ± 0.63* | 24.08% |
| | 8 | 3.98 ± 0.69** | 36.12% |
| X46 | 2 | 4.27 ± 0.18** | 31.46% |
| | 4 | 3.90 ± 0.58** | 37.40% |
| | 8 | 3.70 ± 0.54** | 40.61% |
| X47 | 2 | 4.26 ± 0.64** | 31.62% |
| | 4 | 3.51 ± 0.96** | 43.66% |
| | 8 | 3.28 ± 0.62** | 47.35% |
| X48 | 2 | 5.99 ± 0.13 | 3.85% |
| | 4 | 4.53 ± 0.11* | 27.29% |
| | 8 | 4.32 ± 0.21** | 30.66% |

(continued)

| Group | Dose (mg/kg) | Swelling degree (mg) | Inhibition rate (%) |
|---|---|---|---|
| | 2 | 5.63 ± 0.92 | 9.63% |
| X49 | 4 | 4.74 ± 0.74* | 23.92% |
| | 8 | 3.06 ± 0.86** | 50.88% |
| | 2 | 5.45 ± 0.49 | 12.52% |
| X50 | 4 | 4.29 ± 0.14** | 31.14% |
| | 8 | 3.55 ± 0.74** | 43.02% |
| | 2 | 4.95 ± 0.22* | 20.55% |
| X51 | 4 | 3.20 ± 0.28** | 48.64% |
| | 8 | 3.02 ± 0.33** | 51.52% |
| | 2 | 4.42 ± 0.91* | 29.05% |
| X52 | 4 | 3.49 ± 0.92** | 43.98% |
| | 8 | 3.24 ± 0.43** | 47.99% |
| | 2 | 4.08 ± 0.11** | 34.51% |
| X53 | 4 | 3.99 ± 0.84** | 35.96% |
| | 8 | 3.67 ± 0.64** | 41.09% |
| | 2 | 5.30 ± 0.11 | 14.93% |
| X54 | 4 | 4.71 ± 0.73* | 24.40% |
| | 8 | 3.05 ± 0.28** | 51.04% |
| | 2 | 5.46 ± 0.02 | 12.36% |
| X55 | 4 | 4.98 ± 0.55* | 20.06% |
| | 8 | 3.16 ± 0.77** | 49.28% |
| | 2 | 4.50 ± 0.07* | 27.77% |
| X56 | 4 | 3.54 ± 0.9** | 43.18% |
| | 8 | 3.50 ± 0.93** | 43.82% |
| | 2 | 5.25 ± 0.24 | 15.73% |
| X57 | 4 | 4.99 ± 0.71 | 19.90% |
| | 8 | 4.09 ± 0.69** | 34.35% |
| | 2 | 5.51 ± 0.96 | 11.56% |
| X58 | 4 | 5.33 ± 0.94 | 14.45% |
| | 8 | 4.87 ± 0.72* | 21.83% |
| | 2 | 4.65 ± 0.12* | 25.36% |
| X59 | 4 | 4.12 ± 0.29** | 34.19% |
| | 8 | 3.12 ± 0.68** | 50.24% |
| | 2 | 5.43 ± 0.30 | 12.84% |
| X60 | 4 | 3.83 ± 0.94** | 38.52% |
| | 8 | 3.05 ± 0.71** | 51.04% |
| | 2 | 5.99 ± 0.27 | 3.85% |
| X61 | 4 | 5.29 ± 0.22 | 15.09% |
| | 8 | 4.44 ± 0.33* | 28.73% |
| | 2 | 4.9 ± 0.16* | 21.35% |
| X62 | 4 | 4.62 ± 0.37* | 25.84% |
| | 8 | 3.23 ± 0.25** | 48.15% |
| | 2 | 5.87 ± 0.54 | 5.78% |
| X63 | 4 | 3.90 ± 0.14** | 37.40% |
| | 8 | 3.31 ± 0.21** | 46.87% |
| | 2 | 5.01 ± 0.59 | 19.58% |
| X64 | 4 | 5.00 ± 0.74 | 19.74% |
| | 8 | 4.93 ± 0.36* | 20.87% |
| | 2 | 5.46 ± 0.61 | 12.36% |

(continued)

| Group | Dose (mg/kg) | Swelling degree (mg) | Inhibition rate (%) |
|---|---|---|---|
| X65 | 4 | 5.16 ± 0.99 | 17.17% |
| | 8 | 4.23 ± 0.45** | 32.10% |
| | 2 | 4.78 ± 0.86* | 23.27% |
| X66 | 4 | 3.74 ± 0.95* | 39.97% |
| | 8 | 3.24 ± 0.37** | 47.99% |
| | 2 | 3.42 ± 0.11** | 45.10% |
| X67 | 4 | 3.09 ± 0.87** | 50.40% |
| | 8 | 3.02 ± 0.05** | 51.52% |
| | 2 | 5.96 ± 0.55 | 4.33% |
| X68 | 4 | 5.69 ± 0.97 | 8.67% |
| | 8 | 4.93 ± 0.13* | 20.87% |
| | 2 | 5.67 ± 0.75 | 8.99% |
| X69 | 4 | 4.45 ± 0.99* | 28.57% |
| | 8 | 3.03 ± 0.91** | 51.36% |
| | 2 | 4.60 ± 0.25* | 26.16% |
| X70 | 4 | 4.02 ± 0.72** | 35.47% |
| | 8 | 3.58 ± 0.41** | 42.54% |
| | 2 | 5.96 ± 0.55 | 8.03% |
| X71 | 4 | 5.15 ± 0.32 | 17.34% |
| | 8 | 4.43 ± 0.13* | 28.89% |
| | 2 | 5.11 ± 0.99 | 17.98% |
| X72 | 4 | 4.77 ± 0.81* | 23.43% |
| | 8 | 3.58 ± 0.48** | 42.54% |
| | 2 | 4.72 ± 0.72* | 24.24% |
| X73 | 4 | 4.42 ± 0.57* | 29.05% |
| | 8 | 3.58 ± 0.63** | 42.54% |
| | 2 | 5.56 ± 0.67 | 10.75% |
| X74 | 4 | 4.79 ± 0.29* | 23.11% |
| | 8 | 3.69 ± 0.85** | 40.77% |
| | 2 | 5.94 ± 0.47 | 4.65% |
| X75 | 4 | 4.28 ± 0.41** | 31.30% |
| | 8 | 3.32 ± 0.69** | 46.71% |
| | 2 | 5.65 ± 0.24 | 9.31% |
| X76 | 4 | 5.20 ± 0.91 | 16.53% |
| | 8 | 3.08 ± 0.39** | 50.56% |
| | 2 | 5.03 ± 0.78 | 19.26% |
| X77 | 4 | 4.39 ± 0.95* | 29.53% |
| | 8 | 3.99 ± 0.39** | 35.96% |
| | 2 | 4.38 ± 0.19* | 29.70% |
| X78 | 4 | 3.59 ± 0.66** | 42.38% |
| | 8 | 3.41 ± 0.67** | 45.26% |
| | 2 | 4.72 ± 0.72* | 24.24% |
| X79 | 4 | 4.42 ± 0.57* | 29.05% |
| | 8 | 3.58 ± 0.63** | 42.54% |
| | 2 | 5.56 ± 0.67 | 10.75% |
| X80 | 4 | 4.79 ± 0.29* | 23.11% |
| | 8 | 3.69 ± 0.85** | 40.77% |
| | 2 | 5.94 ± 0.47 | 4.65% |
| X81 | 4 | 4.28 ± 0.41** | 31.30% |

(continued)

| Group | Dose (mg/kg) | Swelling degree (mg) | Inhibition rate (%) |
|---|---|---|---|
| | 8 | 3.32 ± 0.69** | 46.71% |
| | 2 | 5.65 ± 0.24 | 9.31% |
| X82 | 4 | 5.20 ± 0.91 | 16.53% |
| | 8 | 3.08 ± 0.39** | 50.56% |
| | 2 | 5.03 ± 0.78 | 19.26% |
| X83 | 4 | 4.39 ± 0.95* | 29.53% |
| | 8 | 3.99 ± 0.39** | 35.96% |
| | 2 | 5.15 ± 0.62 | 17.34% |
| X84 | 4 | 5.11 ± 0.84 | 17.98% |
| | 8 | 4.52 ± 0.43* | 27.45% |
| | 2 | 4.83 ± 0.77* | 22.47% |
| X85 | 4 | 4.81 ± 0.03* | 22.79% |
| | 8 | 3.66 ± 0.62** | 41.25% |
| | 2 | 5.72 ± 0.64 | 8.19% |
| X86 | 4 | 4.69 ± 0.72* | 24.72% |
| | 8 | 4.06 ± 0.46** | 34.83% |
| | 2 | 5.52 ± 0.64 | 11.40% |
| X87 | 4 | 5.21 ± 0.39 | 16.37% |
| | 8 | 3.58 ± 0.38** | 42.54% |
| | 2 | 4.04 ± 0.34 | 35.15% |
| X88 | 4 | 3.68 ± 0.52** | 40.93% |
| | 8 | 3.50 ± 0.65** | 43.82% |
| | 2 | 5.08 ± 0.69 | 18.46% |
| X89 | 4 | 4.42 ± 0.67* | 29.05% |
| | 8 | 4.08 ± 0.06** | 34.51% |
| | 2 | 4.99 ± 0.57 | 19.90% |
| X90 | 4 | 4.20 ± 0.9** | 32.58% |
| | 8 | 3.22 ± 0.96** | 48.31% |
| Aspirin | 200 | 3.08±0.31** | 51.04% |
| control | - | 6.23 ± 0.29 | |

Results: Polypeptides X1-X90 could have obvious inhibitory effects on xylene-induced mouse ear swelling. The inhibition rate of the polypeptide X51 reached 51.52%, which was better than that of the positive group and showed a dose-dependent relationship.

**Example 13**

In vivo immunoprotective effect of polypeptides X1-X90 on a mouse collagen-induced arthritis (CIA) animal model

A mouse CIA animal model was established to study the therapeutic effects of X1-X90 on mouse CIA. Mice were used as test animals. SPF DBA/1 mice, male, 7-8 weeks old, were used. They each weighed 18-22 g and were randomly divided into a normal control group, a model control group, X1-X90 groups and a positive drug control group (methotrexate at 1 mg/kg). Except the normal group, mouse CIA models were established in each test group on day 0 by dissolving chicken cartilage type III collagen (cIII) into 4 mg/mL solution with 0.1 mol/L acetic acid and standing overnight in a refrigerator at 4°C. On the day of the test, type III collagen and complete Freund's adjuvant (CFA) containing 4 mg/mL Myeobaeterium tuberculosis strain H37Rv were fully emulsified in the same volume. After DB.A/1 mice were anesthetized, 50 μL of emulsifier was injected into the tail skin of each mouse for sensitization. After 21 days, 4 mg/mL type III collagen (cIII) and incomplete Freund's adjuvant (IFA) were fully emulsified in the same volume and then re-immunization was performed with the same dose of emulsifier in the tail. Subcutaneous injection was started from day 30 of modeling: the X1-X90 groups: once every three days; the positive drug control group (methotrexate at 1 mg/kg): once every 5 d, 3 times consecutively; and the normal control group and the model control group (normal saline):

continuous for 10 d. Every 3 days from day 21 to day 70 after modeling, the body weights were weighed, joint scores were made, and the diameters of left and right hind foot ankles were measured respectively to observe the effect of drugs on mouse CIA. On day 70, the mice were killed by dislocation.

Arthritis evaluation indexes are as follows: (1) Joint score: limbs: score on a scale of 0-4: 0 = no erythema or swelling; 1 = slight erythema or swelling, one of the foretoe/hind toe joints has erythema or swelling; 2 = erythema or swelling of more than one toe; 3 = paw swelling under ankle or wrist joints; and 4 = swelling of all paws including ankle joints. The four paws of the mice were scored respectively, with the highest score being 16 points. Every 3 days from day 21 to day 70 after modeling, joint scores were made, and results were recorded. (2) Measurement of ankle diameter: The diameters from medial to lateral of left and right ankles and ankle thicknesses of mice were measured with vernier calipers every 3 days before modeling and from day 21 to day 70 after modeling, and the results were recorded.

The experiment was repeated independently three times. The results were expressed as mean ± SD, and statistical T test was conducted. In the table, *P< 0.05 indicates significant difference, and ** P< 0.01 indicates extremely significant difference.

Table 18 In vivo immunoprotective effect of polypeptides X1-X90 on a mouse CIA animal model

| Group | Number | Dose (mg/kg) | Left and right paw swelling degree (mm) | Joint swelling degree (mm) | Clinical score |
|---|---|---|---|---|---|
| Normal control group | 10 | - | 0.18±0.07 | 0.16±0.05 | 0.00±0.00 |
| Model control group | 10 | - | 2.29±0.39 | 2.00±0.47 | 15.65±1.90 |
| Positive control group | 10 | 1 | 0.70±0.12** | 0.73±0.12** | 8.32±1.35** |
| X1 | 10 | 4.0 | 0.81 ± 0. 11** | 0.79 ± 0.15** | 9.17 ±1.44** |
| X2 | 10 | 4.0 | 0.71 ± 0.15** | 0.77 ± 0.18** | 9.18 ±1.41** |
| X3 | 10 | 4.0 | 0.72 ± 0.11** | 0.81 ± 0.17** | 8.88 ±1.25** |
| X4 | 10 | 4.0 | 0.76 ± 0.14** | 0.82 ± 0.11** | 8.91 ±1.43** |
| X5 | 10 | 4.0 | 0.85 ± 0.12** | 0.81 ± 0.17** | 8.89 ±1.28** |
| X6 | 10 | 4.0 | 0.71 ± 0.14** | 0.81 ± 0.11** | 8.77 ±1.26** |
| X7 | 10 | 4.0 | 0.79 ± 0.11** | 0.73 ± 0.14** | 8.79 ±1.23** |
| X8 | 10 | 4.0 | 0.74 ± 0.13** | 0.82 ± 0.17** | 8.99 ±1.31** |
| X9 | 10 | 4.0 | 0.71 ± 0.15** | 0.81 ± 0.12** | 8.91 ±1.25** |
| X10 | 10 | 4.0 | 0.82 ± 0.11** | 0.72 ± 0.18** | 8.83 ±1.36** |
| X11 | 10 | 4.0 | 0.78 ± 0.18** | 0.76 ± 0.17** | 8.99 ±1.43** |
| X12 | 10 | 4.0 | 0.74 ± 0.18** | 0.74 ± 0.19** | 8.84 ±1.41** |
| X13 | 10 | 4.0 | 0.79 ± 0.12** | 0.75 ± 0.15** | 8.94 ±1.26** |
| X14 | 10 | 4.0 | 0.84 ± 0.13** | 0.83 ± 0.11** | 8.97 ±1.36** |
| X15 | 10 | 4.0 | 0.83 ± 0.13** | 0.71 ± 0.11** | 8.84 ±1.42** |
| X16 | 10 | 4.0 | 0.70± 0.13** | 0.68 ± 0.18** | 8.32 ±1.27** |
| X17 | 10 | 4.0 | 0.78 ± 0.17** | 0.79 ± 0.12** | 9.13 ±1.31** |
| X18 | 10 | 4.0 | 0.79 ± 0.12** | 0.72 ± 0.11** | 8.91 ±1.29** |
| X19 | 10 | 4.0 | 0.82 ± 0.14** | 0.84 ± 0.18** | 8.72 ±1.41** |
| X20 | 10 | 4.0 | 0.74 ± 0.14** | 0.82 ± 0.17** | 9.07 ±1.33** |
| X21 | 10 | 4.0 | 0.83 ± 0.16** | 0.81 ± 0.14** | 8.79 ±1.43** |
| X22 | 10 | 4.0 | 0.83 ± 0.13** | 0.75 ± 0.14** | 8.67 ±1.32** |
| X23 | 10 | 4.0 | 0.77 ± 0.15** | 0.77 ± 0.17** | 9.15 ±1.47** |
| X24 | 10 | 4.0 | 0.71 ± 0.12** | 0.82 ± 0.11** | 8.94 ±1.41** |
| X25 | 10 | 4.0 | 0.74 ± 0.14** | 0.71 ± 0.18** | 9.21 ±1.33** |
| X26 | 10 | 4.0 | 0.84 ± 0.12** | 0.71 ± 0.14** | 9.07 ±1.32** |
| X27 | 10 | 4.0 | 0.75 ± 0.13** | 0.78 ± 0.19** | 8.75 ±1.43** |
| X28 | 10 | 4.0 | 0.73 ± 0.18** | 0.72 ± 0.18** | 8.83 ±1.26** |
| X29 | 10 | 4.0 | 0.71 ± 0.13** | 0.81 ± 0.15** | 8.98 ±1.44** |
| X30 | 10 | 4.0 | 0.79 ± 0.16** | 0.83 ± 0.16** | 8.73 ±1.29** |

(continued)

| Group | Number | Dose (mg/kg) | Left and right paw swelling degree (mm) | Joint swelling degree (mm) | Clinical score |
|---|---|---|---|---|---|
| X31 | 10 | 4.0 | 0.77 ± 0.15** | 0.78 ± 0.15** | 8.75 ±1.38** |
| X32 | 10 | 4.0 | 0.81 ± 0.13** | 0.81 ± 0.16** | 9.06 ±1.26** |
| X33 | 10 | 4.0 | 0.74 ± 0.17** | 0.82 ± 0.11** | 9.08 ±1.25** |
| X34 | 10 | 4.0 | 0.72 ± 0.15** | 0.84 ± 0.13** | 8.8 ±1.34** |
| X35 | 10 | 4.0 | 0.85 ± 0.14** | 0.76 ± 0.13** | 8.78 ±1.23** |
| X36 | 10 | 4.0 | 0.77 ± 0.13** | 0.74 ± 0.14** | 8.86 ±1.23** |
| X37 | 10 | 4.0 | 0.83 ± 0.16** | 0.8± 0.12** | 9.16 ±1.31** |
| X38 | 10 | 4.0 | 0.76 ± 0.13** | 0.71 ± 0.11** | 9.18 ±1.33** |
| X39 | 10 | 4.0 | 0.74 ± 0.19** | 0.81 ± 0.13** | 8.93 ±1.34** |
| X40 | 10 | 4.0 | 0.84 ± 0.12** | 0.83 ± 0.13** | 8.92 ±1.45** |
| X41 | 10 | 4.0 | 0.72 ± 0.12** | 0.79 ± 0.12** | 9.11 ±1.41** |
| X42 | 10 | 4.0 | 0.79 ± 0.17** | 0.76 ± 0.18** | 8.79 ±1.32** |
| X43 | 10 | 4.0 | 0.73 ± 0.18** | 0.71 ± 0.16** | 8.83 ±1.33** |
| X44 | 10 | 4.0 | 0.81 ± 0.16** | 0.73 ± 0.11** | 8.78 ±1.24** |
| X45 | 10 | 4.0 | 0.77 ± 0.17** | 0.75 ± 0.11** | 9.14 ±1.43** |
| X46 | 10 | 4.0 | 0.83 ± 0.11** | 0.77 ± 0.13** | 9.11 ±1.47** |
| X47 | 10 | 4.0 | 0.75 ± 0.18** | 0.77 ± 0.13** | 8.94 ±1.45** |
| X48 | 10 | 4.0 | 0.82 ± 0.13** | 0.74 ± 0.19** | 8.97 ±1.23** |
| X49 | 10 | 4.0 | 0.81 ± 0.19** | 0.84 ± 0.14** | 9.08 ±1.34** |
| X50 | 10 | 4.0 | 0.83 ± 0.12** | 0.74 ± 0.18** | 9.19 ±1.42** |
| X51 | 10 | 4.0 | 0.83 ± 0.16** | 0.8 1± 0.19** | 9.15 ±1.31** |
| X52 | 10 | 4.0 | 0.71 ± 0.16** | 0.79 ± 0.18** | 9.07 ±1.13** |
| X53 | 10 | 4.0 | 0.79 ± 0.13** | 0.78 ± 0.15** | 8.73 ±1.14** |
| X54 | 10 | 4.0 | 0.77 ± 0.18** | 0.73 ± 0.18** | 9.09 ±1.32** |
| X55 | 10 | 4.0 | 0.72 ± 0.18** | 0.84 ± 0.11** | 8.93 ±1.33** |
| X56 | 10 | 4.0 | 0.81 ± 0.18** | 0.73 ± 0.17** | 8.70±1.33** |
| X57 | 10 | 4.0 | 0.72 ± 0.13** | 0.81 ± 0.12** | 8.87 ±1.41** |
| X58 | 10 | 4.0 | 0.71 ± 0.15** | 0.83 ± 0.12** | 8.77 ±1.44** |
| X59 | 10 | 4.0 | 0.72 ± 0.15** | 0.85 ± 0.13** | 8.99 ±1.43** |
| X60 | 10 | 4.0 | 0.72 ± 0.16** | 0.85 ± 0.17** | 9.01 ±1.26** |
| X61 | 10 | 4.0 | 0.85 ± 0.13** | 0.72 ± 0.12** | 8.82 ±1.39** |
| X62 | 10 | 4.0 | 0.82 ± 0.12** | 0.78 ± 0.15** | 8.76 ±1.25** |
| X63 | 10 | 4.0 | 0.72 ± 0.14** | 0.72 ± 0.15** | 8.85 ±1.28** |
| X64 | 10 | 4.0 | 0.81 ± 0.12** | 0.84 ± 0.17** | 8.72 ±1.39** |
| X65 | 10 | 4.0 | 0.72 ± 0.17** | 0.8 2± 0.12** | 8.66 ±1.32** |
| X66 | 10 | 4.0 | 0.71 ± 0.13** | 0.84 ± 0.13** | 8.96 ±1.26** |
| X67 | 10 | 4.0 | 0.79 ± 0.10** | 0.71 ± 0.13** | 9.05 ±1.26** |
| X68 | 10 | 4.0 | 0.84 ± 0.11** | 0.81 ± 0.14** | 8.75 ±1.29** |
| X69 | 10 | 4.0 | 0.73 ± 0.15** | 0.78 ± 0.14** | 8.78 ±1.24** |
| X70 | 10 | 4.0 | 0.78 ± 0.18** | 0.73 ± 0.12** | 8.96 ±1.43** |
| X71 | 10 | 4.0 | 0.82 ± 0.14** | 0.77 ± 0.15** | 8.78 ±1.46** |
| X72 | 10 | 4.0 | 0.82 ± 0.13** | 0.74 ± 0.16** | 9.03 ±1.33** |
| X73 | 10 | 4.0 | 0.74 ± 0.12** | 0.72 ± 0.17** | 8.66 ±1.38** |
| X74 | 10 | 4.0 | 0.79 ± 0.17** | 0.7 ± 0.17** | 8.92 ±1.46** |
| X75 | 10 | 4.0 | 0.85 ± 0.13** | 0.77 ± 0.14** | 8.99 ±1.38** |
| X76 | 10 | 4.0 | 0.75 ± 0.14** | 0.85 ± 0.14** | 9.07 ±1.31** |
| X77 | 10 | 4.0 | 0.8 ± 0.18** | 0.74 ± 0.12** | 8.98 ±1.44** |
| X78 | 10 | 4.0 | 0.77 0.12** | 0.73 ± 0.19** | 8.71 ±1.35** |

(continued)

| Group | Number | Dose (mg/kg) | Left and right paw swelling degree (mm) | Joint swelling degree (mm) | Clinical score |
|---|---|---|---|---|---|
| X79 | 10 | 4.0 | 0.84 ± 0.18** | 0.78 ± 0.14** | 9.03 ±1.31** |
| X80 | 10 | 4.0 | 0.75 ± 0.12** | 0.79 ± 0.18** | 8.88 ±1.35** |
| X81 | 10 | 4.0 | 0.75 ± 0.11** | 0.78 ± 0.14** | 8.82 ±1.39** |
| X82 | 10 | 4.0 | 0.74 ± 0.14** | 0.75 ± 0.14** | 8.89 ±1.22** |
| X83 | 10 | 4.0 | 0.81± 0.15** | 0.83± 0.13** | 8.65 ±1.43** |
| X84 | 10 | 4.0 | 0.78 ± 0.17** | 0.79 ± 0.14** | 8.65 ±1.38** |
| X85 | 10 | 4.0 | 0.79 ± 0.12** | 0.73 ± 0.11** | 9.13 ±1.36** |
| X86 | 10 | 4.0 | 0.78 ± 0.17** | 0.72± 0.12** | 8.71 ±1.26** |
| X87 | 10 | 4.0 | 0.81 ± 0.16** | 0.77 ± 0.15** | 9.12 ±1.33** |
| X88 | 10 | 4.0 | 0.79 ± 0.14** | 0.72 ± 0.11** | 8.81 ±1.42** |
| X89 | 10 | 4.0 | 0.85 ± 0.13** | 0.76 ± 0.17** | 8.68 ±1.41** |
| X90 | 10 | 4.0 | 0.81 ± 0.11** | 0.73 ± 0.19** | 8.72 ±1.26** |

Results: Compared with normal mice, mice after modeling were intracutaneously injected in the tail with an emulsifier containing inactivated mycobacterium tuberculosis CFA and collagen in the same volume. After 21 days, mice were intracutaneously injected in the tail with an emulsifier containing IFA and collagen in the same volume. On day 27 after immunization, CIA mice's paw became red and swollen, and the arthritis index score increased. The swelling peak occurred on day 45 to 60 in the model group. The body weight of the model group hardly increased from day 35 and slightly decreased in the later period. Polypeptide X1-X90 could play an in vivo immunoprotective role in mouse CIA animal models. Compared with the model group, the positive control group and X1-X90 groups had extremely significant differences (p** < 0.01). The limb score of the polypeptide X16 group was significantly lower than that of the model control group, and had the most significant protective effect.

## Example 14

In vivo immunoprotective effect of X1-X90 on a rat adjuvant arthritis (AA) animal model

A rat AA animal model was established to study the therapeutic effects of X1-X90 on rats infected with AA. Rats were used as test animals. SPF SD mice, male, were used. They each weighed 140-160 g and were randomly divided into a normal control group, a model control group, X1-X90 groups and a positive drug control group (methotrexate at 1 mg/kg). Except the normal group, the rat AA model was established for each test group on day 0. The method was to inject 0.08 mL of CFA containing inactivated Mycobacterium tuberculosis (H37RA, 10 mg/mL) into the hind feet of the left sides of the rats to create the rat AA model. The drug was injected intravenously in the tail from day 10 of modeling: X1-X90: once every five days; the positive drug control group (methotrexate at 1 mg/kg): once every five days, 3 times consecutively; and the normal control group and the model control group (normal saline): continuous for ten days. On days 8, 11, 14, 17, 20, 23 and 26 after modeling, joint scores were made and the ankle diameters of left and right hind feet were measured respectively to observe the effect of drugs on rat AA

Arthritis evaluation indexes are as follows: (1) Joint score: limbs: score on a scale of 0-4: 0 = no erythema or swelling; 1 = slight erythema or swelling, one of the foretoe/hind toe joints has erythema or swelling; 2 = erythema or swelling of more than one toe; 3 = paw swelling under ankle or wrist joints; and 4 = swelling of all paws including ankle joints. The four paws of the rats were scored respectively, with the highest score being 16 points. Joint scores were made on days 8, 11, 14th, 17, 20, 23 and 26 after modeling, and results were recorded. (2) Measurement of ankle diameter: The diameters from medial to lateral of left and right ankles and ankle thicknesses of rats were measured with vernier calipers before modeling and on days 8, 11, 14, 17, 20, 23 and 26 after modeling, and the results were recorded. The experiment was repeated independently three times. The results were expressed as mean ± SD, and statistical T test was conducted. In the table, *P< 0.05 indicates significant difference, and ** P< 0.01 indicates extremely significant difference.

Table 19 In vivo immunoprotective effect of X1-X90 on a rat AA animal model

| Group | Number | Dose (mg/kg) | Left and right paw swelling degree (mm) | Joint swelling degree (mm) | Clinical score |
|---|---|---|---|---|---|
| Normal control group | 10 | - | 0.93±0.14 | 0.30±0.15 | 0.00±0.00 |
| Model control group | 10 | - | 6.98±1.27 | 3.74±0.72 | 13.86±1.65 |
| Positive control group | 10 | 1 | 3.29±0.39** | 0.64±0.21** | 5.06±1.07** |
| X1 | 10 | 4.0 | 3.94 ± 0.95** | 0.71 ± 0.14** | 5.87 ±1.04** |
| X2 | 10 | 4.0 | 3.78 ± 0.55** | 0.72 ± 0.12** | 5.57 ±1.08** |
| X3 | 10 | 4.0 | 3.86 ± 0.21** | 0.83 ± 0.15** | 5.69 ±1.07** |
| X4 | 10 | 4.0 | 3.91 ± 0.35** | 0.78 ± 0.19** | 5.76 ±1.1** |
| X5 | 10 | 4.0 | 3.90 ± 0.26** | 0.74 ± 0.12** | 5.73 ±1.1** |
| X6 | 10 | 4.0 | 3.79 ± 0.94** | 0.77 i 0.13** | 5.76 ±1.13** |
| X7 | 10 | 4.0 | 3.82 ± 0.22** | 0.82 ± 0.18** | 5.55 ±1.11** |
| X8 | 10 | 4.0 | 3.92 ± 0.79** | 0.75 i 0.18** | 5.55 ±1.06** |
| X9 | 10 | 4.0 | 3.95 ± 0.55** | 0.82 ± 0.15** | 5.57 ±1.16** |
| X10 | 10 | 4.0 | 3.74 ± 0.76** | 0.82 ± 0.11** | 5.61 ±1.07** |
| X11 | 10 | 4.0 | 3.88 ± 0.34** | 0.81 ± 0.14** | 5.54 ±1.06** |
| X12 | 10 | 4.0 | 3.87 ± 0.89** | 0.72 ± 0.17** | 5.85 ±1.04** |
| X13 | 10 | 4.0 | 3.91 ± 0.44** | 0.78 ± 0.16** | 5.78 ±1.15** |
| X14 | 10 | 4.0 | 3.94 ± 0.22** | 0.84 ± 0.15** | 5.60 ±1.15** |
| X15 | 10 | 4.0 | 3.76 ± 0.92** | 0.84 ± 0.14** | 5.6 1±1.16** |
| X16 | 10 | 4.0 | 3.75 ± 0.77** | 0.77± 0.11** | 5.8 ±1.09** |
| X17 | 10 | 4.0 | 3.84 ± 0.88** | 0.78 ± 0.15** | 5.82 ±1.05** |
| X18 | 10 | 4.0 | 3.81 ± 0.85** | 0.83 ± 0.15** | 5.67 ±1.04** |
| X19 | 10 | 4.0 | 3.74± 0.4** | 0.81 ± 0.16** | 5.63 ±1.04** |
| X20 | 10 | 4.0 | 3.87 0.95** | 0.8± 0.17** | 5.74 ±1.11** |
| X21 | 10 | 4.0 | 3.98 ± 0.17** | 0.82 ± 0.18** | 5.61 ±1.14** |
| X22 | 10 | 4.0 | 3.92 ± 0.18** | 0.83 ± 0.14** | 5.67 ±1.12** |
| X23 | 10 | 4.0 | 3.85 ± 0.29** | 0.73 ± 0.15** | 5.79 ±1.06** |
| X24 | 10 | 4.0 | 3.94 ± 0.70** | 0.76 ± 0.15** | 5.73 ±1.15** |
| X25 | 10 | 4.0 | 3.76 ± 0.09** | 0.71 ± 0.16** | 5.72 ±1.11** |
| X26 | 10 | 4.0 | 3.81 ± 0.59** | 0.74 ± 0.18** | 5.65 ±1.07** |
| X27 | 10 | 4.0 | 3.86 ± 0.85** | 0.74 ± 0.17** | 5.63 ±1.12** |
| X28 | 10 | 4.0 | 3.98 ± 0.12** | 0.75 ± 0.16** | 5.79 ±1.15** |
| X29 | 10 | 4.0 | 3.72 ± 0.80** | 0.84 ± 0.16** | 5.72 ±1.06** |
| X30 | 10 | 4.0 | 3.76 ± 0.64** | 0.82 ± 0.12** | 5.73 ±1.05** |
| X31 | 10 | 4.0 | 3.72 ± 0.98** | 0.84 ± 0.12** | 5.74 ±1.12** |
| X32 | 10 | 4.0 | 3.91 ± 0.64** | 0.81 ± 0.18** | 5.81 ±1.14** |
| X33 | 10 | 4.0 | 3.93 ± 0.08** | 0.76 ± 0.15** | 5.62 ±1.08** |
| X34 | 10 | 4.0 | 3.84 ± 0.88** | 0.83 ± 0.17** | 5.66 ±1.09** |
| X35 | 10 | 4.0 | 3.78 ± 0.56** | 0.85 ± 0.13** | 5.83 ±1.07** |
| X36 | 10 | 4.0 | 4.00 ± 0.44** | 0.81 ± 0.12** | 5.71 ±1.09** |
| X37 | 10 | 4.0 | 3.92 ± 0.36** | 0.73 ± 0.16** | 5.75 ±1.14** |
| X38 | 10 | 4.0 | 3.95 ± 0.09** | 0.82 ± 0.14** | 5.87 ±1.13** |
| X39 | 10 | 4.0 | 3.78 ± 0.22** | 0.83 ± 0.17** | 5.82 ±1.05** |
| X40 | 10 | 4.0 | 3.71 ± 0.15** | 0.75 ± 0.17** | 5.54 ±1.16** |
| X41 | 10 | 4.0 | 3.87 ± 0.86** | 0.85 ± 0.15** | 5.57 ±1.12** |
| X42 | 10 | 4.0 | 3.86 ± 0.56** | 0.85 ± 0.17** | 5.84 ±1.16** |
| X43 | 10 | 4.0 | 3.87 ± 0.26** | 0.81 ± 0.19** | 5.61 ±1.03** |

(continued)

| Group | Number | Dose (mg/kg) | Left and right paw swelling degree (mm) | Joint swelling degree (mm) | Clinical score |
|---|---|---|---|---|---|
| X44 | 10 | 4.0 | 3.82 ± 0.37** | 0.72 ± 0.15** | 5.78 ±1.09** |
| X45 | 10 | 4.0 | 3.90 ± 0.75** | 0.83 ± 0.12** | 5.85 ±1.06** |
| X46 | 10 | 4.0 | 3.78 ± 0.46** | 0.83 ± 0.17** | 5.81 ±1.05** |
| X47 | 10 | 4.0 | 3.86 ± 0.07** | 0.85 ± 0.17** | 5.82 ±1.05** |
| X48 | 10 | 4.0 | 3.85 ± 0.98** | 0.72 ± 0.18** | 5.72 ±1.03** |
| X49 | 10 | 4.0 | 3.82 ± 0.45** | 0.83 ± 0.16** | 5.58 ±1.12** |
| X50 | 10 | 4.0 | 3.75 ± 0.47** | 0.73 ± 0.11** | 5.72 ±1.15** |
| X51 | 10 | 4.0 | 3.96 ± 0.31** | 0.71 ± 0.17** | 5.73 ±1.06** |
| X52 | 10 | 4.0 | 3.98 ± 0.10** | 0.75 ± 0.16** | 5.64 ±1.12** |
| X53 | 10 | 4.0 | 3.92 ± 0.62** | 0.78 ± 0.13** | 5.58 ±1.09** |
| X54 | 10 | 4.0 | 3.85 ± 0.28** | 0.83 ± 0.15** | 5.66 ±1.04** |
| X55 | 10 | 4.0 | 3.89 ± 0.38** | 0.82 ± 0.14** | 5.73 ±1.15** |
| X56 | 10 | 4.0 | 3.72 ± 0.63** | 0.76 ± 0.18** | 5.54 ±1.05** |
| X57 | 10 | 4.0 | 3.79 ± 0.39** | 0.77 ± 0.11** | 5.85 ±1.09** |
| X58 | 10 | 4.0 | 3.79 ± 0.48** | 0.77 ± 0.17** | 5.62 ±1.15** |
| X59 | 10 | 4.0 | 3.93 ± 0.6** | 0.74 ± 0.16** | 5.86 ±1.06** |
| X60 | 10 | 4.0 | 3.75 ± 0.33** | 0.79 ± 0.15** | 5.58 ±1.09** |
| X61 | 10 | 4.0 | 3.87 ± 0.49** | 0.78 ± 0.12** | 5.76 ±1.13** |
| X62 | 10 | 4.0 | 3.70 ± 0.23** | 0.82 ± 0.12** | 5.61 ±1.12** |
| X63 | 10 | 4.0 | 3.74 ± 0.58** | 0.75 ± 0.13** | 5.82 ±1.07** |
| X64 | 10 | 4.0 | 3.76 ± 0.45** | 0.84 ± 0.15** | 5.89 ± 1.12** |
| X65 | 10 | 4.0 | 3.99 ± 0.06** | 0.82 ± 0.18** | 5.68 ± 1.09** |
| X66 | 10 | 4.0 | 3.86 ± 0.49** | 0.81 ± 0.11** | 5.73 ±1.04** |
| X67 | 10 | 4.0 | 3.84 ± 0.34** | 0.72 ± 0.12** | 5.75 ±1.08** |
| X68 | 10 | 4.0 | 3.88 ± 0.32** | 0.79 ± 0.16** | 5.82 ±1.11** |
| X69 | 10 | 4.0 | 3.70 ± 0.12** | 0.75 ± 0.13** | 5.83 ±1.05** |
| X70 | 10 | 4.0 | 3.81 ± 0.14** | 0.73 ± 0.15** | 5.74 ± 1.06** |
| X71 | 10 | 4.0 | 3.95 ± 0.57** | 0.85 ± 0.11** | 5.55 ± 1.04** |
| X72 | 10 | 4.0 | 3.83 ± 0.92** | 0.84 ± 0.18** | 5.63 ±1.08** |
| X73 | 10 | 4.0 | 3.72 ± 0.79** | 0.84 ± 0.17** | 5.78 ± 1.05** |
| X74 | 10 | 4.0 | 3.9 ± 0.09** | 0.82 ± 0.17** | 5.86 ± 1.04** |
| X75 | 10 | 4.0 | 3.94 ± 0.91** | 0.79 ± 0.11** | 5.73 ± 1.11** |
| X76 | 10 | 4.0 | 3.67 ± 0.36** | 0.70 ± 0.16** | 5.51 ±1.13** |
| X77 | 10 | 4.0 | 3.71 ± 0.7** | 0.78 ± 0.12** | 5.57 ±1.1** |
| X78 | 10 | 4.0 | 3.73 ± 0.42** | 0.83 ± 0.16** | 5.77 ± 1.05** |
| X79 | 10 | 4.0 | 3.71 ± 0.83** | 0.85 ± 0.16** | 5.74 ±1.03** |
| X80 | 10 | 4.0 | 3.95 ± 0.19** | 0.72 ± 0.18** | 5.56 ± 1.12** |
| X81 | 10 | 4.0 | 3.82 ± 0.66** | 0.71 ± 0.14** | 5.55 ± 1.09** |
| X82 | 10 | 4.0 | 3.93 ± 0.16** | 0.81 ± 0.19** | 5.75 ± 1.11** |
| X83 | 10 | 4.0 | 3.81 ± 0.79** | 0.74 ± 0.18** | 5.67 ± 1.06** |
| X84 | 10 | 4.0 | 3.84 ± 0.27** | 0.72 ± 0.15** | 5.83 ±1.15** |
| X85 | 10 | 4.0 | 3.76 ± 0.43** | 0.73 ± 0.13** | 5.85 ±1.03** |
| X86 | 10 | 4.0 | 3.91 ± 0.69** | 0.77 ± 0.13** | 5.79 ± 1.12** |
| X87 | 10 | 4.0 | 3.99 ± 0.21** | 0.77 ± 0.11** | 5.82 ± 1.06** |
| X88 | 10 | 4.0 | 3.88 ± 0.55** | 0.77 ± 0.11** | 5.81 ± 1.09** |
| X89 | 10 | 4.0 | 3.76 ± 0.15** | 0.82 ± 0.18** | 5.84 ±1.14** |
| X90 | 10 | 4.0 | 3.95 ± 0.78** | 0.73 ± 0.18** | 5.61 ±1.07** |

Results: Compared with normal rats, the rats after modeling were injected in the left hind feet with inactivated mycobacterium tuberculosis CFA, and then the left hind feet were rapidly subjected to primary arthritis with obvious swelling and ulceration. Secondary arthritis began to appear in the right hind feet about 10 days later, and the scores gradually increased. At the same time, the ear vascular proliferation was obvious, and swelling was obvious; and swelling appeared in the tail joint. Compared with the model group, X1-X90 groups with different molecular weights could exert in vivo immunoprotective effect on rat AA animal models, and X76 had the most significant effect.

**Example 15**

Effect of X1-X90 on acute inflammation of carrageenan-induced toe swelling in rats

SD rats were taken and divided into a blank model group, a Dex positive group (5 mg/kg) and X1-X90 test groups. The rats were injected once a day, and the model group was given the same volume of normal saline for 3 d and fed normally. One hour after the last administration, 0.1 mL of 1% carrageenan was injected subcutaneously into the right hind soles of rats to induce inflammation, and the foot volume was measured at 1 h, 3 h, 5 h and 7 h after inflammation. The foot swelling degree was calculated according to the following formula: foot swelling degree (mL) = foot volume after inflammation-foot volume before inflammation. The number of milliliters of overflow liquid was recorded (method: a ballpoint pen is used to circle the protruding point of the right joint as a measurement mark, and the right hind feet of each rat are sequentially placed into the volume measurer, so that the hind limbs are exposed outside the barrel, and the immersion depth is at the coincidence of the circle and the liquid level. After the foot enters the liquid, the liquid level rises, and the volume of overflow liquid is the volume of the right hind foot of the rat, and the normal volume of the right hind foot of each rat is measured in sequence).

Table 20 Effect of X1-X90 on acute inflammation of carrageenan-induced toe swelling in rats

| Group | Dose (mg/kg) | Swelling degree (mg) | | | |
|---|---|---|---|---|---|
| | | 1 h | 3 h | 5 h | 7 h |
| X1 | 2.0 | 0.31±0.16* | 0.39±0.15 | 0.42±0.13* | 0.35±0.18* |
| | 4.0 | 0.25±0.14* | 0.33±0.17 | 0.41±0.13* | 0.33±0.18* |
| X2 | 2.0 | 0.28±0.12* | 0.38±0.17 | 0.43±0.13* | 0.36±0.11* |
| | 4.0 | 0.27±0.13* | 0.36±0.15 | 0.39±0.12* | 0.35±0.11* |
| X3 | 2.0 | 0.30±0.19* | 0.38±0.16 | 0.45±0.17* | 0.38±0.13* |
| | 4.0 | 0.31±0.11* | 0.35±0.12 | 0.43±0.18* | 0.36±0.17* |
| X4 | 2.0 | 0.28±0.11* | 0.38±0.16 | 0.41±0.17* | 0.33±0.12* |
| | 4.0 | 0.27±0.17* | 0.37±0.13 | 0.40±0.14* | 0.31±0.11* |
| X5 | 2.0 | 0.29±0.16* | 0.34±0.11 | 0.41±0.14* | 0.32±0.12* |
| | 4.0 | 0.23±0.18* | 0.30±0.19 | 0.34±0.17* | 0.30±0.16* |
| X6 | 2.0 | 0.29±0.13* | 0.33±0.15 | 0.35±0.19* | 0.34±0.11* |
| | 4.0 | 0.28±0.12* | 0.31±0.16 | 0.34±0.11* | 0.32±0.13* |
| X7 | 2.0 | 0.28±0.16* | 0.37±0.14 | 0.37±0.18* | 0.37±0.13* |
| | 4.0 | 0.27±0.13* | 0.33±0.16 | 0.35±0.18* | 0.36±0.11* |
| X8 | 2.0 | 0.29±0.14* | 0.38±0.12 | 0.43±0.17* | 0.35±0.16* |
| | 4.0 | 0.26±0.11* | 0.36±0.14 | 0.41±0.17* | 0.34±0.16* |
| X9 | 2.0 | 0.30±0.14* | 0.33±0.16 | 0.42±0.15* | 0.39±0.13* |
| | 4.0 | 0.29±0.14* | 0.32±0.16 | 0.38±0.13* | 0.34±0.19* |
| X10 | 2.0 | 0.28±0.11* | 0.37±0.17 | 0.39±0.18* | 0.36±0.14* |
| | 4.0 | 0.25±0.18* | 0.36±0.12 | 0.37±0.13* | 0.33±0.16* |
| X11 | 2.0 | 0.23±0.13* | 0.33±0.15 | 0.39±0.12* | 0.35±0.11* |
| | 4.0 | 0.22±0.16* | 0.32±0.14 | 0.38±0.18* | 0.34±0.11* |
| X12 | 2.0 | 0.25±0.13* | 0.32±0.12 | 0.38±0.16* | 0.34±0.19* |
| | 4.0 | 0.22±0.12* | 0.30±0.12 | 0.37±0.17* | 0.33±0.19* |
| X13 | 2.0 | 0.27±0.15* | 0.36±0.12 | 0.42±0.18* | 0.33±0.14* |
| | 4.0 | 0.25±0.12* | 0.35±0.14 | 0.41±0.18* | 0.32±0.15* |
| X14 | 2.0 | 0.26±0.14* | 0.35±0.15 | 0.41±0.19* | 0.35±0.11* |

(continued)

| Group | Dose (mg/kg) | Swelling degree (mg) | | | |
|---|---|---|---|---|---|
| | | 1 h | 3 h | 5 h | 7 h |
| | 4.0 | 0.25±0.12* | 0.33±0.1 | 0.37±0.14* | 0.34±0.11* |
| X15 | 2.0 | 0.32±0.15* | 0.37±0.18 | 0.41±0.12* | 0.33±0.14* |
| | 4.0 | 0.26±0.12* | 0.31±0.18 | 0.36±0.19* | 0.32±0.15* |
| X16 | 2.0 | 0.29±0.17* | 0.34±0.16 | 0.42±0.12* | 0.33±0.15* |
| | 4.0 | 0.28±0.11* | 0.32±0.16 | 0.41±0.13* | 0.32±0.17* |
| X17 | 2.0 | 0.27±0.15* | 0.32±0.14 | 0.38±0.17* | 0.35±0.19* |
| | 4.0 | 0.24±0.12* | 0.31±0.19 | 0.37±0.17* | 0.34±0.15* |
| X18 | 2.0 | 0.23±0.18* | 0.33±0.16 | 0.36±0.15* | 0.32±0.13* |
| | 4.0 | 0.24±0.17* | 0.39±0.18 | 0.45±0.15* | 0.33±0.12* |
| X19 | 2.0 | 0.21±0.13* | 0.33±0.15 | 0.36±0.17* | 0.32±0.12* |
| | 4.0 | 0.26±0.11* | 0.38±0.17 | 0.42±0.15* | 0.32±0.18* |
| X20 | 2.0 | 0.25±0.18* | 0.34±0.13 | 0.36±0.13* | 0.37±0.12* |
| | 4.0 | 0.24±0.15* | 0.34±0.12 | 0.35±0.16* | 0.35±0.12* |
| X21 | 2.0 | 0.23±0.19* | 0.32±0.17 | 0.39±0.16* | 0.33±0.12* |
| | 4.0 | 0.25±0.17* | 0.33±0.13 | 0.39±0.14* | 0.35±0.18* |
| X22 | 2.0 | 0.24±0.13* | 0.31±0.15 | 0.42±0.14* | 0.38±0.18* |
| | 4.0 | 0.23±0.11* | 0.31±0.16 | 0.36±0.13* | 0.37±0.13* |
| X23 | 2.0 | 0.28±0.17* | 0.34±0.19 | 0.37±0.11* | 0.35±0.18* |
| | 4.0 | 0.26±0.16* | 0.34±0.19 | 0.38±0.13* | 0.34±0.17* |
| X24 | 2.0 | 0.24±0.16* | 0.34±0.18 | 0.43±0.12* | 0.38±0.13* |
| | 4.0 | 0.23±0.17* | 0.35±0.13 | 0.38±0.16* | 0.32±0.12* |
| X25 | 2.0 | 0.29±0.17* | 0.38±0.12 | 0.46±0.18* | 0.34±0.13* |
| | 4.0 | 0.27±0.15* | 0.36±0.19 | 0.44±0.18* | 0.33±0.12* |
| X26 | 2.0 | 0.28±0.17* | 0.31±0.18 | 0.42±0.11* | 0.39±0.12* |
| | 4.0 | 0.25±0.18* | 0.31±0.13 | 0.40±0.15* | 0.30±0.16* |
| X27 | 2.0 | 0.27±0.13* | 0.33±0.14 | 0.39±0.17* | 0.36±0.16* |
| | 4.0 | 0.24±0.12* | 0.33±0.16 | 0.32±0.17* | 0.34±0.14* |
| X28 | 2.0 | 0.29±0.13* | 0.35±0.15 | 0.45±0.17* | 0.36±0.14* |
| | 4.0 | 0.28±0.15* | 0.34±0.11 | 0.37±0.17* | 0.35±0.18* |
| X29 | 2.0 | 0.27±0.16* | 0.35±0.18 | 0.36±0.19* | 0.33±0.17* |
| | 4.0 | 0.22±0.12* | 0.34±0.15 | 0.35±0.13* | 0.32±0.17* |
| X30 | 2.0 | 0.26±0.15* | 0.36±0.18 | 0.40±0.16* | 0.35±0.11* |
| | 4.0 | 0.25±0.19* | 0.33±0.13 | 0.39±0.18* | 0.34±0.15* |
| X31 | 2.0 | 0.23±0.18* | 0.37±0.12 | 0.38±0.16* | 0.33±0.11* |
| | 4.0 | 0.22±0.12* | 0.36±0.16 | 0.38±0.15* | 0.35±0.14* |
| X32 | 2.0 | 0.25±0.18* | 0.35±0.13 | 0.36±0.12* | 0.31±0.17* |
| | 4.0 | 0.24±0.14* | 0.33±0.17 | 0.41±0.15* | 0.35±0.12* |
| X33 | 2.0 | 0.22±0.13* | 0.37±0.11 | 0.42±0.17* | 0.35±0.13* |
| | 4.0 | 0.25±0.14* | 0.31±0.13 | 0.44±0.19* | 0.33±0.18* |
| X34 | 2.0 | 0.26±0.18* | 0.38±0.13 | 0.39±0.11* | 0.35±0.14* |
| | 4.0 | 0.22±0.18* | 0.38±0.15 | 0.36±0.16* | 0.34±0.13* |
| X35 | 2.0 | 0.25±0.11* | 0.38±0.16 | 0.38±0.19* | 0.33±0.15* |
| | 4.0 | 0.23±0.11* | 0.36±0.16 | 0.44±0.14* | 0.32±0.12* |
| X36 | 2.0 | 0.29±0.17* | 0.34±0.18 | 0.43±0.13* | 0.32±0.15* |
| | 4.0 | 0.23±0.15* | 0.33±0.13 | 0.35±0.12* | 0.32±0.17* |
| X37 | 2.0 | 0.24±0.17* | 0.38±0.12 | 0.41±0.11* | 0.33±0.16* |
| | 4.0 | 0.21±0.13* | 0.33±0.16 | 0.37±0.18* | 0.32±0.19* |
| X38 | 2.0 | 0.27±0.17* | 0.32±0.14 | 0.42±0.16* | 0.38±0.14* |

(continued)

| Group | Dose (mg/kg) | Swelling degree (mg) | | | |
|---|---|---|---|---|---|
| | | 1 h | 3 h | 5 h | 7 h |
| | 4.0 | 0.26±0.18* | 0.31±0.13 | 0.41±0.16* | 0.31±0.17* |
| X39 | 2.0 | 0.27±0.15* | 0.37±0.11 | 0.46±0.16* | 0.34±0.18* |
| | 4.0 | 0.22±0.18* | 0.36±0.11 | 0.42±0.19* | 0.31±0.13* |
| X40 | 2.0 | 0.27±0.14* | 0.37±0.14 | 0.38±0.12* | 0.35±0.13* |
| | 4.0 | 0.24±0.15* | 0.36±0.14 | 0.37±0.13* | 0.34±0.18* |
| X41 | 2.0 | 0.28±0.17* | 0.33±0.12 | 0.37±0.11* | 0.35±0.18* |
| | 4.0 | 0.26±0.11* | 0.31±0.15 | 0.43±0.14* | 0.34±0.15* |
| X42 | 2.0 | 0.24±0.17* | 0.39±0.12 | 0.41±0.18* | 0.36±0.18* |
| | 4.0 | 0.21±0.19* | 0.38±0.14 | 0.40±0.15* | 0.35±0.13* |
| X43 | 2.0 | 0.29±0.15* | 0.38±0.13 | 0.44±0.17* | 0.36±0.12* |
| | 4.0 | 0.25±0.18* | 0.35±0.11 | 0.42±0.13* | 0.33±0.16* |
| X44 | 2.0 | 0.21±0.12* | 0.32±0.14 | 0.41±0.15* | 0.37±0.13* |
| | 4.0 | 0.3±0.15* | 0.31±0.14 | 0.40±0.18* | 0.36±0.16* |
| X45 | 2.0 | 0.24±0.13* | 0.35±0.17 | 0.42±0.12* | 0.37±0.12* |
| | 4.0 | 0.21±0.16* | 0.34±0.17 | 0.41±0.11* | 0.31±0.14* |
| X46 | 2.0 | 0.27±0.12* | 0.38±0.15 | 0.45±0.17* | 0.35±0.19* |
| | 4.0 | 0.24±0.18* | 0.36±0.13 | 0.44±0.15* | 0.31±0.12* |
| X47 | 2.0 | 0.27±0.18* | 0.34±0.15 | 0.41±0.12* | 0.32±0.14* |
| | 4.0 | 0.26±0.16* | 0.33±0.12 | 0.39±0.18* | 0.31±0.15* |
| X48 | 2.0 | 0.29±0.12* | 0.37±0.16 | 0.36±0.15* | 0.32±0.17* |
| | 4.0 | 0.29±0.12* | 0.32±0.19 | 0.35±0.16* | 0.31±0.16* |
| X49 | 2.0 | 0.24±0.16* | 0.37±0.12 | 0.43±0.15* | 0.36±0.11* |
| | 4.0 | 0.24±0.13* | 0.31±0.12 | 0.42±0.14* | 0.33±0.15* |
| X50 | 2.0 | 0.28±0.15* | 0.34±0.15 | 0.42±0.16* | 0.36±0.14* |
| | 4.0 | 0.27±0.17* | 0.31±0.11 | 0.36±0.15* | 0.34±0.18* |
| X51 | 2.0 | 0.29±0.17* | 0.33±0.11 | 0.36±0.17* | 0.34±0.15* |
| | 4.0 | 0.23±0.15* | 0.31±0.18 | 0.34±0.18* | 0.33±0.18* |
| X52 | 2.0 | 0.27±0.12* | 0.39±0.13 | 0.43±0.17* | 0.37±0.17* |
| | 4.0 | 0.23±0.17* | 0.35±0.11 | 0.41±0.18* | 0.36±0.11* |
| X53 | 2.0 | 0.32±0.11* | 0.36±0.12 | 0.41±0.19* | 0.36±0.13* |
| | 4.0 | 0.25±0.18* | 0.33±0.12 | 0.38±0.15* | 0.34±0.14* |
| X54 | 2.0 | 0.25±0.11* | 0.35±0.15 | 0.39±0.17* | 0.35±0.17* |
| | 4.0 | 0.24±0.16* | 0.34±0.18 | 0.38±0.13* | 0.32±0.17* |
| X55 | 2.0 | 0.28±0.12* | 0.41±0.12 | 0.44±0.17* | 0.36±0.17* |
| | 4.0 | 0.27±0.19* | 0.32±0.14 | 0.42±0.14* | 0.35±0.18* |
| X56 | 2.0 | 0.24±0.12* | 0.34±0.14 | 0.39±0.11* | 0.32±0.14* |
| | 4.0 | 0.24±0.11* | 0.32±0.12 | 0.35±0.13* | 0.24±0.11* |
| X57 | 2.0 | 0.24±0.16* | 0.31±0.16 | 0.37±0.15* | 0.35±0.12* |
| | 4.0 | 0.27±0.12* | 0.33±0.12 | 0.35±0.12* | 0.32±0.12* |
| X58 | 2.0 | 0.26±0.15* | 0.36±0.18 | 0.42±0.11* | 0.35±0.16* |
| | 4.0 | 0.23±0.12* | 0.35±0.12 | 0.41±0.18* | 0.34±0.15* |
| X59 | 2.0 | 0.26±0.13* | 0.35±0.16 | 0.37±0.14* | 0.36±0.19* |
| | 4.0 | 0.25±0.12* | 0.34±0.17 | 0.36±0.13* | 0.32±0.13* |
| X60 | 2.0 | 0.26±0.11* | 0.36±0.12 | 0.39±0.12* | 0.33±0.12* |
| | 4.0 | 0.25±0.15* | 0.35±0.17 | 0.39±0.11* | 0.32±0.17* |
| X61 | 2.0 | 0.27±0.13* | 0.38±0.11 | 0.39±0.16* | 0.37±0.15* |
| | 4.0 | 0.26±0.16* | 0.35±0.11 | 0.36±0.13* | 0.36±0.17* |
| X62 | 2.0 | 0.21±0.13* | 0.32±0.16 | 0.41±0.11* | 0.34±0.12* |

(continued)

| Group | Dose (mg/kg) | Swelling degree (mg) | | | |
|---|---|---|---|---|---|
| | | 1 h | 3 h | 5 h | 7 h |
| | 4.0 | 0.27±0.11* | 0.37±0.18 | 0.37±0.15* | 0.31±0.11* |
| X63 | 2.0 | 0.27±0.16* | 0.38±0.13 | 0.41±0.18* | 0.33±0.16* |
| | 4.0 | 0.26±0.17* | 0.32±0.17 | 0.40±0.19* | 0.31±0.16* |
| X64 | 2.0 | 0.25±0.17* | 0.35±0.11 | 0.43±0.13* | 0.36±0.14* |
| | 4.0 | 0.25±0.13* | 0.34±0.12 | 0.38±0.16* | 0.31±0.11* |
| X65 | 2.0 | 0.27±0.15* | 0.38±0.16 | 0.43±0.13* | 0.36±0.11* |
| | 4.0 | 0.25±0.11* | 0.35±0.11 | 0.42±0.14* | 0.33±0.15* |
| X66 | 2.0 | 0.29±0.13* | 0.37±0.18 | 0.38±0.13* | 0.32±0.18* |
| | 4.0 | 0.22±0.11* | 0.34±0.14 | 0.38±0.19* | 0.32±0.14* |
| X67 | 2.0 | 0.23±0.13* | 0.34±0.19 | 0.42±0.17* | 0.38±0.16* |
| | 4.0 | 0.23±0.18* | 0.31±0.18 | 0.38±0.15* | 0.33±0.15* |
| X68 | 2.0 | 0.24±0.15* | 0.37±0.13 | 0.44±0.12* | 0.31±0.12* |
| | 4.0 | 0.23±0.18* | 0.33±0.14 | 0.38±0.18* | 0.35±0.18* |
| X69 | 2.0 | 0.28±0.11* | 0.35±0.15 | 0.41±0.18* | 0.37±0.14* |
| | 4.0 | 0.25±0.12* | 0.34±0.13 | 0.40±0.14* | 0.36±0.12* |
| X70 | 2.0 | 0.21±0.18* | 0.31±0.15 | 0.41±0.12* | 0.34±0.15* |
| | 4.0 | 0.23±0.13* | 0.32±0.16 | 0.42±0.12* | 0.34±0.17* |
| X71 | 2.0 | 0.25±0.11* | 0.31±0.12 | 0.38±0.14* | 0.33±0.11* |
| | 4.0 | 0.24±0.12* | 0.30±0.18 | 0.34±0.12* | 0.32±0.12* |
| X72 | 2.0 | 0.26±0.15* | 0.37±0.18 | 0.43±0.13* | 0.35±0.12* |
| | 4.0 | 0.25±0.17* | 0.35±0.17 | 0.42±0.16* | 0.31±0.12* |
| X73 | 2.0 | 0.26±0.12* | 0.36±0.12 | 0.43±0.11* | 0.34±0.13* |
| | 4.0 | 0.21±0.14* | 0.35±0.12 | 0.39±0.17* | 0.32±0.19* |
| X74 | 2.0 | 0.28±0.16* | 0.32±0.13 | 0.39±0.18* | 0.38±0.14* |
| | 4.0 | 0.26±0.14* | 0.33±0.15 | 0.38±0.16* | 0.34±0.17* |
| X75 | 2.0 | 0.27±0.12* | 0.39±0.12 | 0.41±0.11* | 0.35±0.18* |
| | 4.0 | 0.26±0.16* | 0.35±0.15 | 0.39±0.19* | 0.34±0.18* |
| X76 | 2.0 | 0.27±0.18* | 0.38±0.19 | 0.42±0.12* | 0.32±0.14* |
| | 4.0 | 0.24±0.18* | 0.33±0.18 | 0.38±0.16* | 0.34±0.14* |
| X77 | 2.0 | 0.25±0.13* | 0.37±0.13 | 0.43±0.12* | 0.32±0.17* |
| | 4.0 | 0.23±0.12* | 0.36±0.18 | 0.39±0.16* | 0.35±0.13* |
| X78 | 2.0 | 0.26±0.11* | 0.31±0.11 | 0.44±0.17* | 0.36±0.16* |
| | 4.0 | 0.27±0.16* | 0.38±0.11 | 0.43±0.19* | 0.35±0.13* |
| X79 | 2.0 | 0.29±0.14* | 0.35±0.11 | 0.38±0.13* | 0.39±0.12* |
| | 4.0 | 0.22±0.16* | 0.33±0.19 | 0.36±0.19* | 0.37±0.11* |
| X80 | 2.0 | 0.22±0.11* | 0.31±0.15 | 0.44±0.17* | 0.33±0.19* |
| | 4.0 | 0.23±0.11* | 0.32±0.12 | 0.41±0.13* | 0.35±0.19* |
| X81 | 2.0 | 0.24±0.18* | 0.35±0.13 | 0.42±0.11* | 0.36±0.19* |
| | 4.0 | 0.22±0.13* | 0.34±0.15 | 0.36±0.16* | 0.33±0.13* |
| X82 | 2.0 | 0.25±0.16* | 0.33±0.12 | 0.38±0.17* | 0.34±0.19* |
| | 4.0 | 0.24±0.14* | 0.39±0.13 | 0.43±0.18* | 0.33±0.18* |
| X83 | 2.0 | 0.26±0.11* | 0.34±0.14 | 0.44±0.18* | 0.35±0.15* |
| | 4.0 | 0.24±0.11* | 0.36±0.11 | 0.44±0.13* | 0.35±0.14* |
| X84 | 2.0 | 0.26±0.12* | 0.34±0.16 | 0.38±0.11* | 0.34±0.11* |
| | 4.0 | 0.23±0.13* | 0.33±0.15 | 0.37±0.11* | 0.32±0.14* |
| X85 | 2.0 | 0.24±0.11* | 0.34±0.13 | 0.41±0.14* | 0.39±0.13* |
| | 4.0 | 0.23±0.11* | 0.32±0.13 | 0.40±0.16* | 0.32±0.16* |
| X86 | 2.0 | 0.29±0.13* | 0.31±0.11 | 0.45±0.17* | 0.34±0.13* |

(continued)

| Group | Dose (mg/kg) | Swelling degree (mg) | | | |
|---|---|---|---|---|---|
| | | 1 h | 3 h | 5 h | 7 h |
| | 4.0 | 0.25±0.14* | 0.30±0.13 | 0.41±0.17* | 0.31±0.12* |
| X87 | 2.0 | 0.26±0.16* | 0.39±0.16 | 0.43±0.19* | 0.36±0.14* |
| | 4.0 | 0.25±0.16* | 0.31±0.12 | 0.41±0.13* | 0.34±0.19* |
| X88 | 2.0 | 0.35±0.14* | 0.32±0.19 | 0.42±0.14* | 0.35±0.15* |
| | 4.0 | 0.26±0.18* | 0.31±0.11 | 0.41±0.18* | 0.34±0.16* |
| X89 | 2.0 | 0.23±0.12* | 0.36±0.16 | 0.42±0.18* | 0.33±0.17* |
| | 4.0 | 0.22±0.17* | 0.35±0.17 | 0.38±0.17* | 0.32±0.15* |
| X90 | 2.0 | 0.27±0.11* | 0.34±0.17 | 0.42±0.16* | 0.33±0.12* |
| | 4.0 | 0.26±0.12* | 0.33±0.14 | 0.39±0.16* | 0.32±0.16* |
| Dex | 10 | 0.22±0.10** | 0.24±0.11** | 0.29±0.11** | 0.23±0.08* |
| control | - | 0.26±0.18 | 0.45±0.19 | 0.56±0.05 | 0.39±0.20 |

Results: the hind toes of rats in each group swelled rapidly after modeling, reaching the peak of swelling at about 3-5 h, and the swelling began to fade at 7 h. Polypeptide X1-X90 groups could have obvious inhibitory effects on carrageenan-induced toe swelling in rats, and the effect of a high-dose group had a better effect than a low-dose group, among which X5 had the most significant effect when the dose was 2.0 mg/kg.

**Example 16**

Proliferation inhibition effect of polypeptides X1-X90 on human retinal capillary endothelial cells (HRCECs)

An MTT method was used to detect the inhibitory activity of the angiogenesis inhibitor polypeptide on proliferation of HRCECs. HRCECs were digested and collected with trypsin after being cultured in an incubator at 37°C with 5% $CO_2$ to a density of 90% or more. The cells were resuspended with a culture solution and counted under a microscope, the cell concentration was adjusted to $3.0 \times 10^4$ cells/mL, and the cell suspension was inoculated into a 96-well plate at 100 μL/well, and cultured overnight in an incubator at 37°C with 5% $CO_2$. After the cells were completely adhered to the wall, cells with the addition of the angiogenesis inhibitor polypeptide were used as administration groups, cells with the addition of Avastin were used as a positive control group, and cells with the addition of culture solution without any drugs were used as a blank control group. The cells were diluted to respective predetermined concentrations with the culture solution. The diluent were added into 96-well plates respectively at 100 μL per well, and the cells were cultured at 37°C for 48 h in an incubator with 5% $CO_2$. 20 μL of 5 mg/mL MTT was added into each well of the 96-well plate, and the cultivation was continued for 4 h. The culture medium was removed, and 100 μL of DMSO was added to each well for dissolution. The absorbance was measured at a detection wavelength of 570 nm and a reference wavelength of 630 nm using a microplate reader, and the PIR was calculated by the formula as follows:

$$\text{PIR (\%)} = 1 - \text{administration group/negative group}$$

The experiment was repeated independently three times. The results were expressed as mean ± SD, and statistical T test was conducted. *P< 0.05 indicates significant difference, and **P< 0.01 indicates extremely significant difference. The experimental results are shown in Table 21.

Table 21 Proliferation inhibition effect of polypeptides X1-X90 on HRCECs

| Group | Dose (μM) | A570 nm-A630 nm | Inhibition rate (%) |
|---|---|---|---|
| | 0.05 | 0.6900 ± 0.09402 | 47.79%* |
| X1 | 0.1 | 0.5959 ± 0.0814 | 54.91%** |
| | 0.2 | 0.5154 ± 0.00343 | 61.00%* |
| | 0.05 | 0.6952 ± 0.04815 | 47.40%* |
| X2 | 0.1 | 0.6597 ± 0.05816 | 50.09%** |
| | 0.2 | 0.4139 ± 0.01863 | 68.68%** |

(continued)

| Group | Dose (μM) | A570 nm-A630 nm | Inhibition rate (%) |
|---|---|---|---|
| | 0.05 | 0.6832 ± 0.00473 | 48.31%* |
| X3 | 0.1 | 0.5950 ± 0.00446 | 54.98%** |
| | 0.2 | 0.5784 ± 0.07639 | 56.24%* |
| | 0.05 | 0.6807 ± 0.06246 | 48.50%* |
| X4 | 0.1 | 0.6427 ± 0.08818 | 51.37%** |
| | 0.2 | 0.5107 ± 0.09081 | 61.36%* |
| | 0.05 | 0.772 ± 0.00377 | 41.59%* |
| X5 | 0.1 | 0.7666 ± 0.05195 | 42.00%** |
| | 0.2 | 0.7106 ± 0.00437 | 46.24%* |
| | 0.05 | 0.7612 ± 0.08455 | 42.41%* |
| X6 | 0.1 | 0.7031 ± 0.02017 | 46.80%** |
| | 0.2 | 0.4613 ± 0.07983 | 65.10%* |
| | 0.05 | 0.6845 ± 0.03192 | 48.21%* |
| X7 | 0.1 | 0.6126 ± 0.02039 | 53.65%** |
| | 0.2 | 0.4791 ± 0.06283 | 63.75%* |
| | 0.05 | 0.7692 ± 0.08937 | 41.80%* |
| X8 | 0.1 | 0.4646 ± 0.03545 | 64.85%** |
| | 0.2 | 0.4092 ± 0.06909 | 69.06%* |
| | 0.05 | 0.7595 ± 0.03958 | 42.54%* |
| X9 | 0.1 | 0.4953 ± 0.01919 | 62.53%** |
| | 0.2 | 0.4704 ± 0.03443 | 64.41%* |
| | 0.05 | 0.5234 ± 0.00498 | 60.40%* |
| X10 | 0.1 | 0.4511 ± 0.06292 | 65.87%** |
| | 0.2 | 0.4501 ± 0.06223 | 65.95%* |
| | 0.05 | 0.7098 ± 0.07736 | 46.30%* |
| X11 | 0.1 | 0.6334 ± 0.09489 | 52.08%** |
| | 0.2 | 0.4571 ± 0.01564 | 65.42%* |
| | 0.05 | 0.7739 ± 0.09091 | 41.45%* |
| X12 | 0.1 | 0.7007 ± 0.03361 | 46.98%** |
| | 0.2 | 0.5484 ± 0.09013 | 58.51%* |
| | 0.05 | 0.6041 ± 0.03298 | 54.29%* |
| X13 | 0.1 | 0.5231 ± 0.0749 | 60.42%** |
| | 0.2 | 0.4230 ± 0.02522 | 68.00%* |
| | 0.05 | 0.7206 ± 0.01593 | 45.48%* |
| X14 | 0.1 | 0.6032 ± 0.02038 | 54.36%** |
| | 0.2 | 0.4592 ± 0.05767 | 65.26%* |
| | 0.05 | 0.6971 ± 0.00527 | 47.26%* |
| X15 | 0.1 | 0.6356 ± 0.05105 | 51.91%** |
| | 0.2 | 0.6335 ± 0.04622 | 52.07%* |
| | 0.05 | 0.6661 ± 0.07224 | 49.60%* |
| X16 | 0.1 | 0.5209 ± 0.00575 | 60.59%** |
| | 0.2 | 0.4429 ± 0.09044 | 66.49%* |
| | 0.05 | 0.5934 ± 0.03887 | 55.10%* |
| X17 | 0.1 | 0.5001 ± 0.02347 | 62.16%** |
| | 0.2 | 0.4616 ± 0.02184 | 65.08%* |
| | 0.05 | 0.7586 ± 0.05265 | 42.60%* |
| X18 | 0.1 | 0.6304 ± 0.03508 | 52.30%** |
| | 0.2 | 0.5051 ± 0.08266 | 61.78%* |
| | 0.05 | 0.6472 ± 0.01829 | 51.03%* |

(continued)

| Group | Dose (μM) | A570 nm-A630 nm | Inhibition rate (%) |
|---|---|---|---|
| X19 | 0.1 | 0.4852 ± 0.07133 | 63.29%** |
| | 0.2 | 0.4664 ± 0.01886 | 64.71%* |
| | 0.05 | 0.7365 ± 0.05032 | 44.28%* |
| X20 | 0.1 | 0.7121 ± 0.0349 | 46.12%** |
| | 0.2 | 0.5268 ± 0.01299 | 60.14%* |
| | 0.05 | 0.6761 ± 0.01737 | 48.85%* |
| X21 | 0.1 | 0.4342 ± 0.04189 | 67.15%** |
| | 0.2 | 0.4076 ± 0.06848 | 69.16%* |
| | 0.05 | 0.6701 ± 0.07545 | 49.30%* |
| X22 | 0.1 | 0.4738 ± 0.00344 | 64.15%** |
| | 0.2 | 0.4425 ± 0.04816 | 66.52%* |
| | 0.05 | 0.7191 ± 0.07483 | 45.59%* |
| X23 | 0.1 | 0.7043 ± 0.07872 | 46.74%** |
| | 0.2 | 0.6753 ± 0.00095 | 48.91%* |
| | 0.05 | 0.7929 ± 0.02399 | 40.01%* |
| X24 | 0.1 | 0.7286 ± 0.00571 | 44.87%** |
| | 0.2 | 0.6661 ± 0.02372 | 49.60%* |
| | 0.05 | 0.6578 ± 0.08044 | 50.23%* |
| X25 | 0.1 | 0.6052 ± 0.09358 | 54.21%** |
| | 0.2 | 0.4847 ± 0.06551 | 63.33%* |
| | 0.05 | 0.7736 ± 0.06908 | 41.47%* |
| X26 | 0.1 | 0.6810 ± 0.02943 | 48.48%** |
| | 0.2 | 0.4162 ± 0.00082 | 68.51%* |
| | 0.05 | 0.6629 ± 0.02448 | 49.84%* |
| X27 | 0.1 | 0.5753 ± 0.02814 | 56.47%** |
| | 0.2 | 0.5731 ± 0.04774 | 56.64%* |
| | 0.05 | 0.6796 ± 0.03488 | 48.58%* |
| X28 | 0.1 | 0.6249 ± 0.09489 | 52.72%** |
| | 0.2 | 0.4004 ± 0.02963 | 69.71%* |
| | 0.05 | 0.6256 ± 0.09549 | 52.67%* |
| X29 | 0.1 | 0.5549 ± 0.02011 | 58.02%** |
| | 0.2 | 0.5151 ± 0.01135 | 61.03%* |
| | 0.05 | 0.7605 ± 0.00513 | 42.46%* |
| X30 | 0.1 | 0.5268 ± 0.06831 | 60.14%** |
| | 0.2 | 0.4517 ± 0.03378 | 65.82%* |
| | 0.05 | 0.7676 ± 0.04187 | 41.92%* |
| X31 | 0.1 | 0.7183 ± 0.03361 | 45.65%** |
| | 0.2 | 0.4824 ± 0.04754 | 63.50%* |
| | 0.05 | 0.6224 ± 0.08338 | 52.91%* |
| X32 | 0.1 | 0.6030 ± 0.05575 | 54.38%** |
| | 0.2 | 0.4030 ± 0.00097 | 69.51%* |
| | 0.05 | 0.7579 ± 0.03744 | 42.66%* |
| X33 | 0.1 | 0.7024 ± 0.04477 | 46.86%** |
| | 0.2 | 0.4991 ± 0.04854 | 62.24%* |
| | 0.05 | 0.6536 ± 0.08102 | 50.55%* |
| X34 | 0.1 | 0.6420 ± 0.08726 | 51.43%** |
| | 0.2 | 0.5298 ± 0.07541 | 59.92%* |
| | 0.05 | 0.7413 ± 0.08995 | 43.91%* |
| X35 | 0.1 | 0.5720 ± 0.01284 | 56.72%** |

(continued)

| Group | Dose (μM) | A570 nm-A630 nm | Inhibition rate (%) |
|---|---|---|---|
| | 0.2 | 0.5624 ± 0.01279 | 57.45%* |
| | 0.05 | 0.5915 ± 0.05135 | 55.25%* |
| X36 | 0.1 | 0.5237 ± 0.03194 | 60.38%** |
| | 0.2 | 0.4664 ± 0.03733 | 64.71%* |
| | 0.05 | 0.6812 ± 0.09473 | 48.46%* |
| X37 | 0.1 | 0.4537 ± 0.03356 | 65.67%** |
| | 0.2 | 0.4053 ± 0.09286 | 69.33%* |
| | 0.05 | 0.5470 ± 0.08782 | 58.61%* |
| X38 | 0.1 | 0.4341 ± 0.02751 | 67.16%** |
| | 0.2 | 0.4319 ± 0.01987 | 67.32%* |
| | 0.05 | 0.6346 ± 0.04812 | 51.99%* |
| X39 | 0.1 | 0.5761 ± 0.04869 | 56.41%** |
| | 0.2 | 0.5379 ± 0.07322 | 59.30%* |
| | 0.05 | 0.7728 ± 0.04511 | 41.53%* |
| X40 | 0.1 | 0.5990 ± 0.01488 | 54.68%** |
| | 0.2 | 0.5890 ± 0.08771 | 55.44%* |
| | 0.05 | 0.7140 ± 0.00296 | 45.98%* |
| X41 | 0.1 | 0.6940 ± 0.04213 | 47.49%** |
| | 0.2 | 0.6212 ± 0.03696 | 53.00%* |
| | 0.05 | 0.7927 ± 0.00393 | 40.02%* |
| X42 | 0.1 | 0.7502 ± 0.04961 | 43.24%** |
| | 0.2 | 0.5605 ± 0.08514 | 57.59%* |
| | 0.05 | 0.7585 ± 0.06573 | 42.61%* |
| X43 | 0.1 | 0.6311 ± 0.06472 | 52.25%** |
| | 0.2 | 0.5077 ± 0.02457 | 61.59%* |
| | 0.05 | 0.7080 ± 0.02655 | 46.43%* |
| X44 | 0.1 | 0.5709 ± 0.01156 | 56.81%** |
| | 0.2 | 0.5140 ± 0.06297 | 61.11%* |
| | 0.05 | 0.7645 ± 0.08831 | 42.16%* |
| X45 | 0.1 | 0.5517 ± 0.06286 | 58.26%** |
| | 0.2 | 0.5097 ± 0.05998 | 61.44%* |
| | 0.05 | 0.7214 ± 0.05055 | 45.42%* |
| X46 | 0.1 | 0.6007 ± 0.02905 | 54.55%** |
| | 0.2 | 0.4615 ± 0.02667 | 65.08%* |
| | 0.05 | 0.7323 ± 0.05099 | 44.59%* |
| X47 | 0.1 | 0.5705 ± 0.01511 | 56.84%** |
| | 0.2 | 0.4145 ± 0.09859 | 68.64%* |
| | 0.05 | 0.7421 ± 0.02423 | 43.85%* |
| X48 | 0.1 | 0.6677 ± 0.01791 | 49.48%** |
| | 0.2 | 0.4615 ± 0.02514 | 65.08%* |
| | 0.05 | 0.4682 ± 0.01676 | 64.58%* |
| X49 | 0.1 | 0.4492 ± 0.06833 | 66.01%** |
| | 0.2 | 0.4010 ± 0.05362 | 69.66%* |
| | 0.05 | 0.6098 ± 0.01925 | 53.86%* |
| X50 | 0.1 | 0.6071 ± 0.03868 | 54.07%** |
| | 0.2 | 0.5590 ± 0.07807 | 57.71%* |
| | 0.05 | 0.7370 ± 0.04085 | 44.24%* |
| X51 | 0.1 | 0.4626 ± 0.01562 | 65.00%** |
| | 0.2 | 0.4308 ± 0.05013 | 67.41%* |

(continued)

| Group | Dose (μM) | A570 nm-A630 nm | Inhibition rate (%) |
|---|---|---|---|
| | 0.05 | 0.7292 ± 0.00462 | 44.83%* |
| X52 | 0.1 | 0.6922 ± 0.07646 | 47.63%** |
| | 0.2 | 0.5217 ± 0.01318 | 60.53%* |
| | 0.05 | 0.6058 ± 0.06277 | 54.17%* |
| X53 | 0.1 | 0.5519 ± 0.03497 | 58.24%** |
| | 0.2 | 0.5346 ± 0.01624 | 59.55%* |
| | 0.05 | 0.6960 ± 0.03088 | 47.34%* |
| X54 | 0.1 | 0.4847 ± 0.09197 | 63.32%** |
| | 0.2 | 0.4203 ± 0.07008 | 68.20%* |
| | 0.05 | 0.7288 ± 0.03166 | 44.85%* |
| X55 | 0.1 | 0.6577 ± 0.00076 | 50.23%** |
| | 0.2 | 0.4451 ± 0.00508 | 66.32%* |
| | 0.05 | 0.6110 ± 0.01015 | 53.77%* |
| X56 | 0.1 | 0.4387 ± 0.02343 | 66.81%** |
| | 0.2 | 0.4218 ± 0.01031 | 68.08%* |
| | 0.05 | 0.6057 ± 0.06594 | 54.17%* |
| X57 | 0.1 | 0.4783 ± 0.03174 | 63.81%** |
| | 0.2 | 0.4125 ± 0.0892 | 68.79%* |
| | 0.05 | 0.7350 ± 0.04037 | 44.39%* |
| X58 | 0.1 | 0.5535 ± 0.04093 | 58.12%** |
| | 0.2 | 0.5020 ± 0.06759 | 62.02%* |
| | 0.05 | 0.6513 ± 0.08257 | 50.72%* |
| X59 | 0.1 | 0.5967 ± 0.07994 | 54.85%** |
| | 0.2 | 0.4118 ± 0.09488 | 68.84%* |
| | 0.05 | 0.7965 ± 0.09236 | 39.73%* |
| X60 | 0.1 | 0.6741 ± 0.01064 | 48.99%** |
| | 0.2 | 0.4310 ± 0.06113 | 67.39%* |
| | 0.05 | 0.6654 ± 0.03872 | 49.65%* |
| X61 | 0.1 | 0.5495 ± 0.00776 | 58.42%** |
| | 0.2 | 0.5150 ± 0.05485 | 61.03%* |
| | 0.05 | 0.7115 ± 0.01869 | 46.16%* |
| X62 | 0.1 | 0.6619 ± 0.02252 | 49.92%** |
| | 0.2 | 0.4690 ± 0.01251 | 64.51%* |
| | 0.05 | 0.7325 ± 0.04718 | 44.57%* |
| X63 | 0.1 | 0.4304 ± 0.04148 | 67.43%** |
| | 0.2 | 0.4184 ± 0.09622 | 68.34%* |
| | 0.05 | 0.7396 ± 0.00238 | 44.04%* |
| X64 | 0.1 | 0.7051 ± 0.05311 | 46.65%** |
| | 0.2 | 0.4070 ± 0.07432 | 69.20%* |
| | 0.05 | 0.7765 ± 0.01256 | 41.25%* |
| X65 | 0.1 | 0.7264 ± 0.07863 | 45.04%** |
| | 0.2 | 0.6533 ± 0.04283 | 50.57%* |
| | 0.05 | 0.7863 ± 0.03541 | 40.50%* |
| X66 | 0.1 | 0.7815 ± 0.05931 | 40.87%** |
| | 0.2 | 0.6563 ± 0.03135 | 50.34%* |
| | 0.05 | 0.7007 ± 0.00869 | 46.98%* |
| X67 | 0.1 | 0.6861 ± 0.02168 | 48.09%** |
| | 0.2 | 0.5419 ± 0.03429 | 59.00%* |
| | 0.05 | 0.7466 ± 0.08994 | 43.51%* |

(continued)

| Group | Dose (μM) | A570 nm-A630 nm | Inhibition rate (%) |
|---|---|---|---|
| X68 | 0.1 | 0.6496 ± 0.06733 | 50.85%** |
| | 0.2 | 0.6456 ± 0.01534 | 51.15%* |
| | 0.05 | 0.7592 ± 0.03113 | 42.55%* |
| X69 | 0.1 | 0.7117 ± 0.03907 | 46.15%** |
| | 0.2 | 0.4354 ± 0.02235 | 67.06%* |
| | 0.05 | 0.7793 ± 0.05924 | 41.03%* |
| X70 | 0.1 | 0.6176 ± 0.01467 | 53.27%** |
| | 0.2 | 0.5270 ± 0.06539 | 60.12%* |
| | 0.05 | 0.7504 ± 0.03175 | 43.22%* |
| X71 | 0.1 | 0.5795 ± 0.05806 | 56.15%** |
| | 0.2 | 0.4639 ± 0.01851 | 64.90%* |
| | 0.05 | 0.7920 ± 0.03144 | 40.07%* |
| X72 | 0.1 | 0.5688 ± 0.07895 | 56.96%** |
| | 0.2 | 0.4627 ± 0.01624 | 64.99%* |
| | 0.05 | 0.6761 ± 0.03535 | 48.84%* |
| X73 | 0.1 | 0.6738 ± 0.01619 | 49.02%** |
| | 0.2 | 0.6044 ± 0.03818 | 54.27%* |
| | 0.05 | 0.7835 ± 0.04916 | 40.72%* |
| X74 | 0.1 | 0.6877 ± 0.03265 | 47.96%** |
| | 0.2 | 0.5071 ± 0.07328 | 61.63%* |
| | 0.05 | 0.6155 ± 0.01446 | 53.43%* |
| X75 | 0.1 | 0.5841 ± 0.04054 | 55.80%** |
| | 0.2 | 0.5267 ± 0.05865 | 60.15%* |
| | 0.05 | 0.6315 ± 0.04026 | 52.22%* |
| X76 | 0.1 | 0.5306 ± 0.00069 | 59.85%** |
| | 0.2 | 0.4825 ± 0.08079 | 63.49%* |
| | 0.05 | 0.6396 ± 0.02907 | 51.60%* |
| X77 | 0.1 | 0.5502 ± 0.05927 | 58.37%** |
| | 0.2 | 0.4973 ± 0.00893 | 62.37%* |
| | 0.05 | 0.7676 ± 0.06622 | 41.92%* |
| X78 | 0.1 | 0.5403 ± 0.04745 | 59.12%** |
| | 0.2 | 0.4331 ± 0.04096 | 67.23%* |
| | 0.05 | 0.6465 ± 0.04753 | 51.08%* |
| X79 | 0.1 | 0.5171 ± 0.00162 | 60.87%** |
| | 0.2 | 0.5039 ± 0.06602 | 61.87%* |
| | 0.05 | 0.7637 ± 0.04841 | 42.21%* |
| X80 | 0.1 | 0.5098 ± 0.08356 | 61.43%** |
| | 0.2 | 0.4724 ± 0.08499 | 64.26%* |
| | 0.05 | 0.7363 ± 0.01771 | 44.29%* |
| X81 | 0.1 | 0.6572 ± 0.00217 | 50.27%** |
| | 0.2 | 0.4376 ± 0.07394 | 66.89%* |
| | 0.05 | 0.7715 ± 0.03515 | 41.62%* |
| X82 | 0.1 | 0.6098 ± 0.07588 | 53.86%** |
| | 0.2 | 0.5004 ± 0.02218 | 62.14%* |
| | 0.05 | 0.7383 ± 0.05988 | 44.14%* |
| X83 | 0.1 | 0.7125 ± 0.02437 | 46.13%** |
| | 0.2 | 0.5649 ± 0.07386 | 57.26%* |
| | 0.05 | 0.7587 ± 0.03942 | 42.59%* |
| X84 | 0.1 | 0.5954 ± 0.04671 | 54.95%** |

(continued)

| Group | Dose (μM) | A570 nm-A630 nm | Inhibition rate (%) |
|---|---|---|---|
| | 0.2 | 0.4487 ± 0.02541 | 66.05%* |
| | 0.05 | 0.6537 ± 0.02097 | 50.54%* |
| X85 | 0.1 | 0.6066 ± 0.07551 | 54.10%** |
| | 0.2 | 0.5765 ± 0.05608 | 56.38%* |
| | 0.05 | 0.6984 ± 0.06728 | 47.15%* |
| X86 | 0.1 | 0.5645 ± 0.01108 | 57.29%** |
| | 0.2 | 0.5643 ± 0.02159 | 57.30%* |
| | 0.05 | 0.6381 ± 0.08827 | 51.72%* |
| X87 | 0.1 | 0.5551 ± 0.05137 | 58.00%** |
| | 0.2 | 0.4162 ± 0.06198 | 68.51%* |
| | 0.05 | 0.6294 ± 0.07175 | 52.38%* |
| X88 | 0.1 | 0.5393 ± 0.07136 | 59.19%** |
| | 0.2 | 0.5189 ± 0.02832 | 60.74%* |
| | 0.05 | 0.7991 ± 0.08908 | 39.54%* |
| X89 | 0.1 | 0.6056 ± 0.08945 | 54.18%** |
| | 0.2 | 0.4076 ± 0.05343 | 69.16%* |
| | 0.05 | 0.7823 ± 0.07108 | 40.81%* |
| X90 | 0.1 | 0.5321 ± 0.03975 | 59.74%** |
| | 0.2 | 0.4695 ± 0.06418 | 64.47%* |
| Avastin | 0.5 | 0.4325 ± 0.01108 | 67.30%** |
| control | - | 1.3217±0.08914 | 0.00% |

Results: X1-X90 with different molecular weights could have obvious inhibitory effects on the proliferation of HRCECs, and showed a dose-dependent relationship. The inhibition rate in the high-dose group was close to that of the positive drug. The inhibition rate reached 69.71% when X28 was administered at a dose of 0.2 μM, slightly higher than that of the positive drug.

**Example 17**

Effect of polypeptides X1-X90 on corneal neovascularization in BALB/c mice

(1) Preparation of a model of corneal neovascularization induced by alkali burn in BALB/c mice: The mice were randomly grouped, and marked as X1-X90 test groups and control group, with 5 mice in each group. After alkali burn, X1-X90 and normal saline were injected in the vitreous chamber once a day for 1 week. Inflammatory reaction and neovascularization of corneas in each group were observed under a slit lamp microscope on days 1, 7 and 14 after alkali burn. On day 14 after alkali burn, corneal neovascularization of each group was photographed and recorded under a slit lamp microscope with anterior segment photography. Then all mice were killed by a cervical dislocation method and eyeballs were extracted. Blood was washed with normal saline, the eyeballs were fixed with 4% paraformaldehyde for 1.5 h, dehydrated overnight in PBS containing 30% sucrose, embedded with OCT frozen section embedding agent and stored in a refrigerator at -80°C. Frozen section was performed to a thickness of 8 μm, and expression of CD31 was detected by immunohistochemistry.

(2) Quantitative measurement of corneal microvascular density: Microvessel density (MVD) is an indicator for evaluating angiogenesis. An anti-CD31 antibody immunohistochemical method was used to label vascular endothelial cells and count the number of microvessels in unit area to measure the degree of neoangiogenesis. The standard for statistics of microvessels: endothelial cells or cell clusters in a corneal tissue that are clearly demarcated from adjacent tissues and dyed brownish yellow or brown are counted as neovascularization under a microscope. The number of neovascularization in the whole section was counted under a 10 × 20 microscope. After the corneal tissue section was photographed, the area of the whole corneal tissue section was calculated by image processing software Image J, and the neovascularization density of the whole section was calculated.

The experiment was repeated independently three times. The results were expressed as mean ± SD, and statistical T test was conducted. *P< 0.05 indicates significant difference, and **P< 0.01 indicates extremely significant

difference. The experimental results are shown in Table 22.

Table 22 Effect of polypeptides X1-X90 on corneal neovascularization in mice

| Group | MVD | Inhibition rate (%) |
|---|---|---|
| X1 | 39.28 ± 4.061* | 41.58% |
| X2 | 33.84 ± 3.805** | 49.67% |
| X3 | 34.20 ± 6.078** | 49.14% |
| X4 | 37.39 ± 3.344* | 44.39% |
| X5 | 36.23 ± 4.86** | 46.12% |
| X6 | 31.53 ± 6.34** | 53.11% |
| X7 | 32.19 ± 6.641** | 52.13% |
| X8 | 32.16 ± 5.44** | 52.17% |
| X9 | 39.39 ± 3.884* | 41.42% |
| XI0 | 39.00 ± 4.194* | 42.00% |
| X11 | 32.51 ± 4.294** | 51.65% |
| X12 | 37.84 ± 6.113* | 43.72% |
| X13 | 34.19 ± 5.411** | 49.15% |
| X14 | 30.54 ± 5.794** | 54.58% |
| X15 | 39.85 ± 3.153* | 40.73% |
| X16 | 38.83 ± 4.871* | 42.25% |
| X17 | 34.93 ± 4.725** | 48.05% |
| X18 | 33.50 ± 5.649** | 50.18% |
| X19 | 39.38 ± 4.833* | 41.43% |
| X20 | 37.24 ± 6.113* | 44.62% |
| X21 | 36.36 ± 3.663** | 45.93% |
| X22 | 35.84 ± 6.594** | 46.70% |
| X23 | 35.67 ± 6.861** | 46.95% |
| X24 | 36.15 ± 4.199** | 46.24% |
| X25 | 31.67 ± 6.856** | 52.90% |
| X26 | 37.44 ± 4.646* | 44.32% |
| X27 | 37.70 ± 3.805* | 43.93% |
| X28 | 35.91 ± 4.891** | 46.59% |
| X29 | 30.27 ± 4.918** | 54.98% |
| X30 | 35.55 ± 5.717** | 47.13% |
| X31 | 36.70 ± 5.505** | 45.42% |
| X32 | 33.45 ± 6.453** | 50.25% |
| X33 | 35.50 ± 3.223** | 47.20% |
| X34 | 34.72 ± 6.051** | 48.36% |
| X35 | 37.25 ± 4.806** | 44.60% |
| X36 | 36.63 ± 5.284** | 45.52% |
| X37 | 30.12 ± 6.961** | 55.21% |
| X38 | 33.63 ± 6.163** | 49.99% |
| X39 | 34.06 ± 6.441** | 49.35% |
| X40 | 31.19 ± 5.089** | 53.61% |
| X41 | 34.49 ± 6.982** | 48.71% |
| X42 | 34.66 ± 5.340** | 48.45% |
| X43 | 30.78 ± 4.182** | 54.22% |
| X44 | 39.89 ± 6.464* | 40.68% |
| X45 | 33.58 ± 4.495** | 50.06% |
| X46 | 32.30 ± 4.971** | 51.96% |
| X47 | 37.45 ± 3.897* | 44.30% |
| X48 | 38.39 ± 4.908* | 42.91% |

(continued)

| Group | MVD | Inhibition rate (%) |
|---|---|---|
| X49 | 39.48 ± 3.684* | 41.28% |
| X50 | 31.41 ± 4.385** | 53.29% |
| X51 | 33.79 ± 3.902** | 49.75% |
| X52 | 31.75 ± 4.837** | 52.78% |
| X53 | 35.73 ± 6.955** | 46.86% |
| X54 | 32.09 ± 3.526** | 52.28% |
| X55 | 32.62 ± 5.356** | 51.49% |
| X56 | 30.87 ± 4.765** | 54.09% |
| X57 | 30.94 ± 3.893** | 53.99% |
| X58 | 35.87 ± 5.015** | 46.65% |
| X59 | 34.04 ± 6.473** | 49.38% |
| X60 | 33.59 ± 4.055** | 50.04% |
| X61 | 36.31 ± 4.693** | 46.00% |
| X62 | 37.50 ± 5.197* | 44.23% |
| X63 | 32.96 ± 4.213** | 50.98% |
| X64 | 38.20 ± 6.446* | 43.19% |
| X65 | 34.72 ± 4.489** | 48.36% |
| X66 | 31.96 ± 4.478** | 52.47% |
| X67 | 35.94 ± 4.335** | 46.55% |
| X68 | 34.80 ± 3.496** | 48.25% |
| X69 | 39.34 ± 3.192* | 41.49% |
| X70 | 32.83 ± 4.159** | 51.17% |
| X71 | 33.33 ± 3.004** | 50.43% |
| X72 | 38.75 ± 5.524* | 42.37% |
| X73 | 38.89 ± 4.593* | 42.16% |
| X74 | 35.04 ± 5.106** | 47.89% |
| X75 | 39.54 ± 4.543* | 41.20% |
| X76 | 30.37 ± 6.648** | 54.83% |
| X77 | 33.48 ± 6.956** | 50.21% |
| X78 | 33.47 ± 6.931** | 50.22% |
| X79 | 36.78 ± 4.743** | 45.30% |
| X80 | 39.64 ± 4.856* | 41.05% |
| X81 | 38.49 ± 6.821* | 42.76% |
| X82 | 30.94 ± 5.093** | 53.99% |
| X83 | 39.67 ± 4.978* | 41.00% |
| X84 | 39.66 ± 4.989* | 41.02% |
| X85 | 37.36 ± 4.485* | 44.44% |
| X86 | 30.43 ± 5.512** | 54.74% |
| X87 | 38.66 ± 4.202* | 42.50% |
| X88 | 36.50 ± 6.089** | 45.72% |
| X89 | 33.05 ± 5.016** | 50.85% |
| X90 | 34.91 ± 3.786** | 48.08% |
| control | 67.24±7.341 | 0.00% |

The experimental results showed that different polypeptides X1-X90 could significantly inhibit the growth of corneal neovascularization, and the inhibition rate of X37 reached 55.21%.

**Example 18**

Effect of X1-X90 on iris neovascularization in rabbits

577 nm argon ion laser was used to coagulate main and branch veins of rabbit retina, and fundus fluorescein angiography (FFA) confirmed the success of vein occlusion. 5-12 days later, the iris fluorescein angiography (IFA) showed that the leakage of fluorescein was obvious compared with that of the normal control group, confirming the formation of an animal model of iris neovascularization (NVI).

273 eyes with successful modeling were taken and randomly grouped with 3 eyes in each group. The groups were respectively marked as a negative control group and X1-X90 treatment groups, and every 3 eyes fell into one group. The doses of normal saline and X1-X90 were each 0.05 μM, and were injected in the vitreous chamber once a day for 2 weeks. In the third week, observation was made with optical and electron microscopes.

Results: Under the optical microscope, the anterior surface of iris had fibrovascular membrane remnants mainly composed of fibrous tissues, with only a few open vascular cavities. Vascular remnants could be seen in iris stroma, which were necrotic cells and cell fragments. The iris surface of the control eye under the light microscope had the fibrovascular membrane with branching and potential lumen. The ultrastructure of iris in the treatment group had a series of degenerative changes: endothelial cells of large blood vessels in the middle of the iris stroma had normal nucleus, cytoplasm and cell junctions, capillary remnants were found in the iris stroma and the anterior surface of the iris, cell fragments and macrophage infiltration were found around the iris stroma, and there were no capillaries with potential lumen and no degenerated parietal cells, indicating the fading of neovascularization.

The experimental results showed that X1-X90 could inhibit the formation of iris neovascularization in rabbits and degrade the formed blood vessels.

**Example 19**

Effect of X1-X90 on choroidal neovascularization in rats

6-8 weeks male BN rats were fully anesthetized by intraperitoneal injection of 846 compound anesthetic with a concentration of 0.5 mL/kg. Compound tropicamide eye drops were used once 5 min before laser photocoagulation to fully dilate the pupils of both eyes. The animals were fixed and subjected to krypton laser photocoagulation equidistantly around an optic disc with the aid of a - 53.00D corneal contact lens at a distance of 2PD from the optic disc. A total of 8 photocoagulation spots were obtained. The laser wavelength was 647.1 nm, the power was 350 mW, and the diameter and time of the photocoagulation spots were 50 μm and 0.05 s respectively. Fundus photography was performed immediately after photocoagulation. FFA, histopathology and transmission electron microscopy were performed on the days 3, 7, 14, 21 and 28 after photocoagulation respectively.

Through fundus photography and FFA examination, it was confirmed that the fluorescein leakage of the photocoagulation spot reached its peak on day 21 after photocoagulation. At the same time, histopathological examination was carried out. On day 21 after photocoagulation, CNV showed significant fibrovascular proliferation under a light microscope, in which a large number of neovascularization could be seen, and red blood cells were seen in lumen. Under the microscope, capillaries between choroidal melanocytes showed coagulative changes and endothelial cells coagulated. The results showed that the choroidal neovascularization model of rats was formed 21 days later.

Rats with successful modeling were randomly grouped, with 5 rats in each group. The groups were marked as a negative control group and X1-X90 treatment groups respectively. The rats were injected with normal saline and X1-X90 (the doses of X1-X90 were each 0.05 μM) in the vitreous chamber once a day for 1 week. FFA examination was carried out 3 days, 7 days, 14 days and 28 days after administration. The experimental results are shown in Table 23.

Table 23 Effect of polypeptides X1-X90 on choroidal neovascularization in rats

| Group | Detection time | | | | | | | |
|---|---|---|---|---|---|---|---|---|
| | day 3 The total light spot number was 297 | | day 7 The total light spot number was 186 | | day 14 The total light spot number was 137 | | day 28 The total light spot number was 69 | |
| | Leakage Light spot number | CNV Incidence (%) | Leakage Light spot number | CNV Incidence (%) | Leakage Light spot number | CNV Incidence (%) | Leakage Light spot number | CNV Incidence (%) |
| control | 248 | 83.50% | 139 | 74.73% | 88 | 64.23% | 43 | 62.31% |
| X1 | 161 | 54.21% | 87 | 46.77% | 62 | 45.26% | 25 | 36.23% |
| X2 | 157 | 52.86% | 85 | 45.70% | 54 | 39.42% | 25 | 36.23% |

(continued)

| Group | Detection time | | | | | | | |
|---|---|---|---|---|---|---|---|---|
| | day 3 The total light spot number was 297 | | day 7 The total light spot number was 186 | | day 14 The total light spot number was 137 | | day 28 The total light spot number was 69 | |
| | Leakage Light spot number | CNV Incidence (%) | Leakage Light spot number | CNV Incidence (%) | Leakage Light spot number | CNV Incidence (%) | Leakage Light spot number | CNV Incidence (%) |
| X3 | 150 | 50.51% | 89 | 47.85% | 62 | 45.26% | 30 | 43.48% |
| X4 | 157 | 52.86% | 85 | 45.70% | 57 | 41.61% | 27 | 39.13% |
| X5 | 153 | 51.52% | 85 | 45.70% | 60 | 43.80% | 25 | 36.23% |
| X6 | 148 | 49.83% | 87 | 46.77% | 55 | 40.15% | 27 | 39.13% |
| X7 | 149 | 50.17% | 84 | 45.16% | 59 | 43.07% | 27 | 39.13% |
| X8 | 157 | 52.86% | 92 | 49.46% | 64 | 46.72% | 29 | 42.03% |
| X9 | 160 | 53.87% | 84 | 45.16% | 64 | 46.72% | 25 | 36.23% |
| X10 | 148 | 49.83% | 87 | 46.77% | 62 | 45.26% | 29 | 42.03% |
| X11 | 158 | 53.20% | 85 | 45.70% | 58 | 42.34% | 24 | 34.78% |
| X12 | 147 | 49.49% | 92 | 49.46% | 64 | 46.72% | 27 | 39.13% |
| X13 | 158 | 53.20% | 90 | 48.39% | 64 | 46.72% | 26 | 37.68% |
| X14 | 160 | 53.87% | 84 | 45.16% | 60 | 43.80% | 30 | 43.48% |
| X15 | 151 | 50.84% | 88 | 47.31% | 63 | 45.99% | 25 | 36.23% |
| X16 | 157 | 52.86% | 88 | 47.31% | 55 | 40.15% | 29 | 42.03% |
| X17 | 163 | 54.88% | 90 | 48.39% | 58 | 42.34% | 28 | 40.58% |
| X18 | 160 | 53.87% | 86 | 46.24% | 63 | 45.99% | 31 | 44.93% |
| X19 | 155 | 52.19% | 85 | 45.70% | 57 | 41.61% | 31 | 44.93% |
| X20 | 156 | 52.53% | 91 | 48.92% | 58 | 42.34% | 27 | 39.13% |
| X21 | 151 | 50.84% | 89 | 47.85% | 64 | 46.72% | 28 | 40.58% |
| X22 | 160 | 53.87% | 89 | 47.85% | 60 | 43.80% | 26 | 37.68% |
| X23 | 157 | 52.86% | 85 | 45.70% | 61 | 44.53% | 26 | 37.68% |
| X24 | 161 | 54.21% | 92 | 49.46% | 63 | 45.99% | 31 | 44.93% |
| X25 | 154 | 51.85% | 88 | 47.31% | 59 | 43.07% | 31 | 44.93% |
| X26 | 164 | 55.22% | 92 | 49.46% | 62 | 45.26% | 30 | 43.48% |
| X27 | 163 | 54.88% | 86 | 46.24% | 58 | 42.34% | 25 | 36.23% |
| X28 | 149 | 50.17% | 87 | 46.77% | 62 | 45.26% | 28 | 40.58% |
| X29 | 161 | 54.21% | 85 | 45.70% | 56 | 40.88% | 27 | 39.13% |
| X30 | 154 | 51.85% | 91 | 48.92% | 63 | 45.99% | 28 | 40.58% |
| X31 | 159 | 53.54% | 87 | 46.77% | 57 | 41.61% | 31 | 44.93% |
| X32 | 161 | 54.21% | 86 | 46.24% | 59 | 43.07% | 29 | 42.03% |
| X33 | 161 | 54.21% | 86 | 46.24% | 62 | 45.26% | 26 | 37.68% |
| X34 | 155 | 52.19% | 88 | 47.31% | 63 | 45.99% | 29 | 42.03% |
| X35 | 155 | 52.19% | 89 | 47.85% | 64 | 46.72% | 31 | 44.93% |
| X36 | 150 | 50.51% | 92 | 49.46% | 55 | 40.15% | 30 | 43.48% |
| X37 | 154 | 51.85% | 85 | 45.70% | 59 | 43.07% | 26 | 37.68% |
| X38 | 163 | 54.88% | 88 | 47.31% | 57 | 41.61% | 26 | 37.68% |
| X39 | 154 | 51.85% | 88 | 47.31% | 59 | 43.07% | 31 | 44.93% |
| X40 | 157 | 52.86% | 91 | 48.92% | 55 | 40.15% | 26 | 37.68% |
| X41 | 157 | 52.86% | 91 | 48.92% | 60 | 43.80% | 28 | 40.58% |
| X42 | 151 | 50.84% | 88 | 47.31% | 63 | 45.99% | 29 | 42.03% |
| X43 | 148 | 49.83% | 89 | 47.85% | 59 | 43.07% | 25 | 36.23% |
| X44 | 152 | 51.18% | 91 | 48.92% | 59 | 43.07% | 29 | 42.03% |
| X45 | 155 | 52.19% | 92 | 49.46% | 58 | 42.34% | 27 | 39.13% |
| X46 | 153 | 51.52% | 86 | 46.24% | 60 | 43.80% | 31 | 44.93% |

(continued)

| Group | Detection time | | | | | | | |
|---|---|---|---|---|---|---|---|---|
| | day 3 The total light spot number was 297 | | day 7 The total light spot number was 186 | | day 14 The total light spot number was 137 | | day 28 The total light spot number was 69 | |
| | Leakage Light spot number | CNV Incidence (%) | Leakage Light spot number | CNV Incidence (%) | Leakage Light spot number | CNV Incidence (%) | Leakage Light spot number | CNV Incidence (%) |
| X47 | 161 | 54.21% | 89 | 47.85% | 62 | 45.26% | 30 | 43.48% |
| X48 | 161 | 54.21% | 85 | 45.70% | 59 | 43.07% | 25 | 36.23% |
| X49 | 157 | 52.86% | 91 | 48.92% | 57 | 41.61% | 27 | 39.13% |
| X50 | 157 | 52.86% | 84 | 45.16% | 60 | 43.80% | 25 | 36.23% |
| X51 | 158 | 53.20% | 91 | 48.92% | 60 | 43.80% | 26 | 37.68% |
| X52 | 150 | 50.51% | 84 | 45.16% | 58 | 42.34% | 31 | 44.93% |
| X53 | 161 | 54.21% | 92 | 49.46% | 63 | 45.99% | 27 | 39.13% |
| X54 | 164 | 55.22% | 92 | 49.46% | 56 | 40.88% | 26 | 37.68% |
| X55 | 156 | 52.53% | 92 | 49.46% | 62 | 45.26% | 29 | 42.03% |
| X56 | 162 | 54.55% | 92 | 49.46% | 55 | 40.15% | 31 | 44.93% |
| X57 | 149 | 50.17% | 90 | 48.39% | 56 | 40.88% | 26 | 37.68% |
| X58 | 157 | 52.86% | 84 | 45.16% | 59 | 43.07% | 28 | 40.58% |
| X59 | 153 | 51.52% | 84 | 45.16% | 63 | 45.99% | 29 | 42.03% |
| X60 | 159 | 53.54% | 86 | 46.24% | 58 | 42.34% | 26 | 37.68% |
| X61 | 161 | 54.21% | 91 | 48.92% | 62 | 45.26% | 27 | 39.13% |
| X62 | 164 | 55.22% | 88 | 47.31% | 62 | 45.26% | 31 | 44.93% |
| X63 | 150 | 50.51% | 87 | 46.77% | 61 | 44.53% | 31 | 44.93% |
| X64 | 153 | 51.52% | 86 | 46.24% | 58 | 42.34% | 30 | 43.48% |
| X65 | 157 | 52.86% | 85 | 45.70% | 61 | 44.53% | 26 | 37.68% |
| X66 | 162 | 54.55% | 92 | 49.46% | 56 | 40.88% | 29 | 42.03% |
| X67 | 153 | 51.52% | 89 | 47.85% | 60 | 43.80% | 27 | 39.13% |
| X68 | 158 | 53.20% | 87 | 46.77% | 60 | 43.80% | 29 | 42.03% |
| X69 | 164 | 55.22% | 87 | 46.77% | 62 | 45.26% | 31 | 44.93% |
| X70 | 160 | 53.87% | 90 | 48.39% | 61 | 44.53% | 31 | 44.93% |
| X71 | 154 | 51.85% | 88 | 47.31% | 58 | 42.34% | 26 | 37.68% |
| X72 | 150 | 50.51% | 89 | 47.85% | 64 | 46.72% | 27 | 39.13% |
| X73 | 160 | 53.87% | 91 | 48.92% | 63 | 45.99% | 26 | 37.68% |
| X74 | 151 | 50.84% | 88 | 47.31% | 60 | 43.80% | 28 | 40.58% |
| X75 | 162 | 54.55% | 88 | 47.31% | 59 | 43.07% | 30 | 43.48% |
| X76 | 148 | 49.83% | 91 | 48.92% | 55 | 40.15% | 28 | 40.58% |
| X77 | 148 | 49.83% | 92 | 49.46% | 63 | 45.99% | 29 | 42.03% |
| X78 | 147 | 49.49% | 89 | 47.85% | 60 | 43.80% | 31 | 44.93% |
| X79 | 153 | 51.52% | 92 | 49.46% | 57 | 41.61% | 30 | 43.48% |
| X80 | 156 | 52.53% | 84 | 45.16% | 61 | 44.53% | 30 | 43.48% |
| X81 | 163 | 54.88% | 91 | 48.92% | 57 | 41.61% | 30 | 43.48% |
| X82 | 159 | 53.54% | 87 | 46.77% | 61 | 44.53% | 27 | 39.13% |
| X83 | 163 | 54.88% | 88 | 47.31% | 60 | 43.80% | 30 | 43.48% |
| X84 | 161 | 54.21% | 92 | 49.46% | 58 | 42.34% | 25 | 36.23% |
| X85 | 157 | 52.86% | 91 | 48.92% | 63 | 45.99% | 25 | 36.23% |
| X86 | 155 | 52.19% | 90 | 48.39% | 64 | 46.72% | 30 | 43.48% |
| X87 | 163 | 54.88% | 88 | 47.31% | 58 | 42.34% | 25 | 36.23% |
| X88 | 147 | 49.49% | 84 | 45.16% | 63 | 45.99% | 28 | 40.58% |
| X89 | 161 | 54.21% | 84 | 45.16% | 63 | 45.99% | 29 | 42.03% |
| X90 | 156 | 52.53% | 84 | 45.16% | 59 | 43.07% | 28 | 40.58% |

Results: The FFA examination showed that, at 3 days after administration, the leakage of fluorescein in X1-X90 treatment groups was significantly changed compared with that before administration. At 7 and 14 days after administration, the leakage of fluorescein in the treatment groups decreased gradually. On day 28 after administration, the leakage of fluorescein was less than that on day 14 after administration. It showed that X1-X90 could treat choroidal neovascularization in rats, among which X11 had the most obvious effect, and the CNV incidence was the lowest (34.78%) on day 28 after administration.

**Example 20**

Effects of X1-X90 on retinal vessels in OIR mice

Establishment of an OIR model: Young mice and their mothers were exposed in a 75% hyperoxic environment from day 7 to day 12 after the birth of C57/B 16 mice, which caused capillaries in their central retina to disappear rapidly. The mice were returned to the indoor air on day 12, retinal vessels exposed to hyperoxia quickly disappeared, causing extensive abnormal neoangiogenesis, and the central part of the retina remained largely avascular for a long time. After the blood vessels disappeared completely, the mice were injected with normal saline (a negative group), X1-X12, X13-X44, X45-X90 in the vitreous chamber on day 13 respectively, and retinal vessels were evaluated on day 17 (50 mL of Texas red labeled tomato agglutinin was injected into the left ventricle to mark unclosed vessels and circulated for 5 min). The experimental results are shown in Table 24.

Table 24 Effect of polypeptides X1-X90 on retinal vessels in OIR mice

| Group | Dose (μM) | Area of neovascular plexus ($mm^2$) | Inhibition rate (%) |
|---|---|---|---|
| control | - | 0.212±0.008 | 0.00% |
| X1 | 0.05 | 0.115 ± 0.009 | 45.75%* |
| X2 | 0.05 | 0.081 ± 0.005 | 61.79%** |
| X3 | 0.05 | 0.084 ± 0.012 | 60.38%** |
| X4 | 0.05 | 0.116 ± 0.014 | 45.28%* |
| X5 | 0.05 | 0.101 ± 0.021 | 52.36%** |
| X6 | 0.05 | 0.108 ± 0.005 | 49.06%** |
| X7 | 0.05 | 0.108 ± 0.004 | 49.06%** |
| X8 | 0.05 | 0.091 ± 0.003 | 57.08%** |
| X9 | 0.05 | 0.110 ± 0.016 | 48.11%* |
| X10 | 0.05 | 0.097 ± 0.004 | 54.25%** |
| X11 | 0.05 | 0.103 ± 0.003 | 51.42%** |
| X12 | 0.05 | 0.090 ± 0.002 | 57.55%** |
| X13 | 0.05 | 0.080 ± 0.015 | 62.26%** |
| X14 | 0.05 | 0.101 ± 0.005 | 52.36%** |
| X15 | 0.05 | 0.091 ± 0.003 | 57.08%** |
| X16 | 0.05 | 0.110 ± 0.016 | 48.11%* |
| X17 | 0.05 | 0.084 ± 0.009 | 60.38%** |
| X18 | 0.05 | 0.119 ± 0.011 | 43.87%* |
| X19 | 0.05 | 0.083 ± 0.019 | 60.85%** |
| X20 | 0.05 | 0.118 ± 0.014 | 44.34%* |
| X21 | 0.05 | 0.118 ± 0.012 | 44.34%* |
| X22 | 0.05 | 0.082 ± 0.005 | 61.32%** |
| X23 | 0.05 | 0.101 ± 0.002 | 52.36%** |
| X24 | 0.05 | 0.107 ± 0.012 | 49.53%* |
| X25 | 0.05 | 0.096 ± 0.012 | 54.72%** |
| X26 | 0.05 | 0.104 ± 0.001 | 50.94%** |
| X27 | 0.05 | 0.106 ± 0.002 | 50.00%** |
| X28 | 0.05 | 0.088 ± 0.004 | 58.49%** |
| X29 | 0.05 | 0.109 ± 0.012 | 48.58%* |
| X30 | 0.05 | 0.118 ± 0.012 | 44.34%* |

(continued)

| Group | Dose (μM) | Area of neovascular plexus ($mm^2$) | Inhibition rate (%) |
|---|---|---|---|
| X31 | 0.05 | 0.101 ± 0.013 | 52.83%** |
| X32 | 0.05 | 0.096 ± 0.001 | 54.72%** |
| X33 | 0.05 | 0.089 ± 0.029 | 58.02%** |
| X34 | 0.05 | 0.120 ± 0.014 | 43.40%* |
| X35 | 0.05 | 0.101 ± 0.005 | 52.36%** |
| X36 | 0.05 | 0.084 ± 0.009 | 60.38%** |
| X37 | 0.05 | 0.084 ± 0.011 | 60.38%** |
| X38 | 0.05 | 0.110 ± 0.016 | 48.11%* |
| X39 | 0.05 | 0.111 ± 0.012 | 47.64%* |
| X40 | 0.05 | 0.089 ± 0.002 | 58.02%** |
| X41 | 0.05 | 0.092 ± 0.006 | 56.60%** |
| X42 | 0.05 | 0.092 ± 0.019 | 56.60%** |
| X43 | 0.05 | 0.112 ± 0.004 | 47.17%* |
| X44 | 0.05 | 0.084 ± 0.017 | 60.38%** |
| X45 | 0.05 | 0.100 ± 0.013 | 52.83%** |
| X46 | 0.05 | 0.110 ± 0.019 | 48.11%** |
| X47 | 0.05 | 0.114 ± 0.014 | 46.23%* |
| X48 | 0.05 | 0.102 ± 0.012 | 51.89%** |
| X49 | 0.05 | 0.089 ± 0.006 | 58.02%** |
| X50 | 0.05 | 0.118 ± 0.012 | 44.34%* |
| X51 | 0.05 | 0.095 ± 0.019 | 55.19%** |
| X52 | 0.05 | 0.117 ± 0.011 | 44.81%* |
| X53 | 0.05 | 0.083 ± 0.009 | 60.85%** |
| X54 | 0.05 | 0.116 ± 0.018 | 45.28%* |
| X55 | 0.05 | 0.116 ± 0.015 | 45.28%* |
| X56 | 0.05 | 0.107 ± 0.007 | 49.53%** |
| X57 | 0.05 | 0.103 ± 0.008 | 51.42% |
| X58 | 0.05 | 0.112 ± 0.013 | 47.17%* |
| X59 | 0.05 | 0.081 ± 0.013 | 61.79%** |
| X60 | 0.05 | 0.116 ± 0.005 | 45.28%* |
| X61 | 0.05 | 0.085 ± 0.002 | 59.91%** |
| X62 | 0.05 | 0.112 ± 0.019 | 47.17%* |
| X63 | 0.05 | 0.100 ± 0.006 | 52.83%** |
| X64 | 0.05 | 0.118 ± 0.017 | 44.34%* |
| X65 | 0.05 | 0.083 ± 0.012 | 60.85%** |
| X66 | 0.05 | 0.081 ± 0.008 | 61.79%** |
| X67 | 0.05 | 0.102 ± 0.003 | 51.89%** |
| X68 | 0.05 | 0.098 ± 0.019 | 53.77%** |
| X69 | 0.05 | 0.104 ± 0.012 | 50.94%** |
| X70 | 0.05 | 0.090 ± 0.022 | 57.55%** |
| X71 | 0.05 | 0.087 ± 0.018 | 58.96%** |
| X72 | 0.05 | 0.084 ± 0.010 | 60.38%** |
| X73 | 0.05 | 0.110 ± 0.015 | 48.11%* |
| X74 | 0.05 | 0.094 ± 0.009 | 55.66%** |
| X75 | 0.05 | 0.111 ± 0.009 | 47.64%* |
| X76 | 0.05 | 0.100 ± 0.016 | 52.83%** |
| X77 | 0.05 | 0.087 ± 0.001 | 58.96%** |
| X78 | 0.05 | 0.091 ± 0.017 | 57.08%** |
| X79 | 0.05 | 0.113 ± 0.005 | 46.70%* |

(continued)

| Group | Dose (μM) | Area of neovascular plexus ($mm^2$) | Inhibition rate (%) |
|---|---|---|---|
| X80 | 0.05 | 0.095 ± 0.011 | 55.19%* |
| X81 | 0.05 | 0.083 ± 0.011 | 60.85%** |
| X82 | 0.05 | 0.108 ± 0.017 | 49.06%** |
| X83 | 0.05 | 0.106 ± 0.007 | 50.00%** |
| X84 | 0.05 | 0.094 ± 0.018 | 55.66%** |
| X85 | 0.05 | 0.119 ± 0.002 | 43.87%* |
| X86 | 0.05 | 0.103 ± 0.015 | 51.42%** |
| X87 | 0.05 | 0.081 ± 0.003 | 61.79%** |
| X88 | 0.05 | 0.112 ± 0.017 | 47.17%* |
| X89 | 0.05 | 0.094 ± 0.006 | 55.66%** |
| X90 | 0.05 | 0.106 ± 0.014 | 50.00%** |

Compared with negative control, the neovascular plexus in retinas of OIR mice treated with polypeptide X1-X90 was significantly reduced. X13 in the polypeptides X1-X90 administration groups had the best effect, and the inhibition rate reached 62.26% when the dose was 0.05 μm.

**Example 21**

Effect of X1-X90 on neovascularization in a rat model of premature retinopathy

Using a fluctuating oxygen-induced animal model, newborn rats born naturally on the same day (within 12 h) were randomly divide into three groups: an oxygen model group, an oxygen treatment group and a normal control group. The oxygen model was further divided into three sub-group model groups, and the sub-group model groups and the treatment group were all placed in a semi-closed oxygen chamber made of organic glass; medical oxygen was introduced into the chamber, and the concentration was adjusted to 80% ± 2% by an oxygen meter; nitrogen was introduced into the oxygen chamber after 24 h, and the oxygen concentration was adjusted to 10% ± 2% rapidly and maintained for 24 h. This was repeated to keep the oxygen concentration in the oxygen chamber alternating between 80% and 10% every 24 h, the oxygen concentration was maintained for 7 d, and then the rats were fed in the air. The oxygen concentration was monitored 8 times a day, and the ambient temperature in the chamber was controlled at 23°C ± 2°C. Bedding was replaced, feed was added, and water and mother rats were replaced once. The normal control group was placed in the animal room feeding environment. Compared with the control group, if the ADP enzyme staining of retinal serial sections in the model group showed obvious vascular changes, and the number of vascular endothelial cell nuclei that broke through the inner limiting membrane of the retina and grew into the vitreous body increased with a statistically significant difference, the modeling was successful.

The oxygen treatment group was divided into three subgroups. On day 7 of modeling, X1-X30, X31-X60 and X61-X90 were injected in the vitreous chamber respectively, and the oxygen model group and the control group were only given normal saline for one week. On day 14, after the rats were anesthetized by diethyl ether and killed, eyeballs were extracted and fixed in 40 g/L paraformaldehyde solution for 24 h. Gradient dehydration by alcohol was performed, and the eyeballs were processed by xylene to be transparent. After waxing, the eyeballs were sectioned continuously to a thickness of 4 μm, and the periphery of the optic disc should be avoided as much as possible. The sections were parallel to the sagittal plane from cornea to the optic disc. Ten sections of each eyeball were randomly taken and stained with hematoxylin eosin, the number of vascular endothelial nuclei that broke through the inner limiting membrane of the retina (only the number of vascular endothelial nuclei closely related to the inner limiting membrane were counted) was counted, and statistics of the average number of cells per section per eyeball was performed.

Results: In the control group, there were no vascular endothelial nuclei that broke through the inner limiting membrane of the retina and grew into vitreous body, or the vascular endothelial nuclei were occasionally found in only a few sections. In the model group, there were many vascular endothelial nuclei that broke through the inner limiting membrane of the retina, some appeared alone, and some appeared in clusters. At the same time, on some sections, these vascular endothelial nuclei were also found adjacent to deep retinal vessels, confirming that they originated from the retina rather than the vitreous body or other ocular tissues. In the treatment group, only a few vascular endothelial nuclei that broke through the inner limiting membrane of retina could be seen in the sections. The experimental results are shown in Table 25.

Table 25 Retinal vascular endothelial nuclei count in each group

| Group | Dose (μM) | Nuclei count |
|---|---|---|
| X1 | 0.05 | 8.755 ± 2.888 |
| X2 | 0.05 | 8.561 ± 2.673 |
| X3 | 0.05 | 7.169 ± 1.551 |
| X4 | 0.05 | 7.604 ± 3.526 |
| X5 | 0.05 | 9.043 ± 2.651 |
| X6 | 0.05 | 9.085 ± 2.938 |
| X7 | 0.05 | 9.425 ± 3.563 |
| X8 | 0.05 | 9.066 ± 1.229 |
| X9 | 0.05 | 7.249 ± 3.321 |
| X10 | 0.05 | 7.741 ± 3.738 |
| X11 | 0.05 | 9.059 ± 1.301 |
| X12 | 0.05 | 8.232 ± 1.126 |
| X13 | 0.05 | 8.331 ± 3.318 |
| X14 | 0.05 | 8.539 ± 3.671 |
| X15 | 0.05 | 8.814 ± 1.616 |
| X16 | 0.05 | 8.221 ± 1.355 |
| X17 | 0.05 | 7.012±1.321 |
| X18 | 0.05 | 8.002 ± 3.606 |
| X19 | 0.05 | 7.236 ± 1.218 |
| X20 | 0.05 | 9.374 ± 3.742 |
| X21 | 0.05 | 7.222 ± 3.567 |
| X22 | 0.05 | 7.368 ± 1.842 |
| X23 | 0.05 | 8.275 ± 1.713 |
| X24 | 0.05 | 7.699 ± 3.108 |
| X25 | 0.05 | 8.923 ± 1.954 |
| X26 | 0.05 | 8.343 ± 2.131 |
| X27 | 0.05 | 8.358 ± 1.328 |
| X28 | 0.05 | 9.261 ± 3.879 |
| X29 | 0.05 | 9.533 ± 2.225 |
| X30 | 0.05 | 8.256 ± 3.403 |
| X31 | 0.05 | 7.228 ± 3.184 |
| X32 | 0.05 | 8.394 ± 2.665 |
| X33 | 0.05 | 7.654 ± 1.142 |
| X34 | 0.05 | 7.292 ± 3.827 |
| X35 | 0.05 | 8.544 ± 3.087 |
| X36 | 0.05 | 8.726 ± 3.273 |
| X37 | 0.05 | 8.921 ± 2.863 |
| X38 | 0.05 | 8.325 ± 3.699 |
| X39 | 0.05 | 8.695 ± 2.322 |
| X40 | 0.05 | 8.963 ± 1.695 |
| X41 | 0.05 | 8.953 ± 1.042 |
| X42 | 0.05 | 7.149 ± 2.242 |
| X43 | 0.05 | 8.781 ± 1.541 |
| X44 | 0.05 | 8.554 ± 1.518 |
| X45 | 0.05 | 7.841 ± 3.399 |
| X46 | 0.05 | 7.316 ± 1.838 |
| X47 | 0.05 | 9.352 ± 2.961 |
| X48 | 0.05 | 8.817 ± 1.029 |
| X49 | 0.05 | 8.059 ± 2.391 |

(continued)

| Group | Dose (μM) | Nuclei count |
|---|---|---|
| X50 | 0.05 | 7.461 ± 1.469 |
| X51 | 0.05 | 9.049 ± 1.682 |
| X52 | 0.05 | 7.727 ± 1.706 |
| X53 | 0.05 | 8.649 ± 1.067 |
| X54 | 0.05 | 7.539 ± 1.845 |
| X55 | 0.05 | 9.378 ± 3.044 |
| X56 | 0.05 | 9.403 ± 3.439 |
| X57 | 0.05 | 7.437 ± 1.847 |
| X58 | 0.05 | 7.215 ± 1.261 |
| X59 | 0.05 | 8.466 ± 2.301 |
| X60 | 0.05 | 9.131 ± 3.321 |
| X61 | 0.05 | 9.151 ± 3.533 |
| X62 | 0.05 | 9.312 ± 1.889 |
| X63 | 0.05 | 8.748 ± 3.796 |
| X64 | 0.05 | 7.686 ± 1.242 |
| X65 | 0.05 | 7.333 ± 3.716 |
| X66 | 0.05 | 7.576 ± 1.162 |
| X67 | 0.05 | 8.823 ± 1.792 |
| X68 | 0.05 | 9.115 ± 1.754 |
| X69 | 0.05 | 8.616 ± 3.698 |
| X70 | 0.05 | 8.274 ± 3.515 |
| X71 | 0.05 | 7.678 ± 2.426 |
| X72 | 0.05 | 8.886 ± 1.692 |
| X73 | 0.05 | 7.601 ± 2.727 |
| X74 | 0.05 | 9.149 ± 1.759 |
| X75 | 0.05 | 8.371 ± 1.232 |
| X76 | 0.05 | 7.354 ± 2.263 |
| X77 | 0.05 | 7.752 ± 3.349 |
| X78 | 0.05 | 8.482 ± 2.669 |
| X79 | 0.05 | 7.974 ± 3.165 |
| X80 | 0.05 | 7.124 ± 2.409 |
| X81 | 0.05 | 8.034 ± 3.187 |
| X82 | 0.05 | 7.399 ± 2.401 |
| X83 | 0.05 | 8.728 ± 1.165 |
| X84 | 0.05 | 7.795 ± 3.921 |
| X85 | 0.05 | 8.157 ± 2.676 |
| X86 | 0.05 | 8.499 ± 2.052 |
| X87 | 0.05 | 7.629 ± 2.335 |
| X88 | 0.05 | 7.353 ± 2.599 |
| X89 | 0.05 | 7.327 ± 2.279 |
| X90 | 0.05 | 8.755 ± 2.888 |
| Model group | - | 27.463±2.213 |
| control | - | 1.329±0.259 |

The results showed that compared with 27.463 ± 2.213 in the oxygen model group, retinal vascular endothelial nuclei count in the X1-X90 treatment groups was significantly reduced, which proved that the X1-X90 treatment groups could inhibit the neoangiogenesis in oxygen-induced retinopathy model of neonatal rats to some extent. Among them, X17 had the best effect, and nuclei count was 7.012 ± 1.321 when the dose was 0.05 μm.

## Claims

1. A modified angiogenesis inhibitor polypeptide, wherein a maleimide group is used to modify the angiogenesis inhibitor polypeptide, and the carboxyl group of the maleimide group forms an amide bond with the amino group of N-terminal Arg of the polypeptide.

2. The modified angiogenesis inhibitor polypeptide according to claim 1, wherein the modified angiogenesis inhibitor polypeptide sequence comprises two functional groups A and B, wherein the functional group A is Arg-Gly-Ala-Asp-Arg-Ala or a derived polypeptide obtained by substituting, deleting or adding one or two amino acid residues in Arg-Gly-Ala-Asp-Arg-Al, and the derived polypeptide has the same angiogenesis inhibition, anti-tumor and anti-inflammatory activities as Arg-Gly-Ala-Asp-Arg-Ala; the functional group B is Arg-Gly-Asp, wherein the functional groups A and B are ligated with each other through a linker, that is, the modified polypeptide sequence structure is A-linker-B.

3. The modified angiogenesis inhibitor polypeptide according to claim 2, wherein the linker is Gly-Gly-Gly-Gly, Glu-Ala-Ala-Ala-Lys or Gly-Ser-Ser-Ser-Ser.

4. The modified angiogenesis inhibitor polypeptide according to claim 1 or 2 or 3, wherein the modified polypeptide sequence is preferably:

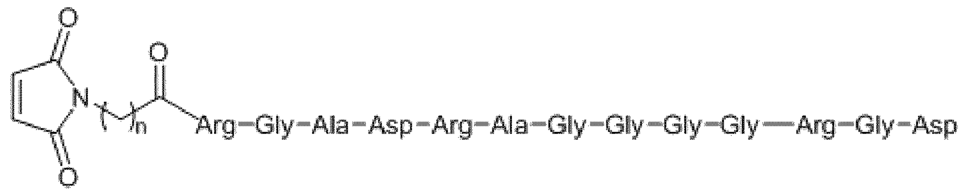

or

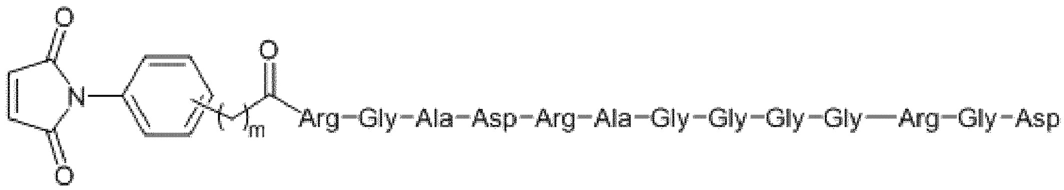

or

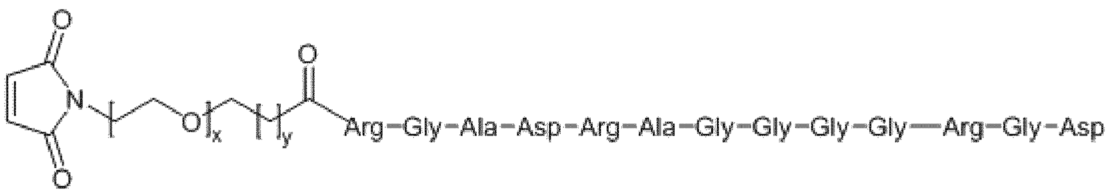

5. The modified angiogenesis inhibitor polypeptide according to claim 4, wherein a polypeptide chain is ligated with the maleimide group by different linkers, wherein n, m, x and y are the numbers of repeating structural unit methylene, methylene, oxyethylene and methylene respectively; the n, m, x and y are all integers, and specific numerical ranges are: n=1-12, m=1-12, x=1-5, y=0-6.

6. Use of the modified angiogenesis inhibitor polypeptide according to claim 4 in the preparation of a medicament for treating tumors, inflammations and ocular neovascular diseases.

7. The use of the modified angiogenesis inhibitor polypeptide according to claim 6 in the preparation of a medicament for treating tumors, inflammations and ocular neovascular diseases, wherein the tumors are primary or secondary cancers, melanoma, hemangioma and sarcoma originating from human head and neck, brain, thyroid, esophagus, pancreas, lung, liver, stomach, breast, kidney, gallbladder, colon or rectum, ovary, blood vessel, cervix, prostate, bladder or testis.

8. The use of the modified angiogenesis inhibitor polypeptide according to claim 6 in the preparation of a medicament for treating tumors, inflammations and ocular neovascular diseases, wherein the inflammations comprise rheumatoid arthritis, gouty arthritis, reactive arthritis, osteoarthritis, psoriasis, infectious arthritis, traumatic arthritis and ankylosing spondylitis.

9. The use of the modified angiogenesis inhibitor polypeptide according to claim 6 in the preparation of a medicament for treating tumors, inflammations and ocular neovascular diseases, wherein the ocular neovascular diseases comprise age-related macular degeneration (AMD), iris neovascular eye diseases, choroidal neovascular eye diseases, retinal neovascular eye diseases or corneal neovascular eye diseases.

**10.** A medicament for treating tumors, inflammations and/or ocular neovascular diseases, comprising the modified angiogenesis inhibitor polypeptide according to claim 1 or 2 or 3 and pharmaceutically acceptable excipients.

**11.** The medicament for treating tumors, inflammations and/or ocular neovascular diseases according to claim 10, wherein the medicament is administered by injection, comprising subcutaneous injection, intramuscular injection, intravenous injection, vitreous injection and intravenous drip.

**INTERNATIONAL SEARCH REPORT**

International application No.
**PCT/CN2018/104578**

**A. CLASSIFICATION OF SUBJECT MATTER**

A61K 38/10(2006.01)i; A61P 35/00(2006.01)i; C07K 17/08(2006.01)i

According to International Patent Classification (IPC) or to both national classification and IPC

**B. FIELDS SEARCHED**

Minimum documentation searched (classification system followed by classification symbols)

A61K; A61P; C07K

Documentation searched other than minimum documentation to the extent that such documents are included in the fields searched

Electronic data base consulted during the international search (name of data base and, where practicable, search terms used)

VEN, CNABS, PubMed, ISI Web of Knowledge, NCBI Genbank, EMBL-EBI, CNKI, 万方, WANFANG, 申请人/发明人, 序列 1, 血管生成抑制剂, 马来酰亚胺, 修饰, HM-3, HM-1, 半衰期, 肿瘤, 癌症, tumor, cancer, angiogenesis inhibitor, maleimide, half-life, modified

**C. DOCUMENTS CONSIDERED TO BE RELEVANT**

| Category* | Citation of document, with indication, where appropriate, of the relevant passages | Relevant to claim No. |
|---|---|---|
| Y | CN 103623394 A (NANJING ANJI BIOTECHNOLOGY CO., LTD.) 12 March 2014 (2014-03-12)<br>claims 1-3 | 1-11 |
| Y | CN 104045718 A (NANJING ANJI BIOTECHNOLOGY CO., LTD.) 17 September 2014 (2014-09-17)<br>description, paragraphs 21 and 29 | 1-11 |
| Y | SHTENBERG, Y. et al. "Alginate Modified with Maleimide-Terminated PEG as Drug Carriers with Enhanced Mucoadhesion"<br>*Carbohydr Polym.*, No. no. 175, 28 July 2016 (2016-07-28),<br>pp. 337-346 | 4, 5 |
| Y | CN 105646667 A (NANJING ANJI BIOTECHNOLOGY CO., LTD.) 08 June 2016 (2016-06-08)<br>abstract | 1-11 |
| A | CN 102417540 A (CHINA PHARMACEUTICAL UNIVERSITY) 18 April 2012 (2012-04-18)<br>entire document | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 November 2018** | **22 November 2018** |

Name and mailing address of the ISA/CN

**State Intellectual Property Office of the P. R. China**
**No. 6, Xitucheng Road, Jimenqiao Haidian District, Beijing 100088**
**China**

Facsimile No. **(86-10)62019451**

Authorized officer

Telephone No.

INTERNATIONAL SEARCH REPORT

International application No.
PCT/CN2018/104578

C. DOCUMENTS CONSIDERED TO BE RELEVANT

| Category* | Citation of document, with indication, where appropriate, of the relevant passages | Relevant to claim No. |
|---|---|---|
| A | CN 1699408 A (CHINA PHARMACEUTICAL UNIVERSITY) 23 November 2005 (2005-11-23)<br>entire document | 1-11 |
| A | EP 1985302 A1 (TSINGHUA UNIVERSITY ET AL.) 29 October 2008 (2008-10-29)<br>entire document | 1-11 |
| A | 程昊冉等 (CHENG, Haoran et al.). "多肽HM_3对人非小细胞肺癌裸鼠移植瘤抑制作用研究 (Antitumor Effects of Peptide HM-3 Against Non-Small Cell Lung Cancer Xenografts in Nude Mice)"<br>*中国药理学通报 (Chinese Pharmacological Bulletin),*<br>Vol. 32, No. (6), 25 May 2016 (2016-05-25),<br>pp. 806-811 | 1-11 |
| A | 张利 (ZHANG, Li). "靶向转铁蛋白受体的聚谷氨酸药物载体的设计和研究 (Design and Research on γ-PGA Based Drug Carrier Targeting Transferrin Receptor, a Tumor over Expressed Maker)"<br>*中国优秀硕士学位论文全文数据库(电子期刊)医药卫生科技辑 (Electronic Medicine & Public Health, China Master's Theses Full-Text Database),* 15 November 2014 (2014-11-15),<br>Document Number E072-71 | 1-11 |

Form PCT/ISA/210 (second sheet) (January 2015)

INTERNATIONAL SEARCH REPORT

International application No.
PCT/CN2018/104578

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2018/104578**

| Patent document cited in search report | Publication date (day/month/year) | Patent family member(s) | Publication date (day/month/year) |
|---|---|---|---|
| CN 103623394 A | 12 March 2014 | None | |
| CN 104045718 A | 17 September 2014 | CN 104045718 B | 17 August 2016 |
| CN 105646667 A | 08 June 2016 | WO 2017173905 A1 | 12 October 2017 |
| CN 102417540 A | 18 April 2012 | WO 2013075600 A1 | 30 May 2013 |
| | | EP 2784093 A1 | 01 October 2014 |
| | | IN 4482CHN2014 A | 04 September 2015 |
| | | AU 2012343020 B2 | 19 May 2016 |
| | | US 2014329759 A1 | 06 November 2014 |
| | | KR 20140096373 A | 05 August 2014 |
| | | US 2017100489 A1 | 13 April 2017 |
| | | EP 2784093 A4 | 05 August 2015 |
| | | AU 2012343020 A1 | 12 June 2014 |
| CN 1699408 A | 23 November 2005 | CN 1314705 C | 09 May 2007 |
| EP 1985302 A1 | 29 October 2008 | EP 1985302 B1 | 03 January 2018 |
| | | CA 2637698 C | 20 September 2016 |
| | | CA 2637698 A1 | 26 July 2007 |
| | | JP 5687823 B2 | 25 March 2015 |
| | | WO 2007082483 A1 | 26 July 2007 |
| | | CN 104043107 A | 17 September 2014 |
| | | JP 2013163674 A | 22 August 2013 |
| | | CN 104043107 B | 07 September 2016 |
| | | EP 1985302 A4 | 02 January 2013 |
| | | JP 2009523743 A | 25 June 2009 |
| | | AU 2007207268 B2 | 18 August 2011 |
| | | WO 2007082483 B1 | 07 September 2007 |
| | | US 2010285103 A1 | 11 November 2010 |
| | | NO 20083287 A | 20 October 2008 |
| | | CN 101405019 B | 04 June 2014 |
| | | AU 2007207268 A1 | 26 July 2007 |
| | | CN 100475270 C | 08 April 2009 |
| | | CN 101002946 A | 25 July 2007 |
| | | CN 101405019 A | 08 April 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201410324568
- CN 201610211000

SEQUENCE LISTING

<110> Nanjing Anji Biological Technology Co., Ltd.

<120> Maleimide group-modified angiogenesis inhibitor HM-1 and use thereof

<160> 1

<170> Patent In version 3.5

<210> 1

<211> 13

<212> PRT

<213> Artificial sequence

<400> 1

Arg Gly Ala Asp Arg Ala Gly Gly Gly Gly Arg Gly Asp

1 5 10

**SEQUENCE LISTING**

**<110> ÄÏ¾©°²¼ªÉúÎï¿Æ¼¼ÓÐÏÞ¹«Ë¾**
**<120> ÂíÀ´õ£ÑÇ°·»ùÍÅÐÞÊÎµÄÑª¹ÜÉú³ÉÒÖÖÆ¼Á¶àëÄ¼°ÆäÓ¦ÓÃ**
**<160> 1**
**<170> Patent In version 3.5**
**<210> 1**
**<211> 13**
**<212> PRT**
**<213> ÈË¹¤ÐòÁÐ**
**<400> 1**
**Arg Gly Ala Asp Arg Ala Gly Gly Gly Gly Arg Gly Asp**
**1 5 10**